(19) Europäisches Patentamt / European Patent Office / Office européen des brevets

(11) EP 4 778 932 A1

(12) # EUROPEAN PATENT APPLICATION

published in accordance with Art. 153(4) EPC

(43) Date of publication:
**22.07.2026 Bulletin 2026/30**

(21) Application number: **24864636.6**

(22) Date of filing: **10.09.2024**

(51) International Patent Classification (IPC):
***C07D 515/22*** *(2006.01)* ***A61P 35/00*** *(2006.01)*
***A61K 31/4995*** *(2006.01)*

(52) Cooperative Patent Classification (CPC):
**A61K 31/4995; A61K 47/64; A61K 47/68; A61P 35/00; C07D 515/22**

(86) International application number:
**PCT/CN2024/118098**

(87) International publication number:
**WO 2025/055920 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.09.2023 CN 202311172522**
**18.06.2024 CN 202410790928**

(71) Applicant: **Abbvie Group Holdings Limited**
**Hamilton Pembroke, HM 11 (BM)**

(72) Inventors:
- **ZOU, Bin**
  **Shanghai 200131 (CN)**
- **DU, Ping**
  **Shanghai 200131 (CN)**
- **YEUNG, Bryan K.S.**
  **Shanghai 200131 (CN)**

(74) Representative: **Mathys & Squire**
**32 London Bridge Street**
**The Shard**
**London SE1 9SG (GB)**

(54) **NOVEL ECTEINASCIDIN DERIVATIVE, PREPARATION METHOD THEREFOR AND USE THEREOF, AND ANTIBODY-DRUG CONJUGATE AND USE THEREOF**

(57) The present invention relates to a novel ecteinascidin derivative, a preparation method therefor and a use thereof, and an antibody-drug conjugate and an application thereof. Provided are novel ecteinascidin derivatives, wherein some of the novel ecteinascidin derivatives have relatively strong in-vitro cytotoxicity (1-10 nM), and some have stronger in-vitro cytotoxicity (less than 1 nM). Additionally, provided are antibody-drug conjugates based on the novel ecteinascidin derivatives, wherein some of the novel antibody-drug conjugates have relatively strong in-vitro cytotoxicity (1-10 nM), and some have stronger in-vitro cytotoxicity (less than 1 nM).

EP 4 778 932 A1

## Description

### Technical Field

The present invention relates to a novel ecteinascidin derivative, a preparation method therefor and a use thereof, an antibody-drug conjugate, and an application thereof.

### Background Art

Ecteinascidin is a family of natural products isolated from tunicates, and the family includes many compounds having biological activity, which generally contain two to three tetrahydroisoquinoline subunits.

Trabectedin (Trabectedin, Yondelis, ET-743) is one of them. It has significant biological activity against a variety of tumor cells and was first isolated in 1990 by Kenneth L. Rinehart from the Caribbean tunicate Ecteinascidia turbinata. It can now be prepared by artificial synthesis (Nat. Prod. Rep., 2015, 32, 328-347). Trabectedin has a unique mechanism of action. It binds to the minor groove of DNA, interfering with cell division, gene transcription processes, and DNA repair mechanisms. On October 23, 2015, it was approved by the U.S. Food and Drug Administration (FDA) for the treatment of specific soft tissue sarcomas. Clinical data show that among more than 500 patients, the median PFS was 4.2 months (n=345; 95% confidence interval (CI): 3.0-4.8 months), and the risk of disease progression or death was reduced by 45% (HR=0.55; 95% CI: 0.44-0.70; p<0.001). The median OS was 13.7 months. The most serious associated risks include neutropenic sepsis (severe infection caused by leukopenia), rhabdomyolysis (severe muscle problems), cardiomyopathy (cardiac muscle problems, including heart failure), hepatotoxicity (liver problems, including liver failure), hypersensitivity reactions, and extravasation (leakage of trabectedin from a vein during infusion), tissue necrosis (damage to or death of tissue cells), and embryotoxicity. Among 378 patients who received at least one dose of trabectedin in randomized trials, the most common (≥20%) adverse reactions were nausea (75%), fatigue (69%), vomiting (46%), constipation (37%), decreased appetite (37%), diarrhea (35%), peripheral edema (28%), dyspnea (25%), and headache (25%). The most common (≥5%) grade 3-4 laboratory abnormalities were neutropenia (43%), alanine transaminase (ALT) increase (31%), thrombocytopenia (21%), anemia (19%), aspartate transaminase (AST) increase (17%), and creatine kinase increase (6.4%).

In summary, although ecteinascidin derivatives have already been used clinically as chemotherapeutic drugs, there remains a high clinical demand to continue developing new ecteinascidin derivatives and corresponding antibody-drug conjugates, and to further improve efficacy while enhancing safety.

### Summary of the Invention

The present invention provides a novel ecteinascidin derivative. The ecteinascidin compound has good antitumor activity, and antibody-drug conjugates based thereon have potential for use in the treatment of tumor diseases.

An ecteinascidin derivative, characterized in that the structure thereof is a compound as shown in general formula (I):

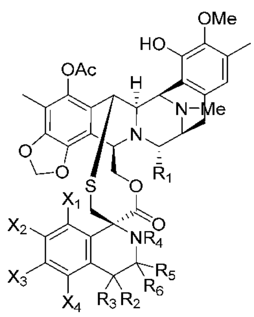

General formula (I)

wherein $R_1$ is selected from hydroxyl or cyano;

$R_2$ and $R_3$ are the same or different, and are each independently selected from hydrogen, deuterium, hydroxyl, mercapto, halogen, deuterated alkyl, haloalkyl, haloalkoxy, hydroxyl-substituted alkyl, amino-substituted alkyl, alkylamino-substituted alkyl, alkyl, alkoxy, alkylthio, $C_{2-6}$ alkenyl, $C_{2-6}$ alkynyl, cycloalkyl, alkyl-substituted amino,

cycloalkyl-substituted alkyl, heterocyclyl, aryl, heteroaryl, sulfonyl,

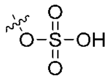, 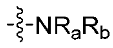, or 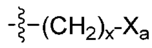;

$R_2$ and $R_3$ together with the carbon atoms to which they are attached form a saturated carbocycle, a saturated heterocycle, cycloalkyl, heterocycloalkyl, or do not form a ring; the x is selected from an integer of 1-10;

$R_a$ and $R_b$ are each independently selected from hydrogen, deuterium, hydroxyl, hydroxyl-substituted alkyl, alkyl, alkoxy, cycloalkyl, heterocyclyl, aryl, heteroaryl, alkyl-substituted sulfonyl, aryl-substituted sulfonyl, or heteroaryl-substituted sulfonyl; $R_a$ and $R_b$ together with the nitrogen atoms to which they are attached form a saturated heterocycle, heterocycloalkyl, or do not form a ring;

$R_4$ is selected from hydrogen, alkyl, deuterated alkyl, haloalkyl, amino-substituted alkyl, alkylamino-substituted alkyl, or

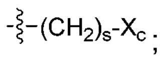;

s is selected from an integer of 1-10;

$R_5$ and $R_6$ are both hydrogen or alkyl, and R5 and R6 together with the carbon atoms to which they are attached form a saturated carbocycle, a saturated heterocycle, cycloalkyl, heterocycloalkyl, or do not form a ring; or $R_5$ and $R_6$ are different, and are each independently selected from hydrogen, alkyl, cycloalkyl, or cycloalkyl-substituted alkyl;

$X_a$, $X_c$, $X_1$, $X_2$, $X_3$, and $X_4$ are each independently selected from hydrogen, halogen, $C_{1-4}$ alkyl, or

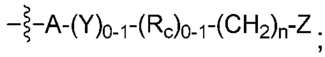;

wherein A is selected from oxygen, sulfur, -NH, $-NCD_3$, $-NCF_3$, $-NCH_3$, $-NCH_2CH_3$, or $-NCH_2CH_2CH_3$; Y is selected from -C(=O)-, $-S(=O)_2-$, or $-CHCF_3$; $R_c$ is selected from a 3- to 10-membered saturated or partially unsaturated heterocyclyl, a $C_{3-10}$ saturated or partially unsaturated carbocycle, or

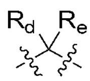;

n is selected from an integer of 0-10, and Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, deuterium, trifluoromethyl, alkyl, $C_{2-6}$ alkenyl, $C_{2-6}$ alkynyl, cycloalkyl, cycloalkyl-substituted alkyl, heterocyclyl, aryl, or heteroaryl; $R_d$ and $R_e$ together with the carbon atoms to which they are attached form a saturated carbocycle, a saturated heterocycle, cycloalkyl, heterocycloalkyl, or do not form a ring;

when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

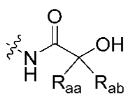, 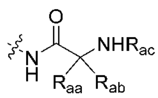, $-NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

According to one embodiment of the present invention, in the general formula (I):

$R_1$ is selected from hydroxyl or cyano;

$R_2$ and $R_3$ are the same or different, and are each independently selected from hydrogen, deuterium, hydroxyl, mercapto, halogen, deuterated alkyl, haloalkyl, haloalkoxy, hydroxyalkyl, amino-substituted alkyl, alkylamino-substituted alkyl, alkyl, alkoxy, alkylthio, $C_{2-6}$ alkenyl, $C_{2-6}$ alkynyl, cycloalkyl, alkyl-substituted amino, cycloalkylalkyl, heterocyclyl, aryl, heteroaryl, sulfonyl group,

$$-\wr-NR_aR_b \text{ or } -\wr-(CH_2)_x-X_a;$$

$R_2$ and $R_3$ together with the carbon atoms to which they are attached form cycloalkyl, heterocycloalkyl, or do not form a ring;

x is selected from an integer of 1-10;

$R_a$ and $R_b$ are each independently selected from hydrogen, deuterium, hydroxyl, hydroxyalkyl, alkyl, alkoxy, cycloalkyl, heterocyclyl, aryl, heteroaryl, alkylsulfonyl, arylsulfonyl, or heteroarylsulfonyl; $R_a$ and $R_b$ together with the nitrogen atoms to which they are attached form heterocycloalkyl or do not form a ring;

$R_4$ is selected from hydrogen, alkyl, deuterated alkyl, haloalkyl, amino-substituted alkyl, alkylamino-substituted alkyl, or

$$-\wr-(CH_2)_s-X_c;$$

s is selected from an integer of 1-10;

$R_5$ and $R_6$ are both hydrogen or alkyl, and $R_5$ and $R_6$ together with the carbon atoms to which they are attached form cycloalkyl, heterocycloalkyl, or do not form a ring; or $R_5$ and $R_6$ are different, and are each independently selected from hydrogen, alkyl, cycloalkyl, or cycloalkylalkyl;

$X_1$ and $X_4$ are hydrogen;

$X_a$, $X_c$, $X_2$, and $X_3$ are each independently selected from hydrogen, halogen, $C_{1-4}$ alkyl, or

$$-\wr-A-(Y)_{0-1}-(R_c)_{0-1}-(CH_2)_n-Z,$$

wherein A is selected from oxygen, sulfur, -NH, $-NCD_3$, $-NCF_3$, $-NCH_3$, $-NCH_2CH_3$, or $-NCH_2CH_2CH_3$; Y is selected from C(=O), $S(=O)_2$, or $-CHCF_3$; $R_c$ is selected from a 3- to 10-membered saturated or partially unsaturated heterocyclyl, a $C_{3-10}$ saturated or partially unsaturated carbocycle, or

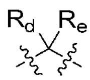;

n is selected from an integer of 0-10, and Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, deuterium, trifluoromethyl, alkyl, $C_{2-6}$ alkenyl, $C_{2-6}$ alkynyl, cycloalkyl, cycloalkylalkyl, heterocyclyl, aryl, or heteroaryl; Rd and Re together with the carbon atoms to which they are attached form cycloalkyl, heterocycloalkyl, or do not form a ring;

when one of $R_2$ or $R_3$ is hydrogen, the other is not $C_{1-4}$ alkyl; when both $R_2$ and $R_3$ are hydrogen, $X_2$ or $X_3$ is not

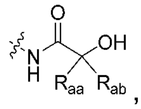, 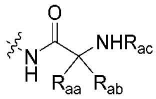,

$-NHCH_3$, $- NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

According to one embodiment of the present invention, in the general formula (I):

$R_1$ is selected from hydroxyl or cyano;

$R_2$ and $R_3$ are the same or different, and are each independently selected from hydrogen, deuterium, hydroxyl, mercapto, halogen, deuterated alkyl, haloalkyl, haloalkoxy, hydroxyl-substituted alkyl, amino-substituted alkyl, alkylamino-substituted alkyl, alkyl, alkoxy, alkylthio, $C_{2-6}$ alkenyl, $C_{2-6}$ alkynyl, cycloalkyl, alkyl-substituted amino, cycloalkyl-substituted alkyl, heterocyclyl, aryl, heteroaryl,

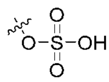, $-\wr-NR_aR_b$, or $-\wr-(CH_2)_x-X_a$;

$R_2$ and $R_3$ together with the carbon atoms to which they are attached form a saturated carbocycle, a saturated heterocycle, or do not form a ring; x is selected from an integer of 1-10;

$R_a$ and $R_b$ are each independently selected from hydrogen, deuterium, hydroxyl, hydroxyl-substituted alkyl, alkyl, alkoxy, cycloalkyl, heterocyclyl, aryl, heteroaryl, alkyl-substituted sulfonyl, aryl-substituted sulfonyl, or heteroaryl-substituted sulfonyl; Ra and Rb together with the nitrogen atoms to which they are attached form a saturated heterocycle or do not form a ring;

$R_4$ is selected from hydrogen, alkyl, deuterated alkyl, haloalkyl, amino-substituted alkyl, alkylamino-substituted alkyl, or

$$-\wr-(CH_2)_s-X_c;$$

s is selected from an integer of 1-10;

$R_5$ and $R_6$ are both hydrogen or alkyl, and $R_5$ and $R_6$ together with the carbon atoms to which they are attached form a saturated carbocycle, a saturated heterocycle, or do not form a ring; or $R_5$ and $R_6$ are different, and are each independently selected from hydrogen, alkyl, cycloalkyl, or cycloalkyl-substituted alkyl;

$X_a$, $X_c$, $X_1$, $X_2$, $X_3$, and $X_4$ are each independently selected from hydrogen, halogen, $C_{1-4}$ alkyl, or

$$-\wr-A-(Y)_{0-1}-(R_c)_{0-1}-(CH_2)_n-Z,$$

wherein A is selected from oxygen, sulfur, -NH, $-NCD_3$, $-NCF_3$, $-NCH_3$, $-NCH_2CH_3$, or $-NCH_2CH_2CH_3$; Y is selected from -C(=O)-, $-S(=O)_2-$, or $-CHCF_3$; $R_c$ is selected from a 3- to 10-membered saturated or partially unsaturated heterocyclyl, a $C_{3-10}$ saturated or partially unsaturated carbocycle, or

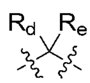;

n is selected from an integer of 0-10, and Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, deuterium, trifluoromethyl, alkyl, $C_{2-6}$ alkenyl, $C_{2-6}$ alkynyl, cycloalkyl, cycloalkyl-substituted alkyl, heterocyclyl, aryl, or heteroaryl; $R_d$ and $R_e$

together with the carbon atoms to which they are attached form a saturated carbocycle, a saturated heterocycle, or do not form a ring;

when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

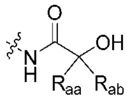, 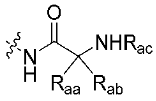, -

$NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

According to one embodiment of the present invention, in the general formula (I):

$R_1$ is selected from hydroxyl or cyano;

$R_2$ and $R_3$ are the same or different, and are each independently selected from hydrogen, deuterium, hydroxyl, mercapto, fluorine, chlorine, deuterated $C_{1-6}$ alkyl, halo $C_{1-6}$ alkyl, halo $C_{1-6}$ alkoxy, $C_{1-6}$ hydroxy-substituted alkyl, amino-substituted $C_{1-6}$ alkyl, $C_{1-6}$ alkylamino-substituted $C_{1-6}$ alkyl, $C_{1-6}$ alkyl, $C_{1-6}$ alkoxy, $C_{1-6}$ alkylthio, $C_{2-6}$ alkenyl, $C_{2-6}$ alkynyl, $C_{3-8}$ cycloalkyl, alkyl-substituted amino, cycloalkyl-substituted alkyl, heterocyclyl, aryl, heteroaryl,

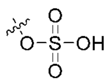, -§-$NR_aR_b$, or -§-$(CH_2)_x$-$X_a$;

$R_2$ and $R_3$ together with the carbon atoms to which they are attached form a 3- to 10-membered saturated carbocycle, a 3- to 10-membered saturated heterocycle, or do not form a ring; x is selected from an integer of 1-6;

$R_a$ and $R_b$ are each independently selected from hydrogen, hydroxyl, hydroxyl-substituted alkyl, alkyl, cycloalkyl, aryl, alkyl-substituted sulfonyl, or aryl-substituted sulfonyl; $R_a$ and $R_b$ together with the nitrogen atoms to which they are attached form a saturated heterocycle or do not form a ring;

$R_4$ is selected from hydrogen, $C_{1-6}$ alkyl, deuterated $C_{1-6}$ alkyl, halo $C_{1-6}$ alkyl, amino-substituted $C_{1-6}$ alkyl, or $C_{1-6}$ alkylamino-substituted $C_{1-6}$ alkyl, or

-§-$(CH_2)_s$-$X_c$;

s is selected from an integer of 1-6;

$R_5$ and $R_6$ are both hydrogen or $C_{1-6}$ alkyl; $R_5$ and $R_6$ together with the carbon atoms to which they are attached form a 3- to 10-membered saturated carbocycle, a 3- to 10-membered saturated heterocycle, or do not form a ring; or $R_5$ and $R_6$ are different, and are each independently selected from hydrogen, $C_{1-6}$ alkyl, $C_{3-10}$ cycloalkyl, or cycloalkyl-substituted alkyl;

$X_a$, $X_c$, $X_1$, $X_2$, $X_3$, and $X_4$ are each independently selected from hydrogen, fluorine, chlorine, methyl, ethyl, or

-§-A-$(Y)_{0-1}$-$(R_c)_{0-1}$-$(CH_2)_n$-Z,

wherein A is selected from oxygen, sulfur, -NH, $-NCD_3$, $-NCF_3$, $-NCH_3$, $-NCH_2CH_3$, or - $NCH_2CH_2CH_3$; Y is selected from -C(=O)-; $R_c$ is selected from a 3- to 6-membered saturated heterocyclyl or a $C_{3-6}$ saturated carbocycle, or

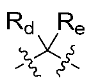;

n is selected from an integer of 0-6, and Z is selected from hydrogen, hydroxyl, mercapto, $-NH_2$, $-NHCH_3$,

$-NHCH_2CH_3$, $-NHCH_2CH_2CH_3$, $-CD_3$, or $-CF_3$;

$R_d$ and $R_e$ are the same or different, and are each independently selected from hydrogen, trifluoromethyl, alkyl, $C_{2-6}$ alkenyl, $C_{2-6}$ alkynyl, cycloalkyl, cycloalkyl-substituted alkyl, or aryl; $R_d$ and $R_e$ together with the carbon atoms to which they are attached form a saturated carbocycle, a saturated heterocycle, or do not form a ring;

when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

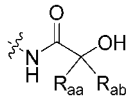, 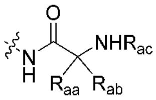, -

$NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

According to one embodiment of the present invention, in the general formula (I):

$R_1$ is selected from hydroxyl or cyano;

$R_2$ and $R_3$ are the same or different, and are each independently selected from hydrogen, hydroxyl, mercapto, fluorine, chlorine, trideuteromethyl, trifluoromethyl, trifluoromethoxy, C1-6 hydroxy-substituted alkyl, amino-substituted $C_{1-6}$ alkyl, $C_{1-6}$ alkylamino-substituted $C_{1-6}$ alkyl, $C_{1-6}$ alkyl, $C_{1-6}$ alkoxy, $C_{1-6}$ alkylthio, $C_{2-6}$ alkenyl, $C_{2-6}$ alkynyl, $C_{3-8}$ cycloalkyl, alkyl-substituted amino, cycloalkyl-substituted alkyl, heterocyclyl, aryl, heteroaryl,

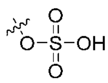,

$-\S-NR_aR_b$, or $-\S-(CH_2)_x-X_a$;

$R_2$ and $R_3$ together with the carbon atoms to which they are attached form a 3- to 6-membered saturated carbocycle, a 3- to 6-membered saturated heterocycle, or do not form a ring; x is selected from an integer of 1-6;

$R_a$ and $R_b$ are each independently selected from hydrogen, hydroxyl, hydroxyl-substituted alkyl, alkyl, cycloalkyl, aryl, or alkyl-substituted sulfonyl; $R_a$ and $R_b$ together with the nitrogen atoms to which they are attached form a saturated heterocycle or do not form a ring;

$R_4$ is selected from hydrogen, $C_{1-6}$ alkyl, trideuteromethyl, trifluoromethyl, amino-substituted $C_{1-6}$ alkyl, or $C_{1-6}$ alkylamino-substituted $C_{1-6}$ alkyl, or

$-\S-(CH_2)_s-X_c$;

s is selected from an integer of 1-6;

$R_5$ and $R_6$ are both hydrogen or $C_{1-6}$ alkyl; $R_5$ and $R_6$ together with the carbon atoms to which they are attached form a 3- to 6-membered saturated carbocycle, a 3- to 6-membered saturated heterocycle, or do not form a ring; or $R_5$ and $R_6$ are different, and are each independently selected from hydrogen, $C_{1-6}$ 6 alkyl, $C_{3-6}$ cycloalkyl, or cycloalkyl-substituted alkyl;

$X_a$, $X_c$, $X_1$, $X_2$, $X_3$, and $X_4$ are each independently selected from hydrogen, fluorine, chlorine, methyl, ethyl, or

$-\S-A-(Y)_{0-1}-(R_c)_{0-1}-(CH_2)_n-Z$,

wherein A is selected from oxygen, sulfur, -NH, $-NCD_3$, $-NCH_3$, $-NCH_2CH_3$, or - $NCH^2CH^2CH^3$; Y is selected from -C(=O)-; $R_c$ is selected from a 3- to 6-membered saturated heterocyclyl, a $C_{3-6}$ saturated carbocycle, or

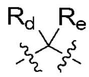;

n is selected from an integer of 0-6, and Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, trifluoromethyl, alkyl, $C_{2-6}$ alkenyl, $C_{2-6}$ alkynyl, cycloalkyl, cycloalkyl-substituted alkyl, or aryl; $R_d$ and $R_e$ together with the carbon atoms to which they are attached form a saturated carbocycle, a saturated heterocycle, or do not form a ring;

when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

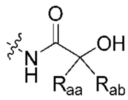, 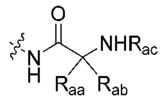, -

$NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

According to one embodiment of the present invention, in the general formula (I):

$R_1$ is selected from hydroxyl or cyano;

$R_2$ and $R_3$ are the same or different, and are each independently selected from hydrogen, hydroxyl, fluorine, trifluoromethyl, trifluoromethoxy, $C_{1-6}$ hydroxy-substituted alkyl, amino-substituted $C_{1-6}$ alkyl, $C_{1-6}$ alkylamino-substituted $C_{1-6}$ alkyl, $C_{1-6}$ alkyl, $C_{1-6}$ alkoxy, $C_{3-8}$ cycloalkyl, alkyl-substituted amino,

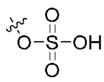, $-\S-NR_aR_b$, or $-\S-(CH_2)_x-X_a$;

$R_2$ and $R_3$ together with the carbon atoms to which they are attached form a 3- to 6-membered saturated carbocycle, a 3- to 6-membered saturated heterocycle, or do not form a ring; x is selected from an integer of 1-2;

$R_a$ and $R_b$ are each independently selected from hydrogen, hydroxyl-substituted alkyl, alkyl, or alkyl-substituted sulfonyl; $R_a$ and $R_b$ together with the nitrogen atoms to which they are attached form a saturated heterocycle or do not form a ring;

$R_4$ is selected from hydrogen, $C_{1-4}$ alkyl, amino-substituted $C_{1-4}$ alkyl, $C_{1-4}$ alkylamino-substituted $C_{1-4}$ alkyl, or

$$-\S-(CH_2)_s-X_c;$$

s is selected from an integer of 1-2;

$R_5$ and $R_6$ are both hydrogen or $C_{1-6}$ alkyl; $R_5$ and $R_6$ together with the carbon atoms to which they are attached form a 3- to 6-membered saturated carbocycle, a 3- to 6-membered saturated heterocycle, or do not form a ring; or $R_5$ and $R_6$ are different, and are each independently selected from hydrogen, $C_{1-6}$ alkyl, $C_{3-6}$ cycloalkyl, or cycloalkyl-substituted alkyl;

$X_a$, $X_c$, $X_1$, $X_2$, $X_3$, and $X_4$ are each independently selected from hydrogen, fluorine, methyl, or

$$-\S-A-(Y)_{0-1}-(R_c)_{0-1}-(CH_2)_n-Z,$$

wherein A is selected from oxygen, $-NCH_3$, or -NH; Y is selected from -C(=O)-; $R_c$ is selected from a $C_{3-6}$ saturated carbocycle or

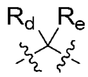;

n is selected from an integer of 0-1, and Z is selected from hydrogen or hydroxyl;

$R_d$ and $R_e$ are the same or different, and are each independently selected from hydrogen, trifluoromethyl, alkyl, cycloalkyl, or cycloalkyl-substituted alkyl; $R_d$ and $R_e$ together with the carbon atoms to which they are attached form a saturated carbocycle, a saturated heterocycle, or do not form a ring;

when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

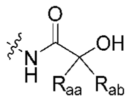, 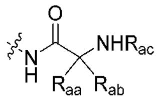, -

$NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

According to one embodiment of the present invention, in the general formula (I):

$R_1$ is selected from hydroxyl or cyano;

$R_2$ and $R_3$ are the same or different, and are each independently selected from hydrogen, deuterium, hydroxyl, mercapto, halogen, deuterated alkyl, haloalkyl, haloalkoxy, hydroxyl-substituted alkyl, amino-substituted alkyl, alkylamino-substituted alkyl, alkyl, alkoxy, alkylthio, $C_{2-6}$ alkenyl, $C_{2-6}$ alkynyl, cycloalkyl, alkyl-substituted amino, cycloalkyl-substituted alkyl, heterocyclyl, aryl, heteroaryl,

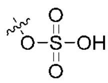, 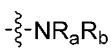, or 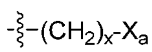;

$R_2$ and $R_3$ together with the carbon atoms to which they are attached form a saturated carbocycle, a saturated heterocycle, or do not form a ring;

x is selected from an integer of 1-10;

$R_a$ and $R_b$ are each independently selected from hydrogen, deuterium, hydroxyl, hydroxyl-substituted alkyl, alkyl, alkoxy, cycloalkyl, heterocyclyl, aryl, heteroaryl, alkyl-substituted sulfonyl, aryl-substituted sulfonyl, or heteroaryl-substituted sulfonyl; $R_a$ and $R_b$ together with the nitrogen atoms to which they are attached form a saturated heterocycle or do not form a ring;

$R_4$ is selected from hydrogen, alkyl, deuterated alkyl, haloalkyl, amino-substituted alkyl, or alkylamino-substituted alkyl, or

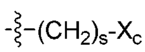;

s is selected from an integer of 1-10;

$R_5$ and $R_6$ are both hydrogen or alkyl, and $R_5$ and $R_6$ together with the carbon atoms to which they are attached form a saturated carbocycle, a saturated heterocycle, or do not form a ring; or $R_5$ and $R_6$ are different, and are each independently selected from hydrogen, alkyl, cycloalkyl, or cycloalkyl-substituted alkyl;

$X_1$ and $X_4$ are hydrogen;

$X_a$, $X_c$, $X_2$, and $X_3$ are each independently selected from hydrogen, halogen, $C_{1-4}$ alkyl, or

$$-\wr-A-(Y)_{0-1}-(R_c)_{0-1}-(CH_2)_n-Z,$$

wherein A is selected from oxygen, sulfur, -NH, $-NCD_3$, $-NCF_3$, $-NCH_3$, $-NCH_2CH_3$, or $-NCH_2CH_2CH_3$; Y is selected from -C(=O)-, $-S(=O)_2-$, or $-CHCF_3$; $R_c$ is selected from a 3- to 10-membered saturated or partially unsaturated heterocyclyl, a $C_{3-10}$ saturated or partially unsaturated carbocycle, or

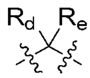;

n is selected from an integer of 0-10, and Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, deuterium, trifluoromethyl, alkyl, $C_{2-6}$ alkenyl, $C_{2-6}$ alkynyl, cycloalkyl, cycloalkyl-substituted alkyl, heterocyclyl, aryl, or heteroaryl; $R_d$ and $R_e$ together with the carbon atoms to which they are attached form a saturated carbocycle, a saturated heterocycle, or do not form a ring;

when one of $R_2$ or $R_3$ is hydrogen, the other is not $C_{1-4}$ alkyl; when $R_2$ or $R_3$ are both hydrogen, $X_2$ or $X_3$ is not

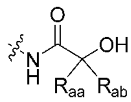, 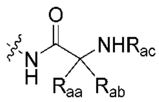, .

$-NHCH_3$, - $NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

According to one embodiment of the present invention, in the general formula (I):

$R_1$ is selected from hydroxyl or cyano;

$R_2$ and $R_3$ are the same or different, and are each independently selected from hydrogen, hydroxyl, fluorine, trifluoromethyl, trifluoromethoxy, methyl, methoxy, cyclopropyl-substituted methyl,

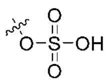, $-\wr-NR_aR_b$,

or

$$-\wr-(CH_2)_x-X_a;$$

$R_a$ and $R_b$ are each independently selected from hydrogen or methyl; $R_2$ and $R_3$ together with the carbon atoms to which they are attached form a 3-membered saturated carbocycle, a 4-membered oxygen-containing saturated heterocycle, or a 4-membered sulfur-containing saturated heterocycle; x is selected from an integer of 1-2;

$R_4$ is selected from hydrogen or methyl;

$R_5$ and $R_6$ are each independently selected from hydrogen or methyl; when both $R_5$ and $R_6$ are are methyl, they together with the carbon atoms to which they are attached form a 3-membered saturated carbocycle;

$X_1$ and $X_4$ are each independently selected from hydrogen or methoxy;

$X_a$, $X_2$, and $X_3$ are each independently selected from hydrogen, fluorine, methyl, or

$$-\S-A-(Y)_{0-1}-(R_c)_{0-1}-(CH_2)_n-Z,$$

wherein A is selected from oxygen, - $NCH_3$, or -NH; Y is selected from -C(=O)-; $R_c$ is selected from a $C_{3-6}$ saturated carbocycle or

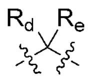

;

n is selected from an integer of 0-1, and Z is selected from hydrogen or hydroxyl;

$R_d$ and $R_e$ are the same or different, and are each independently selected from hydrogen, trifluoromethyl, cyclopropyl, or cyclopropyl-substituted methyl; $R_d$ and $R_e$ together with the carbon atoms to which they are attached form a 3- to 5-membered saturated carbocycle;

when one of $R_2$ or $R_3$ is hydrogen, the other is not methyl; or when $R_2$, $R_3$, $X_1$, and $X_4$ are all hydrogen, $X_2$ or $X_3$ is not

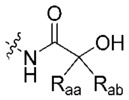,
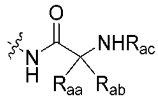,

, $-NHCH_3$, - $NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

According to one embodiment of the present invention, the compound in the formula (I) is selected from at least one of the following structural formula compounds,

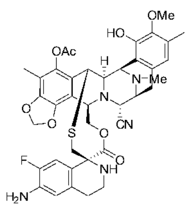
**P1**

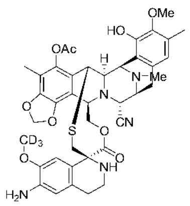
**P2**

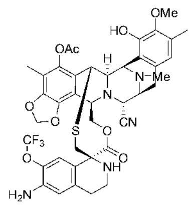
**P3**

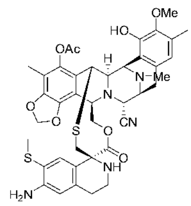
**P4**

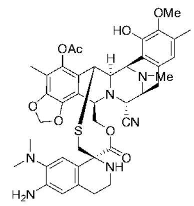
**P5**

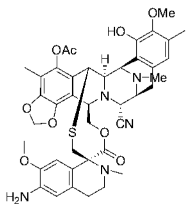
**P6**

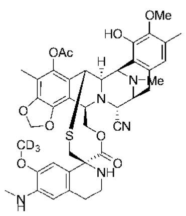
**P7**

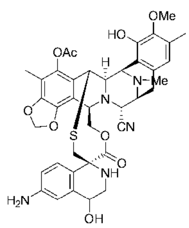
**P8**

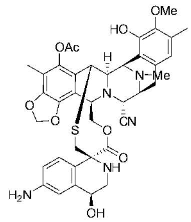
**P9**

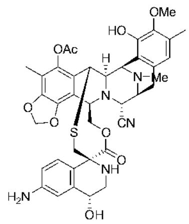
**P10**

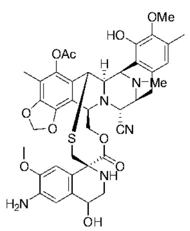
**P11**

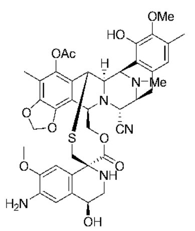
**P12**

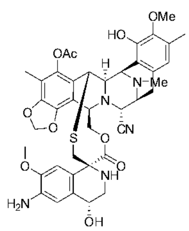
**P13**

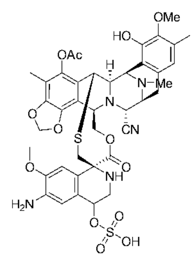
**P14**

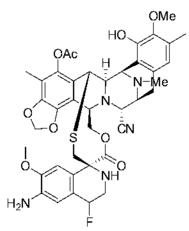
**P15**

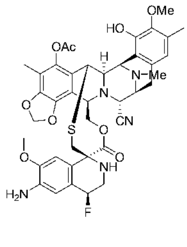

**P16**

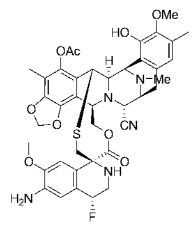

**P17**

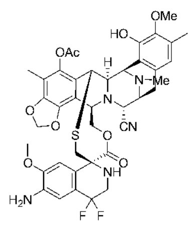

**P18**

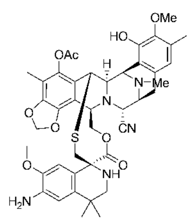

**P19**

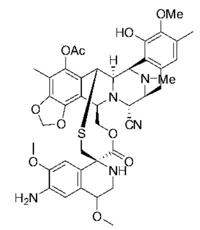

**P20**

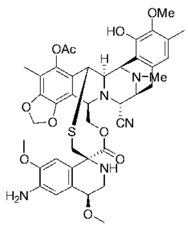

**P21**

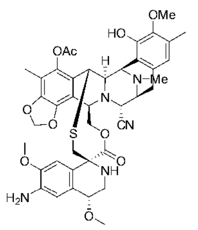

**P22**

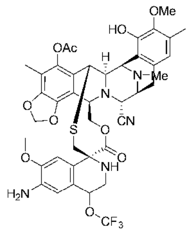

**P23**

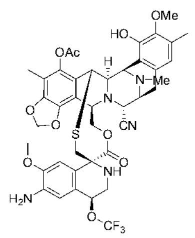

**P24**

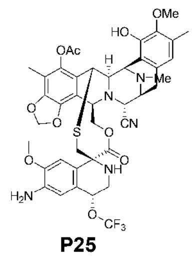

**P25**

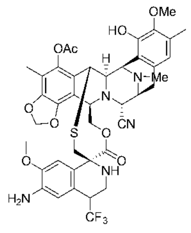

**P26**

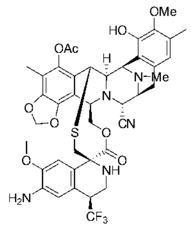

**P27**

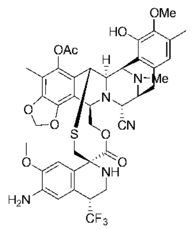

**P28**

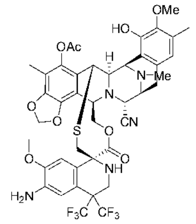

**P29**

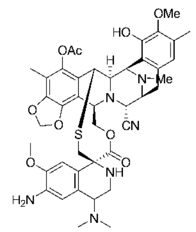

**P30**

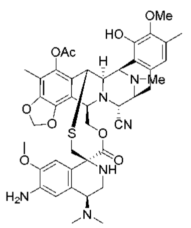

**P31**

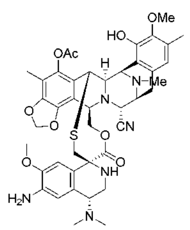

**P32**

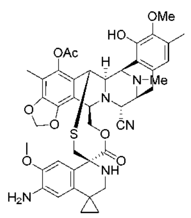

**P36**

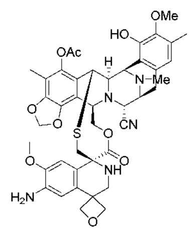

**P37**

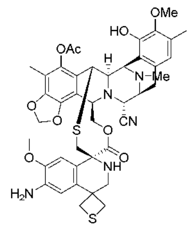

**P38**

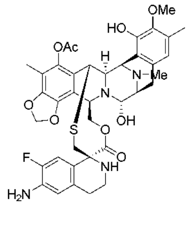

**P43**

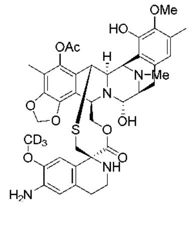

**P44**

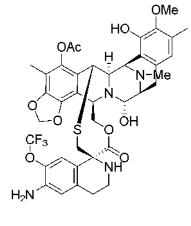

**P45**

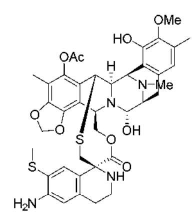

**P46**

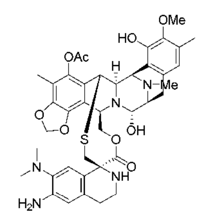

**P47**

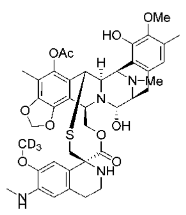

**P48**

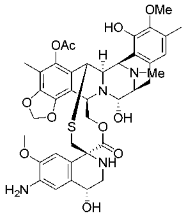

**P49**

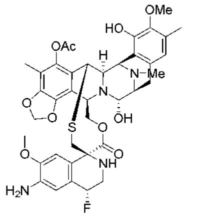

**P50**

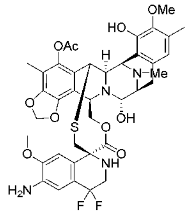

**P51**

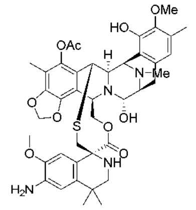

**P52**

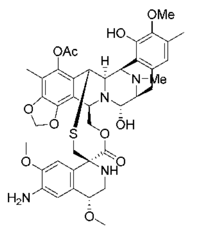
P53
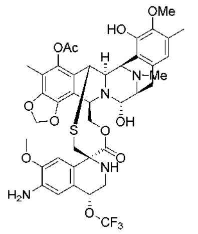
P54
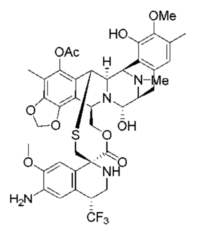
P55
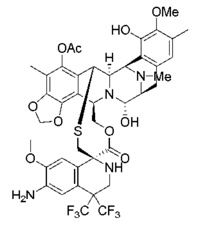
P56
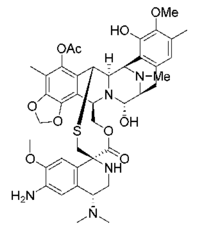
P57

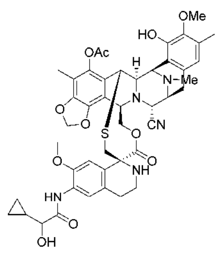
P58
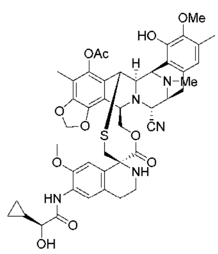
P59
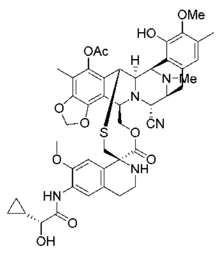
P60
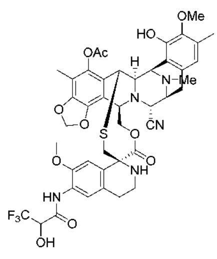
P61
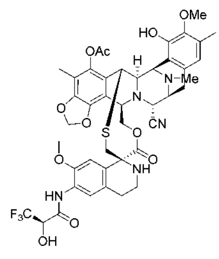
P62

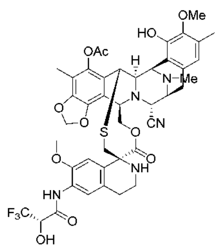
P63
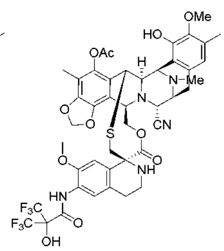
P64
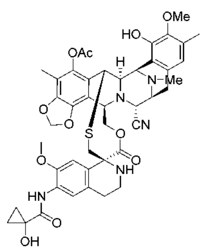
P65
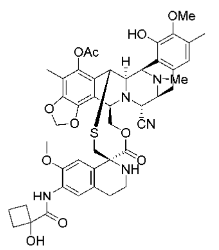
P66
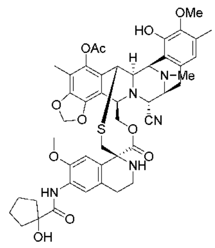
P67

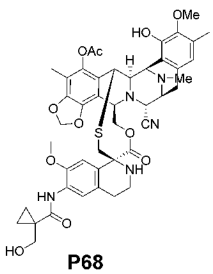
P68
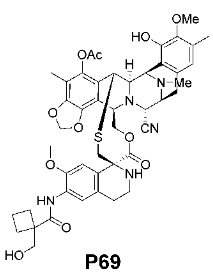
P69
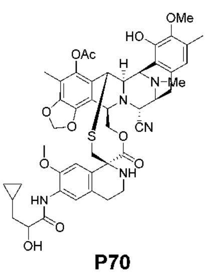
P70
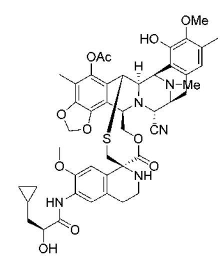
P71
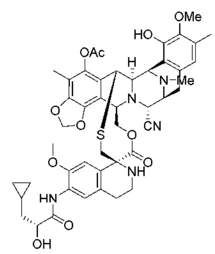
P72

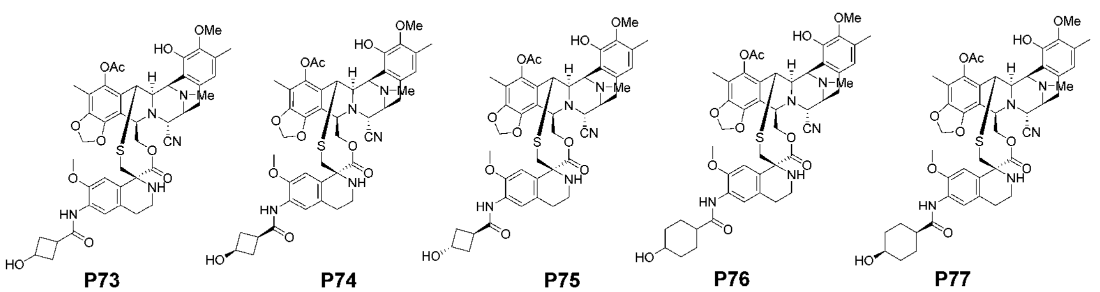
P73 P74 P75 P76 P77

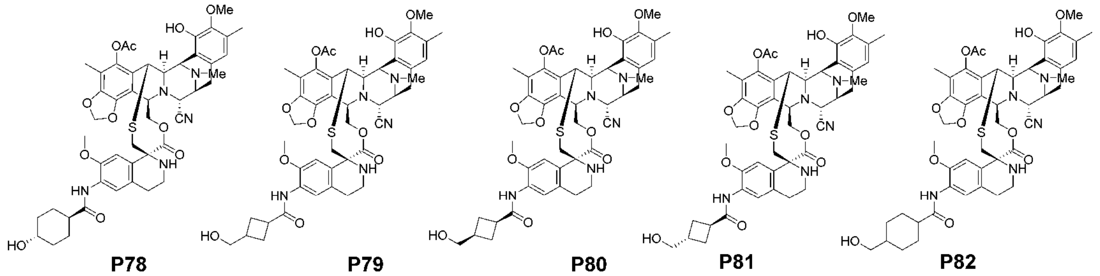

P78 P79 P80 P81 P82

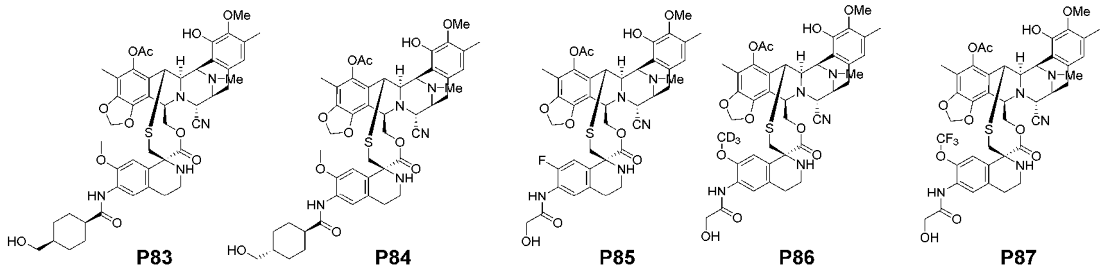

P83 P84 P85 P86 P87

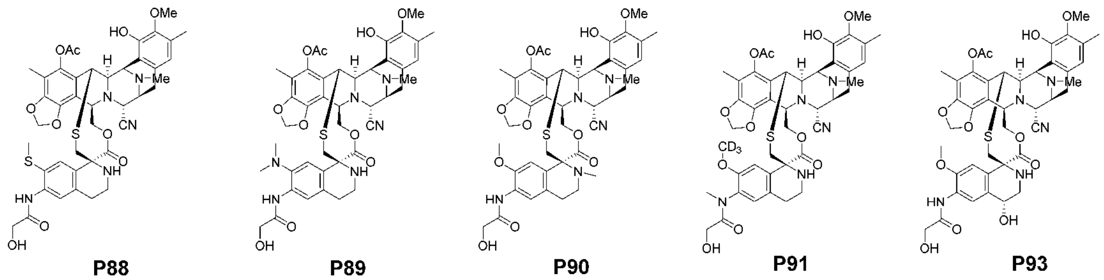

P88 P89 P90 P91 P93

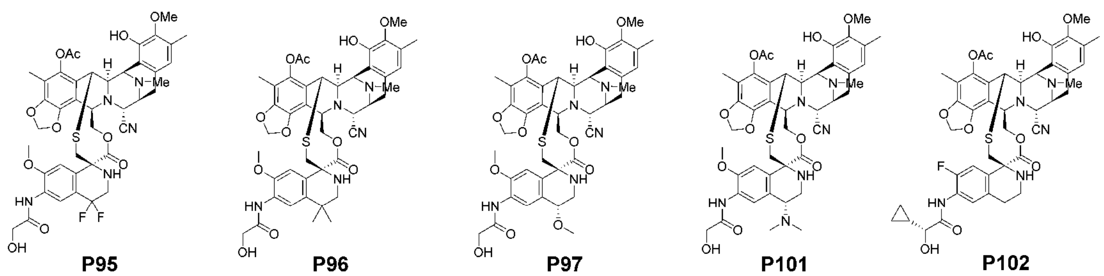

P95 P96 P97 P101 P102

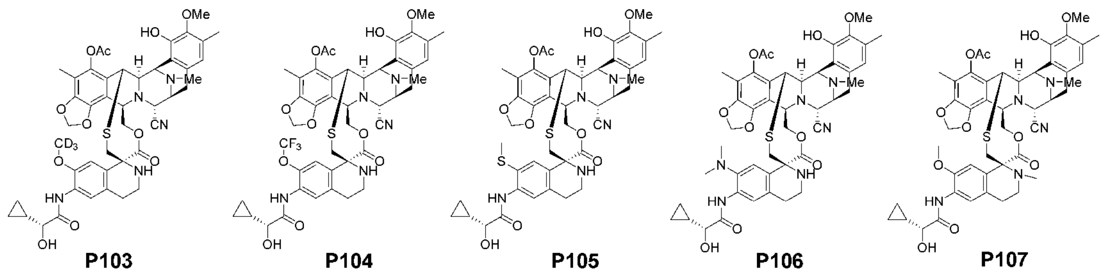

P103 P104 P105 P106 P107

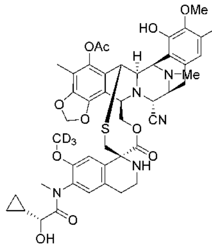
P108

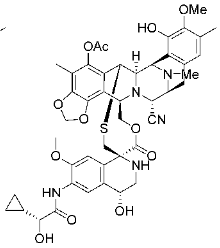
P110

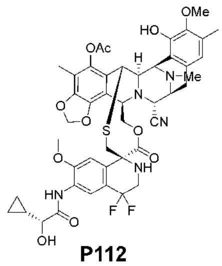
P112

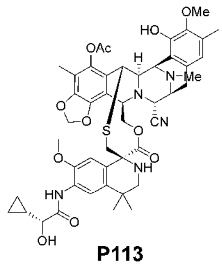
P113

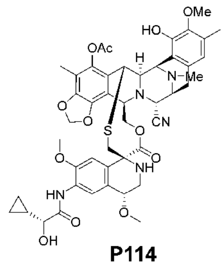
P114

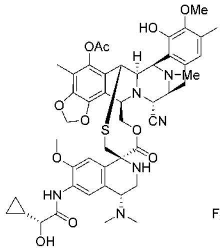
P118

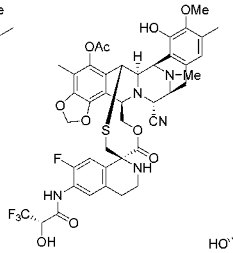
P119

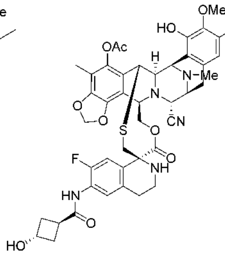
P136

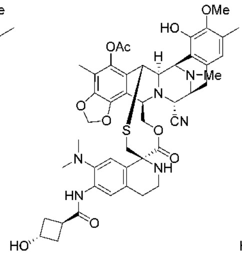
P140

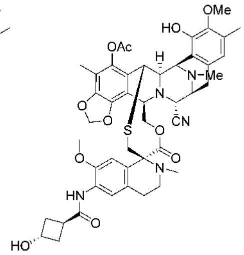
P141

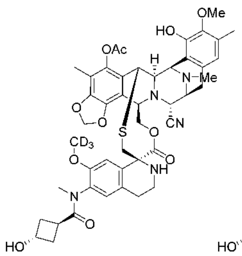
P142

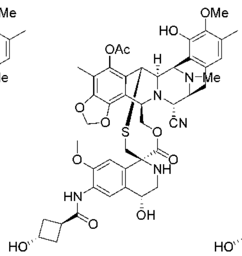
P144

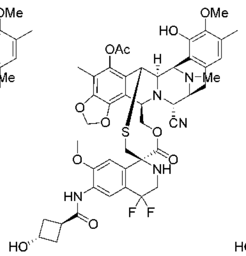
P146

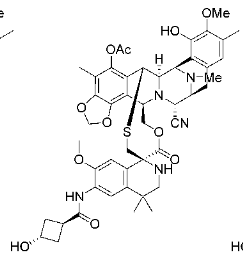
P147

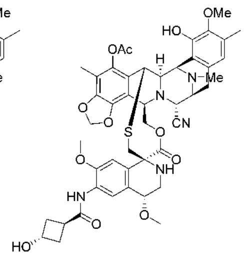
P148

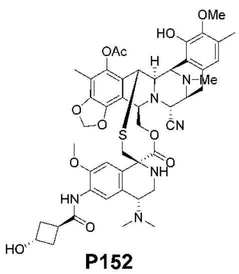
P152

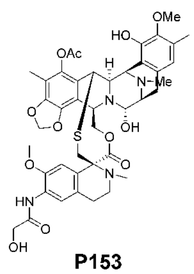
P153

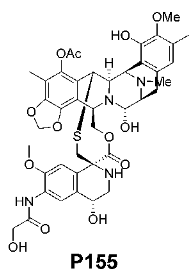
P155

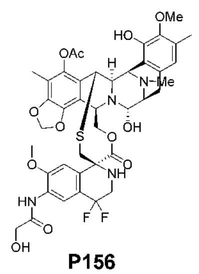
P156

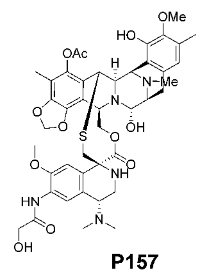
P157

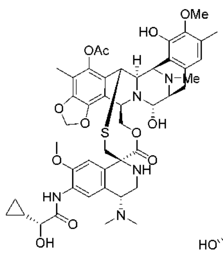
P158

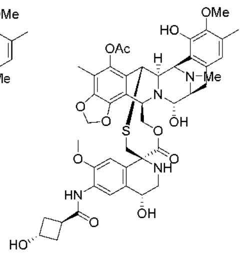
P160

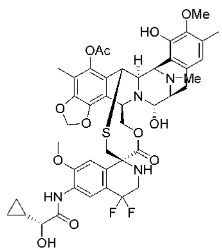
P163

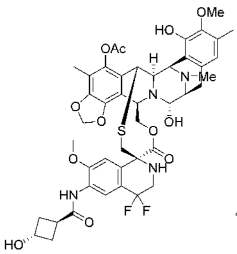
P164

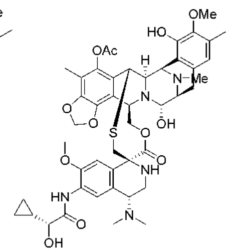
P165

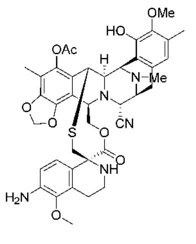
P166

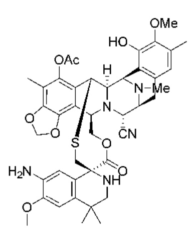
P167

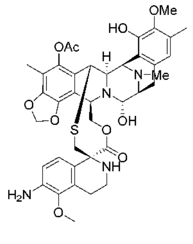
P168

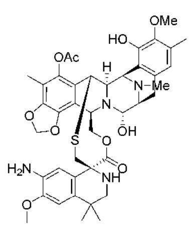
P169

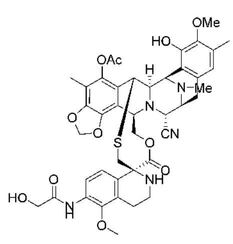
P170

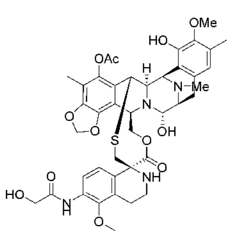
P171

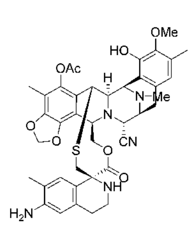
P172

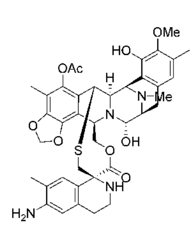
P173

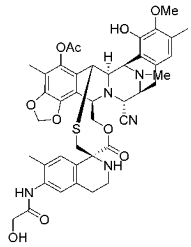
P174

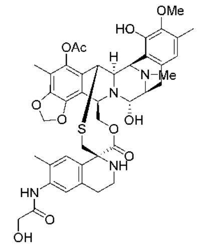
P175

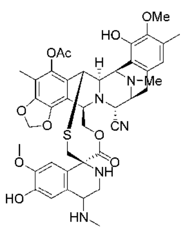
P186

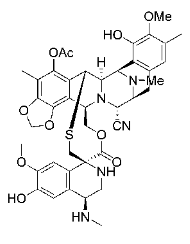
P187

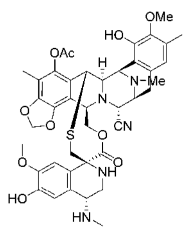
P188

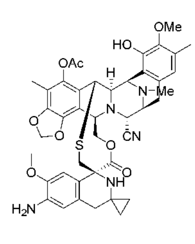
P241

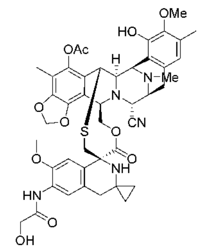
P253

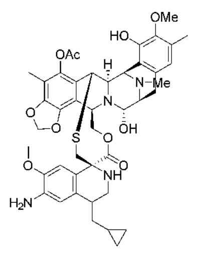
P260

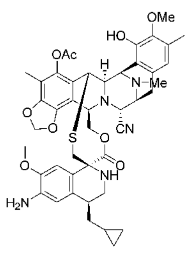
P261

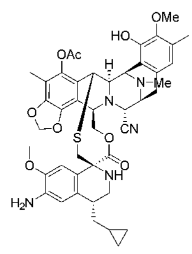
P262

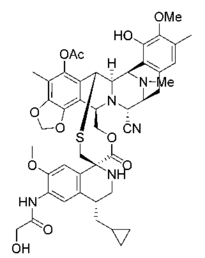
P264

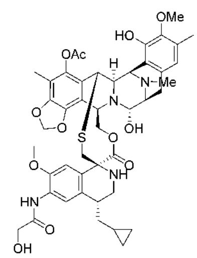
P265

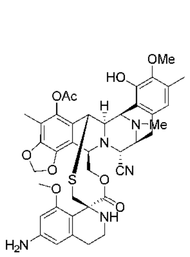
T1

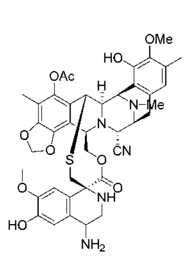
T2

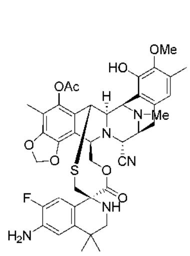
T3

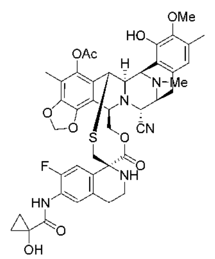
T4

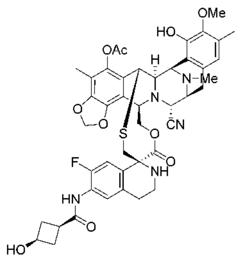
T5

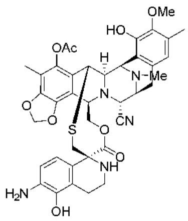

T6

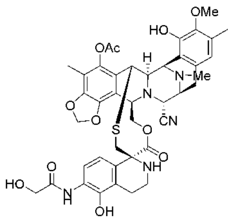

T7

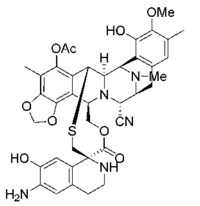

T8

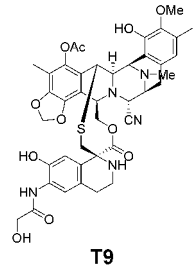

T9

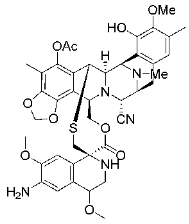

T10

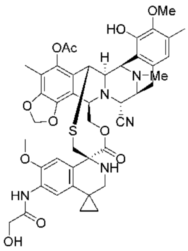

T11

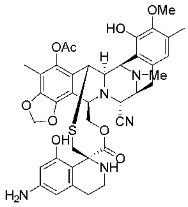

T12

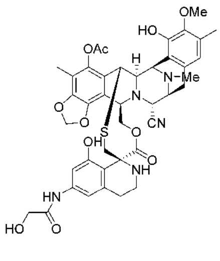

T13

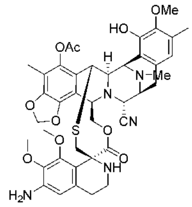

T14

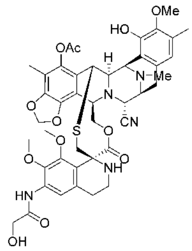

T15

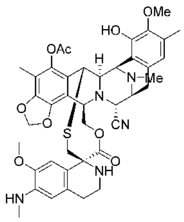

T16

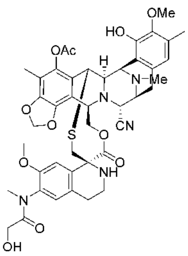

T17

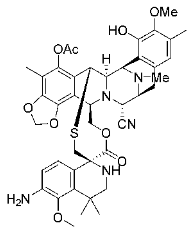

T18

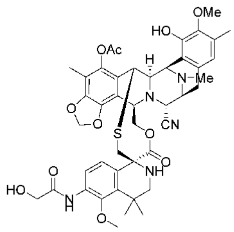

T19

According to one embodiment of the present invention, the compound in general formula (I) is selected from at least one compound of the structural formulas P1-P32, P36-P38, P43-P91, P93, P95-P97, P101-P108, P110, P112-P114, P118, P119, P136, P140-P142, P144, P146-P148, P152, P153, P155-P158, P160, P163-P175, P186-P188, P241, P253, P260-P262, P264, and P265.

According to one embodiment of the present invention, the compound in general formula (I) is selected from at least one compound of the structural formulas T1-T19.

According to one embodiment of the present invention, in the compounds P1-P32, P36-P38, P58-P91, P93, P95-P97, P101-P108, P110, P112-P114, P118, P119, P136, P140-P142, P144, P146-P148, P152, P166, P167, P170, P172, P174, P186-P188, P241, P253, P261-P262, P264, and T1-T19, the - CN at the $R_1$ position is replaced by -OH.

According to one embodiment of the present invention, the derivative comprises a single chiral component or a mixture of non-enantiomeric isomers.

According to one embodiment of the present invention, it further comprises a pharmaceutically acceptable salt, a tautomer, or a stereoisomer of the compound of general formula (I).

In the foregoing compound of general formula (I), a hydroxyl-substituted alkyl is also referred to as a hydroxyalkyl; a cycloalkyl-substituted alkyl is also referred to as a cycloalkylalkyl; an alkyl-substituted sulfonyl is also referred to as an alkylsulfonyl; an aryl-substituted sulfonyl is also referred to as an arylsulfonyl; and a heteroaryl-substituted sulfonyl is also referred to as a heteroarylsulfonyl.

According to one embodiment of the present invention, in general formula (I):

$R_1$ is selected from hydroxyl group or cyano;

$R_2$ and $R_3$ are the same or different, and are each independently selected from hydrogen, deuterium, hydroxyl, mercapto, fluoro, chloro, deuterated $C_{1-6}$ alkyl, halogenated $C_{1-6}$ alkyl, halogenated $C_{1-6}$ alkoxy, $C_{1-6}$ hydroxyalkyl, amino-substituted $C_{1-6}$ alkyl, $C_{1-6}$ alkylamino-substituted $C_{1-6}$ alkyl, $C_{1-6}$ alkyl, $C_{1-6}$ alkoxy, $C_{1-6}$ thioalkyl, $C_{2-6}$ alkenyl, $C_{2-6}$ alkynyl, $C_{3-8}$ cycloalkyl, amino-substituted $C_{1-6}$ alkyl, cycloalkylalkyl, heterocyclyl, aryl, heteroaryl, sulfonyl,

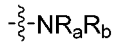 or 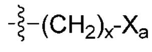;

$R_2$ and $R_3$, together with the carbon atoms to which they are attached, form a $C_{3-10}$ cycloalkyl, a 3- to 10-membered heterocycloalkyl, or do not form a ring;

$R_a$ and $R_b$ are each independently selected from hydrogen, hydroxyl, hydroxyalkyl, alkyl, cycloalkyl, aryl, alkylsulfonyl, and arylsulfonyl; Ra and Rb, together with the nitrogen atom to which they are attached, form a heterocycloalkyl or do not form a ring;

x is an integer selected from 1 to 6;

$R_4$ is selected from hydrogen, $C_{1-6}$ alkyl, deuterated $C_{1-6}$ alkyl, halogenated $C_{1-6}$ alkyl, amino-substituted $C_{1-6}$ alkyl, $C_{1-6}$ alkylamino-substituted $C_{1-6}$ alkyl, or

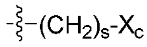;

s is an integer selected from 1 to 6;

$R_5$ and $R_6$ are simultaneously hydrogen or $C_{1-6}$ alkyl; $R_5$ and $R_6$, together with the carbon atoms to which they are attached, form a $C_{3-10}$ cycloalkyl, a 3- to 10-membered heterocycloalkyl, or do not form a ring; or $R_5$ and $R_6$ are different and are each independently selected from hydrogen, $C_{1-6}$ alkyl, $C_{3-10}$ cycloalkyl, or cycloalkylalkyl;

$X_a$, $X_c$, $X_1$, $X_2$, $X_3$, and $X_4$ are each independently selected from hydrogen, fluoro, chloro, methyl, ethyl, or

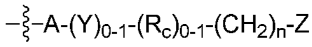,

wherein A is selected from oxygen, sulfur, -NH, $-NCD_3$, $-NCF_3$, $-NCH_3$, $-NCH_2CH_3$, or - $NCH_2CH_2CH_3$; Y is selected from C(=O); $R_c$ is selected from a 3- to 6-membered saturated heterocyclic group, a $C_{3-6}$ saturated carbocyclic group, or

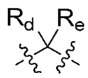;

n is an integer selected from 0 to 6; Z is selected from hydrogen, hydroxyl, mercapto, - $NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, trifluoromethyl, alkyl, $C_{2-6}$ alkenyl, $C_{2-6}$ alkynyl, cycloalkyl, cycloalkylalkyl, or aryl; $R_d$ and $R_e$, together with the carbon atoms to which they are attached, form a cycloalkyl, a heterocycloalkyl, or do not form a ring;

when one of $R_2$ or $R_3$ is hydrogen, the other is not a $C_{1-4}$ alkyl, or when $R_2$, $R_3$, $X_1$, and $X_4$ are all hydrogen, $X_2$ or $X_3$ is not

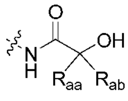, 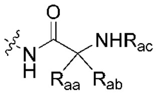, -

$NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

According to one embodiment of the present invention, in general formula (I):

$R_1$ is selected from hydroxyl or cyano;

$R_2$ and $R_3$ are the same or different, and are each independently selected from hydrogen, hydroxyl, mercapto, fluoro, chloro, trideuteromethyl, trifluoromethyl, trifluoromethoxy, $C_{1-6}$ hydroxyalkyl, amino-substituted $C_{1-6}$ alkyl, $C_{1-6}$ alkyl substituted with $C_{1-6}$ alkanamine, $C_{1-6}$ alkyl, $C_{1-6}$ alkoxy, $C_{1-6}$ thioalkyl, $C_{2-6}$ alkenyl, $C_{2-6}$ alkynyl, $C_{3-8}$ cycloalkyl, amino substituted with $C_{1-6}$ alkyl, cycloalkylalkyl, heterocyclyl, aryl, heteroaryl, sulfonyl,

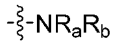 or 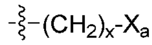 ;

the $R_2$ and $R_3$ together with the carbon atoms to which they are attached form a $C_{3-6}$ cycloalkyl, a 3- to 6-membered heterocyclyl, or do not form a ring;

$R_a$ and $R_b$ are each independently selected from hydrogen, hydroxyl, hydroxyalkyl, alkyl, cycloalkyl, aryl, alkylsulfonyl; the $R_a$ and $R_b$ together with the nitrogen atoms to which they are attached form a heterocycloalkyl or do not form a ring;

x is selected from an integer of 1-6;

$R_4$ is selected from hydrogen, $C_{1-6}$ alkyl, trideuteromethyl, trifluoromethyl, amino-substituted $C_{1-6}$ alkyl, C1-6 alkyl substituted with $C_{1-6}$ alkanamine, or

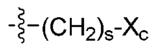 ;

s is selected from an integer of 1-6;

$R_5$ and $R_6$ are simultaneously hydrogen or $C_{1-6}$ alkyl; the $R_5$ and $R_6$ together with the carbon atoms to which they are attached form a $C_{3-6}$ cycloalkyl, a 3- to 6-membered heterocyclyl, or do not form a ring; or $R_5$ and $R_6$ are different and are each independently selected from hydrogen, $C_{1-6}$ alkyl, $C_{3-6}$ cycloalkyl, or cycloalkylalkyl;

$X_a$, $X_c$, $X_1$, $X_2$, $X_3$, and $X_4$ are each independently selected from hydrogen, fluoro, chloro, methyl, ethyl, or

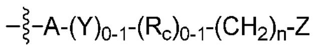 ,

wherein A is selected from oxygen, sulfur, -NH, $-NCD_3$, $-NCH_3$, $-NCH_2CH_3$, or $-NCH_2CH_2CH_3$; Y is selected from C(=O); $R_c$ is selected from a 3- to 6-membered saturated heterocyclyl, $C_{3-6}$ saturated carbocyclyl, or

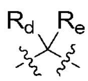 ;

n is selected from an integer of 0-6; Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, trifluoromethyl, alkyl, $C_{2-6}$ alkenyl, $C_{2-6}$ alkynyl, cycloalkyl, cycloalkylalkyl, or aryl; $R_d$ and $R_e$ together with the carbon atoms to which they are attached form a cycloalkyl, heterocycloalkyl, or do not form a ring;

when one of $R_2$ or $R_3$ is hydrogen, the other is not a $C_{1-4}$ alkyl, or when $R_2$, $R_3$, $X_1$, and $X_4$ are all hydrogen, $X_2$ or $X_3$ is not

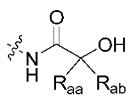 , 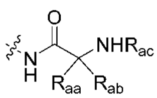 , -

$NHCH_3$, $-NHCH_2CH_3$, or $-NHCH_2CH_2CH_3$; wherein $R_{aa}$, $R_{ab}$, and $R_{ac}$ are each independently selected from hydrogen or $C_{1-4}$ alkyl.

According to one embodiment of the present invention, in general formula (I):

$R_1$ is selected from hydroxyl or cyano;

$R_2$ and $R_3$ are the same or different, and are each independently selected from hydrogen, hydroxyl, fluoro, trifluoromethyl, trifluoromethoxy, $C_{1-6}$ hydroxyalkyl, amino-substituted $C_{1-6}$ alkyl, $C_{1-6}$ alkyl substituted with $C_{1-6}$ alkanamine, $C_{1-6}$ alkyl, $C_{1-6}$ alkoxy, $C_{3-8}$ cycloalkyl, amino substituted with $C_{1-6}$ alkyl, sulfonyl,

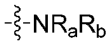 or 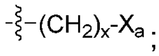;

$R_2$ and $R_3$ together with the carbon atoms to which they are attached form a $C_{3-6}$ cycloalkyl, a 3- to 6-membered heterocyclyl, or do not form a ring;

$R_a$ and $R_b$ are each independently selected from hydrogen, hydroxyalkyl, alkyl, alkylsulfonyl; $R_a$ and $R_b$ together with the nitrogen atoms to which they are attached form a heterocycloalkyl or do not form a ring;

x is selected from an integer of 1-2;

$R_4$ is selected from hydrogen, $C_{1-4}$ alkyl, amino-substituted $C_{1-4}$ alkyl, $C_{1-4}$ alkyl substituted with $C_{1-4}$ alkanamine, or

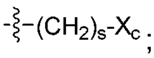;

s is selected from an integer of 1-2;

$R_5$ and $R_6$ are simultaneously hydrogen or $C_{1-6}$ alkyl; the $R_5$ and $R_6$ together with the carbon atoms to which they are attached form a $C_{3-6}$ cycloalkyl, a 3- to 6-membered heterocyclyl, or do not form a ring; or $R_5$ and $R_6$ are different and are each independently selected from hydrogen, $C_{1-6}$ alkyl, $C_{3-6}$ cycloalkyl, or cycloalkylalkyl;

$X_a$, $X_c$, $X_1$, $X_2$, $X_3$, and $X_4$ are each independently selected from hydrogen, fluoro, methyl, or

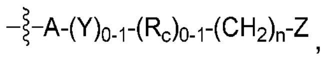,

wherein A is selected from oxygen, $-NCH_3$, or -NH; Y is selected from C(=O); $R_c$ is selected from C3-6 saturated carbocyclyl or

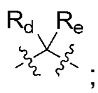;

n is selected from an integer of 0-1; Z is selected from hydrogen or hydroxyl;

$R_d$ and $R_e$ are the same or different, and are each independently selected from hydrogen, trifluoromethyl, alkyl, cycloalkyl, or cycloalkylalkyl; $R_d$ and $R_e$ together with the carbon atom to which they are attached form a cycloalkyl, heterocycloalkyl, or do not form a ring;

when one of $R_2$ or $R_3$ is hydrogen, the other is not a $C_{1-4}$ alkyl, or when $R_2$, $R_3$, $X_1$, and $X_4$ are all hydrogen, $X_2$ or $X_3$ is not

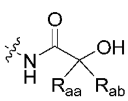, 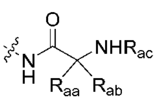, -

$NHCH_3$, $-NHCH_2CH_3$, or $-NHCH_2CH_2CH_3$; wherein $R_{aa}$, $R_{ab}$, and $R_{ac}$ are each independently selected from hydrogen or $C_{1-4}$ alkyl.

According to one embodiment of the present invention, in the general formula (I):

$R_1$ is selected from hydroxyl or cyano;

$R_2$ and $R_3$ are the same or different, and are each independently selected from hydrogen, deuterium, hydroxyl, sulfhydryl, halogen, deuterated alkyl, haloalkyl, haloalkoxy, hydroxyalkyl, amino-substituted alkyl, alkylamino-substituted alkyl, alkyl, alkoxy, alkylthio, $C_{2-6}$ alkenyl, $C_{2-6}$ alkynyl, cycloalkyl, alkyl-substituted amino, cycloalkylalkyl, heterocyclyl, aryl, heteroaryl, sulfonyl,

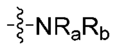,

or

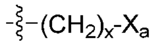;

$R_2$ and $R_3$ together with the carbon atoms to which they are attached form cycloalkyl, heterocycloalkyl, or do not form a ring;

x is selected from an integer of 1-10;

$R_a$ and $R_b$ are each independently selected from hydrogen, deuterium, hydroxyl, hydroxyalkyl, alkyl, alkoxy, cycloalkyl, heterocyclyl, aryl, heteroaryl, alkylsulfonyl, arylsulfonyl, or heteroarylsulfonyl; $R_a$ and $R_b$ together with the nitrogen atoms to which they are attached form heterocycloalkyl or do not form a ring;

$R_4$ is selected from hydrogen, alkyl, deuterated alkyl, haloalkyl, amino-substituted alkyl, alkylamino-substituted alkyl, or

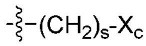;

s is selected from an integer of 1-10;

$R_5$ and $R_6$ are simultaneously hydrogen or alkyl, and $R_5$ and $R_6$ together with the carbon atoms to which they are attached form cycloalkyl, heterocycloalkyl, or do not form a ring; or $R_5$ and $R_6$ are different and are each independently selected from hydrogen, alkyl, cycloalkyl, or cycloalkylalkyl;

$X_1$ and $X_4$ are hydrogen;

$X_a$, $X_c$, $X_2$, and $X_3$ are each independently selected from hydrogen, halogen, $C_{1-4}$ alkyl, or

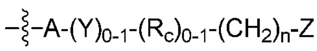;

wherein A is selected from oxygen, sulfur, -NH, $-NCD_3$, $-NCF_3$, $-NCH_3$, $-NCH_2CH_3$, or $-NCH_2CH_2CH_3$; Y is selected from C(=O), $S(=O)_2$, or $-CHCF_3$; $R_c$ is selected from a 3- to 10-membered saturated or partially unsaturated heterocyclyl, a $C_{3-10}$ saturated or partially unsaturated carbocyclyl, or

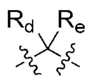;

n is selected from an integer of 0-10, and Z is selected from hydrogen, hydroxyl, sulfhydryl, $-NH_2$, $-NHCH_3$, $-NHCH_2CH_3$, - $NHCH_2CH_2CH_3$, $-CD_3$, or $-CF_3$;

$R_d$ and $R_e$ are the same or different, and are each independently selected from hydrogen, deuterium, trifluoromethyl, alkyl, $C_{2-6}$ alkenyl, $C_{2-6}$ alkynyl, cycloalkyl, cycloalkylalkyl, heterocyclyl, aryl, or heteroaryl; $R_d$ and $R_e$ together with the

carbon atoms to which they are attached form cycloalkyl, heterocycloalkyl, or do not form a ring;

when one of the $R_2$ or $R_3$ is hydrogen, the other is not $C_{1-4}$ alkyl; when both $R_2$ and $R_3$ are hydrogen, $X_2$ or $X_3$ is not

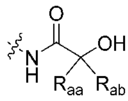, 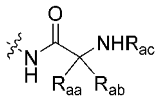,

$-NHCH_3$, $-NHCH_2CH_3$, or $-NHCH_2CH_2CH_3$; wherein $R_{aa}$, $R_{ab}$, and $R_{ac}$ are each independently selected from hydrogen or $C_{1-4}$ alkyl.

According to one embodiment of the present invention, in general formula (I): wherein $R_1$ is selected from hydroxyl or cyano;

$R_2$ and $R_3$ are the same or different, and are each independently selected from hydrogen, deuterium, hydroxyl, sulfhydryl, halogen, deuterated alkyl, haloalkyl, haloalkoxy, hydroxyl-substituted alkyl, amino-substituted alkyl, alkylamino-substituted alkyl, alkyl, alkoxy, alkylthio, $C_{2-6}$ alkenyl, $C_{2-6}$ alkynyl, cycloalkyl, alkyl-substituted amino, cycloalkyl-substituted alkyl, heterocyclyl, aryl, heteroaryl, sulfonyl,

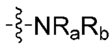, or 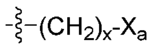;

$R_2$ and $R_3$ together with the carbon atoms to which they are attached form cycloalkyl, heterocycloalkyl, or do not form a ring;

$R_a$ and $R_b$ are each independently selected from hydrogen, deuterium, hydroxyl, hydroxyl-substituted alkyl, alkyl, alkoxy, cycloalkyl, heterocyclyl, aryl, heteroaryl, alkyl-substituted sulfonyl, aryl-substituted sulfonyl, or heteroaryl-substituted sulfonyl; $R_a$ and $R_b$ together with the nitrogen atoms to which they are attached form heterocycloalkyl or do not form a ring;

x is selected from an integer of 1-10;

$R_4$ is selected from hydrogen, alkyl, deuterated alkyl, haloalkyl, amino-substituted alkyl, alkylamino-substituted alkyl, or

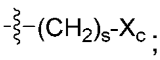;

s is selected from an integer of 1-10;

$R_5$ and $R_6$ are simultaneously hydrogen or alkyl; $R_5$ and $R_6$ together with the carbon atoms to which they are attached form cycloalkyl, heterocycloalkyl, or do not form a ring; or $R_5$ and $R_6$ are different and are each independently selected from hydrogen, alkyl, cycloalkyl, or cycloalkyl-substituted alkyl;

$X_a$, $X_c$, $X_1$, $X_2$, $X_3$, and $X_4$ are each independently selected from hydrogen, halogen, $C_{1-4}$ alkyl, or

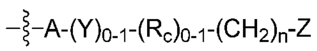;

wherein A is selected from oxygen, sulfur, -NH, $-NCD_3$, $-NCF_3$, $-NCH_3$, $-NCH_2CH_3$, or $-NCH_2CH_2CH_3$; Y is selected from -C(=O)-, $-S(=O)_2-$, or $-CHCF_3$; $R_c$ is selected from a 3- to 10-membered saturated or partially unsaturated heterocyclyl, a $C_{3-10}$ saturated or partially unsaturated carbocyclyl, or

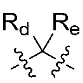;

n is selected from an integer of 0-10, and Z is selected from hydrogen, hydroxyl, sulfhydryl, $-NH_2$, $-NHCH_3$,

$-NHCH_2CH_3$, $-NHCH_2CH_2CH_3$, $-CD_3$, or $-CF_3$;

$R_d$ and $R_e$ are the same or different, and are each independently selected from hydrogen, deuterium, trifluoromethyl, alkyl, $C_{2-6}$ alkenyl, $C_{2-6}$ alkynyl, cycloalkyl, cycloalkyl-substituted alkyl, heterocyclyl, aryl, or heteroaryl; $R_d$ and $R_e$ together with the carbon atoms to which they are attached form cycloalkyl, heterocycloalkyl, or do not form a ring;

when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

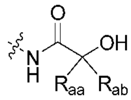, 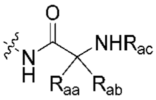, -

$NHCH_3$, $-NHCH_2CH_3$, or $-NHCH_2CH_2CH_3$; wherein $R_{aa}$, $R_{ab}$, and $R_{ac}$ are each independently selected from hydrogen or $C_{1-4}$ alkyl.

According to one embodiment of the present invention, in general formula (I):

$R_1$ is selected from hydroxyl or cyano;

$R_2$ and $R_3$ are the same or different, and are each independently selected from hydrogen, deuterium, hydroxy, mercapto, fluoro, chloro, deuterated $C_{1-6}$ alkyl, halogenated $C_{1-6}$ alkyl, halogenated $C_{1-6}$ alkoxy, $C_{1-6}$ hydroxy-substituted alkyl, amino-substituted $C_{1-6}$ alkyl, $C_{1-6}$ alkylamino-substituted $C_{1-6}$ alkyl, $C_{1-6}$ alkyl, $C_{1-6}$ alkoxy, $C_{1-6}$ alkylthio, $C_{2-6}$ alkenyl, $C_{2-6}$ alkynyl, $C_{3-8}$ cycloalkyl, amino substituted $C_{1-6}$ alkyl, cycloalkyl-substituted alkyl, heterocyclyl, aryl, heteroaryl, sulfonyl,

$$-\xi-NR_aR_b \text{, or } -\xi-(CH_2)_x-X_a;$$

$R_2$ and $R_3$ together with the carbon atoms to which they are attached form a $C_{3-10}$ cycloalkyl, a 3- to 10-membered heterocycloalkyl, or do not form a ring;

$R_a$ and $R_b$ are each independently selected from hydrogen, hydroxy, hydroxy-substituted alkyl, alkyl, cycloalkyl, aryl, alkyl-substituted sulfonyl, or aryl-substituted sulfonyl; $R_a$ and $R_b$ together with the nitrogen atoms to which they are attached form a heterocycloalkyl or do not form a ring;

x is selected from an integer of 1 to 6;

$R_4$ is selected from hydrogen, $C_{1-6}$ alkyl, deuterated $C_{1-6}$ alkyl, halogenated $C_{1-6}$ alkyl, amino-substituted $C_{1-6}$ alkyl, $C_{1-6}$ alkylamino-substituted $C_{1-6}$ alkyl, or do not form a ring; s is selected from an integer of 1-6;

$R_5$ and $R_6$ are simultaneously hydrogen or $C_{1-6}$ alkyl; $R_5$ and $R_6$ together with the carbon atoms to which they are attached form a $C_{3-10}$ cycloalkyl, a 3- to 10-membered heterocycloalkyl, or do not form a ring; or $R_5$ and $R_6$ are different, and are each independently selected from hydrogen, $C_{1-6}$ alkyl, $C_{3-10}$ cycloalkyl, or cycloalkyl-substituted alkyl;

$X_a$, $X_c$, $X_1$, $X_2$, $X_3$, and $X_4$ are each independently selected from hydrogen, fluoro, chloro, methyl, ethyl, or

$$-\xi-A-(Y)_{0-1}-(R_c)_{0-1}-(CH_2)_n-Z;$$

wherein A is selected from oxygen, sulfur, -NH, $-NCD_3$, $-NCF_3$, $-NCH_3$, $-NCH_2CH_3$, or $-NCH_2CH_2CH_3$; Y is selected from -C(=O)-; $R_c$ is selected from a 3- to 6-membered saturated heterocyclyl, a $C_{3-6}$ saturated carbocyclyl, or

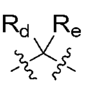;

n is selected from an integer of 0 to 6; Z is selected from hydrogen, hydroxy, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, trifluoromethyl, alkyl, $C_{2-6}$ alkenyl, $C_{2-6}$ alkynyl, cycloalkyl, alkyl-substituted cycloalkyl, or aryl; $R_d$ and $R_e$, together with the carbon atoms to which they are attached, form a cycloalkyl, heterocycloalkyl, or do not form a ring;

when one of $R_2$ or $R_3$ is hydrogen, the other is not a $C_{1-4}$ alkyl, or when $R_2$, $R_3$, $X_1$, and $X_4$ are all hydrogen, $X_2$ or $X_3$ is not

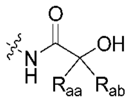, 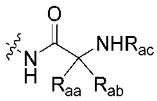, -

$NHCH_3$, $-NHCH_2CH_3$, or $-NHCH_2CH_2CH_3$; wherein $R_{aa}$, $R_{ab}$, and $R_{ac}$ are each independently selected from hydrogen or $C_{1-4}$ alkyl.

According to one embodiment of the present invention, in general formula (I):

$R_1$ is selected from hydroxyl or cyano;

$R_2$ and $R_3$ are the same or different, and are each independently selected from hydrogen, hydroxyl, mercapto, fluoro, chloro, trideuteriomethyl, trifluoromethyl, trifluoromethoxy, $C_{1-6}$ hydroxy-substituted alkyl, amino-substituted $C_{1-6}$ alkyl, C1-6 alkylamino-substituted $C_{1-6}$ alkyl, $C_{1-6}$ alkyl, $C_{1-6}$ alkoxy, $C_{1-6}$ alkylthio, $C_{2-6}$ alkenyl, $C_{2-6}$ alkynyl, $C_{3-8}$ cycloalkyl, amino-substituted $C_{1-6}$ alkyl, cycloalkyl-substituted alkyl, heterocyclyl, aryl, heteroaryl, sulfonyl,

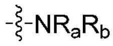, or

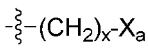;

$R_2$ and $R_3$, together with the carbon atoms to which they are attached, form a C3-6 cycloalkyl, a 3- to 6-membered heterocycloalkyl, or do not form a ring;

$R_a$ and $R_b$ are each independently selected from hydrogen, hydroxyl, hydroxy-substituted alkyl, alkyl, cycloalkyl, aryl, or alkyl-substituted sulfonyl; $R_a$ and $R_b$, together with the nitrogen atoms to which they are attached, form a heterocycloalkyl or do not form a ring;

x is selected from an integer of 1-6;

$R_4$ is selected from hydrogen, $C_{1-6}$ alkyl, trideuteriomethyl, trifluoromethyl, amino-substituted $C_{1-6}$ alkyl, $C_{1-6}$ alkylamino-substituted $C_{1-6}$ alkyl,

or 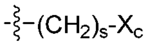;

s is selected from an integer of 1-6;

$R_5$ and $R_6$ are simultaneously hydrogen or $C_{1-6}$ alkyl; $R_5$ and $R_6$, together with the carbon atoms to which they are attached, form a $C_{3-6}$ cycloalkyl, a 3- to 6-membered heterocycloalkyl, or do not form a ring; or $R_5$ and $R_6$ are different, and are each independently selected from hydrogen, $C_{1-6}$ alkyl, $C_{3-6}$ cycloalkyl, or cycloalkyl-substituted alkyl;

$X_a$, $X_c$, $X_1$, $X_2$, $X_3$, and $X_4$ are each independently selected from hydrogen, fluoro, chloro, methyl, ethyl, or

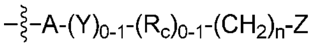;

wherein A is selected from oxygen, sulfur, -NH, $-NCD_3$, $-NCH_3$, $-NCH_2CH_3$, or $-NCH_2CH_2CH_3$; Y is selected from -C(=O)-; $R_c$ is selected from a 3- to 6-membered saturated heterocyclyl, a $C_{3-6}$ saturated carbocyclyl, or

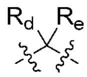;

n is selected from an integer of 0-6; Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, trifluoromethyl, alkyl, $C_{2-6}$ alkenyl, $C_{2-6}$ alkynyl, cycloalkyl, cycloalkyl-substituted alkyl, or aryl; $R_d$ and $R_e$, together with the carbon atoms to which they are attached, form a cycloalkyl, heterocycloalkyl, or do not form a ring;

when one of $R_2$ or $R_3$ is hydrogen, the other is not a $C_{1-4}$ alkyl, or when $R_2$, $R_3$, $X_1$, and $X_4$ are all hydrogen, $X_2$ or $X_3$ is not

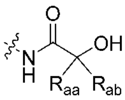, 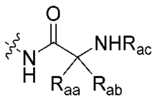,

$-NHCH_3$, $-NHCH_2CH_3$, or $-NHCH_2CH_2CH_3$; wherein $R_{aa}$, $R_{ab}$, and $R_{ac}$ are each independently selected from hydrogen or $C_{1-4}$ alkyl.

According to one embodiment of the present invention, in general formula (I):

$R_1$ is selected from hydroxyl or cyano;

$R_2$ and $R_3$ are the same or different, and are each independently selected from hydrogen, hydroxyl, fluoro, trifluoromethyl, trifluoromethoxy, $C_{1-6}$ hydroxy-substituted alkyl, amino-substituted $C_{1-6}$ alkyl, $C_{1-6}$ alkylamino-substituted $C_{1-6}$ alkyl, $C_{1-6}$ alkyl, $C_{1-6}$ alkoxy, $C_{3-8}$ cycloalkyl, amino-substituted $C_{1-6}$ alkyl, sulfonyl,

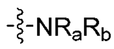, or 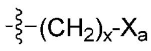;

$R_2$ and $R_3$, together with the carbon atoms to which they are attached, form a $C_{3-6}$ cycloalkyl, a 3- to 6-membered heterocycloalkyl, or do not form a ring;

$R_a$ and $R_b$ are each independently selected from hydrogen, hydroxy-substituted alkyl, alkyl, or alkyl-substituted sulfonyl; $R_a$ and $R_b$, together with the nitrogen atom to which they are attached, form a heterocycloalkyl or do not form a ring;

x is selected from an integer of 1-2;

$R_4$ is selected from hydrogen, $C_{1-4}$ alkyl, amino-substituted $C_{1-4}$ alkyl, $C_{1-4}$ alkylamino-substituted $C_{1-4}$ alkyl, or

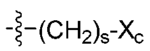;

s is selected from an integer of 1-2;

$R_5$ and $R_6$ are simultaneously hydrogen or $C_{1-6}$ alkyl; $R_5$ and $R_6$, together with the carbon atoms to which they are attached, form a $C_{3-6}$ cycloalkyl, a 3- to 6-membered heterocycloalkyl, or do not form a ring; or $R_5$ and $R_6$ are different, and are each independently selected from hydrogen, $C_{1-6}$ alkyl, $C_{3-6}$ cycloalkyl, or cycloalkyl-substituted alkyl;

$X_a$, $X_c$, $X_1$, $X_2$, $X_3$, and $X_4$ are each independently selected from hydrogen, fluoro, methyl, or

$$-\S-A-(Y)_{0-1}-(R_c)_{0-1}-(CH_2)_n-Z,$$

wherein A is selected from oxygen, $-NCH_3$, or -NH; Y is selected from -C(=O)-; $R_c$ is selected from a $C_{3-6}$ saturated carbocyclyl or

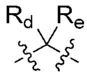;

n is selected from an integer of 0-1; Z is selected from hydrogen or hydroxyl;

$R_d$ and $R_e$ are the same or different, and are each independently selected from hydrogen, trifluoromethyl, alkyl, cycloalkyl, or cycloalkyl-substituted alkyl; $R_d$ and $R_e$, together with the carbon atoms to which they are attached, form a cycloalkyl, heterocycloalkyl, or do not form a ring;

when one of $R_2$ or $R_3$ is hydrogen, the other is not a $C_{1-4}$ alkyl, or when $R_2$, $R_3$, $X_1$, and $X_4$ are all hydrogen, $X_2$ or $X_3$ is not

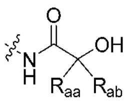, 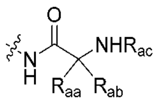,

$-NHCH_3$, $-NHCH_2CH_3$, or $-NHCH_2CH_2CH_3$; wherein $R_{aa}$, $R_{ab}$, and $R_{ac}$ are each independently selected from hydrogen or $C_{1-4}$ alkyl.

According to one embodiment of the present invention, in general formula (I):

$R_1$ is selected from hydroxyl or cyano;

$R_2$ and $R_3$ are the same or different, and are each independently selected from hydrogen, deuterium, hydroxyl, mercapto, halogen, deuterated alkyl, halogenated alkyl, halogenated alkoxy, hydroxy-substituted alkyl, amino-substituted alkyl, alkylamino-substituted alkyl, alkyl, alkoxy, alkylthio, $C_{2-6}$ alkenyl, $C_{2-6}$ alkynyl, cycloalkyl, amino-substituted alkyl, cycloalkyl-substituted alkyl, heterocyclyl, aryl, heteroaryl, sulfonyl,

$$-\S-NR_aR_b \text{ or } -\S-(CH_2)_x-X_a;$$

$R_2$ and $R_3$, together with the carbon atoms to which they are attached, form a cycloalkyl, heterocycloalkyl, or do not form a ring;

x is selected from an integer of 1-10;

$R_a$ and $R_b$ are each independently selected from hydrogen, deuterium, hydroxyl, hydroxy-substituted alkyl, alkyl, alkoxy, cycloalkyl, heterocyclyl, aryl, heteroaryl, alkyl-substituted sulfonyl, aryl-substituted sulfonyl, or heteroaryl-substituted sulfonyl; $R_a$ and $R_b$, together with the nitrogen atoms to which they are attached, form a heterocycloalkyl or do not form a ring;

$R_4$ is selected from hydrogen, alkyl, deuterated alkyl, halogenated alkyl, amino-substituted alkyl, or alkylamino-substituted alkyl, or

$$-\S-(CH_2)_s-X_c;$$

s is selected from an integer of 1-10;

$R_5$ and $R_6$ are simultaneously hydrogen or alkyl; $R_5$ and $R_6$, together with the carbon atoms to which they are attached, form a cycloalkyl, heterocycloalkyl, or do not form a ring; or $R_5$ and $R_6$ are different, and are each independently

selected from hydrogen, alkyl, cycloalkyl, or cycloalkyl-substituted alkyl;

$X_1$ and $X_4$ are hydrogen;

$X_a$, $X_c$, $X_2$, and $X_3$ are each independently selected from hydrogen, halogen, $C_{1-4}$ alkyl, or

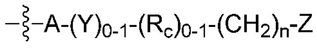,

wherein A is selected from oxygen, sulfur, -NH, $-NCD_3$, $-NCF_3$, $-NCH_3$, $-NCH_2CH_3$, or $-NCH_2CH_2CH_3$; Y is selected from -C(=O)-, $-S(=O)_2-$, or $-CHCF_3$; $R_c$ is selected from a 3- to 10-membered saturated or partially unsaturated heterocyclyl, a $C_{3-10}$ saturated or partially unsaturated carbocyclyl, or

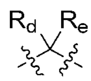;

n is selected from an integer of 0-10; Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, deuterium, trifluoromethyl, alkyl, $C_{2-6}$ alkenyl, $C_{2-6}$ alkynyl, cycloalkyl, cycloalkyl-substituted alkyl, heterocyclyl, aryl, or heteroaryl; $R_d$ and $R_e$, together with the carbon atoms to which they are attached, form a cycloalkyl, heterocycloalkyl, or do not form a ring;

when one of $R_2$ or $R_3$ is hydrogen, the other is not a $C_{1-4}$ alkyl; when $R_2$ and $R_3$ are both hydrogen, $X_2$ or $X_3$ is not

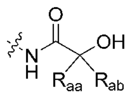, 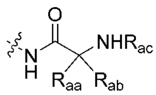,

$-NHCH_3$, $-NHCH_2CH_3$, or $-NHCH_2CH_2CH_3$; wherein $R_{aa}$, $R_{ab}$, and $R_{ac}$ are each independently selected from hydrogen or $C_{1-4}$ alkyl.

An application of the foregoing ecteinascidin derivative in the preparation of anticancer drugs.

An antibody-drug conjugate, characterized in that, the antibody-drug conjugate comprises the foregoing ecteinascidin derivative and an antibody.

An application of the foregoing ecteinascidin derivative in the preparation of anticancer drugs.

A compound of general formula (III), or a pharmaceutically acceptable salt, tautomer, or stereoisomer thereof,

$M-L_1-L_2-D$ General formula (III)

M is selected from

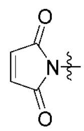, 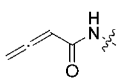, 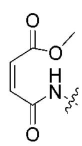, 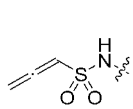, 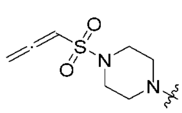,

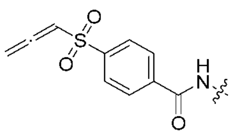, 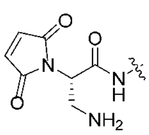, 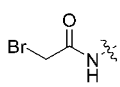 or 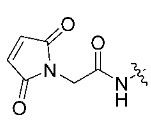;

$L_1$ is selected from

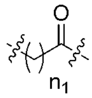, 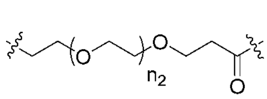,

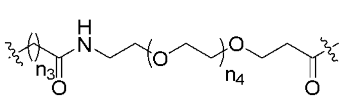, 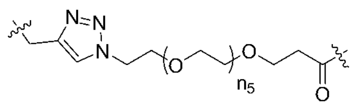,

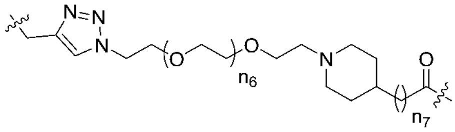,

or a combination thereof; $n_1$ is an integer of 1-20, $n_2$ is an integer of 0-20, $n_3$ is an integer of 1-20, $n_4$ is an integer of 0-20, $n_5$ is an integer of 0-20, $n_6$ is an integer of 0-20, and $n_7$ is an integer of 0-20;

$L_2$ is 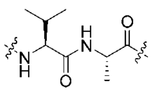, 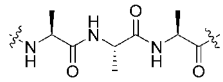, 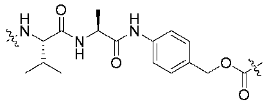,

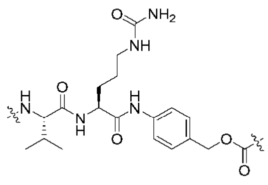, 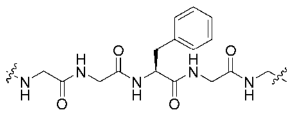, 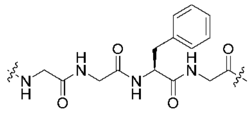,

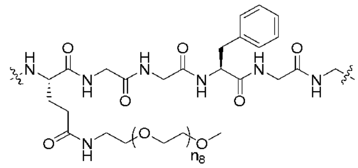, 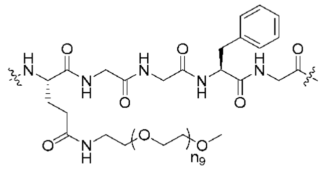, 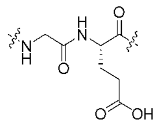,

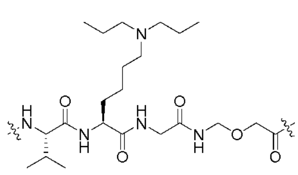, 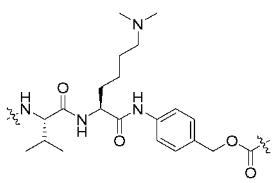, 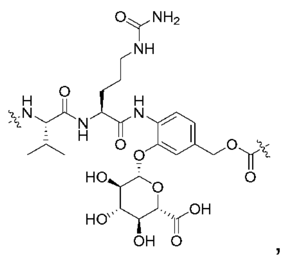,

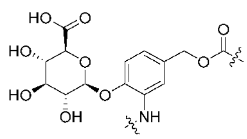, 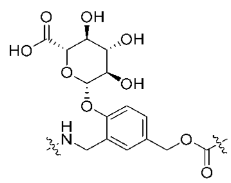, 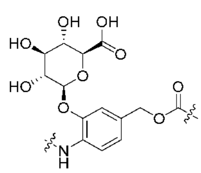, 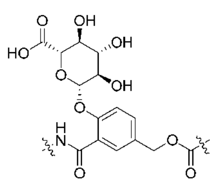,

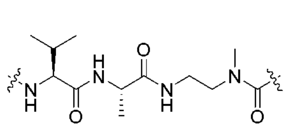, 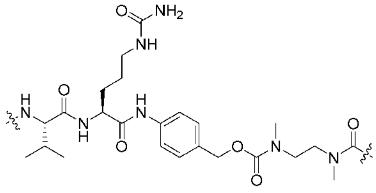,

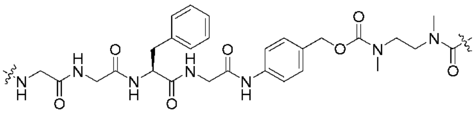, 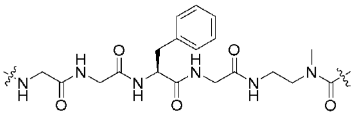,

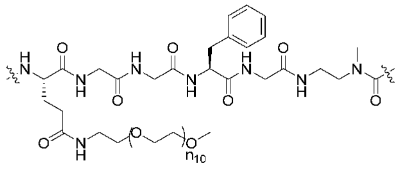 or 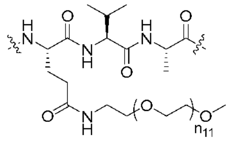;

$n_8$ is an integer of 0-20, $n_9$ is an integer of 0-20, $n_{10}$ is an integer of 0-20, and $n_{11}$ is an integer of 0-20;

D is selected from a product moiety formed by the ecteinascidin derivative as described above by loss of one or more atoms or groups;

wherein D is covalently bonded to $L_2$ through a nitrogen or oxygen atom in D.

According to one embodiment of the present invention, in general formula (III),

M is selected from

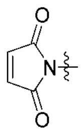, 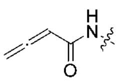, 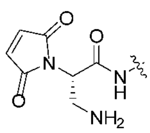, 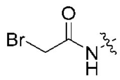 or 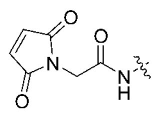;

$L_1$ is selected from

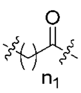, 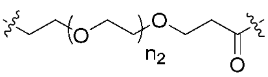,

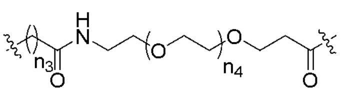,

or a combination thereof; $n_1$ is an integer of 1-20, $n_2$ is an integer of 0-20, $n_3$ is an integer of 1-20, or $n_4$ is an integer of 0-20;

$L_2$ is 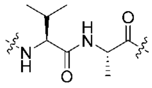, 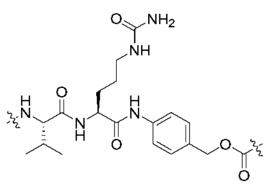, 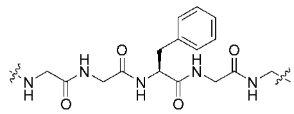,

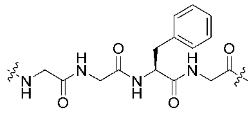, 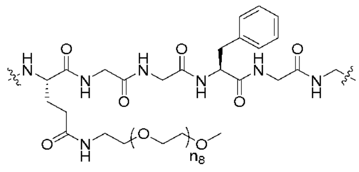, 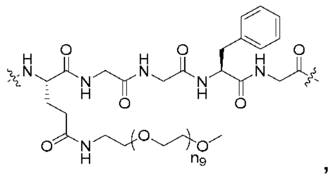,

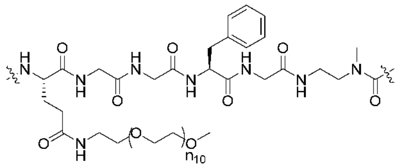 or 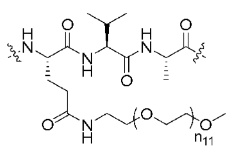;

$n_8$ is an integer of 0-20, $n_{10}$ is an integer of 0-20, or $n_{11}$ is an integer of 0-20;

D is selected from a product moiety formed by the ecteinascidin derivative as described above by loss of one or more atoms or groups;

D is covalently bonded to L2 through a nitrogen or oxygen atom in any one of $X_1$, $X_2$, $X_3$, $X_4$, $R_2$, $R_3$, or $R_4$ in D.

According to one embodiment of the present invention, in general formula (III),

M is selected from

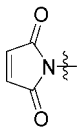, 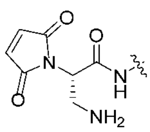, 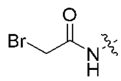 or 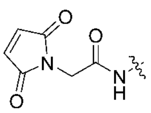;

$L_1$ is selected from

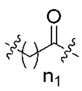, 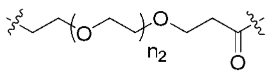,

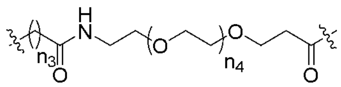,

or a combination thereof; $n_1$ is an integer of 1-18, $n_2$ is an integer of 0-18, $n_3$ is an integer of 1-18, $n_4$ is an integer of 0-18;

$L_2$ is

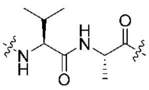, 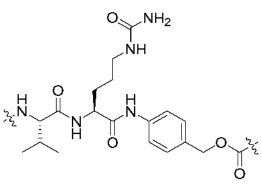, 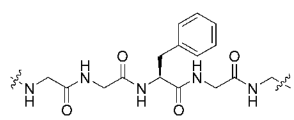,

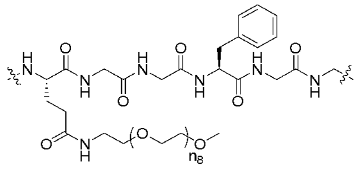, 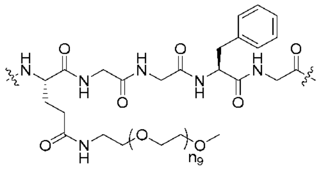 or 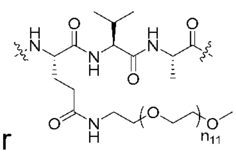;

$n_8$ is an integer of 0-18, or $n_{11}$ is an integer of 0-18;

D is selected from a product moiety formed by the ecteinascidin derivative as described above by loss of one or more atoms or groups;

D is covalently bonded to $L_2$ through a nitrogen or oxygen atom in any one of $X_1$, $X_2$, $X_3$, $X_4$, $R_2$, $R_3$, or $R_4$ in D.

According to one embodiment of the present invention, in general formula (III),

M is selected from

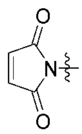, 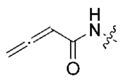 or 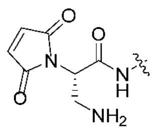;

$L_1$ is selected from

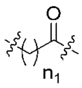, 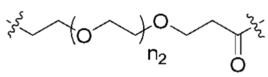,

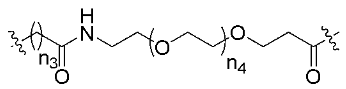,

or a combination thereof; $n_1$ is an integer of 1-20, $n_2$ is an integer of 1-20, $n_3$ is an integer of 1-20, or $n_4$ is an integer of 1-20;

$L_2$ is

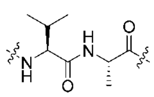, 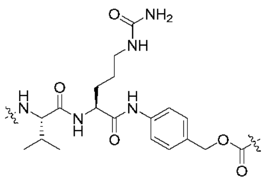, 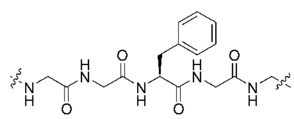,

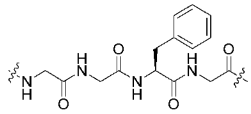, 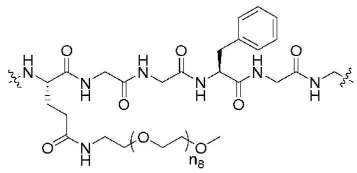 or 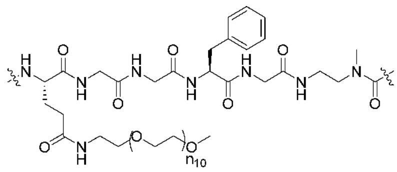;

$n_8$ is an integer of 1-20, $n_{10}$ is an integer of 1-20;

D is selected from a product moiety formed by the ecteinascidin derivative as decribed above by loss of one or more atoms or groups;

D is covalently bonded to $L_2$ through a nitrogen or oxygen atom in any one of $X_1$, $X_2$, $X_3$, $X_4$, $R_2$, $R_3$, or $R_4$ in D.

According to one embodiment of the present invention, in general formula (III), M is selected from

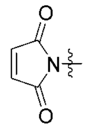, 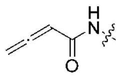, 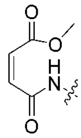, 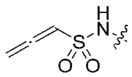, 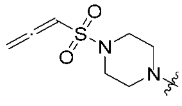,

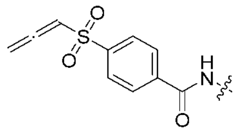, 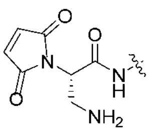 or 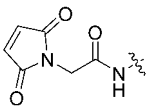;

$L_1$ is selected from

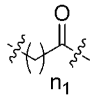, 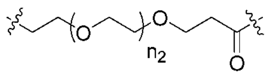,

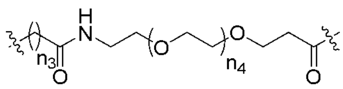, 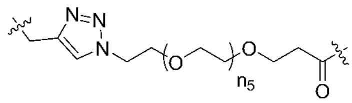,

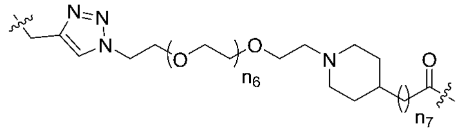,

or a combination thereof; $n_1$ = 1-20, $n_2$ = 1-20, $n_3$ = 1-20, $n_4$ = 1-20, $n_5$ = 1-20, $n_6$ = 1-20, or $n_7$ = 1-20;

$L_2$ is

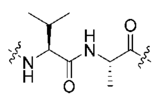, 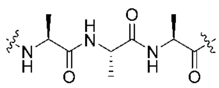, 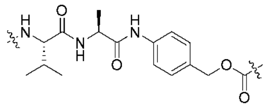,

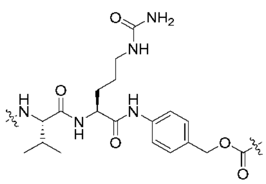, 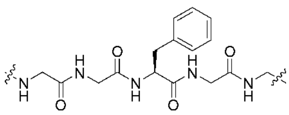, 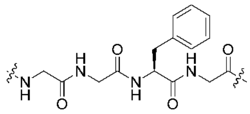,

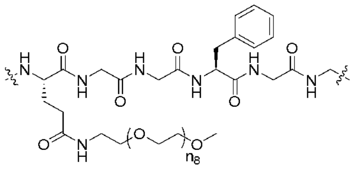, 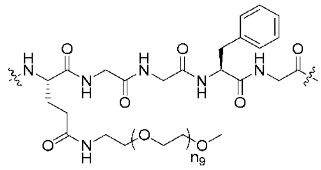, 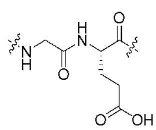,

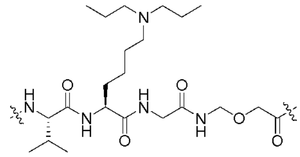, 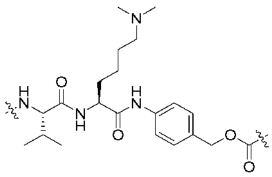, 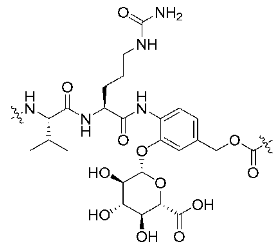,

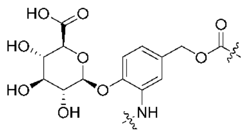, 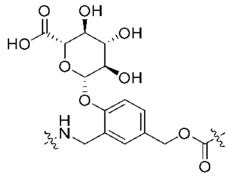, 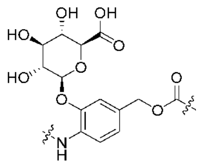, 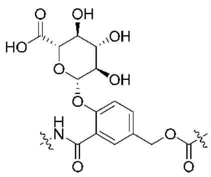,

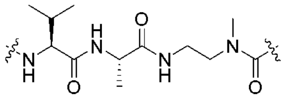, 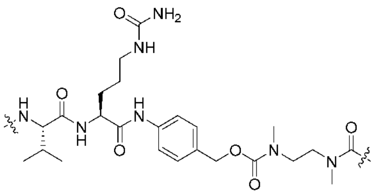,

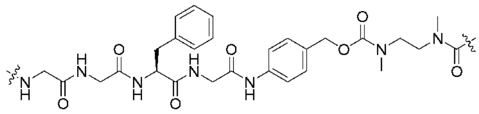, 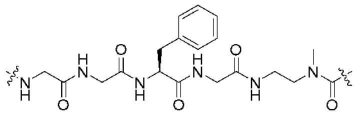,

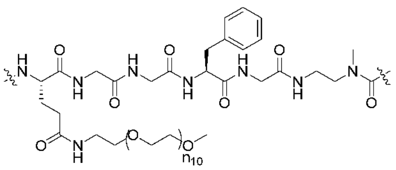;

$n_8$ = 1-20, $n_9$ = 1-20, or $n_{10}$ = 1-20;

D is selected from a product moiety formed by the foregoing ecteinascidin derivative, or a pharmaceutically acceptable salt, tautomer, or stereoisomer thereof by loss of one or more atoms or groups;

wherein D is covalently bonded to $L_2$ through a nitrogen or oxygen atom in D.

According to one embodiment of the present invention, in general formula (III),

wherein M is selected from

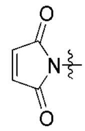, 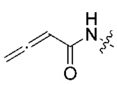 or 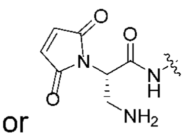;

$L_1$ is selected from

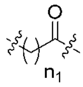, 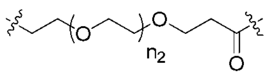,

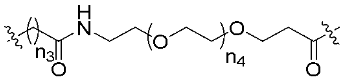

or a combination thereof; $n_1$ = 1-20, $n_2$ = 1-20, $n_3$ = 1-20, or $n_4$ = 1-20;

$L_2$ is

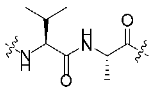, 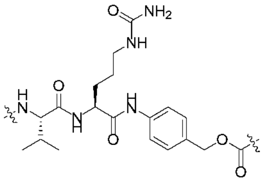, 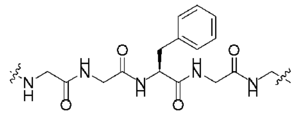,

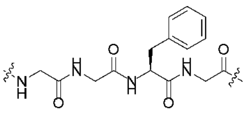, 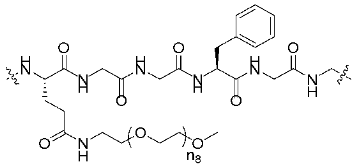 or 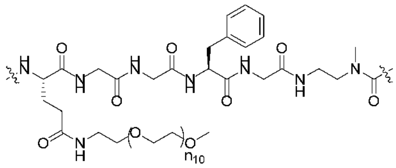;

$n_8$ = 1-20, or $n_{10}$ = 1-20;

D is selected from a product moiety formed by the foregoing ecteinascidin derivative, or a pharmaceutically acceptable salt, tautomer, or stereoisomer thereof by loss of one or more atoms or groups;

D is covalently bonded to $L_2$ through a nitrogen or oxygen atom in D in $X_1$, $X_2$, $X_3$, $X_4$, $R_2$, $R_3$ or $R_4$.

According to one embodiment of the present invention, in general formula (III),

wherein M is selected from

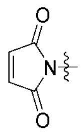;

$L_1$ is selected from

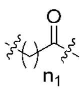, 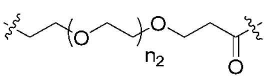,

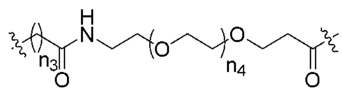

or a combination thereof; $n_1$ = 1-20, $n_2$ = 1-20, $n_3$ = 1-20, $n_4$ = 1-20;

$L_2$ is

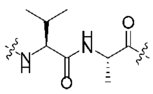, 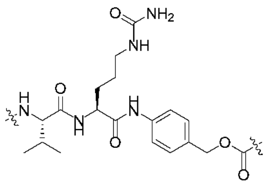, 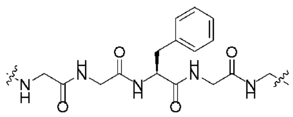

or

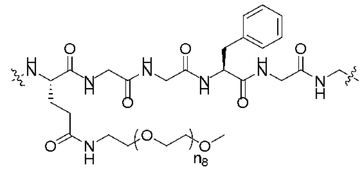;

$n_8$ = 1-20;

D is selected from a product moiety formed by the foregoing ecteinascidin derivative, or a pharmaceutically acceptable salt, tautomer, or stereoisomer thereof by loss of one or more atoms or groups;

D is covalently bonded to $L_2$ through a nitrogen or oxygen atom in D in $X_1$, $X_2$, $X_3$, $X_4$, $R_2$, $R_3$ or $R_4$,

wherein $n_1$, $n_2$, $n_3$, $n_4$, $n_5$, $n_6$, $n_7$, $n_8$, $n_9$, $n_{10}$ are integers.

According to one embodiment of the present invention, the compound of general formula (III) is selected from at least one of the compounds in the following table:

| No. | Structure |
|---|---|
| LP1 | 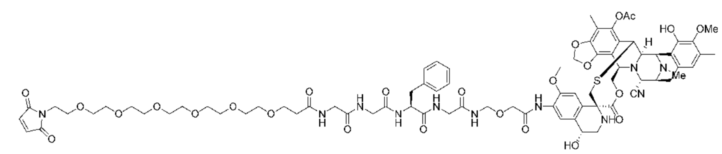 |
| LP2 | 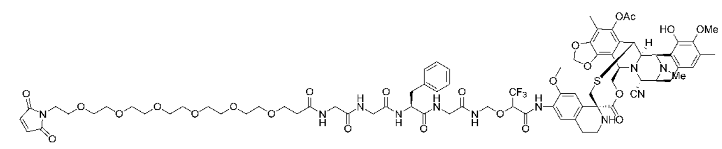 |
| LP3 | 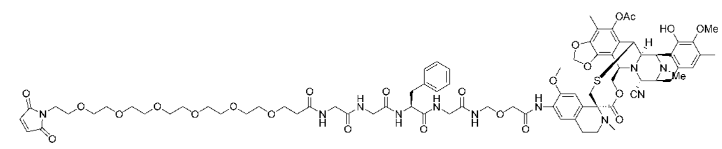 |
| LP4 | 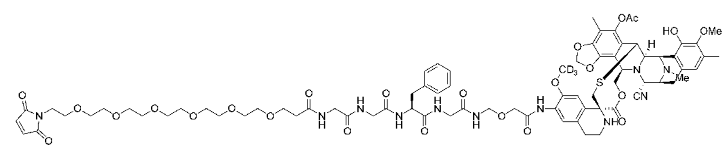 |
| LP5 | 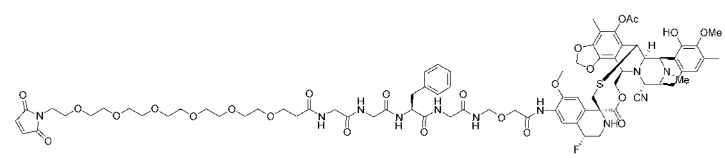 |

(continued)

| No. | Structure |
|---|---|
| LP6 | 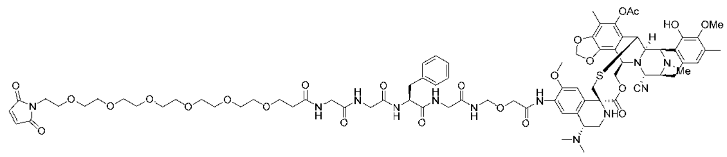 |
| LP7 | 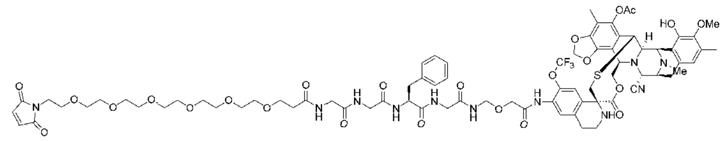 |
| LP8 | 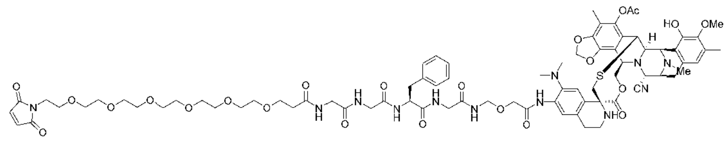 |
| LP9 | 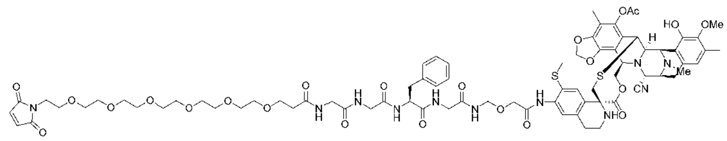 |
| LP10 | 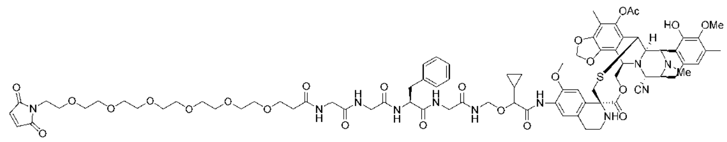 |
| LP11 | 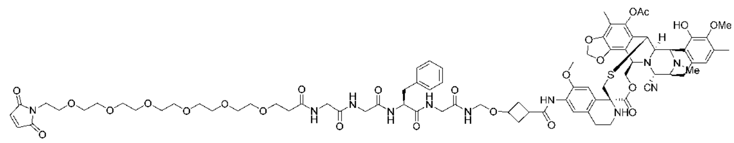 |
| LP12 | 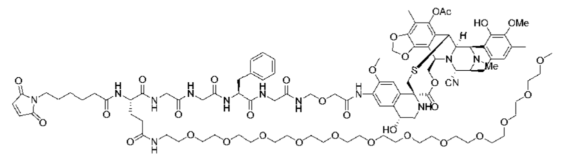 |
| LP13 | 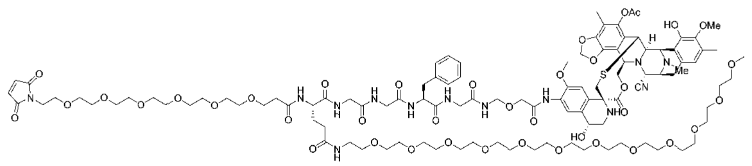 |
| LP14 | 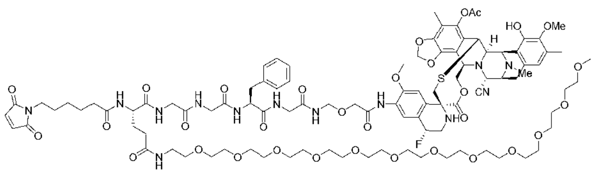 |
| LP15 | 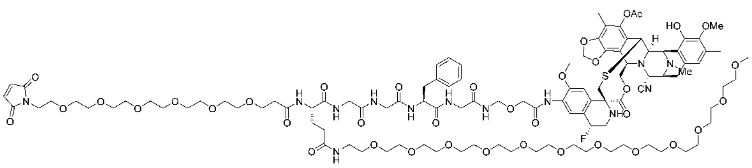 |

(continued)

| No. | Structure |
|---|---|
| LP16 | 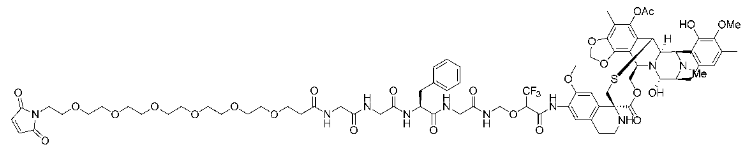 |
| LP17 | 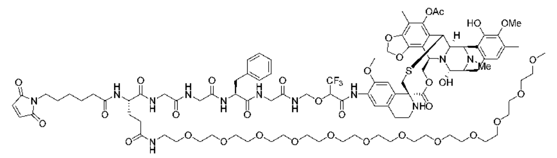 |
| LP18 | 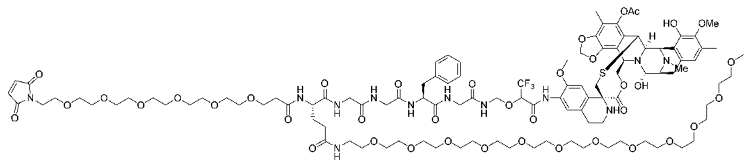 |
| LP19 | 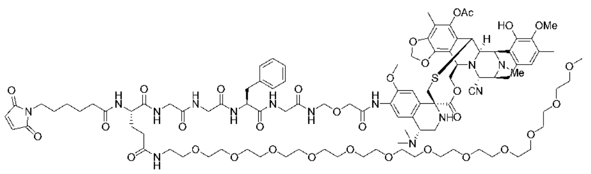 |
| LP27 | 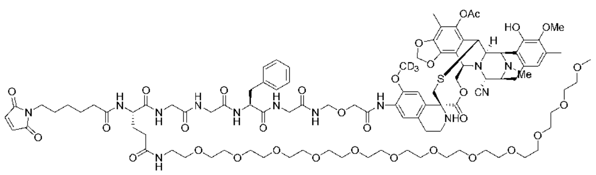 |
| LP28 | 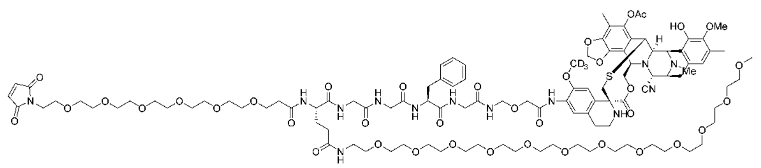 |
| LP29 | 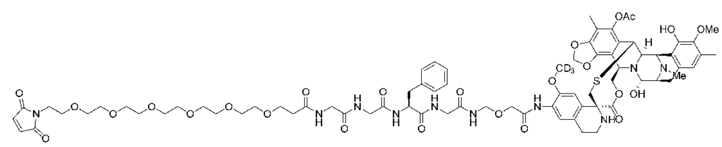 |
| LP30 | 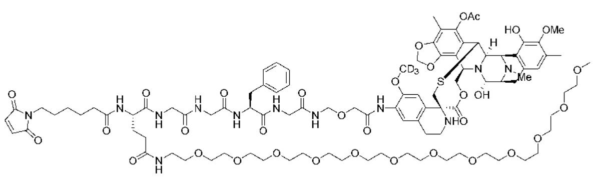 |
| LP31 | 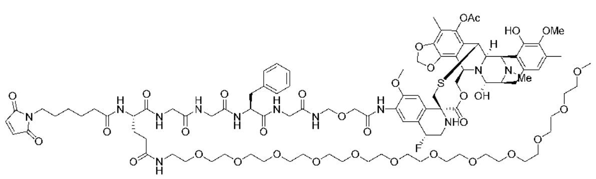 |

(continued)

| No. | Structure |
|---|---|
| LP34 | 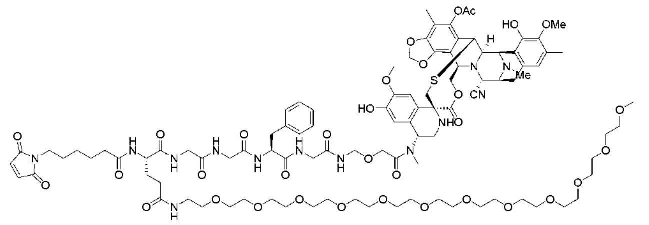 |
| LP58 | 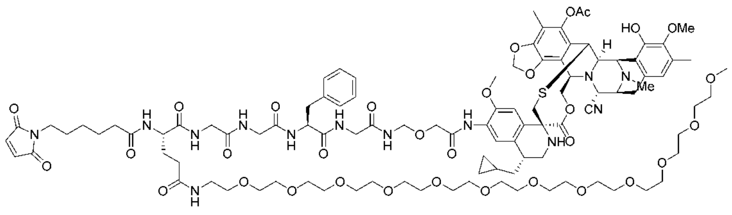 |
| LP59 | 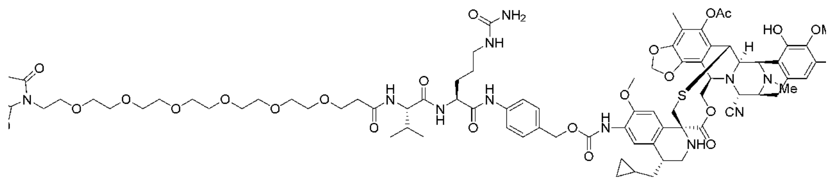 |
| LP62 | 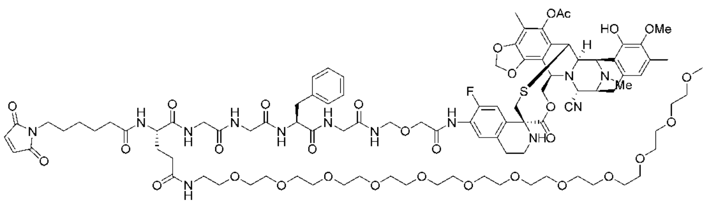 |
| LP63 | 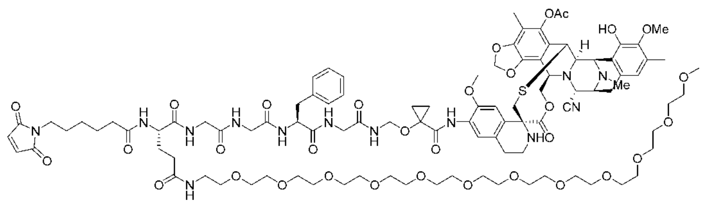 |
| LP64 | 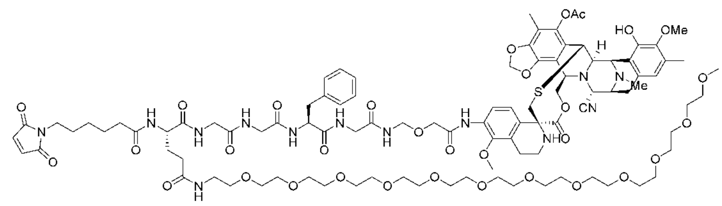 |
| LP65 | 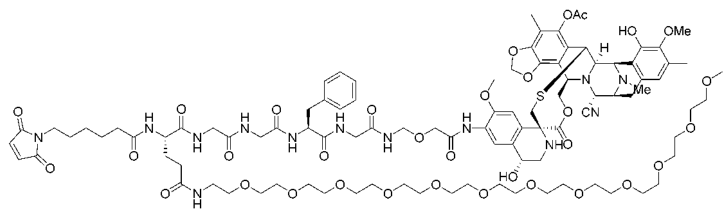 |

(continued)

| No. | Structure |
|---|---|
| LP66 | 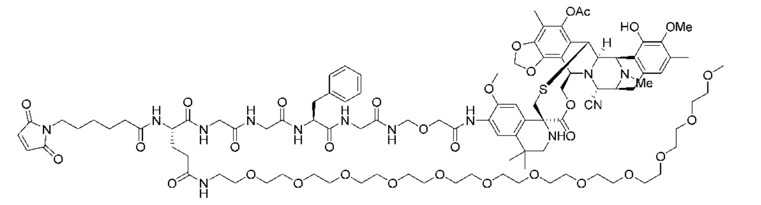 |
| LP67 | 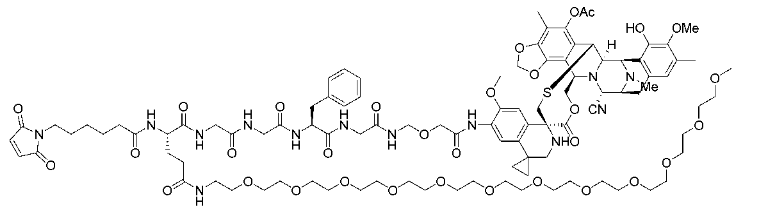 |
| LP68 | 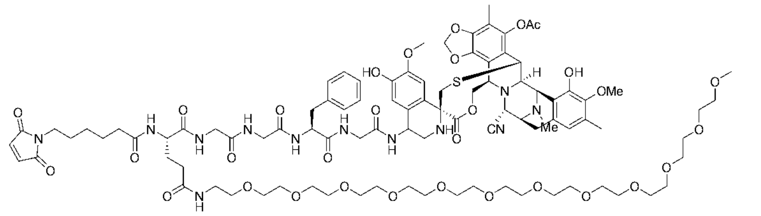 |
| LP69 | 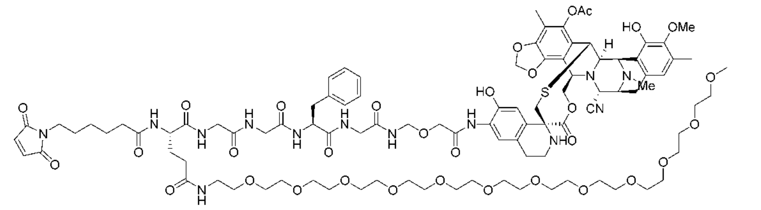 |
| LP70 | 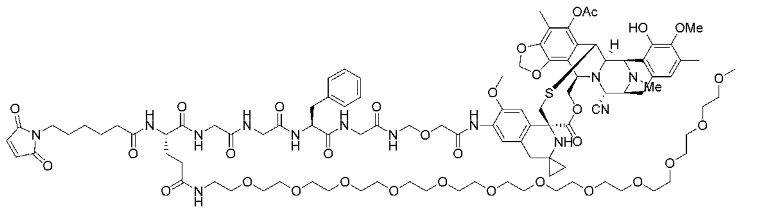 |
| LP71 | 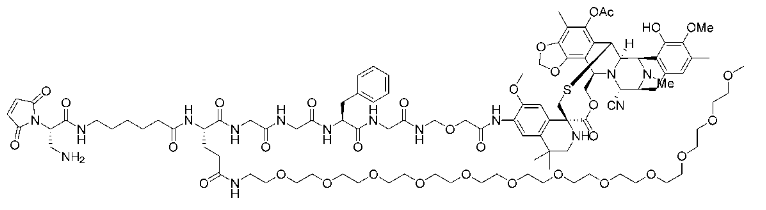 |
| LP72 | 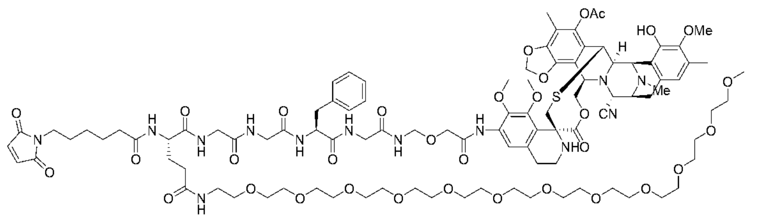 |

(continued)

| No. | Structure |
|---|---|
| LP73 | 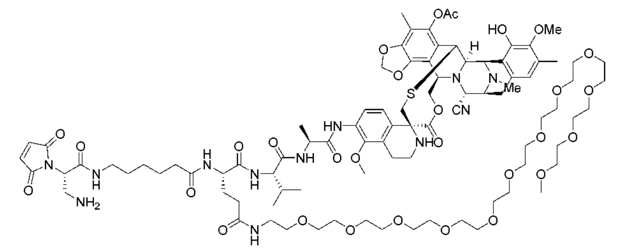 |
| LP74 | 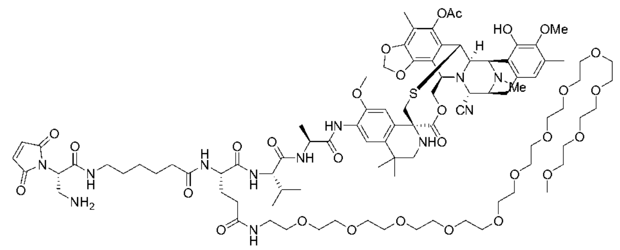 |
| LP75 | 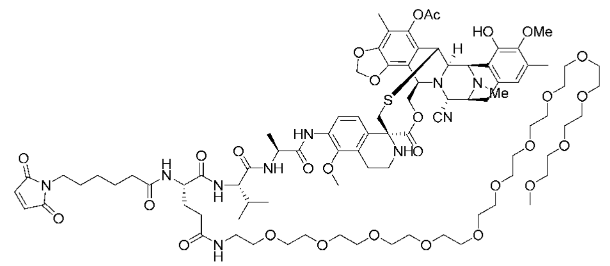 |
| LP76 | 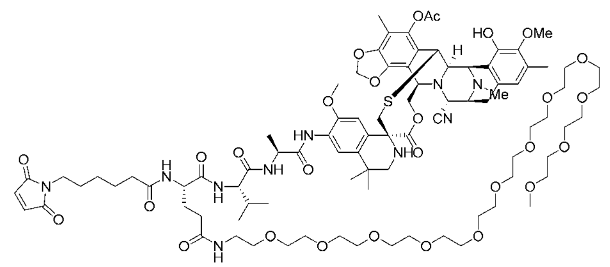 |
| LP77 | 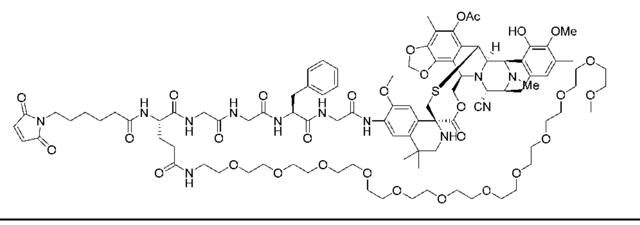 |
| LP78 | 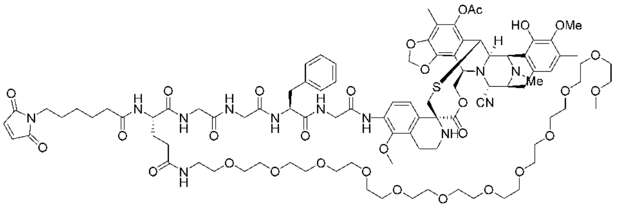 |

(continued)

| No. | Structure |
|---|---|
| LP79 | 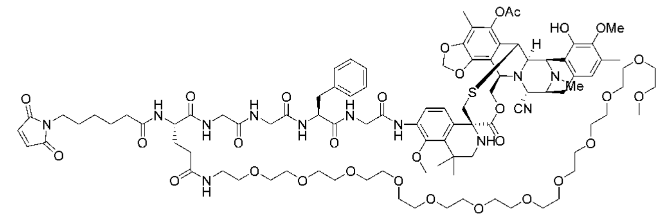 |
| LP80 | 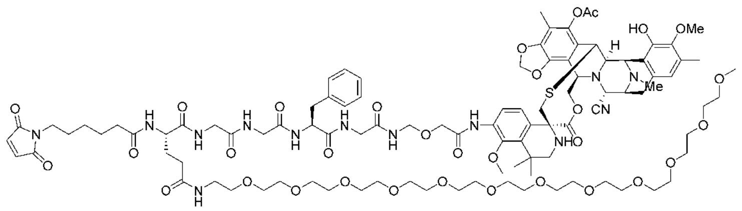 |

An antibody-drug conjugate, characterized in that the antibody-drug conjugate comprises a structure as shown in general formula (IV):

$$Ab\left(-M-L_1-L_2-D\right)_{n_{12}}$$

General formula (IV)

wherein Ab is an antibody, an antibody fragment, or a protein, wherein the antibody is selected from a murine antibody, a rabbit antibody, a phage display-derived antibody, a yeast display-derived antibody, a chimeric antibody, a humanized antibody, a fully human antibody, an antibody fragment, a bispecific antibody, and a multispecific antibody; M-$L_1$-$L_2$-D is selected from any compound as described above, or a pharmaceutically acceptable salt thereof, or a tautomer or stereoisomer thereof; and $n_{12}$ is any numerical value selected from 0 to 20.

In M-$L_1$-$L_2$-D, the carbon-carbon double bond of M is connected to a mercapto group in the antibody Ab via a Michael addition reaction, or a C-S bond is formed through a nucleophilic substitution reaction between the mercapto sulfur atom in the antibody Ab and a bromomethylene group of M.

According to one embodiment of the present invention, $n_{12}$ is any numerical value selected from 1 to 20.

According to one embodiment of the present invention, in the general formula (IV), Ab is a monoclonal antibody.

According to one embodiment of the present invention, in general formula (IV), the Ab is selected from Abciximab, Alemtuzumab, Anetumab, Atezolizumab, Avelumab, Basiliximab, Bevacizumab, Blinatomumab, Brentuximab, Catumaxomab, Cetuximab, Cirmtuzumab, Coltuximab, Daclizumab, Daratumumab, Denintuzumab, Denosumab, Depatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labetuzumab, Ladiratuzumab, Laprituximab, Lifastuzumab, Lorvotuzumab, Milatuzumab, Mirvetuximab, Naratuximab, Necitumumab, Nimotuzumab, Nivolumab, Obinutuzumab, Ofatumumab, Olaratumab, Omalizumab, Palivizumab, Panitumumab, Patritumab, Pembrolizumab, Pertuzumab, Pinatuzumab, Polatuzumab, Ramucirumab, Rovalpituzumab, Sacituzumab, Siltuximab, Sirtratumab, Sofituzumab, Vadastuximab, Vorsetuzumab, Trastuzumab, an anti-CD4 antibody, an anti-CD5 antibody, an anti-CD13 antibody, an anti-CD30 antibody, an antigen-binding fragment thereof, or an immunologically active portion thereof; M-$L_1$-$L_2$-D is selected from a compound according to any one of claims 16 to 20, or a pharmaceutically acceptable salt thereof, or a tautomer or stereoisomer thereof; and $n_{12}$ is any numerical value selected from 0 to 12.

The English name of axicimab is: Abciximab; the English name of alenutuzumab is: Alemtuzumab; the English name of anetumab is: Anetumab; the English name of atezolizumab is: Atezolizumab; the English name of avelumab is: Avelumab; the English name of basiliximab is: Basiliximab; the English name of bevacizumab is: Bevacizumab; the English name of blinatomumab is: Blinatomumab; the English name of brentuximab is: Brentuximab; the English name of catumaxomab is: Catumaxomab; the English name of cetuximab is: Cetuximab; the English name of cirmtuzumab is: Cirmtuzumab; the English name of coltuximab is: Coltuximab; the English name of daclizumab is: Daclizumab; the English name of daratumumab is: Daratumumab; the English name of denintuzumab is: Denintuzumab; the English name of denosumab is: Denosumab; the English name of depatuxizumab is: Depatuxizumab; the English name of dinutuximab is:

Dinutuximab; the English name of durvalumab is: Durvalumab; the English name of elotuzumab is: Elotuzumab; the English name of enfortumab is: Enfortumab; the English name of glembatumumab is: Glembatumumab; the English name of gemtuzumab is: Gemtuzumab; the English name of ibritumomab is: Ibritumomab; the English name of indatuximab is: Indatuximab; the English name of indusatumab is: Indusatumab; the English name of inotuzumab is: Inotuzumab; the English name of ipilimumab is: Ipilimumab; the English name of labetuzumab is: Labetuzumab; the English name of ladiratuzumab is: Ladiratuzumab; the English name of laprituximab is: Laprituximab; the English name of lifastuzumab is: Lifastuzumab; the English name of lorvotuzumab is: Lorvotuzumab; the English name of milatuzumab is: Milatuzumab; the English name of mirvetuximab is: Mirvetuximab; the English name of naratuximab is: Naratuximab; the English name of necitumumab is: Necitumumab; the English name of nimotuzumab is: Nimotuzumab; the English name of nivolumab is: Nivolumab; the English name of obinutuzumab is: Obinutuzumab; the English name of ofatumumab is: Ofatumumab; the English name of olaratumab is: Olaratumab; the English name of omalizumab is: Omalizumab; the English name of palivizumab is: Palivizumab; the English name of panitumumab is: Panitumumab; the English name of patritumab is: Patritumab; the English name of pembrolizumab is: Pembrolizumab; the English name of pertuzumab is: Pertuzumab; the English name of pinatuzumab is: Pinatuzumab; the English name of polatuzumab is: Polatuzumab; the English name of ramucirumab is: Ramucirumab; the English name of rovalpituzumab is: Rovalpituzumab; the English name of sacituzumab is: Sacituzumab; the English name of siltuximab is: Siltuximab; the English name of sirtratumab is: Sirtratumab; the English name of sofituzumab is: Sofituzumab; the English name of vadastuximab is: Vadastuximab; the English name of vorsetuzumab is: Vorsetuzumab; the English name of trastuzumab is: Trastuzumab.

According to one embodiment of the present invention, in general formula (IV), the antibody-drug conjugate in general formula (IV) is selected from at least one compound of the following structural formulas:

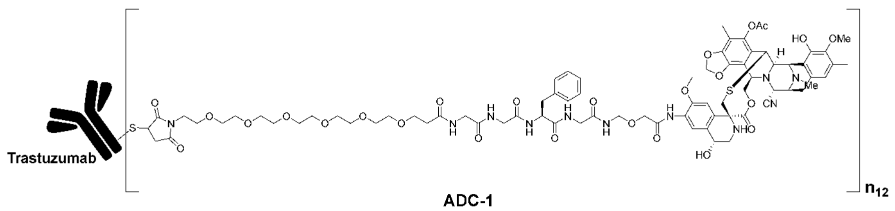

ADC-1

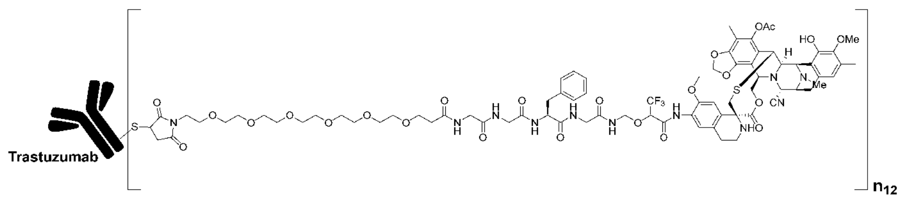

ADC-2

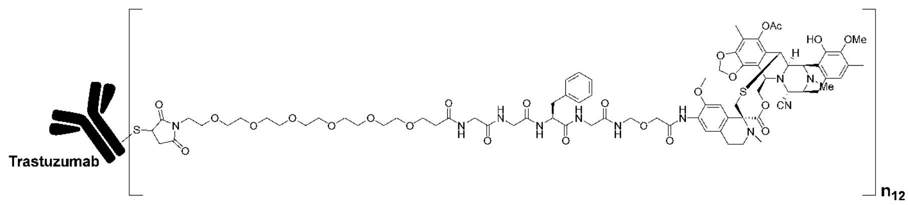

ADC-3

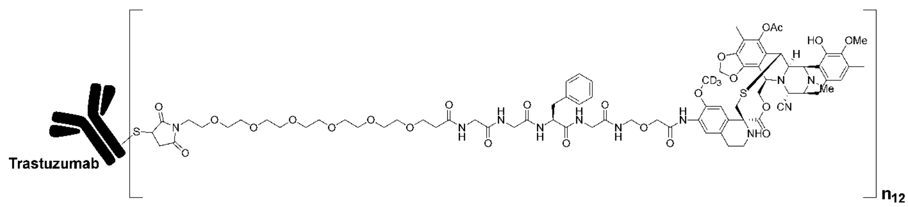

ADC-4

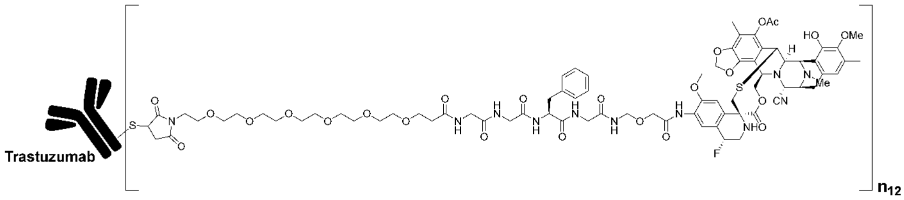

ADC-5

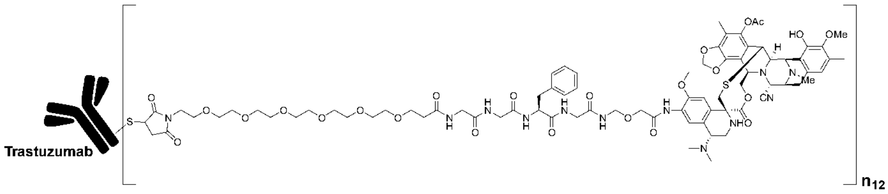

ADC-6

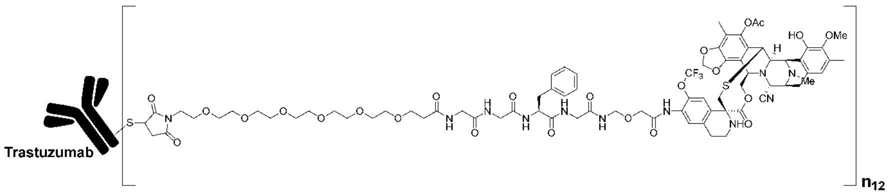

ADC-7

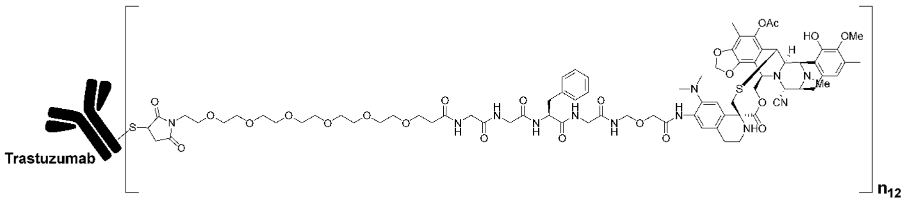

ADC-8

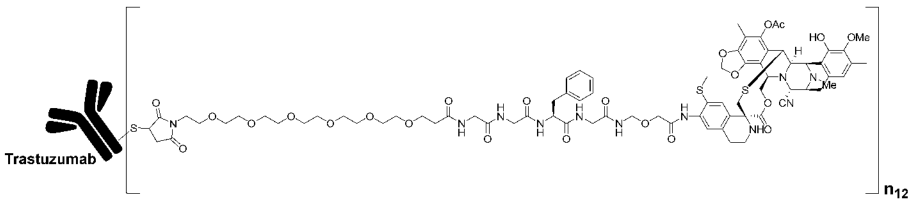

ADC-9

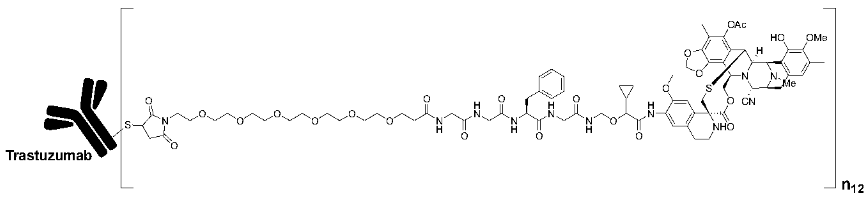

ADC-10

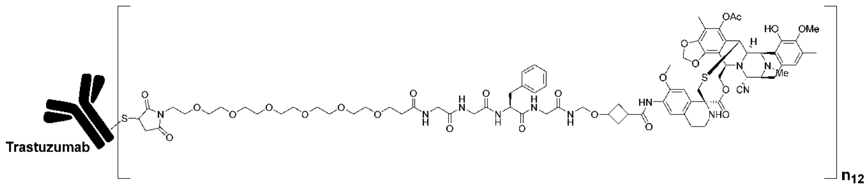

ADC-11

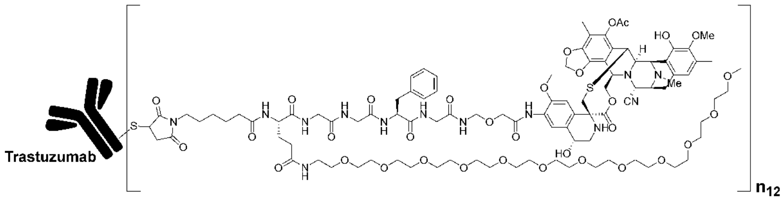

ADC-12

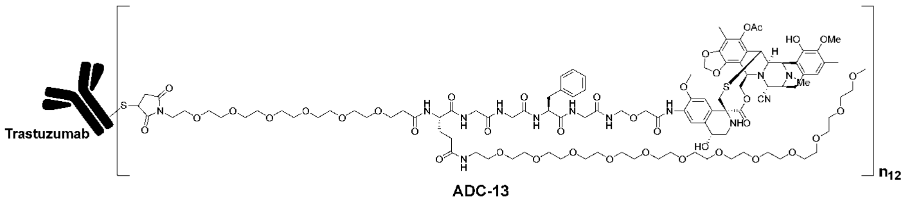

ADC-13

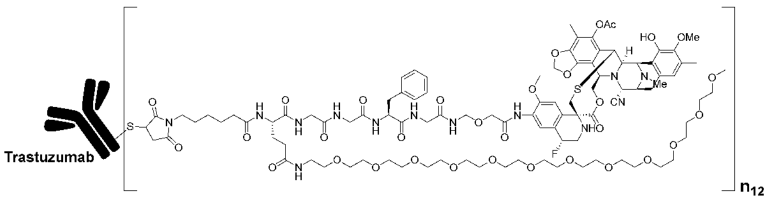

ADC-14

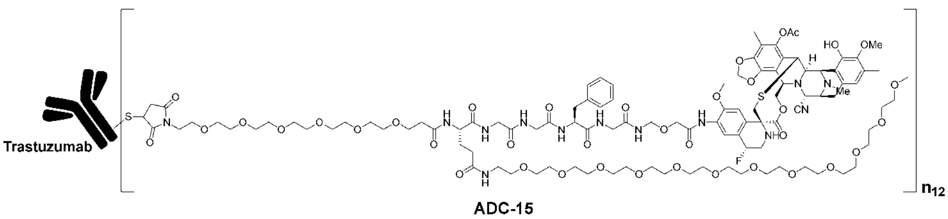

ADC-15

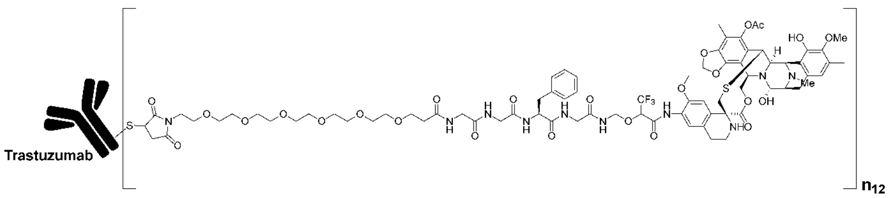

ADC-16

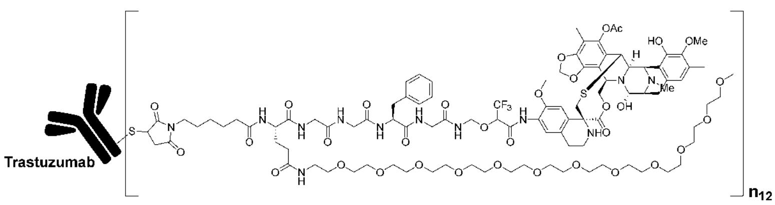

ADC-17

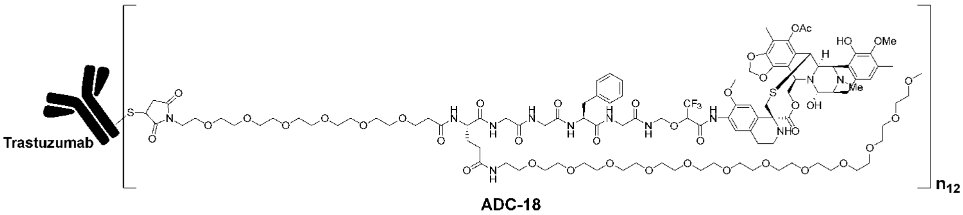

ADC-18

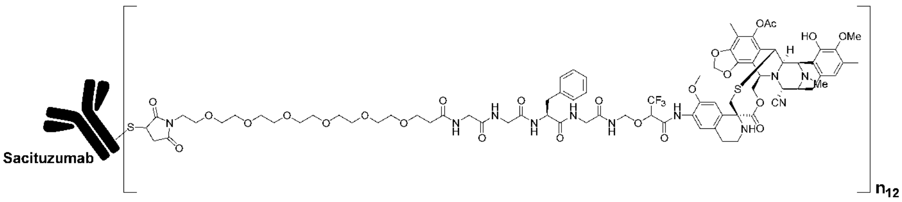

ADC-26

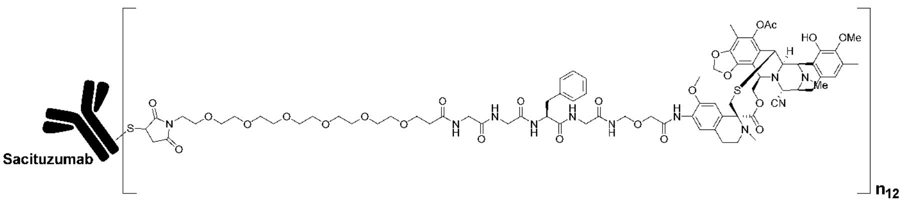

ADC-27

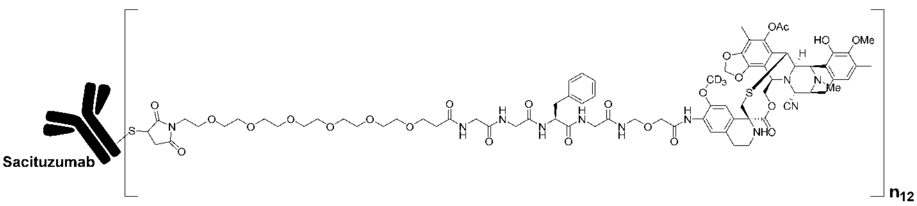

ADC-28

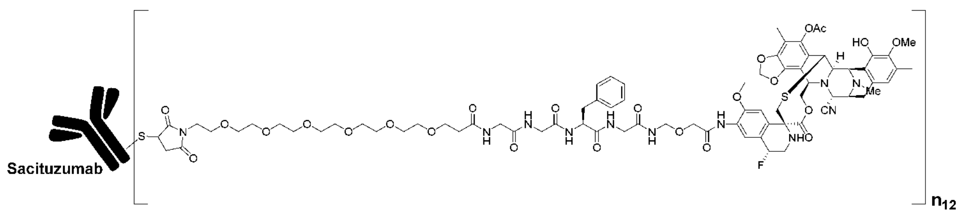

ADC-29

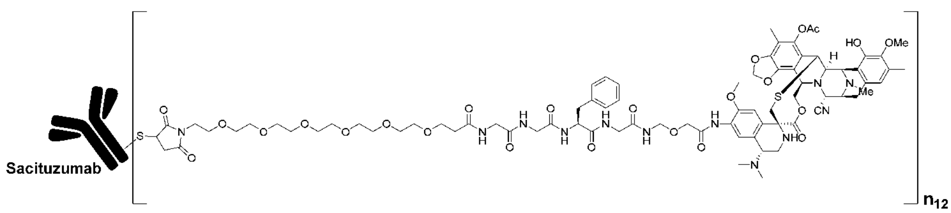

ADC-30

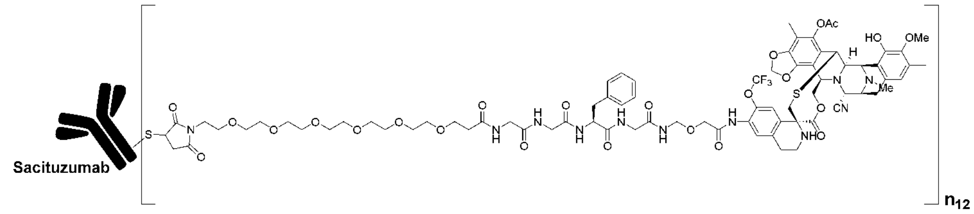

ADC-31

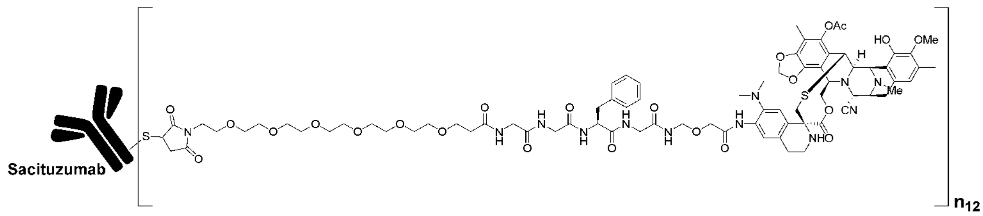

ADC-32

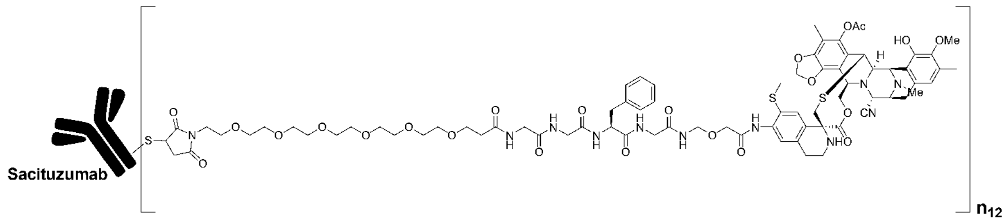

ADC-33

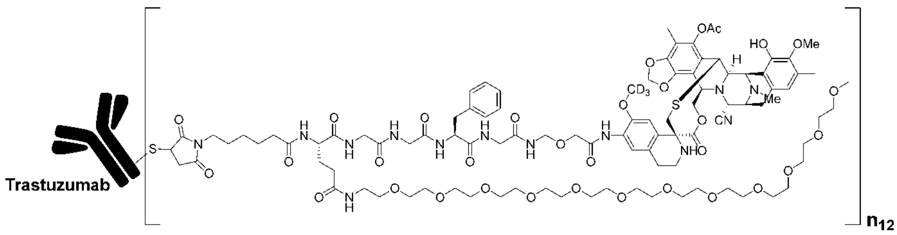

ADC-34

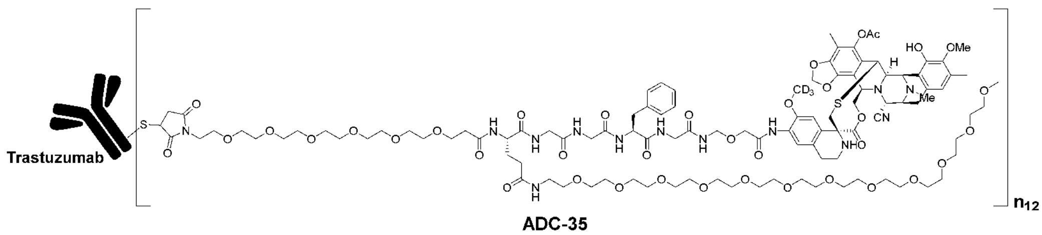

ADC-35

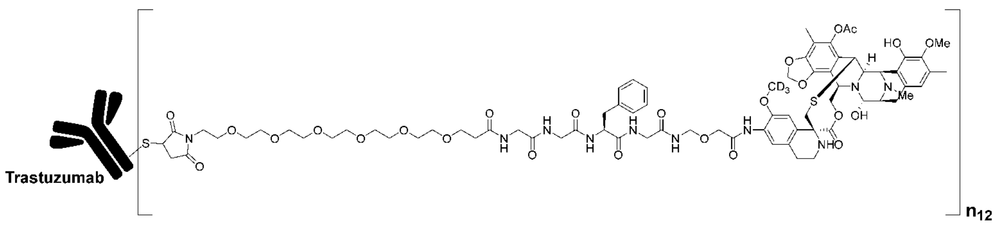

ADC-36

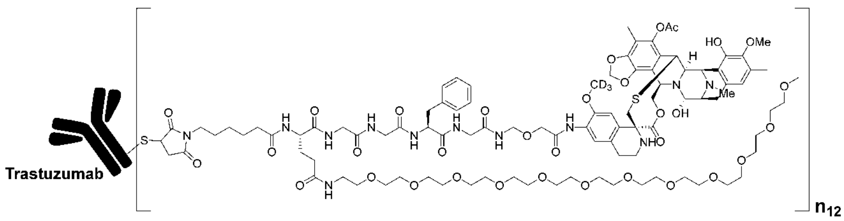

ADC-37

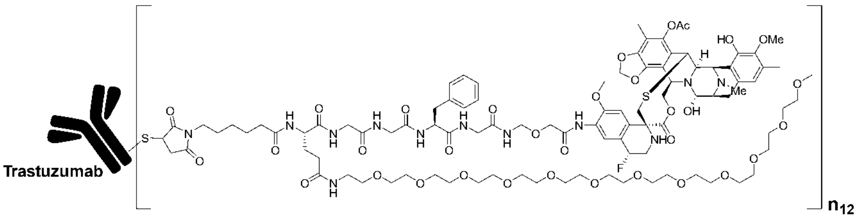

ADC-38

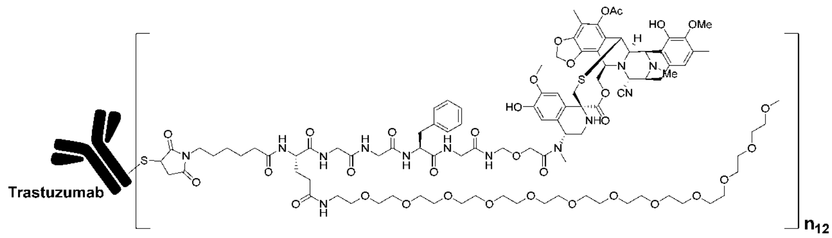

ADC-41

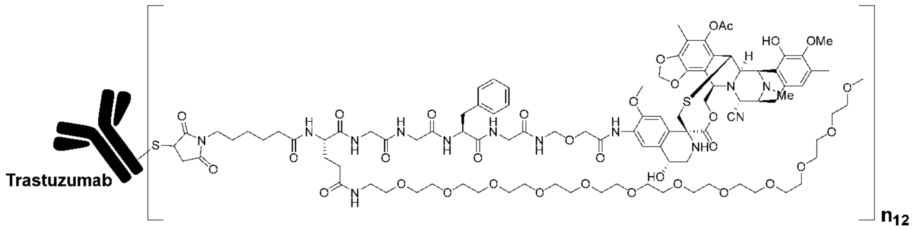

ADC-55

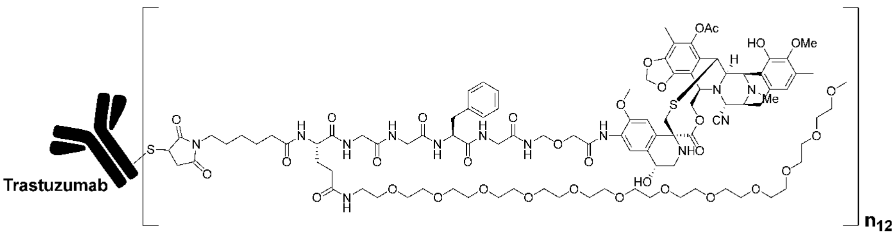

ADC-56

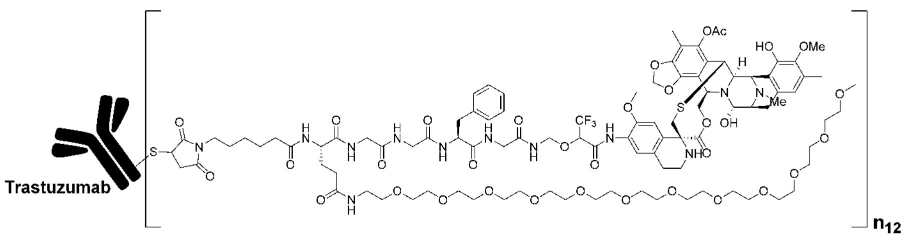

ADC-57

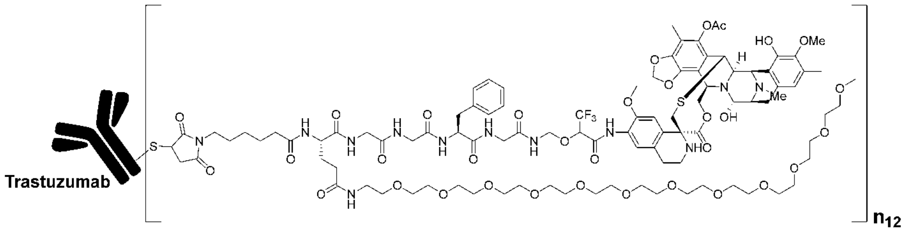

ADC-58

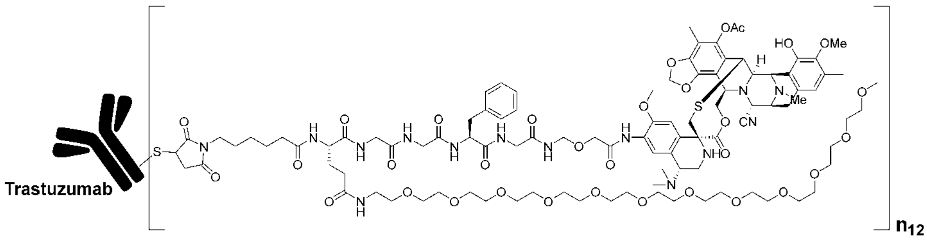

ADC-59

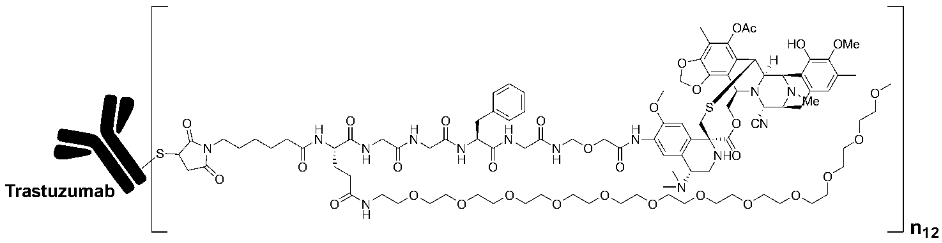

ADC-60

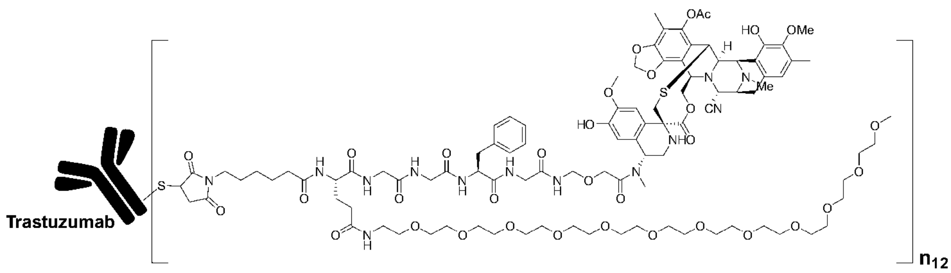

ADC-61

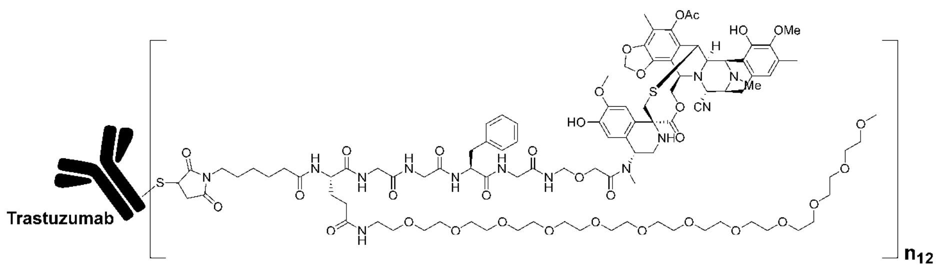

ADC-62

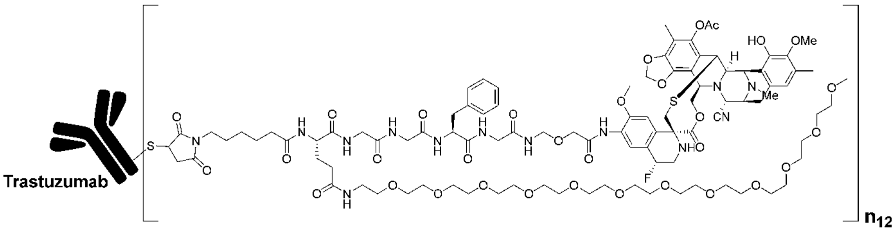

ADC-65

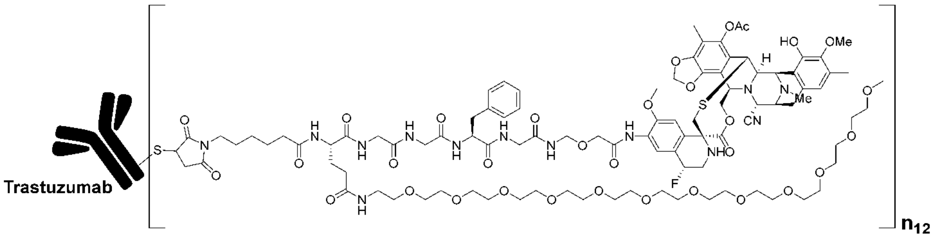

ADC-66

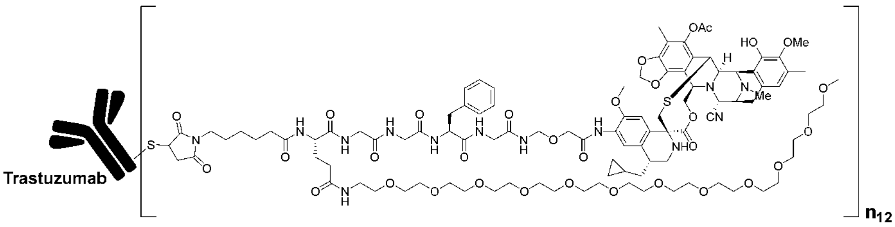

ADC-79

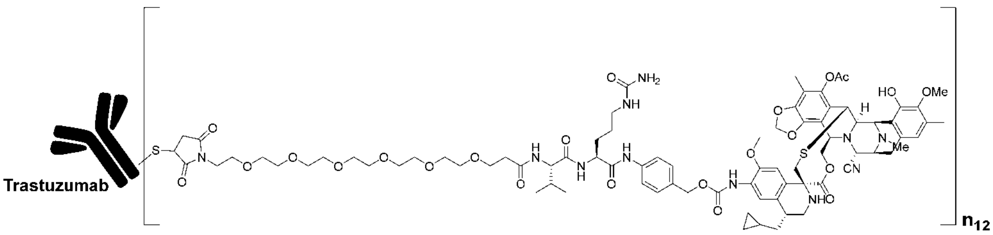

ADC-80

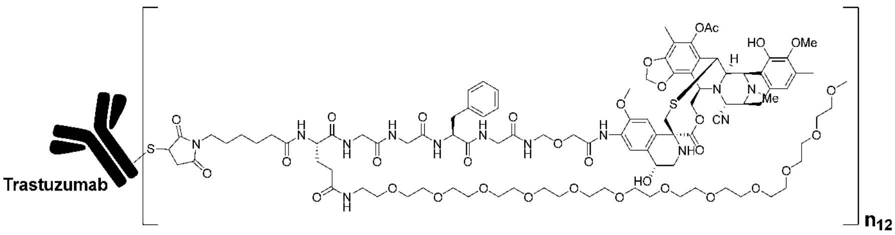

ADC-83

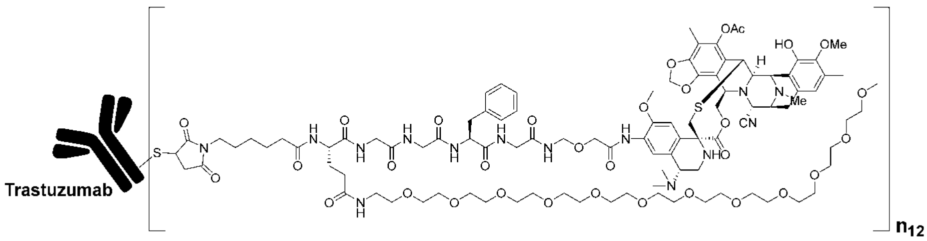

ADC-84

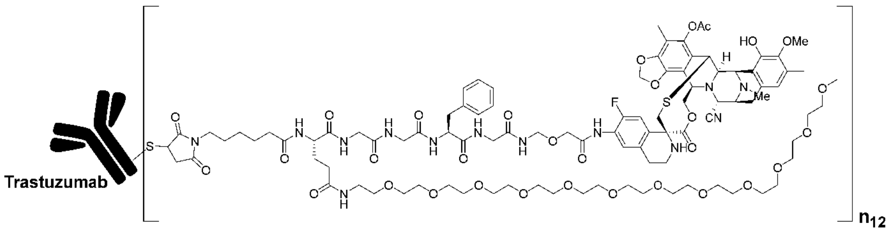

ADC-85

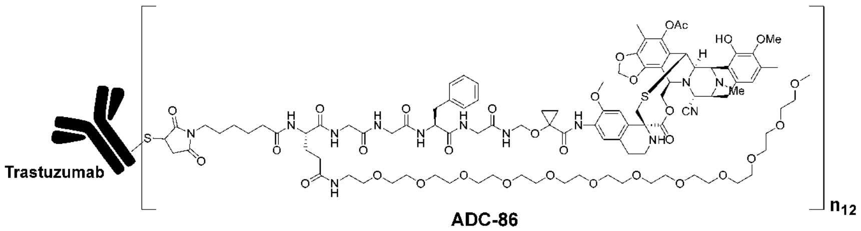

ADC-86

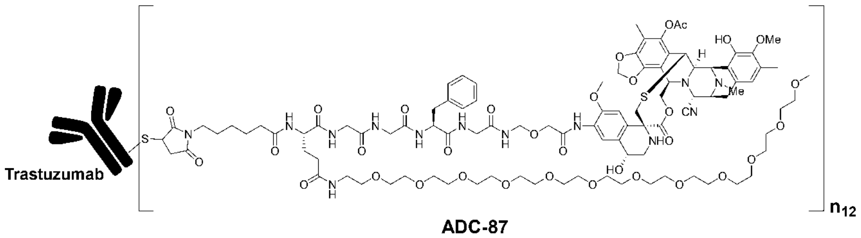

ADC-87

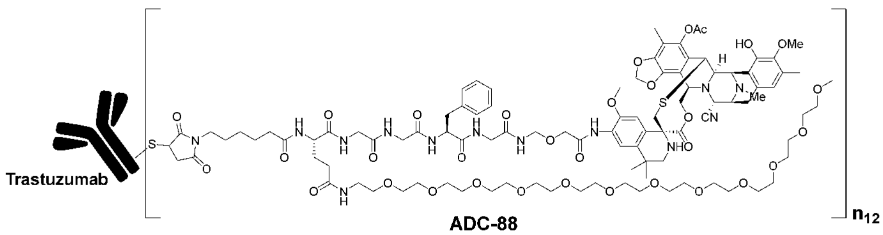

ADC-88

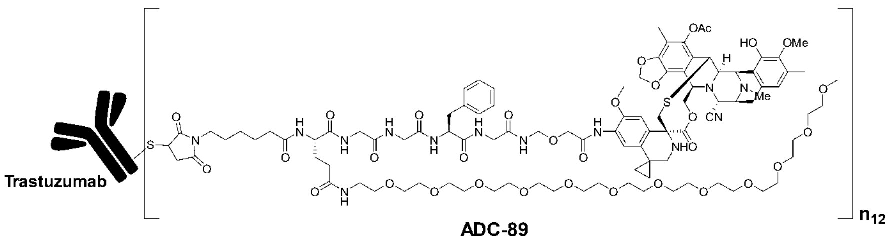

ADC-89

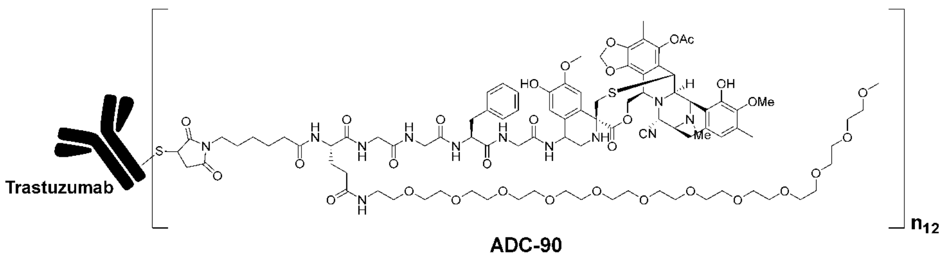

ADC-90

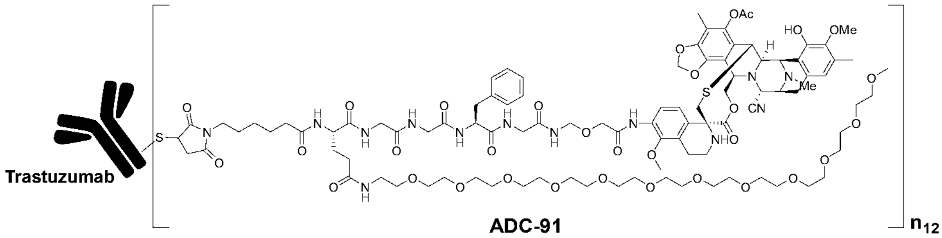

ADC-91

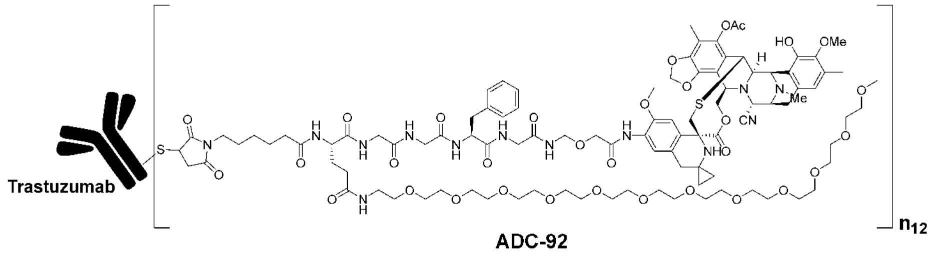

ADC-92

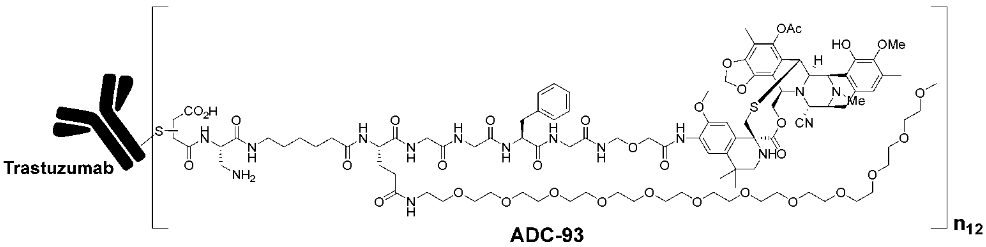

ADC-93

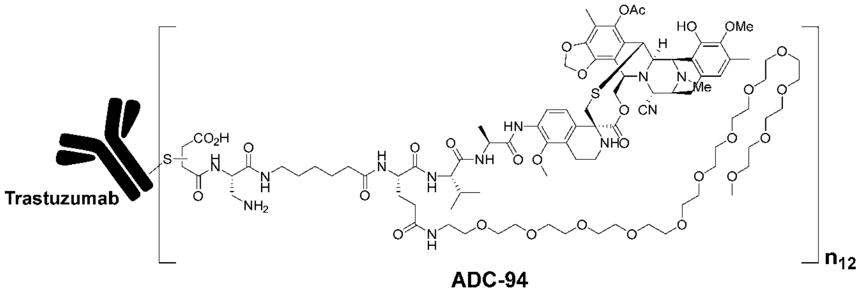

ADC-94

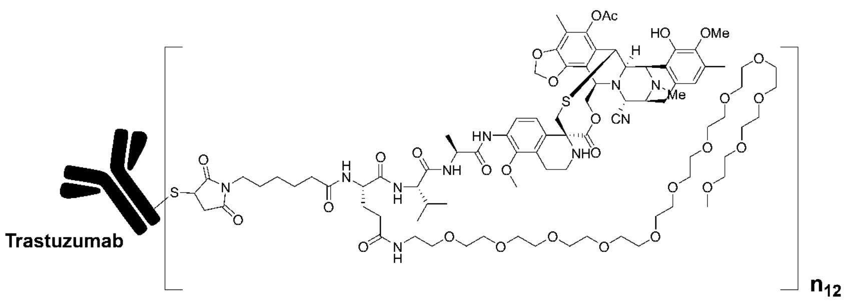

ADC-95

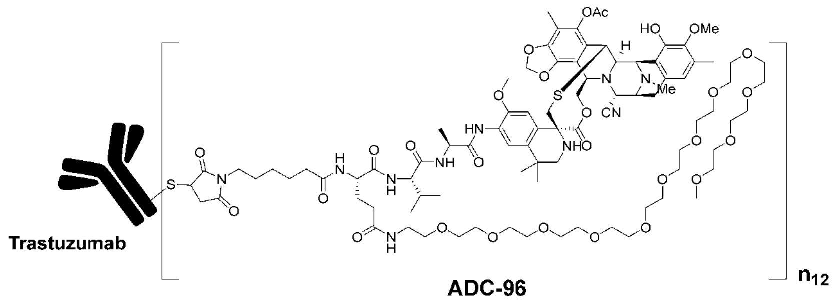

ADC-96

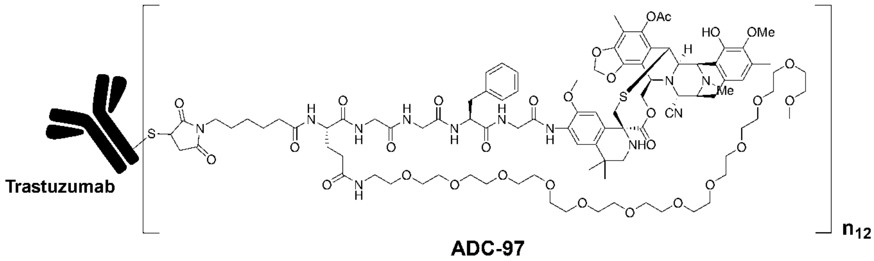

ADC-97

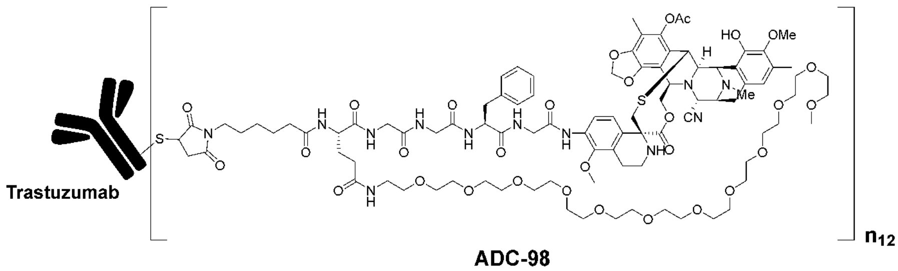

ADC-98

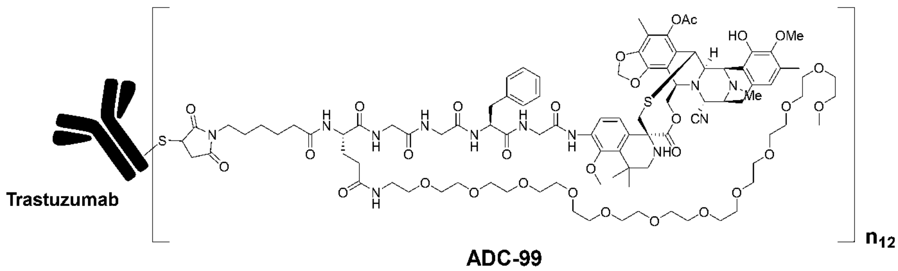

ADC-99

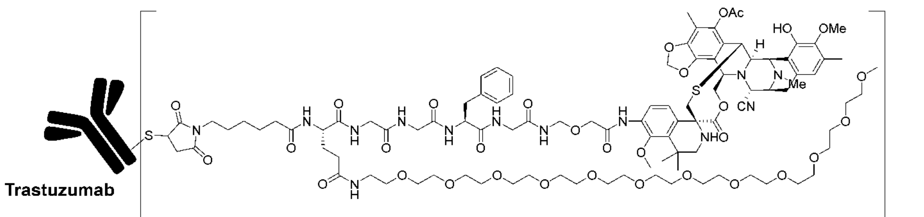

ADC-100

$n_{12}$ is any numerical value selected from 0 to 8.

According to one embodiment of the present invention, $n_{12}$ is selected from any value between 1 and 8.

Another aspect of the present invention provides the ecteinascidin derivative according to the present invention, or a pharmaceutically acceptable salt, tautomer, or stereoisomer thereof, for use in the treatment of cancer, preferably for the treatment of non-small cell lung cancer, small cell lung cancer, sarcoma, gastric cancer, ovarian cancer, colorectal cancer, breast cancer, pancreatic cancer, prostate cancer, liver cancer, kidney cancer, and hematological tumors.

Another aspect of the present invention provides an antibody-drug conjugate based on the ecteinascidin derivative according to the present invention for use in the treatment of cancer, preferably for the treatment of non-small cell lung cancer, small cell lung cancer, sarcoma, gastric cancer, ovarian cancer, colorectal cancer, breast cancer,

pancreatic cancer, prostate cancer, liver cancer, kidney cancer, and hematological tumors.

The main advantages of the present invention are as follows:

1. Some of the novel ecteinascidin derivatives provided by the present invention have strong in-vitro cytotoxicity (1-10 nM), and some have even stronger in-vitro cytotoxicity (<1 nM).

2. The antibody-drug conjugates based on the novel ecteinascidin derivatives provided by the present invention have strong in-vitro cytotoxicity (1-10 nM), and some antibody-drug conjugates have even stronger in-vitro cytotoxicity (<1 nM).

Description of the Embodiments

The following are examples of the synthesis and analysis of the compounds described in this patent (including ecteinascidin derivatives, linkers, and linker-drug conjugates) and antibody-drug conjugates. It should be noted that these examples do not limit the protection scope of the present invention.

Unless otherwise specified, all anhydrous reagents were purchased directly from suppliers and stored under nitrogen. All other purchased reagents and solvents were of high purity and were used without further purification before use.

Synthesis and characterization of ecteinascidin derivatives

Example 1: Synthesis of compound P1

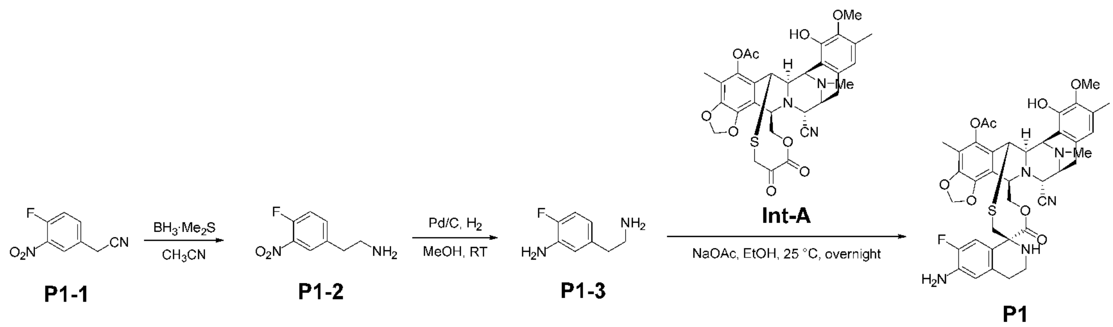


Step 1: Synthesis of 2-(4-fluoro-3-nitrophenyl)ethylenediamine

To a THF solution of 2-(4-fluoro-3-nitrophenyl)acetonitrile (100 mg, 0.55 mmol, 1.0 eq) was added borane-dimethyl sulfide complex (2.5 M, 0.69 mL, 2.5 eq), and the mixture was refluxed with stirring for 1 hour. The reaction was quenched with MeOH and concentrated. The target product was purified by thin-layer chromatography to give the desired compound (70 mg, yield: 97.8%) as a colorless oil.
LC-MS (ESI) m/z: 185.3 $[M+H]^+$

Step 2: Synthesis of 5-(2-aminoethyl)-2-fluoroaniline

Under $N_2$ protection, 20 mg of 5% wet Pd/C was added to a MeOH (20 mL) solution of 2-(4-fluoro-3-nitrophenyl) ethylenediamine (70 mg, 454 mmol, 1.0 eq). The suspension was subjected to multiple $H_2$ purges under vacuum and then stirred at room temperature under $H_2$ overnight. The suspension was filtered through diatomaceous earth, and the filter cake was washed with methanol. The combined filtrate was concentrated to dryness and used directly in the next step.

Step 3: Synthesis of P1

A mixture of intermediate Int-A (10 mg, 0.016 mmol, 1.0 eq), 5-(2-aminoethyl)-2-fluoroaniline (24.7 mg, 0.16 mmol, 10 eq), and NaOAc (6.56 mg, 0.08 mmol, 10 eq) was dissolved in anhydrous ethanol and stirred at 25°C overnight. Upon completion of the reaction, the solution was filtered, and the residue was purified by preparative high-performance liquid chromatography to afford the target product (1.5 mg, yield: 12.3%) as a white solid.

LC-MS (ESI) m/z: 758.1 $[M+H]^+$

$^1H$ NMR (400 MHz, DMSO-$d_6$) δ 8.78 (s, 1H), 6.46 (s, 1H), 6.40 (d, *J* = 13.1 Hz, 1H), 6.36 (d, *J* = 9.4 Hz, 1H), 6.25 (s, 1H), 6.16 (s, 1H), 5.06 (d, *J* = 12.1 Hz, 3H), 4.49 - 4.42 (m, 2H), 4.20 (d, *J* = 4.5 Hz, 1H), 4.08 (s, 1H), 4.00 (s, 1H), 3.64 (s, 4H), 3.22 (s, 1H), 3.08 (s, 1H), 2.79 (s, 2H), 2.35 (s, 1H), 2.30 (s, 3H), 2.23 (s, 3H), 2.05 (s, 3H).

Example 2: Synthesis of compound P5

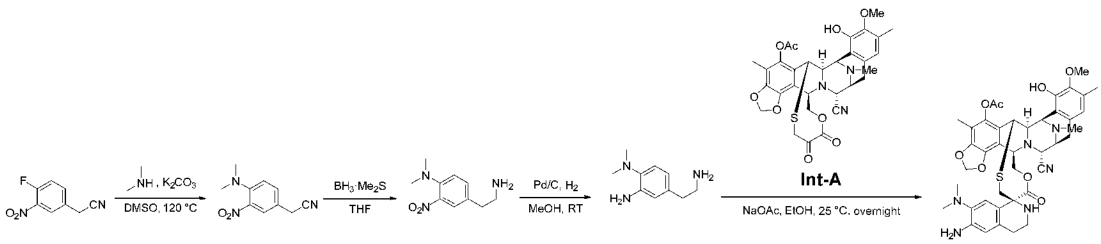


P5-1 P5-2 P5-3 P5-4 P5

Step 1: Synthesis of 2-(4-(dimethylamino)-3-nitrophenyl)acetonitrile

2-(4-fluoro-3-nitrophenyl)acetonitrile (100 mg, 0.55 mmol, 1.0 eq), dimethylamine (40%, w/w, 25 mg, 0.55 mmol, 1 eq), and $K_2CO_3$ (115.05 mg, 0.832 mmol, 1.5 eq) were added to a sealed tube containing DMSO (1.5 mL). The mixture was stirred at 120°C under argon overnight. After quenching with water, the mixture was extracted with ethyl acetate, and the organic layer was washed twice with saturated brine. The organic layer was dried over sodium sulfate, filtered, and concentrated to afford the crude product (125 mg, yield: 100%) as a brown solid, which was used directly in the next step without further purification.
LC-MS (ESI) m/z: 206.4 $[M+H]^+$

Step 2: Synthesis of 4-(2-aminoethyl)-N,N-dimino-2-nitroaniline

The crude product 2-(4-(dimethylamino)-3-nitrophenyl)acetonitrile (125 mg, 0.61 mmol, 1.0 eq) was dissolved in THF (3 mL), and borane-dimethyl sulfide complex (2.5 M, 0.76 mL, 2.5 eq) was added. The mixture was refluxed with stirring for 1 hour. After quenching with MeOH, the solution was concentrated. The residue was purified by thin-layer chromatography to afford the target compound (90 mg, yield: 70.0%) as a colorless oil.
LC-MS (ESI) m/z: 210.5 $[M+H]^+$

Step 3: Synthesis of 4-(2-aminoethyl)-N,N-dimethylenediaminobenzene

Under $N_2$ protection, 20 mg of 5% wet Pd/C was added to a MeOH (10 mL) solution of 4-(2-aminoethyl)-N,N-dimethyl-2-nitroaniline (90 mg, 430 mmol, 1.0 eq). The suspension was degassed under vacuum and subjected to multiple $H_2$ purges. The mixture was then stirred at room temperature under $H_2$ overnight. The suspension was filtered through diatomaceous earth, and the filter cake was washed with methanol. The combined filtrate was concentrated and used directly in the next step.
LC-MS (ESI) m/z: 180.2 $[M+H]^+$

Step 4: Synthesis of P5

A mixture of intermediate Int-A (10 mg, 0.016 mmol, 1.0 eq), 4-(2-aminoethyl)-N,N-dimethylenediaminobenzene (28.83 mg, 0.16 mmol, 10 eq), and NaOAc (6.56 mg, 0.08 mmol, 10 eq) in anhydrous ethanol was stirred at 25°C overnight. Upon completion of the reaction, the reaction mixture was filtered, and the filtrate was purified by preparative high-performance liquid chromatography to afford the target product (5.47 mg, yield: 43.4%) as a white solid.

LC-MS (ESI) m/z: 783.0 $[M+H]^+$

$^1H$ NMR (500 MHz, DMSO-$d_6$) δ 8.68 (s, 1H), 6.40 (d, *J* = 11.3 Hz, 2H), 6.17 (d, *J* = 8.0 Hz, 2H), 6.12 (s, 1H), 5.00 (d, *J* = 11.5 Hz, 1H), 4.61 (s, 2H), 4.45 (d, *J* = 2.9 Hz, 2H), 4.21 - 4.13 (m, 1H), 4.08 (d, *J* = 2.3 Hz, 1H), 3.94 (dd, *J* = 11.6, 2.5 Hz,

1H), 3.62 (s, 3H), 3.24 (d, *J* = 4.7 Hz, 1H), 2.97 (td, *J* = 11.5, 3.7 Hz, 1H), 2.83 - 2.61 (m, 3H), 2.43 - 2.34 (m, 2H), 2.33 (s, 6H), 2.28 (s, 3H), 2.22 (d, *J* = 3.3 Hz, 1H), 2.19 (s, 4H), 2.12 (d, *J* = 14.6 Hz, 1H), 2.03 (s, 3H), 1.96 (s, 4H).

Example 3: Synthesis of compounds P9 and P10

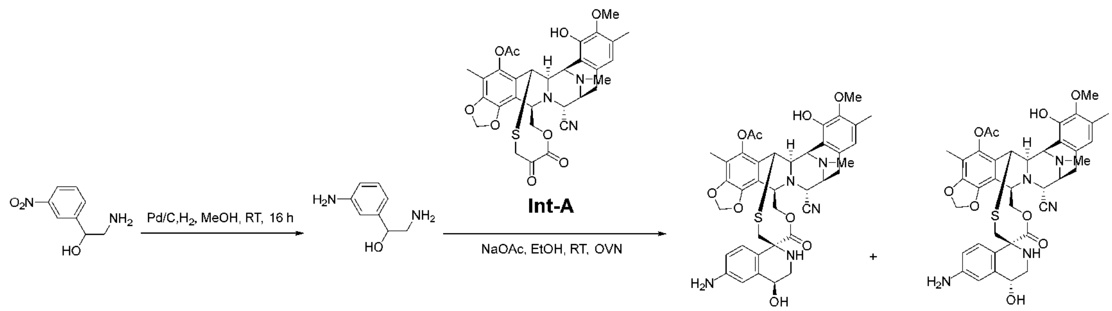


Int-A

P9-1 P9-2 P9 P10

Step 1: Synthesis of 2-amino-1-(3-aminophenyl)ethanol

Under $N_2$ protection, 5% wet Pd/C (20 mg) was added to a MeOH (50 mL) solution of 2-amino-1-(3-nitrophenyl) ethanol (200 mg, 0.0188 mmol, 1.0 eq). The suspension was degassed under vacuum and subjected to multiple $H_2$ purges. The mixture was then stirred at room temperature under $H_2$ for 16 hours. The suspension was filtered through diatomaceous earth, and the filter cake was washed with methanol. The combined filtrate was concentrated to afford the crude product (190 mg) as an oil.

LC-MS (ESI) m/z: 153.2 $[M+H]^+$.

$^1$H NMR (400 MHz, DMSO-$d_6$) δ 7.97 (s, 2H), 7.01 - 6.98 (m, 1H), 6.59 - 6.57 (m, 1H), 6.47 - 6.50 (m, 2H), 6.92 (s, 1H), 5.12 (s, 2H), 4.66 - 4.62 (m, 1H), 2.90 - 2.94 (m, 1H), 2.77 - 2.72(m, 1H).

Step 2: Synthesis of P9 and P10

Intermediate Int-A (10 mg, 0.016 mmol, 1.0 eq), 3-(2-aminoethyl)aniline (12 mg, 0.081 mmol, 5.0 eq), and NaOAc (19 mg, 0.16 mmol, 10 eq) were dissolved in anhydrous ethanol (2 mL) and stirred at room temperature overnight. The solvent was removed under reduced pressure, and the residues were purified by preparative high-performance liquid chromatography to afford P9 (1.67 mg, yield: 13.91%) and P10 (2.43 mg, yield: 20%) as white solids.

LC-MS (ESI) m/z: 756.2 $[M+H]^+$

$^1$H NMR (400 MHz, DMSO-$d_6$) δ 8.76 (s, 1H), 7.62 - 7.56 (d, *J* = 24 Hz 2H), 6.63 (s, 1H), 6.51 (d, *J* = 8 Hz, 2H), 6.46 (s, 1H), 6.25 (s, 2H), 6.15 (s, 1H), 5.05 - 4.98 (m, 7H), 4.45(s, 5H), 4.19(s, 4H), 3.96(s, 3H), 2.79(s, 4H), 2.28 - 2.23(m, 6H), 2.04 - 1.98(m, 6H).

$^1$H NMR (400 MHz, DMSO-$d_6$) δ 8.85 (s, 1H), 6.63 (d, *J* = 8 Hz, 1H), 6.51 (d, *J* = 12 Hz, 2H), 6.35 - 6.32 (m, 2H), 5.14 - 5.15 (m, 3H), 4.74 - 4.72 (m, 1H), 4.49 (s, 2H), 4.26 - 4.19 (m, 8H), 4.15 - 4.01(m, 3H), 3.17(s, 5H), 2.85 - 2.81(m, 4H), 2.35 - 2.30 (m, 10H), 2.10 - 2.04 (m, 8H).

Example 4: Synthesis of compounds P12 and P13

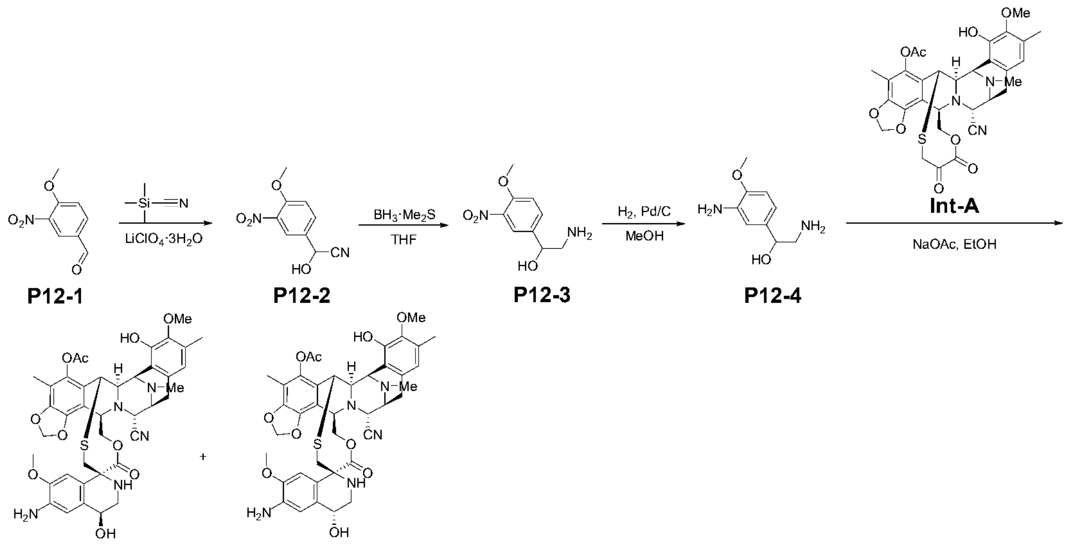


P12-1 P12-2 P12-3 P12-4

P12 P13

Step 1: Synthesis of 2-hydroxy-2-(4-methoxy-3-nitrophenyl)acetonitrile

4-methoxy-3-nitrobenzaldehyde (4.0 g, 22 mmol, 1 eq) and trimethylsilyl cyanide (8.7 g, 88 mmol, 4 eq) were added to a reaction flask and dissolved in dichloromethane (50 mL). $LiClO_4 \cdot 3H_2O$ (3.53 g, 22 mmol, 1 eq) was added dropwise, and the mixture was stirred at room temperature overnight. TLC (EA:n-heptane = 1/3) indicated completion of the reaction. The reaction mixture was filtered, and the filtrate was washed twice with 10% dilute HCl. The organic phase was dried over anhydrous sodium sulfate, filtered, and concentrated to afford the crude product (3.9 g, yield: 85%) as a yellow solid, which was used directly in the next step.

Step 2: Synthesis of 2-amino-1-(4-methoxy-3-nitrophenyl)ethanol

2-hydroxy-2-(4-methoxy-3-nitrophenyl)acetonitrile (3.9 g, 18.7 mmol, 1 eq) was dissolved in anhydrous THF (80 mL), and 1 M borane-dimethyl sulfide complex (46.7 mL, 46.75 mmol, 2.5 eq) was added slowly. The mixture was heated to 60°C and stirred until LC-MS indicated completion of the reaction. The reaction was cooled to 0°C and quenched slowly with methanol, during which the solution gradually turned purple. The solution was concentrated, and the residue was purified by rapid silica gel column chromatography (DCM/MeOH = 30/1 to 10/1) to afford the target compound (3.8 g, yield: 96%) as a pale yellow solid.

LC-MS (ESI) m/z: 195.2 $[M-OH]^+$

$^1H$ NMR (400 MHz, DMSO-$d_6$) δ 7.79 (d, $J$ = 2.2 Hz, 1H), 7.59 (dd, $J$ = 8.7, 2.2 Hz, 1H), 7.31 (d, $J$ = 8.7 Hz, 1H), 4.48 (dd, $J$ = 7.3, 4.5 Hz, 1H), 3.90 (s, 3H), 2.72 - 2.56 (m, 2H).

Step 3: Synthesis of 2-amino-1-(3-amino-4-methoxyphenyl)ethanol

To a 100 mL methanol solution of 2-amino-1-(4-methoxy-3-nitrophenyl)ethanol (3.8 g, 17.9 mmol) was added 5% wet Pd/C (200 mg). The suspension was degassed under vacuum and subjected to multiple $H_2$ purges. The mixture was stirred at room temperature under $H_2$ for 2 hours. LC-MS indicated complete conversion of the starting material. The Pd/C was removed by filtration, and the filtrate was concentrated to afford the target compound (1.76 g, yield: 54%) as a brown solid.

Step 4: Synthesis of P12 and P13

Intermediate Int-A (200 mg, 0.321 mmol, 1 eq), 2-amino-1-(3-amino-4-methoxyphenyl)ethanol (586 mg, 3.2 mmol, 10 eq), and glacial acetic acid (192 mg, 3.2 mmol, 10 eq) were added to a reaction flask, followed by 20 mL of anhydrous ethanol. The mixture was stirred at 60°C for 1 hour. LC-MS indicated completion of the reaction. The ethanol was concentrated, and the residue was adjusted to pH 7-8 with saturated sodium carbonate solution. The mixture was

extracted with ethyl acetate, and the organic layer was dried over anhydrous sodium sulfate, filtered, and concentrated. The residue was purified by rapid silica gel column chromatography (MeOH/DCM = 1/50) to afford P12 (96 mg, yield: 76%) and P13 (100 mg, yield: 79%) as brown solids.

P12

LC-MS (ESI) m/z: 786.1 $[M+H]^+$

$^1$H NMR (400 MHz, Chloroform-d) δ 6.59 (s, 1H), 6.51 (s, 1H), 6.34 (s, 1H), 6.01 (d, *J* = 25.3 Hz, 2H), 5.78 (s, 1H), 4.99 (d, *J* = 11.6 Hz, 1H), 4.57 (s, 1H), 4.36 - 4.26 (m, 2H), 4.23 - 4.14 (m, 2H), 4.11 (d, *J* = 11.7 Hz, 1H), 3.81 (s, 3H), 3.59 (s, 3H), 3.51 (d, *J* = 4.8 Hz, 1H), 3.42 (s, 1H), 3.34 (s, 1H), 2.94 (d, *J* = 5.3 Hz, 2H), 2.33 (s, 4H), 2.29 (s, 3H), 2.21 (s, 3H), 2.04 (s, 3H).

P13

LC-MS (ESI) m/z: 786.1 $[M+H]^+$

$^1$H NMR (400 MHz, Chloroform-*d*) δ 7.89 (d, *J* = 2.3 Hz, 3H), 7.54 (dd, *J* = 8.7, 2.4 Hz, 3H), 7.05 (d, *J* = 8.6 Hz, 3H), 6.64 (d, *J* = 6.0 Hz, 1H), 6.42 (s, 1H), 6.03 (ddd, *J* = 21.2, 10.1, 4.2 Hz, 4H), 5.49 (d, *J* = 17.3 Hz, 3H), 5.35 (d, *J* = 10.5 Hz, 4H), 4.70 (d, *J* = 5.0 Hz, 6H), 4.46 (s, 6H), 4.36 - 4.26 (m, 2H), 4.20 - 4.12 (m, 1H), 3.79 (s, 2H), 3.61 (s, 2H), 3.50 (d, *J* = 4.8 Hz, 1H), 2.92 (dd, *J* = 14.3, 5.2 Hz, 3H), 2.35 (s, 3H), 2.27 (s, 3H), 2.20 (s, 3H), 2.03 (s, 3H).

Example 5: Synthesis of compound P14

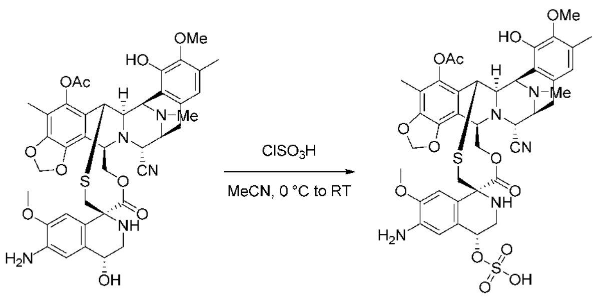


P13 P14

P13 (5.0 mg, 0.0063 mmol, 1 eq) was dissolved in 1.5 mL of anhydrous acetonitrile and cooled to 0°C. Chlorosulfonyl (9.0 mg, 0.075 mmol, 12 eq) was added, and the reaction was monitored by LC-MS until completion. The reaction mixture was slowly quenched with saturated ammonium chloride solution, and the resulting solution was purified by preparative high-performance liquid chromatography to afford the target product (0.2 mg, yield: 3.6%) as a brown solid.
LC-MS (ESI) m/z: 866.1 $[M+H]^+$

Example 6: Synthesis of compound P19

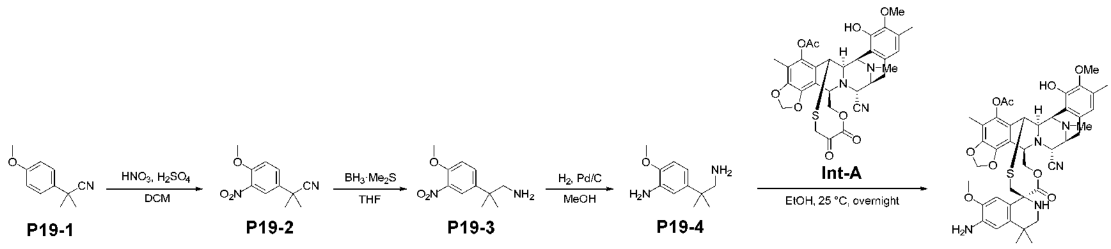


P19-1 P19-2 P19-3 P19-4 P19

Step 1: Synthesis of 2-(4-methoxy-3-nitrophenyl)-2-methylpropanenitrile

To a dichloromethane (1.5 mL) solution of 2-(4-methoxyphenyl)-2-methylpropanenitrile (100 mg, 0.57 mmol, 1.0 eq) was added a mixture of $HNO_3$ (65%, w/w, 82.9 mg, 0.86 mmol, 1.5 eq) and $H_2SO_4$ (88.8 mg, 0.86 mmol, 1.5 eq). The reaction was stirred at room temperature overnight. After completion, the pH was adjusted to 7 with saturated $NaHCO_3$ aqueous solution. The mixture was extracted with dichloromethane, and the organic layer was separated, dried over anhydrous sodium sulfate, filtered, and concentrated. The residue was purified by preparative thin-layer chromatography (EA:n-heptane = 1:2) to afford the target product (75 mg, yield: 60.1%) as a white solid.
LC-MS (ESI) m/z: 220.8 $[M+H]^+$

Step 2: Synthesis of 2-(4-methoxy-3-nitrophenyl)-2-methylpropanamine

2-(4-methoxy-3-nitrophenyl)-2-methylpropanenitrile (75 mg, 0.34 mmol, 1.0 eq) was dissolved in anhydrous THF (3 mL), and borane-dimethyl sulfide complex (2.5 M, 0.43 mL, 2.5 eq) was added. The mixture was refluxed with stirring for 1 hour. After quenching with MeOH, the solution was concentrated. The residue was purified by preparative thin-layer chromatography to afford the target compound (50 mg, yield: 65.1%) as a colorless oil.
LC-MS (ESI) m/z: 225.2 $[M+H]^+$

Step 3: Synthesis of 5-(1-amino-2-methylpropyl)-2-methoxyaniline

Under $N_2$ protection, 20 mg of 5% wet Pd/C was added to a MeOH (10 mL) solution of 2-(4-methoxy-3-nitrophenyl)-2-methylpropan-1-amine (50 mg, 223 mmol, 1.0 eq). The suspension was degassed under vacuum and subjected to multiple $H_2$ purges. The mixture was stirred at room temperature under $H_2$ overnight. The suspension was filtered through diatomaceous earth, and the filter cake was washed with methanol. The combined filtrate was concentrated and used directly in the next step.
LC-MS (ESI) m/z: 194.9 $[M+H]^+$

Step 4: Synthesis of P19

A mixture of intermediate Int-A (10 mg, 0.016 mmol, 1.0 eq) and 5-(1-amino-2-methylpropyl)-2-methoxyaniline (20 mg, 0.08 mmol, 5 eq) was dissolved in anhydrous ethanol (1 mL) and stirred at 25°C overnight. Upon completion of the reaction, the reaction mixture was filtered, and the filtrate was purified by preparative high-performance liquid chromatography to afford P19 (4.67 mg, yield: 36.3%) as a white solid.

LC-MS (ESI) m/z: 798.0 $[M+H]^+$

$^1$H NMR (500 MHz, DMSO-$d_6$) δ 8.71 (s, 1H), 6.43 (d, *J* = 1.5 Hz, 2H), 6.21 - 6.09 (m, 3H), 5.02 (d, *J* = 11.5 Hz, 1H), 4.59 (s, 2H), 4.47 (d, *J* = 2.9 Hz, 2H), 4.22 - 4.16 (m, 1H), 4.09 (s, 1H), 3.99 (dd, *J* = 11.6, 2.5 Hz, 1H), 3.63 (s, 3H), 3.46 (s, 4H), 3.26 (d, *J* = 4.8 Hz, 1H), 2.91 - 2.69 (m, 3H), 2.30 (s, 4H), 2.20 (s, 3H), 2.12 (d, *J* = 14.9 Hz, 1H), 2.04 (s, 3H), 1.97 (s, 3H), 0.99 (d, *J* = 13.0 Hz, 6H).

Example 7: Synthesis of compound P30

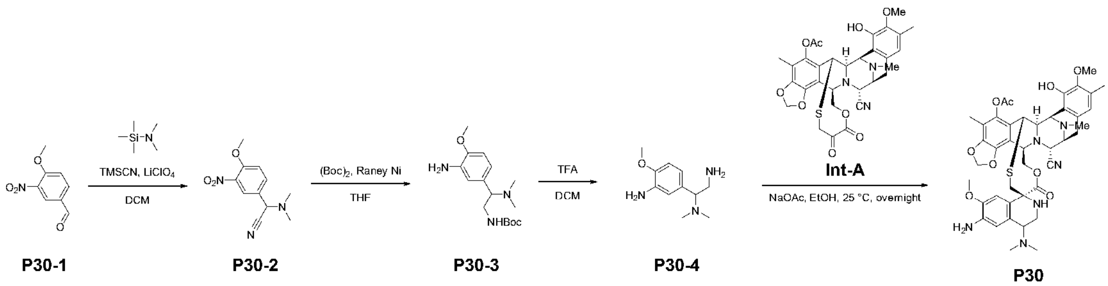


P30-1 P30-2 P30-3 P30-4 P30

Step 1: Synthesis of 2-(dimethylamino)-2-(4-methoxy-3-nitrophenyl)acetonitrile

To a dichloromethane (10 mL) solution of 4-methoxy-3-nitrobenzaldehyde (1 g, 5.52 mmol, 1.0 eq) was added $LiClO_4$ (1.17 g, 11.04 mmol, 2 eq) and stirred for 1 minute. N,N,1,1,1-pentamethylsilylamine (1.94 g, 16.56 mmol, 3 eq) was then added and stirred for 5 minutes, followed by addition of TMSCN (657 mg, 6.62 mmol, 1.2 eq) and stirring for another 5 minutes. LC-MS indicated completion of the reaction. The reaction mixture was diluted with dichloromethane (50 mL) and washed twice with saturated $NaHCO_3$ aqueous solution. The organic layer was dried over sodium sulfate, filtered, and concentrated. The resulting orange oil was purified by rapid silica gel column chromatography to afford the target compound (840 mg, yield: 65%).
LC-MS (ESI) m/z: 236 $[M+H]^+$

Step 2: Synthesis of 3-butyl (2-(3-amino-4-methoxyphenyl)-2-(dimethylamino)ethyl) carbamate

To a THF (3 mL) solution of 2-(dimethylamino)-2-(4-methoxy-3-nitrophenyl)acetonitrile (100 mg, 0.425 mmol, 1.0 eq) were added $Boc_2O$ (139 mg, 0.638 mmol, 1.5 eq) and 20 mg of Raney Ni. The mixture was stirred overnight at room temperature under a hydrogen balloon. LC-MS indicated completion of the reaction. The mixture was filtered, and the filtrate was concentrated to remove the solvent. The residue was used directly in the next step.
LC-MS (ESI) m/z: 310 $[M+H]^+$

Step 3: Synthesis of 1-(3-amino-4-methoxyphenyl)-$N^1$,$N^1$-dimethylethane-1,2-diamine TFA salt

The above 3-butyl (2-(3-amino-4-methoxyphenyl)-2-(dimethylamino)ethyl) carbamate was dissolved in DCM/TFA (3:1, v/v, 6 mL) and stirred at room temperature for 1 hour. The solvent was concentrated to afford 256 mg of crude product, which was used directly in the next step.
LC-MS (ESI) m/z: 310 $[M+H]^+$

Step 4: Synthesis of P30

Intermediate Int-A (4 mg, 0.0064 mmol, 1.0 eq), 1-(3-amino-4-methoxyphenyl)-$N^1$,$N^1$-dimethylethane-1,2-diamine TFA salt (16 mg, 0.08 mmol, 10 eq), and NaOAc (6.4 mg, 0.08 mmol, 10 eq) were dissolved in anhydrous ethanol (1 mL) and stirred at 25°C overnight. Upon completion of the reaction, the crude product was purified by preparative high-performance liquid chromatography to afford P30 (1.37 mg, yield: 26%) as a white solid.
LC-MS (ESI) m/z: 813 $[M+H]^+$

Example 8: Synthesis of compound P36

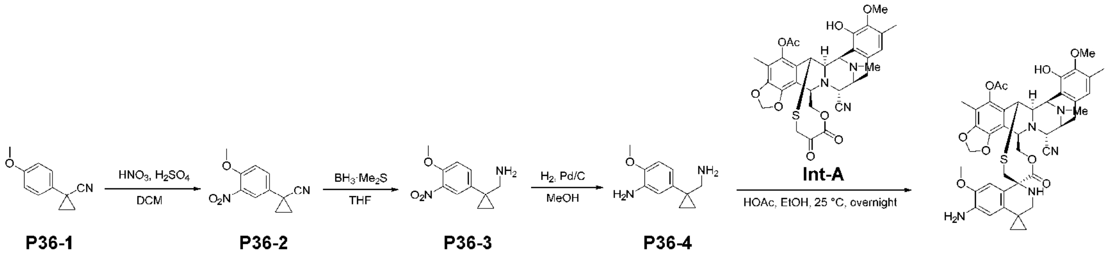


P36-1 P36-2 P36-3 P36-4 P36

Step 1: Synthesis of 1-(4-methoxy-3-nitrophenyl)cyclopropanecarbonitrile

At 0°C, a mixture of 65% $HNO_3$ (454.56 mg, 0.865 mmol) and concentrated $H_2SO_4$ (84.83 mg, 0.865 mmol) was added slowly to a dichloromethane (1 mL) solution of 1-(4-methoxyphenyl)-1'-cyclopropanecarbonitrile (100 mg, 0.577 mmol, 1.0 eq), and the mixture was stirred overnight. LC-MS indicated completion of the reaction. The pH was adjusted to 7 with saturated $NaHCO_3$ aqueous solution, and the aqueous phase was extracted with dichloromethane. The combined organic layers were dried over anhydrous sodium sulfate, filtered, and concentrated. The residue was purified by preparative thin-layer chromatography to afford the target compound (110 mg, yield: 87%) as a yellow solid.
LC-MS (ESI) m/z: 219 $[M+H]^+$

Step 2: Synthesis of (1-(4-methoxy-3-nitrophenyl)cyclopropylimethylamine

1-(4-methoxy-3-nitrophenyl)cyclopropanecarbonitrile (120 mg, 0.549 mmol, 1.0 eq) was dissolved in THF (3 mL), and 2.5 M $BH_3 \cdot Me_2S$ (0.1 mL, 2.5 eq) was added. The reaction mixture was refluxed for 1 hour, then quenched with MeOH and concentrated. The residue was purified by preparative thin-layer chromatography to afford the target product (30 mg, yield: 24.5%).
LC-MS (ESI) m/z: 223 $[M+H]^+$

Step 3: Synthesis of 5-(1-(aminomethyl)cyclopropyl)-2-methoxyaniline

Under $N_2$ protection, 10 mg of 5% wet Pd/C was added to a MeOH (2 mL) solution of (1-(4-methoxy-3-nitrophenyl)cyclopropyl)methylamine (30 mg, 0.135 mmol, 1.0 eq). The suspension was degassed and purged with hydrogen three times, then stirred at room temperature under hydrogen overnight. The Pd/C was removed by filtration, and the filtrate was concentrated to afford the crude product (37 mg) as a brown oil, which was used directly in the next step.
LC-MS (ESI) m/z: 193 $[M+H]^+$

Step 4: Synthesis of P36

Intermediate Int-A (4 mg, 0.0064 mmol, 1.0 eq), 5-(1-(aminomethyl)cyclopropyl)-2-methoxyaniline (12 mg, 0.064 mmol, 10 eq), and HOAc (3.84 mg, 0.064 mmol, 10 eq) were dissolved in anhydrous ethanol (1 mL) and stirred at 25°C overnight. Upon completion of the reaction, the crude product was purified by preparative high-performance liquid chromatography to afford P36 (3.95 mg, yield: 61%) as a white solid.
LC-MS (ESI) m/z: 796 $[M+H]^+$

Example 9: Synthesis of compound P52

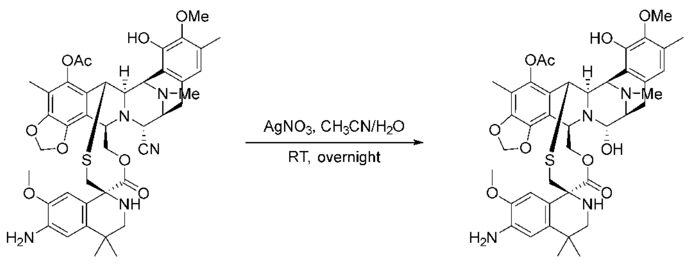


P19 P52

P19 (15 mg, 0.0188 mmol) was suspended in $CH_3CN/H_2O$ (3:2, v/v, 2 mL), and $AgNO_3$ (80 mg, 0.47 mmol) was added. The mixture was stirred at room temperature overnight. LC-MS indicated completion of the reaction. The reaction was quenched with saturated $NaHCO_3$ solution and filtered. The filtrate was extracted with ethyl acetate, dried over anhydrous sodium sulfate, filtered, and concentrated. The residue was purified by preparative high-performance liquid chromatography to afford the target compound (3.87 mg, yield: 26%) as a white solid.

LC-MS (ESI) m/z: 789.6 $[M+H]^+$

$^1H$ NMR (400 MHz, Chloroform-*d*) δ 6.63 (s, 1H), 6.50 (s, 1H), 6.35 (s, 1H), 6.04 (d, *J* = 1.3 Hz, 1H), 5.96 (d, *J* = 1.2 Hz,

1H), 5.76 (s, 1H), 5.13 (d, $J$ = 11.1 Hz, 1H), 4.82 (s, 1H), 4.49 (d, $J$ = 3.3 Hz, 1H), 4.18 (d, $J$ = 5.2 Hz, 1H), 4.03 (d, $J$ = 10.1 Hz, 1H), 3.83 (d, $J$ = 3.5 Hz, 3H), 3.68 (s, 1H), 3.62 - 3.56 (m, 5H), 3.24 (s, 1H), 3.05 (d, $J$ = 11.6 Hz, 1H), 2.89 (d, $J$ = 11.3 Hz, 2H), 2.44 (d, $J$ = 11.6 Hz, 2H), 2.36 (s, 4H), 2.32 (s, 3H), 2.24 (d, $J$ = 7.6 Hz, 1H), 2.21 (s, 3H), 2.05 (s, 3H), 1.11 (d, $J$ = 11.7 Hz, 6H).

Example 10: Synthesis of compound P58

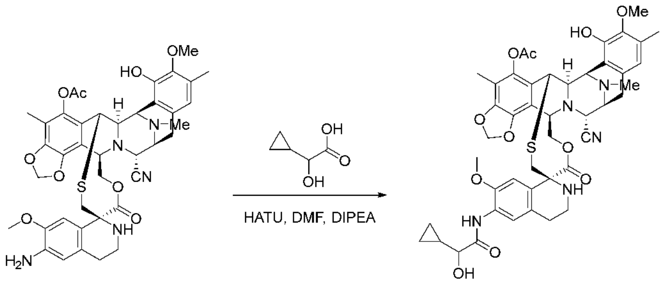


**P74-6** **P58**

To a DMF (2 mL) solution of P74-6 (see Example 12) (20 mg, 0.026 mmol, 1.0 eq) and 2-cyclopropyl-2-hydroxyacetic acid (30 mg, 0.26 mmol, 10 eq) was added HATU (99 mg, 0.26 mmol, 10 eq). After stirring the reaction mixture for 5-10 minutes, DIPEA (33.6 mg, 0.26 mmol, 10 eq) was added. The mixture was stirred at room temperature for 3 hours. The crude product was purified by preparative high-performance liquid chromatography to afford compound P58 (9.25 mg, yield: 41.1%) as a white solid.

LC-MS (ESI) m/z: 868.2 $[M+H]^+$

$^1$H NMR (400 MHz, DMSO-$d_6$) δ 8.99 (s, 1H), 8.70 (s, 1H), 7.89 (d, $J$ = 2.7 Hz, 1H), 6.43 (d, $J$ = 5.9 Hz, 2H), 6.15 (s, 2H), 6.07 (d, $J$ = 4.8 Hz, 1H), 5.04 (d, $J$ = 11.4 Hz, 1H), 4.49 (d, $J$ = 2.8 Hz, 2H), 4.20 (s, 1H), 4.11 (s, 1H), 4.06 (d, $J$ = 11.5 Hz, 1H), 3.72 (s, 1H), 3.63 (s, 3H), 3.58 (s, 3H), 3.08 - 2.94 (m, 3H), 2.86 - 2.65 (m, 3H), 2.41 - 2.32 (m, 1H), 2.29 (s, 3H), 2.21 (s, 3H), 2.19 - 2.12 (m, 1H), 2.05 (s, 3H), 1.98 (s, 3H), 1.08 (t, $J$ = 7.1 Hz, 1H), 0.40 (dd, $J$ = 8.9, 4.6 Hz, 2H), 0.32 - 0.25 (m, 2H).

Example 11: Synthesis of compound P65

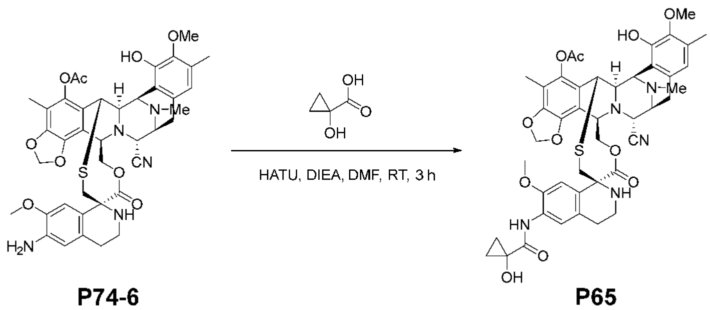


**P74-6** **P65**

P74-6 (see Example 12) (10 mg, 0.013 mmol, 1.0 eq) and 1-hydroxycyclopropane-1-carboxylic acid (13.3 mg, 0.13 mmol, 10 eq) were dissolved in DMF (2 mL), and HATU (49.5 mg, 0.13 mmol, 10 eq) was added. After stirring the reaction mixture for 5-10 minutes, DIPEA (16.8 mg, 0.13 mmol, 10 eq) was added, and the mixture was stirred at room temperature for 1 hour. The crude product was purified by preparative high-performance liquid chromatography to afford compound P65 (3.14 mg, yield: 30.7%) as a white solid.

LC-MS (ESI) m/z: 845.0 $[M+H]^+$

$^1$H NMR (400 MHz, DMSO-$d_6$) δ 9.24 (s, 1H), 8.73 (s, 1H), 7.89 (s, 1H), 6.83 (s, 1H), 6.46 (s, 2H), 6.17 (s, 2H), 5.07 (d, $J$ = 11.5 Hz, 1H), 4.51 (d, $J$ = 2.9 Hz, 2H), 4.22 (d, $J$ = 4.8 Hz, 1H), 4.16 - 4.05 (m, 2H), 3.64 (d, $J$ = 14.7 Hz, 6H), 3.30 (s,

1H), 3.19 (d, *J* = 5.2 Hz, 1H), 3.08 - 2.96 (m, 2H), 2.89 - 2.66 (m, 2H), 2.37 (d, *J* = 16.3 Hz, 1H), 2.32 (s, 3H), 2.23 (s, 3H), 2.19 (d, *J* = 14.5 Hz, 1H), 2.07 (s, 3H), 2.00 (s, 3H), 1.13 (q, *J* = 3.7 Hz, 2H), 0.97 (q, *J* = 3.8 Hz, 2H).

Example 12: Synthesis of compound P74

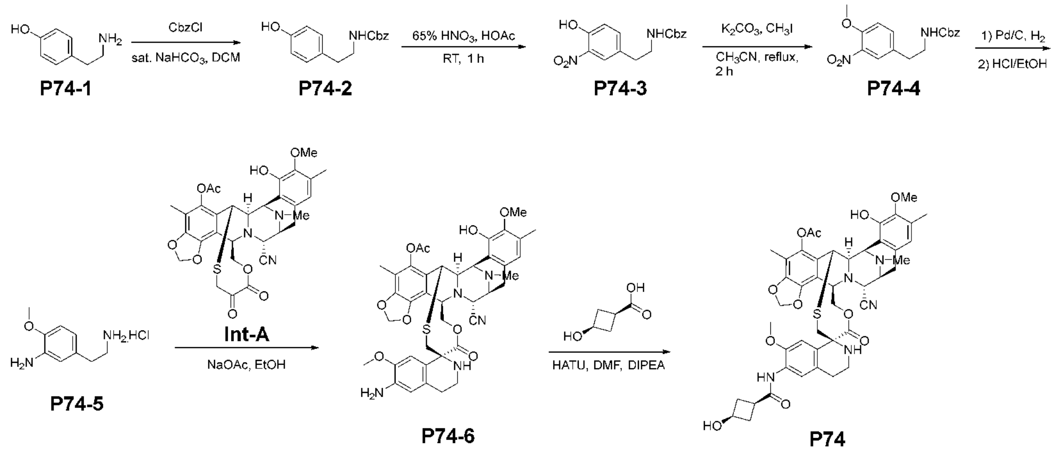


Step 1: Synthesis of P74-2

P74-1 (10 g, 0.0729 mol, 1.0 eq) was dissolved in dichloromethane (100 mL), and saturated $NaHCO_3$ solution (50 mL) was added. The mixture was cooled to 0-10°C, and Cbz-Cl (22.3 g, 0.13 mol, 1.8 eq) was added dropwise. After the addition, the mixture was allowed to warm to room temperature and stirred overnight. The layers were separated, and the aqueous layer was extracted twice with dichloromethane (100 mL each). The combined organic layers were dried over anhydrous sodium sulfate and concentrated. The residue was dissolved in ethyl acetate, and n-heptane was added to induce crystallization. The solid was filtered, and the filtrate was concentrated and recrystallized again from ethyl acetate/n-heptane. The combined solids were dried to afford compound P74-2 (16.2 g, yield: 81.8%).

LC-MS (ESI) m/z: 272.0 $[M+H]^+$

$^1$H NMR (300 MHz, Chloroform-d) δ 7.35 (s, 5H), 7.05 (d, *J* = 7.8 Hz, 2H), 6.77 (d, *J* = 7.8 Hz, 2H), 5.10 (s, 2H), 4.73 (s, 1H), 3.42 (d, *J* = 6.7 Hz, 2H), 2.74 (s, 2H).

Step 2: Synthesis of P74-3

P74-2 (14 g, 0.0516 mol, 1.0 eq) was dissolved in acetic acid (140 mL), and a mixture of acetic acid (140 mL) and 65% nitric acid (3.57 mL, 0.0516 mol, 1.0 eq) was added dropwise at room temperature. The reaction was stirred for 1 hour. The mixture was washed with ethyl acetate and saturated $NaHCO_3$ solution, and the aqueous layer was extracted with ethyl acetate. The combined organic layers were dried over anhydrous sodium sulfate, filtered, and concentrated. The crude product was purified by rapid silica gel column chromatography (eluting with EA/n-heptane = 1:4, v/v) to afford the target compound P74-3 (15.4 g, yield: 94%).

LC-MS (ESI) m/z: 317.0 $[M+H]^+$

$^1$H NMR (300 MHz, DMSO-$d_6$) δ 10.49 (s, 1H), 7.93 (s, 1H), 7.48-7.31 (m, 6H), 7.10 (d, *J* = 8.5 Hz, 1H), 5.32 (d, *J* = 5.7 Hz, 1H), 5.13 (d, *J* = 15.2 Hz, 2H), 4.83 (s, 1H), 3.45 (q, *J* = 6.8 Hz, 2H), 2.99-2.66 (m, 2H).

Steps 3 and 4: Synthesis of P74-5

P74-3 (12 g, 0.0379 mol, 1.0 eq) and potassium carbonate (15.7 g, 0.113 mol, 3.0 eq) were added to a reaction vessel, and acetonitrile (240 mL) was added with stirring to dissolve the solids. Methyl iodide (8.08 g, 0.0569 mol, 1.5 eq) was then added, and the mixture was refluxed for 2 hours. After cooling, the mixture was filtered, and the filtrate was concentrated. The residue was dissolved in dichloromethane (200 mL) and extracted with water (100 mL). The aqueous

layer was further extracted with dichloromethane, and the combined organic layers were dried over anhydrous sodium sulfate and concentrated to afford P74-4.

P74-4 was dissolved in methanol (300 mL), and 5% wet Pd/C (1.5 g) was added. The suspension was degassed under vacuum and purged with hydrogen several times, then stirred at room temperature under hydrogen overnight. The suspension was filtered through diatomaceous earth, and the filtrate was concentrated. The residue was dissolved in ethyl acetate, and 1.0 eq of 2 N HCl/EtOH solution was added to precipitate the solid. The solid was filtered to afford the target compound P74-5 (7.08 g, two-step yield: 93%).

P74-4:

$^1$H NMR (300 MHz, Chloroform-*d*) δ 7.80-7.61 (m, 1H), 7.37 (d, *J* = 9.8 Hz, 6H), 7.02 (d, *J* = 8.6 Hz, 1H), 5.10 (s, 2H), 4.97-4.77 (m, 1H), 3.95 (d, *J* = 5.5 Hz, 3H), 3.45 (q, *J* = 6.9 Hz, 2H), 2.97-2.68 (m, 2H).

P74-5:

$^1$H NMR (300 MHz, $D_2O$) δ 6.98-6.88 (m, 1H), 6.88-6.73 (m, 2H), 3.79 (d, *J* = 3.4 Hz, 3H), 3.15 (t, *J* = 7.2 Hz, 2H), 2.92-2.75 (m, 2H).

Step 5: Synthesis of P74-6

Intermediate Int-A (20 mg, 0.0322 mmol, 1.0 eq), P74-5 (65.2 mg, 0.322 mmol, 10.0 eq), and sodium acetate (29 mg, 0.354 mmol, 11 eq) were dissolved in anhydrous ethanol (1.5 mL) and stirred at room temperature overnight. TLC indicated complete consumption of the starting material. The mixture was filtered, and the inorganic salts were washed with ethyl acetate. The filtrate was concentrated, and the crude product was purified by rapid silica gel column chromatography (eluting with EA/n-heptane = 1:1, v/v) to afford the target compound P74-6 (19 mg, yield: 76.7%).

$^1$H NMR (300 MHz, Chloroform-d) δ 6.60 (s, 1H), 6.37 (s, 1H), 6.27 (s, 1H), 6.06 (s, 1H), 5.99 (s, 1H), 5.31 (s, 1H), 5.02 *(d, J* = 11.4 Hz, 1H), 4.33 (s, 1H), 4.29 (d, *J* = 4.5 Hz, 1H), 4.19 (s, 1H), 4.12 *(d, J* = 12.3 Hz, 1H), 3.82 *(d, J* = 8.3 Hz, 3H), 3.67-3.57 (m, 3H), 3.52 (d, *J* = 5.1 Hz, 1H), 3.43 (s, 1H), 3.13 (s, 1H), 2.96 (s, 2H), 2.83 (s, 2H), 2.60 (s, 3H), 2.33 (s, 3H), 2.32 (s, 1H), 2.28 (d, *J* = 2.7 Hz, 3H), 2.21 (s, 3H), 2.11-2.02 (m, 4H).

Step 6: Synthesis of P74

P74-6 (10 mg, 0.013 mmol, 1.0 eq) and (1S,3S)-3-hydroxycyclobutane-1-carboxylic acid (15 mg, 0.13 mmol, 10 eq) were dissolved in DMF (1 mL), and HATU (49.5 mg, 0.13 mmol, 10 eq) was added. After stirring the reaction mixture for 5-10 minutes, DIPEA (16.8 mg, 0.13 mmol, 10 eq) was added, and the mixture was stirred at room temperature for 1 hour. The crude product was purified by preparative high-performance liquid chromatography to afford compound P74 (6.59 mg, yield: 58.4%) as a white solid.

LC-MS (ESI) m/z: 868.2 $[M+H]^+$

$^1$H NMR (400 MHz, DMSO-$d_6$) δ 6.50 (s, 1H), 6.36 (s, 1H), 6.19 (d, *J* = 7.9 Hz, 2H), 5.13 (s, 1H), 4.85 (dt, *J* = 13.2, 6.9 Hz, 1H), 4.53 (s, 1H), 4.21 (d, *J* = 13.6 Hz, 3H), 3.95 (q, *J* = 8.3 Hz, 2H), 3.66 (s, 4H), 3.56 (s, 4H), 2.87 - 2.67 (m, 4H), 2.39 (d, *J* = 8.0 Hz, 3H), 2.32 (s, 4H), 2.29 (s, 1H), 2.25 (s, 4H), 2.20 - 2.08 (m, 3H), 2.05 (s, 4H), 1.99 (s, 4H), 1.96 (d, *J* = 9.9 Hz, 3H).

Example 13: Synthesis of compound P75

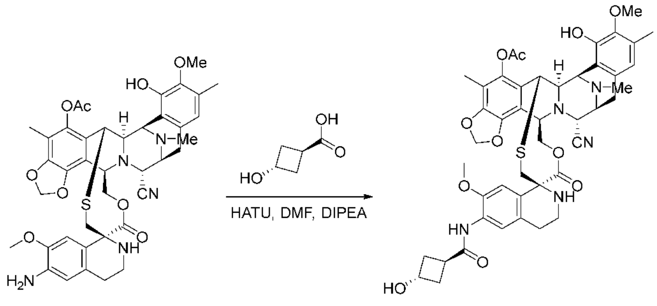


P74-6 P75

P74-6 (10 mg, 0.013 mmol, 1.0 eq) and (1R,3R)-3-hydroxycyclobutane-1-carboxylic acid (15 mg, 0.13 mmol, 10 eq) were dissolved in DMF (1 mL), and HATU (49.5 mg, 0.13 mmol, 10 eq) was added. After stirring the reaction mixture for 5-10 minutes, DIPEA (16.8 mg, 0.13 mmol, 10 eq) was added, and the mixture was stirred at room temperature for 1 hour. The crude product was purified by preparative high-performance liquid chromatography to afford compound P75 (4.25 mg, yield: 37.7%) as a white solid.

LC-MS (ESI) m/z: 868.1 $[M+H]^+$

$^1$H NMR (400 MHz, DMSO-$d_6$) δ 8.88 (s, 1H), 8.77 (d, *J* = 14.2 Hz, 2H), 7.64 (s, 1H), 6.47 (s, 1H), 6.39 (s, 1H), 6.17 (d, *J* = 5.4 Hz, 3H), 5.05 (dt, *J* = 13.4, 7.0 Hz, 3H), 4.51 (s, 2H), 4.25 (dd, *J* = 15.3, 8.2 Hz, 3H), 4.12 (d, *J* = 18.3 Hz, 2H), 3.65 (s, 4H), 3.54 (s, 4H), 3.23 - 2.67 (m, 9H), 2.41 - 2.32 (m, 4H), 2.31 (s, 4H), 2.28 (d, *J* = 3.0 Hz, 1H), 2.23 (s, 4H), 2.14 - 2.07 (m, 2H), 2.06 (s, 3H), 2.03 - 2.00 (m, 2H), 1.99 (s, 3H).

Example 14: Synthesis of compound P85

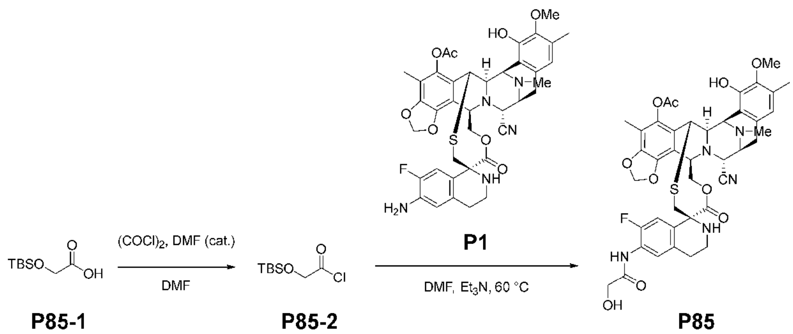


P1

P85-1 P85-2 P85

At 0°C, oxalyl chloride (0.25 mL, 0.48 mmol) and 0.1 eq catalytic DMF were added to a solution of 2-(tert-butyldimethylsilyloxy)acetic acid (200 mg, 0.96 mmol) in dichloromethane (3 mL). The reaction was monitored using $BnNH_2$, and upon completion, the resulting solution of 2-((tert-butyldimethylsilyl)oxy)acetyl chloride was used directly in the next step.

P1 (4 mg, 0.0052 mmol) was dissolved in DMF, and $Et_3N$ (2.6 mg, 0.026 mmol) was added. The mixture was heated to 60°C. The solution of 2-((tertbutyldimethylsilyl)oxy)acetyl chloride (0.1 mL) was added to the reaction mixture, and additional portions (0.1 mL each) were added until the reaction of P1 was complete. Under these conditions, the TBS group was simultaneously deprotected, directly yielding the target compound P85. The crude product was purified by preparative high-performance liquid chromatography to afford P85 (1.64 mg, yield: 38%) as a white solid.

LC-MS (ESI) m/z: 816 $[M+H]^+$

$^1$H NMR (400 MHz, DMSO-$d_6$) δ 8.80 (s, 1H), 6.48 (s, 1H), 6.26 (s, 1H), 6.17 (s, 1H), 5.88 (t, *J* = 5.8 Hz, 1H), 4.49 (d, *J* = 2.8 Hz, 1H), 3.99 (d, *J* = 5.7 Hz, 2H), 3.65 (s, 3H), 3.24 (d, *J* = 4.6 Hz, 1H), 2.81 (d, *J* = 7.3 Hz, 1H), 2.76 - 2.65 (m, 1H), 2.38 - 2.33 (m, 1H), 2.30 (s, 3H), 2.24 (s, 3H), 2.09 (s, 1H), 2.06 (s, 3H), 2.01 (s, 3H).

Example 15: Synthesis of compound P90

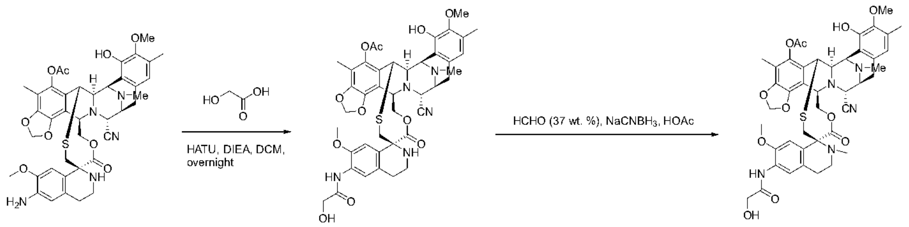


P74-6 P90-1 P90

Step 1: Synthesis of P90-1

P74-6 (20 mg, 0.026 mmol, 1.0 eq) and glycolic acid (8 mg, 0.105 mmol, 4.0 eq) were dissolved in dichloromethane (2 mL), and HATU (29.64 mg, 0.078 mmol, 3.0 eq) and DIPEA (16.77 mg, 0.13 mmol, 5 eq) were added. The reaction mixture was stirred at room temperature overnight. The crude product was purified by preparative high-performance liquid chromatography to afford P90-1 (3.3 mg, yield: 15.3%) as a white solid.

LC-MS (ESI) m/z: 828.0 $[M+H]^+$

$^1$H NMR (400 MHz, DMSO-$d_6$) $\delta$ 9.03 (s, 1H), 8.75 (s, 1H), 7.90 (s, 1H), 6.47 (s, 2H), 6.18 (s, 2H), 6.13 (t, *J* = 5.5 Hz, 1H), 5.07 (d, *J* = 11.6 Hz, 1H), 4.52 (d, *J* = 2.8 Hz, 2H), 4.22 (d, *J* = 4.8 Hz, 1H), 4.18-4.05 (m, 2H), 3.96 (d, *J* = 5.5 Hz, 2H), 3.66 (s, 3H), 3.61 (s, 3H), 3.30 (s, 1H), 3.01 (s, 1H), 2.78 (s, 2H), 2.70 (t, *J* = 1.9 Hz, 1H), 2.39-2.34 (m, 1H), 2.32 (s, 3H), 2.24 (s, 3H), 2.18 (d, *J* = 14.6 Hz, 1H), 2.07 (s, 4H), 2.00 (s, 3H).

Step 2: Synthesis of P90

P90-1 (3.77 mg, 0.0045 mmol, 1.0 eq) was dissolved in acetonitrile (0.5 mL) at room temperature. Formaldehyde (37 wt%, 200 eq) and $NaCNBH_3$ (10 eq) were added, and the mixture was stirred for 1 hour. Acetic acid (40 eq) was then added, and the reaction mixture was stirred at room temperature for 2 hours. The crude product was purified by preparative high-performance liquid chromatography to afford P90 (0.23 mg, yield: 6%) as a white solid.

LC-MS (ESI) m/z: 842.1 $[M+H]^+$

$^1$H NMR (400 MHz, DMSO-$d_6$) $\delta$ 9.03 (s, 1H), 8.76 (s, 1H), 7.84 (s, 1H), 6.35 (s, 1H), 6.25 (s, 1H), 6.22 (s, 1H), 6.13 (s, 1H), 4.94 (d, *J* = 11.4 Hz, 1H), 4.50 (d, *J* = 2.7 Hz, 1H), 4.19 (d, *J* = 13.4 Hz, 2H), 3.94 (s, 2H), 3.78 (d, *J* = 11.1 Hz, 1H), 3.66 (s, 3H), 3.52 (s, 4H), 3.25 (d, *J* = 4.7 Hz, 1H), 3.10 - 2.98 (m, 2H), 2.86 (dd, *J* = 17.6, 9.2 Hz, 1H), 2.57 (d, *J* = 18.6 Hz, 3H), 2.42 (s, 1H), 2.35 (s, 3H), 2.14 (s, 3H), 2.11 (s, 3H), 2.03 (s, 3H), 1.98 (s, 3H).

Example 16: Synthesis of compound P96

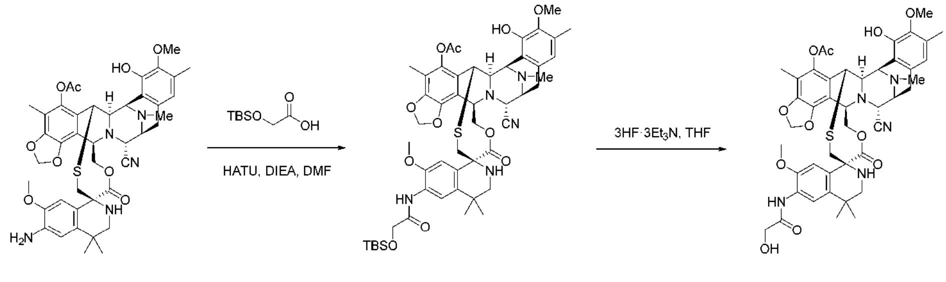


P19 P96-1 P96

Steps 1 and 2: Synthesis of P96

P19 (19 mg, 0.024 mmol) was dissolved in DMF (1 mL), and 2-((tertbutyldimethylsilyl)oxy)acetic acid (45.6 mg, 0.24 mmol), HATU (91.3 mg, 0.24 mmol), and DIPEA (31 mg, 0.24 mmol) were added. LC-MS indicated the reaction was complete. Water was added to quench the reaction, and the mixture was extracted with ethyl acetate and concentrated.
The residue was dissolved in anhydrous THF (1 mL), and $3HF\cdot 3Et_3N$ (0.9 mL) was added. The reaction was stirred at room temperature for 1 hour, and LC-MS showed completion. Saturated $NaHCO_3$ solution was added, and the mixture was extracted with dichloromethane. The combined organic layers were dried over anhydrous $Na_2SO_4$, filtered, and concentrated. The crude product was purified by preparative high-performance liquid chromatography to afford P96 (5.86 mg, yield: 28.7%) as a white solid.

LC-MS (ESI) m/z: 856.7 $[M+H]^+$

$^1H$ NMR (400 MHz, Chloroform-d) δ 8.51 (s, 1H), 8.15 (s, 1H), 6.52 (s, 1H), 6.37 (s, 1H), 5.97 (d, *J* = 1.3 Hz, 1H), 5.90 (d, *J* = 1.2 Hz, 1H), 5.78 (s, 1H), 5.23 (s, 1H), 5.07-4.94 (m, 1H), 4.92 (d, *J* = 11.4 Hz, 1H), 4.50 (s, 1H), 4.24 (s, 1H), 4.21 (dd, *J* = 4.8, 1.5 Hz, 1H), 4.11 (s, 2H), 4.08 (d, *J* = 2.8 Hz, 1H), 4.05-4.00 (m, 1H), 3.73 (s, 3H), 3.52 (s, 3H), 3.46-3.42 (m, 1H), 3.34 (s, 1H), 2.94 (d, *J* = 11.8 Hz, 1H), 2.87 (d, *J* = 5.3 Hz, 2H), 2.38 (s, 1H), 2.26 (s, 3H), 2.22 (s, 3H), 2.14 (s, 3H), 2.02 (d, *J* = 14.0 Hz, 2H), 1.96 (s, 3H), 1.06 (d, *J* = 1.6 Hz, 6H).

Example 17: Synthesis of compound P166

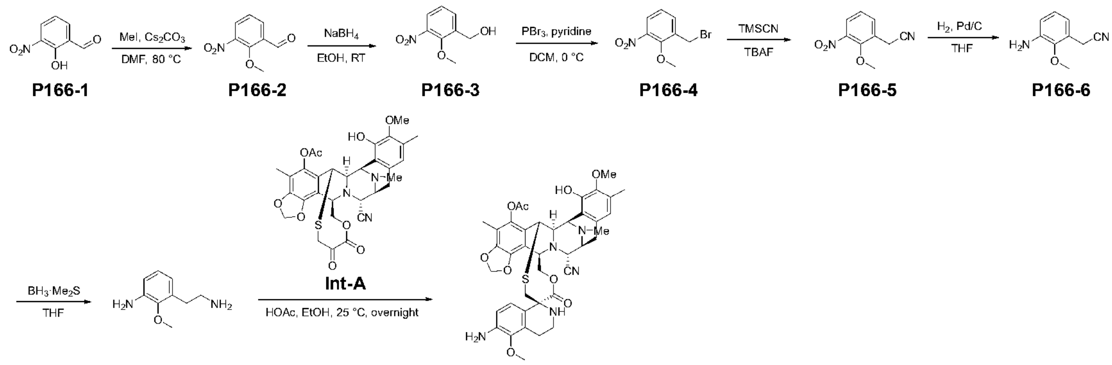


P166-1 P166-2 P166-3 P166-4 P166-5 P166-6

P166-7 P166

Step 1: Synthesis of 2-methoxy-3-nitrobenzaldehyde

2-Hydroxy-3-nitrobenzaldehyde (5 g, 30 mmol), $Cs_2CO_3$ (29 g, 89 mmol), and MeI (5.1 g, 35.9 mmol) were dissolved in DMF (40 mL). The mixture was stirred at 80°C for 1.5 hours. The reaction mixture was filtered, washed with water, and extracted with MTBE. The organic layer was dried over anhydrous $Na_2SO_4$, filtered, and concentrated to afford the target compound (4 g, yield: 73.8%) as a yellow solid.

Step 2: Synthesis of (2-methoxy-3-nitrophenyl)methanol

2-Methoxy-3-nitrobenzaldehyde (370 mg, 2.04 mmol) was dissolved in EtOH (5 mL), and $NaBH_4$ (85 mg, 2.24 mmol) was added. The reaction mixture was stirred at room temperature for 1 hour. Upon completion, the reaction was quenched with 1 N HCl and extracted with dichloromethane. The organic layer was dried over anhydrous $Na_2SO_4$, filtered, and concentrated to give the crude product (350 mg) as a solid, which was used directly in the next step.
$^1H$ NMR (400 MHz, DMSO-$d_6$) δ 7.81 (m, 2H), 7.37 (t, 1H), 5.45 (t, *J* = 5.7 Hz, 1H), 4.63 (d, *J* = 5.7 Hz, 2H), 3.85 (s, 3H).

Step 3: Synthesis of 1-(Bromomethyl)-2-methoxy-3-nitrobenzene

To a solution of (2-methoxy-3-nitrophenyl)methanol (120 mg, 0.655 mmol) in dichloromethane (3 mL) was added pyridine (37 mg), and the solution was cooled to 0°C. $PBr_3$ (266 mg, 0.982 mmol) was added gradually. Upon completion of the reaction, water was added, and the mixture was extracted with dichloromethane. The organic layer was separated,

dried over anhydrous $Na_2SO_4$, filtered, and concentrated. The residue was purified by preparative thin-layer chromatography to afford the target compound (65 mg, yield: 40%).

$^1$H NMR (400 MHz, DMSO-$d_6$) $\delta$ 7.95 (dd, $J$ = 8.2, 1.7 Hz, 1H), 7.86 (dd, $J$ = 7.7, 1.7 Hz, 1H), 7.38 (t, $J$ = 7.9 Hz, 1H), 4.78 (s, 2H), 3.93 (s, 3H).

Step 4: Synthesis of 2-(2-methoxy-3-nitrophenyl)acetonitrile

To a solution of TMSCN (31.42 mg, 0.29 mmol) in THF (2 mL) was added TBAF (1 M in THF, 0.29 mL) and stirred for 1 hour. Then, a solution of 1-(bromoethyl)-2-methoxy-3-nitrobenzene (65 mg, 0.264 mmol) in acetonitrile was added. Upon completion of the reaction, the residue after concentration was purified by preparative thin-layer chromatography to afford the target compound (25 mg, yield: 48.9%).
LC-MS (ESI) m/z: 166.3 [M-CN]$^+$

Step 5: Synthesis of 2-(3-amino-2-methoxyphenyl)acetonitrile

Under $N_2$ protection, a solution of 2-(2-methoxy-3-nitrophenyl)acetonitrile (25 mg, 0.56 mmol, 1.0 eq) in THF (3 mL) was treated with 5% wet Pd/C. The suspension was degassed under vacuum and subjected to multiple $H_2$ replacements. The reaction was stirred at room temperature under $H_2$ overnight. The suspension was filtered through diatomaceous earth, and the filter cake was washed with methanol. The combined filtrate was concentrated and used directly in the next step.
LC-MS (ESI) m/z: 163.2 [M+H]$^+$

Step 6: Synthesis of 3-(2-aminoethyl)-2-methoxyaniline

Under Ar protection, the crude 2-(3-amino-2-methoxyphenyl)acetonitrile was dissolved in anhydrous THF (1 mL). Borane-dimethyl sulfide complex (2 M, 0.5 mL) was added dropwise at 0°C, and the mixture was then heated to reflux for 1 hour. LC-MS indicated the reaction was complete. The mixture was cooled to 0°C and quenched with MeOH (1 mL), then concentrated to remove the solvent. The residue was purified by preparative thin-layer chromatography to afford the target product (10 mg).
LC-MS (ESI) m/z: 167.3 [M+H]$^+$

Step 7: Synthesis of P166

The intermediate Int-A (5 mg, 0.008 mmol), 3-(2-aminoethyl)-2-methoxyaniline (14.6 mg, 0.08 mmol), and AcOH (6.4 mg, 0.08 mmol) were dissolved in anhydrous ethanol (1 mL) and stirred at 25°C overnight. Upon completion, the solvent was removed under reduced pressure to afford a crude solid. The crude product was purified by preparative HPLC to give the target compound P166 (0.62 mg, yield: 10%) as a white solid.
LC-MS (ESI) m/z: 770.4 [M+H]$^+$

Example 18: Synthesis of the compound P170

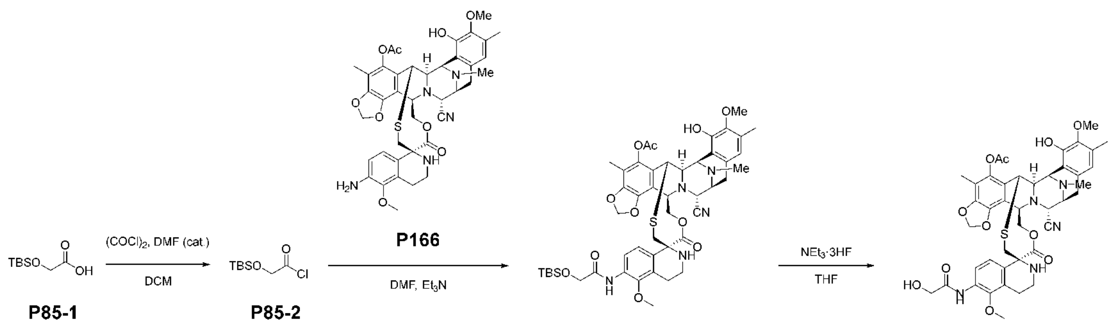

P85-1 P85-2 P166 P170-1 P170

Step 1: Synthesis of P170-1

At 0 °C, oxalyl chloride (2 M solution in dichloromethane, 0.08 mL, 0.15 mmol) was added to a solution of 2-((tert-butyldimethylsilyl)oxy)acetic acid (57 mg, 0.3 mmol) in dichloromethane (5 mL), followed by dropwise addition of two drops of catalytic DMF. After stirring at 0 °C for 1 hour, the reaction solution was added to a solution of P166 (24 mg, 0.03 mmol) and triethylamine (30.3 mg, 0.3 mmol) in anhydrous DMF (1 mL). Stirring was continued at room temperature for 2 hours. LC-MS showed that the reaction was complete. The reaction was quenched with water and extracted with ethyl acetate. The organic layer was separated, dried over anhydrous sodium sulfate, filtered, and concentrated. The crude product was purified by flash column chromatography on silica gel to obtain the target product (15 mg, yield: 53.2%) as a yellow liquid. LC-MS (ESI) m/z: $[M+H]^+$ 942

Step 2: Synthesis of P170

Triethylamine trifluoroacetate (1 mL) was added to a solution of P170-1 (14 mg, 0.014 mmol, 1 eq) in anhydrous THF (1 mL), and the mixture was stirred at room temperature for 1 hour. LC-MS showed that the reaction was complete. The reaction mixture was neutralized with a saturated aqueous $NaHCO_3$ solution and extracted with ethyl acetate. The organic phase was dried over anhydrous sodium sulfate, filtered, and concentrated, and then purified by high-performance liquid chromatography to obtain the target product (2.35 mg, yield: 19.1%) as a white solid.

LC-MS (ESI) m/z: $[M+H]^+$ 828

$^1$H NMR (400 MHz, DMSO-$d_6$) δ 9.06 (s, 1H), 8.77 (s, 1H), 7.88 (d, $J$ = 8.8 Hz, 1H), 6.73 (d, $J$ = 8.8 Hz, 1H), 6.46 (s, 1H), 6.25 (s, 1H), 6.17 (s, 1H), 6.12 (m, 1H), 5.06 (d, $J$ = 11.5 Hz, 1H), 4.45 (d, $J$ = 2.8 Hz, 3H), 4.20 (d, $J$ = 4.5 Hz, 2H), 4.08 (s, 1H), 4.03 (d, $J$ = 11.3 Hz, 2H), 3.97 (d, $J$ = 5.4 Hz, 3H), 3.64 (s, 3H), 3.61 (s, 3H), 3.23 (m, 2H), 3.08 (m, 1H), 2.80 (m, 2H), 2.75-2.56 (m, 3H), 2.41 (m, 2H), 2.28 (s, 3H), 2.24 (s, 3H), 2.08 (s, 1H), 2.05 (s, 3H), 1.98 (s, 3H).

Example 19: Synthesis of the compound P172

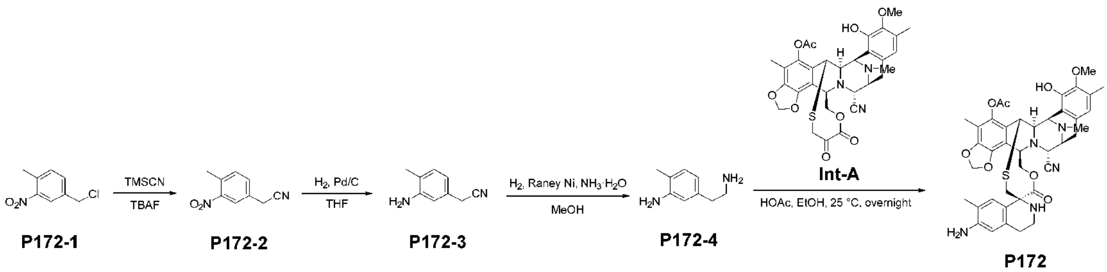


Step 1: Synthesis of 2-(4-methyl-3-nitrophenyl)acetonitrile

TBAF (1 M solution in THF, 12 mL) was added to a solution of TMSCN (1.3 g, 13 mmol) in THF (15 mL), and the mixture was stirred for 1 hour. Then, a solution of 4-(chloromethyl)-1-methyl-2-nitrobenzene (1 g, 5.4 mmol) in $CH_3CN$ (9 mL) was added. After completion of the reaction, the mixture was concentrated, and the crude product was purified by flash column chromatography on silica gel to obtain the target compound (688 mg, yield: 72.3%).

LC-MS (ESI) m/z: 177 $[M+H]^+$

$^1$H NMR (400 MHz, Chloroform-d) δ 7.98 (d, $J$ = 2.0 Hz, 1H), 7.55 (dd, $J$ = 7.9, 2.0 Hz, 1H), 7.43 (d, $J$ = 7.9 Hz, 1H), 3.86 (s, 2H), 2.65 (s, 3H).

Step 2: Synthesis of 2-(3-amino-4-methylphenyl)acetonitrile

Under $N_2$ protection, 5% wet Pd/C (40 mg) was added to a solution of 2-(4-methyl-3-nitrophenyl)acetonitrile (680 mg) in THF (10 mL). The suspension was degassed under vacuum and purged with $H_2$ several times. The mixture was stirred overnight at room temperature under $H_2$ protection. The suspension was filtered through diatomite, and the filter cake was washed with methanol. The combined filtrates were concentrated and directly used in the next step.
LC-MS (ESI) m/z: 147 $[M+H]^+$

Step 3: Synthesis of 5-(2-aminoethyl)-2-methylbenzenamine

Raney Ni (5 mg) and 0.1-0.5 eq. $NH_3 \cdot H_2O$ were added to a MeOH solution of 2-(3-amino-4-methylphenyl) acetonitrile (38 mg). The mixture was stirred at room temperature for 2 hours. After completion of the reaction, the solid was filtered off and the filtrate was concentrated. The target product (27 mg, yield: 69%) was obtained by purification through preparative thin-layer chromatography.

LC-MS (ESI) m/z: 151 $[M+H]^+$

$^1$H NMR (400 MHz, Chloroform-d) δ 7.01 (d, *J* = 7.4 Hz, 1H), 6.58 (d, *J* = 8.2 Hz, 2H), 2.98 (t, *J* = 6.8 Hz, 2H), 2.69 (t, *J* = 6.8 Hz, 2H), 2.18 (s, 3H).

Step 4: Synthesis of P172

Intermediate Int-A (4 mg, 0.0065 mmol), 5-(2-aminoethyl)-2-methylbenzenamine (9.8 mg, 0.065 mmol), and HOAc (3.9 mg, 0.065 mmol) were dissolved in anhydrous ethanol (1 mL) and stirred at 25 °C overnight. After completion of the reaction, the solvent was removed under reduced pressure to obtain a crude solid. The crude product was purified by preparative high-performance liquid chromatography to obtain the target product P172 (3.32 mg, yield: 68%) as a white solid.

LC-MS (ESI) m/z: 754 $[M+H]^+$

$^1$H NMR (400 MHz, DMSO-$d_6$) δ 6.45 (s, 1H), 6.41 (s, 1H), 6.24 - 6.15 (m, 3H), 5.03 (d, *J* = 11.6 Hz, 1H), 4.71 (s, 2H), 4.47 (d, *J* = 2.8 Hz, 1H), 4.20 (d, *J* = 4.7 Hz, 1H), 4.08 (s, 1H), 4.01 (d, *J* = 12.2 Hz, 1H), 3.65 (s, 4H), 3.24 (d, *J* = 4.8 Hz, 2H), 3.08 - 3.00 (m, 1H), 2.79 (d, *J* = 7.5 Hz, 2H), 2.60 (s, 1H), 2.47 - 2.33 (m, 1H), 2.31 (s, 4H), 2.27 (dt, *J* = 4.8, 2.1 Hz, 1H), 2.23 (s, 3H), 2.05 (s, 3H), 2.01 (s, 4H), 1.86 (s, 3H).

Example 20: Synthesis of the compound P241

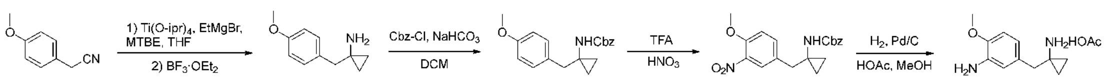


P241-1 P241-2 P241-3 P241-4 P241-5

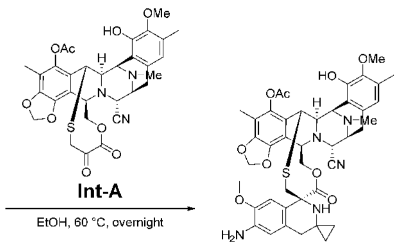


Int-A

P241

Step 1: Synthesis of 1-(4-methoxybenzyl)cyclopropan-1-amine

At 0 °C, $Ti(O\text{-}iPr)_4$ (15.46 g, 54.39 mmol, 2 eq) and EtMgBr (7.2 g, 54.39 mmol, 2 eq) were sequentially added to a solution of 2-(4-methoxyphenyl)acetonitrile (4 g, 27.19 mmol, 1 eq) in THF (50 mL) and MTBE (50 mL), and the mixture was maintained for 1 hour. Then, $BF_3 \cdot OEt_2$ (7.6 g, 54.39 mmol, 2 eq) was added to the reaction mixture and stirred for 1 hour. After completion of the reaction, the mixture was quenched with 2 M NaOH and filtered. The mixture was extracted with ethyl acetate (100 mL), and the organic phase was separated, dried over anhydrous sodium sulfate, filtered, and concentrated. The residue was purified by flash column chromatography on silica gel to obtain the target product (2.7 g, yield: 56.5%) as a white solid.

$^1$H NMR (400 MHz, DMSO-$d_6$) δ 7.05 - 6.96 (m, 2H), 6.73 - 6.65 (m, 2H), 3.63 (s, 3H), 2.50 (s, 2H), 0.51 - 0.34 (m, 4H).

Step 2: Synthesis of benzyl (1-(4-methoxybenzyl)cyclopropyl)carbamate

Cbz-Cl (4.6 mg, 27.41 mmol, 1.8 eq) was slowly added to a mixture of 1-(4-methoxybenzyl)cyclopropan-1-amine (2.7 g, 15.23 mmol, 1 eq) in dichloromethane (30 mL) and saturated aqueous $NaHCO_3$. The mixture was stirred at room temperature overnight. The reaction mixture was extracted with dichloromethane, and the organic phase was dried over anhydrous sodium sulfate, filtered, and concentrated. The residue was purified by flash column chromatography on silica gel to obtain the target product (3 g, yield: 63.8%) as a white solid.
LC-MS (ESI) m/z: 312 $[M+H]^+$

Step 3: Synthesis of benzyl (1-(4-methoxy-3-nitrobenzyl)cyclopropyl)carbamate

At 0 °C, 65% $HNO_3$ (467 mg, 4.81 mmol, 1.5 eq) was added to a solution of benzyl (1-(4-methoxybenzyl)cyclopropyl)carbamate (1 g, 3.21 mmol, 1 eq) in TFA (10 mL), and the mixture was stirred for 2 hours. After completion of the reaction, the mixture was quenched with saturated aqueous $NaHCO_3$, and the solvent was removed under reduced pressure. The pH was adjusted to 7-8. The mixture was extracted with dichloromethane, and the organic phase was dried over anhydrous sodium sulfate, filtered, and concentrated. The residue was purified by flash column chromatography on silica gel to obtain the target compound (250 mg, yield: 21%) as a yellow solid.
LC-MS (ESI) m/z: 357 $[M+H]^+$

Step 4: Synthesis of 5-((1-aminocyclopropyl)methyl)-2-methoxyaniline

Under $N_2$ protection, HOAc (57.3 mg, 0.954 mmol, 2 eq) and 50 mg of 10% wet Pd/C were added to a methanol (3 mL) solution of (1-(4-methoxy-3-nitrobenzyl)cyclopropyl)carbamate (170 mg, 0.477 mmol). The suspension was degassed under vacuum and purged with $H_2$ several times. The mixture was stirred overnight at room temperature under $H_2$ protection. The suspension was filtered through diatomite, and the filter cake was washed with methanol. The combined filtrates were concentrated and directly used in the next step.
LC-MS (ESI) m/z: 193 $[M+H]^+$

Step 5: Synthesis of P241

Intermediate Int-A (10 mg, 0.016 mmol, 1 eq) and 5-((1-aminocyclopropyl)methyl)-2-methoxyaniline (5.19 mg, 0.16 mmol, 10 eq) were dissolved in anhydrous ethanol (2 mL) and stirred at 60 °C for 1 hour. After completion of the reaction, the solvent was removed under reduced pressure to obtain a solid. The crude product was purified by preparative high-performance liquid chromatography to obtain the target compound P241 (1.87 mg, yield: 11%) as a white solid.
LC-MS (ESI) m/z: 796 $[M+H]^+$

Example 21: Synthesis of the compounds P261 and P262

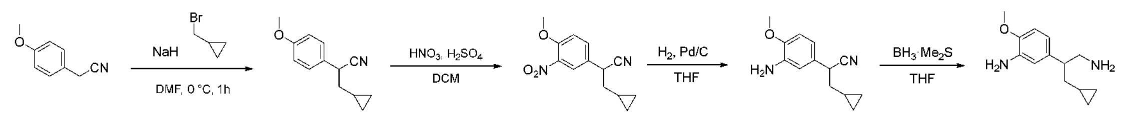


**P261-1** **P261-2** **P261-3** **P261-4** **P261-5**

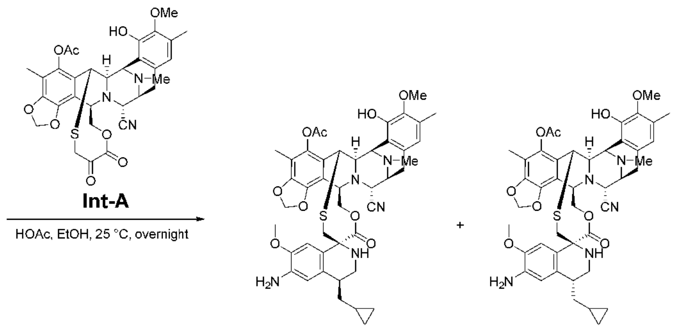


Int-A

**P261** **P262**

Step 1: Synthesis of 3-cyclopropyl-2-(4-methoxyphenyl)propanenitrile

2-(4-methoxyphenyl)acetonitrile (1.0 g, 6.8 mmol) was dissolved in DMF (10 mL), and NaH (60% w/w, 300 mg, 7.48 mmol) was added at 0 °C. The mixture was stirred for 1 hour. Then, (bromoethyl)cyclopropane (918 mg, 6.8 mmol) was added at 0 °C. After completion of the reaction, the mixture was poured into a saturated aqueous $NH_4Cl$ solution and extracted with MTBE. The organic phase was dried over sodium sulfate, filtered, and concentrated. The crude product was purified by flash column chromatography on silica gel to obtain the target compound (1.2 g, yield: 87.7%) as a solid.
LC-MS (ESI) m/z: 202 $[M+H]^+$

Step 2: Synthesis of 3-cyclopropyl-2-(4-methoxy-3-nitrophenyl)propanenitrile

A mixture of conc. $HNO_3$ (65%, 2.6 g, 26.8 mmol) and conc. $H_2SO_4$ (2.77 g, 26.8 mmol) in 20 mL was added to a solution of 3-cyclopropyl-2-(4-methoxyphenyl)propanenitrile (1.2 g, 5.96 mmol) in dichloromethane (20 mL). After completion of the reaction, the pH was adjusted to 7-8 with saturated aqueous $NaHCO_3$. The mixture was extracted with dichloromethane, and the organic phase was separated, dried over anhydrous sodium sulfate, filtered, and concentrated. The crude product was purified by flash column chromatography on silica gel to obtain the target compound (400 mg, yield: 27%).
LC-MS (ESI) m/z: 247 $[M+H]^+$

Step 3: Synthesis of 2-(3-amino-4-methoxyphenyl)-3-cyclopropylpropanenitrile

Under $N_2$ protection, 5% wet Pd/C was added to a THF (3 mL) solution of 3-cyclopropyl-2-(4-methoxy-3-nitrophenyl)propanenitrile (110 mg, 0.45 mmol). The suspension was degassed under vacuum and purged with $H_2$ several times. The mixture was stirred overnight at room temperature under $H_2$ protection. The suspension was filtered through diatomite, and the filter cake was washed with methanol. The combined filtrates were concentrated and directly used in the next step.
LC-MS (ESI) m/z: 217 $[M+H]^+$

Step 4: Synthesis of 5-(1-amino-3-cyclopropylpropan-2-yl)-2-methoxyaniline

Under Ar protection at 0 °C, a solution of the crude 2-(3-amino-4-methoxyphenyl)-3-cyclopropylpropanenitrile in anhydrous THF was treated dropwise with borane-dimethyl sulfide complex (2 M, 0.5 mL). The reaction mixture was then heated to reflux for 1 hour. LC-MS indicated completion of the reaction, and the mixture was cooled to 0 °C. The reaction was quenched with 1 mL MeOH, and the solvent was removed under reduced pressure. The residue was purified by preparative thin-layer chromatography to obtain the target product (10 mg, two-step yield: 10%).

Step 5: Synthesis of P261 and P262

Intermediate Int-A (5 mg, 0.008 mmol), 5-(1-amino-3-cyclopropylpropan-2-yl)-2-methoxyaniline (10 mg, 0.04 mmol), and HOAc (4 mg, 0.064 mmol) were dissolved in anhydrous ethanol (1 mL) and stirred at 25 °C overnight. After completion of the reaction, the solvent was removed under reduced pressure to obtain a solid. The crude product was purified by preparative high-performance liquid chromatography to obtain P261 (0.58 mg, yield: 17%) and P262 (1 mg, yield: 30%).
LC-MS (ESI) m/z: 824 $[M+H]^+$

Example 22: Synthesis of the compound T1

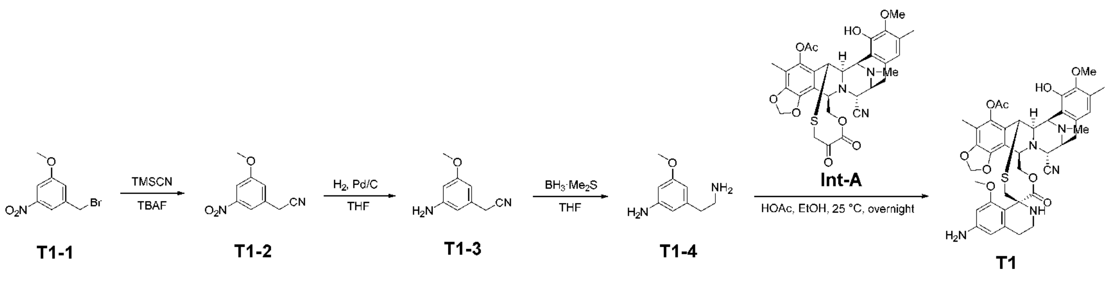

Step 1: Synthesis of 2-(3-methoxy-5-nitrophenyl)acetonitrile

TBAF (1 M solution in THF, 0.9 mL) was added to a solution of TMSCN (97 mg, 0.97 mmol) in THF (5 mL), and the mixture was stirred for 1 hour. Then, a solution of 1-(bromoethyl)-3-methoxy-5-nitrobenzene (200 mg, 0.81 mmol) in acetonitrile was added. After completion of the reaction, the mixture was concentrated to obtain the crude product, which was purified by preparative thin-layer chromatography to afford the target compound (50 mg, yield: 31.7%).
LC-MS (ESI) m/z: 193 $[M+H]^+$

Step 2: Synthesis of 2-(3-amino-5-methoxyphenyl)acetonitrile

Under $N_2$ protection, 10 mg of 5% wet Pd/C was added to a THF (3 mL) solution of 2-(3-methoxy-5-nitrophenyl) acetonitrile (50 mg). The suspension was degassed under vacuum and purged with $H_2$ several times. The mixture was stirred overnight at room temperature under $H_2$ protection. The suspension was filtered through diatomite, and the filter cake was washed with methanol. The combined filtrates were concentrated and directly used in the next step.
LC-MS (ESI) m/z: 163 $[M+H]^+$

Step 3: Synthesis of 3-(2-aminoethyl)-5-methoxyaniline

Under Ar protection at 0 °C, a solution of the crude 2-(3-amino-5-methoxyphenyl)acetonitrile (40 mg, 0.25 mmol) in anhydrous THF was treated dropwise with borane-dimethyl sulfide complex (2 M, 0.6 mL). The reaction mixture was then heated to reflux for 1 hour. LC-MS indicated completion of the reaction, and the mixture was cooled to 0 °C. The reaction was quenched with 1 mL MeOH, and the solvent was removed under reduced pressure. The residue was purified by preparative thin-layer chromatography to obtain the target compound (7 mg, two-step yield: 16%).
LC-MS (ESI) m/z: 167 $[M+H]^+$

Step 4: Synthesis of T1

Intermediate Int-A (5 mg, 0.008 mmol), 3-(2-aminoethyl)-5-methoxyaniline (5 mg, 0.032 mmol), and HOAc (4 mg, 0.064 mmol) were dissolved in anhydrous ethanol (1 mL) and stirred at 25 °C overnight. After completion of the reaction, the solvent was removed under reduced pressure to obtain a crude solid. The crude product was purified by preparative high-performance liquid chromatography to afford the target compound T1 (3.35 mg, yield: 54%) as a white solid.
LC-MS (ESI) m/z: 770 $[M+H]^+$

Example 23: Synthesis of the compound T2

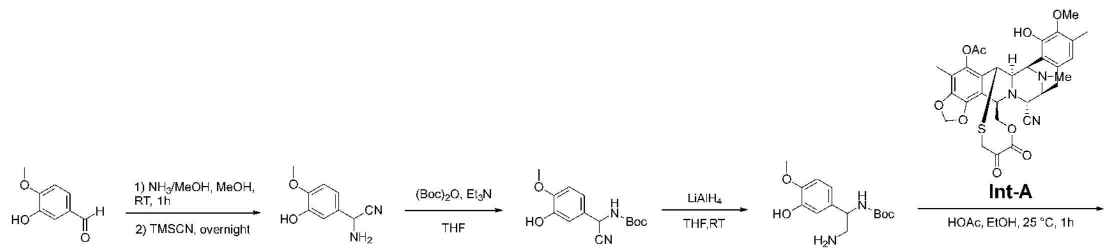


T2-1 T2-2 T2-3 T2-4

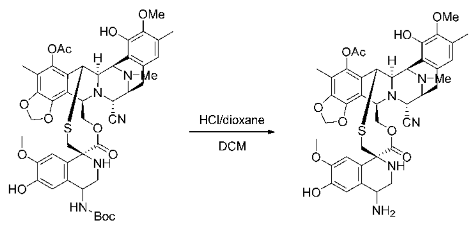


T2-5 T2

Step 1: Synthesis of 2-amino-2-(3-hydroxy-4-methoxyphenyl)acetonitrile

$NH_3$/MeOH (30% w/w, 50 mL) was added to a methanol (50 mL) solution of 3-hydroxy-4-methoxybenzaldehyde (10 g, 65.72 mmol, 1 eq), and the resulting solution was stirred at room temperature for 1 hour. Trimethylsilyl cyanide (20 mL) was then added, and the mixture was stirred overnight. After completion of the reaction, the mixture was quenched with ice water, and the solvent was removed under reduced pressure. The residue was extracted with ethyl acetate, and the organic phase was dried over anhydrous sodium sulfate, filtered, and concentrated. The crude product was purified by flash column chromatography on silica gel to afford the target compound (880 mg, yield: 7.5%) as a white solid.

LC-MS (ESI) m/z: 179 $[M+H]^+$

$^1$H NMR (400 MHz, DMSO-$d_6$) δ9.12(brs, 1H), 6.98 - 6.91 (m, 2H), 6.88 (m, 1H), 4.88 (s, 1H), 3.78 (s, 3H).

Step 2: Synthesis of tert-butyl (cyano(3-hydroxy-4-methoxyphenyl)methyl)carbamate

$Boc_2O$ (836.2 mg, 3.83 mmol, 1.1 eq) and $Et_3N$ (702.9 mg, 6.96 mmol, 2.0 eq) were added to a THF (10 mL) solution of 2-amino-2-(3-hydroxy-4-methoxyphenyl)acetonitrile (620 mg, 3.48 mmol, 1.0 eq). The mixture was stirred at room temperature overnight. After completion of the reaction, the solvent was removed under reduced pressure, and the residue was extracted with ethyl acetate (50 mL) and water (50 mL). The organic phase was dried over anhydrous sodium sulfate, filtered, concentrated, and purified by flash column chromatography on silica gel to afford the target compound (500 mg, yield: 38%) as a white solid.

Step 3: Synthesis of tert-butyl (2-amino-1-(3-hydroxy-4-methoxyphenyl)ethyl)carbamate

Tert-butyl (cyano(3-hydroxy-4-methoxyphenyl)methyl)carbamate (50 mg, 0.179 mmol, 1.0 eq) was dissolved in anhydrous THF (2 mL). $LiAlH_4$ (2 M, 1.1 eq) was added, and the mixture was stirred at room temperature for 2 hours. After completion of the reaction, the mixture was quenched with $Na_2SO_4 \cdot 10H_2O$, filtered, and the filtrate was concentrated and used directly in the next step.
LC-MS (ESI) m/z: 283.4 $[M+H]^+$

Step 4: Synthesis of T2-5

Intermediate Int-A (10 mg, 0.016 mmol, 1.0 eq), tert-butyl (2-amino-1-(3-hydroxy-4-methoxyphenyl)ethyl) carbamate (44.9 mg, 0.16 mmol, 10 eq), and HOAc (9.54 mg, 0.16 mmol, 10 eq) were dissolved in anhydrous EtOH (2 mL) and stirred at room temperature for 1 hour. After completion of the reaction, the solvent was removed under reduced pressure to obtain a crude solid. The crude product was purified by preparative high-performance liquid chromatography to afford the target compound (11 mg, yield: 77%) as a white solid.
LC-MS (ESI) m/z: 886.3 $[M+H]^+$

Step 5: Synthesis of T2

HCl/1,4-dioxane (4 M, 2 mL) was added to a dichloromethane (2 mL) solution of T2-5 (11 mg). The mixture was stirred at room temperature overnight. After completion of the reaction, the product was purified by preparative high-performance liquid chromatography to obtain the target compound T2 (2.37 mg, yield: 26.9%) as a white solid.
LC-MS (ESI) m/z: 786.9 $[M+H]^+$

Example 24: Synthesis of the compound T3

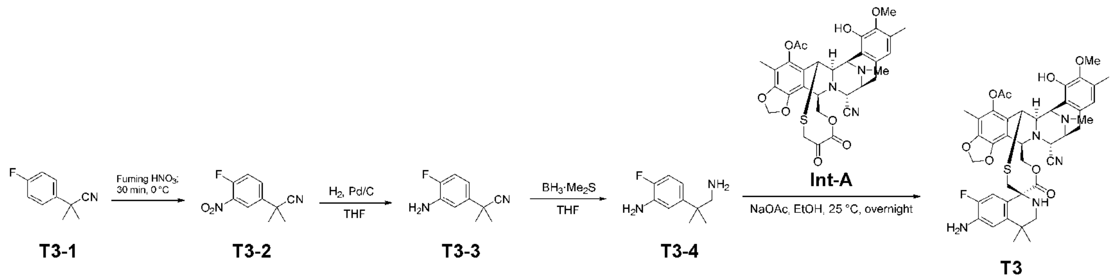


T3-1 T3-2 T3-3 T3-4 T3

Step 1: Synthesis of 2-(4-fluoro-3-nitrophenyl)-2-methylpropanenitrile

At 0 °C, fuming nitric acid (2 mL) was added dropwise to a roundbottom flask containing 2-(4-fluorophenyl)-2-methylpropanenitrile (500 mg, 3.0 mmol). The mixture was stirred at 0 °C for an additional 30 minutes. The reaction mixture was poured into ice and extracted with ethyl acetate. The combined organic layers were washed with saturated brine, dried over $MgSO_4$, filtered, and concentrated. The crude product was purified by flash column chromatography on silica gel (eluted with DCM/MeOH from 100/0 to 96/4) to afford the target product (100 mg, yield: 16%).

Step 2: Synthesis of 2-(3-amino-4-fluorophenyl)-2-methylpropanenitrile

Under $N_2$ protection, 2-(4-fluoro-3-nitrophenyl)-2-methylpropanenitrile (100 mg, 0.56 mmol) was dissolved in THF (3 mL), and 20 mg of 5% wet Pd/C was added. The suspension was degassed under vacuum and purged with $H_2$ several times. The mixture was stirred overnight at room temperature under $H_2$ protection. The suspension was filtered through diatomite, and the filter cake was washed with methanol. The combined filtrates were concentrated and used directly in the next step.
LC-MS (ESI) m/z: 179.2 $[M+H]^+$

Step 3: Synthesis of 5-(1-amino-2-methylpropan-2-yl)-2-fluoroaniline

Under Ar protection at 0 °C, a solution of the crude 2-(3-amino-4-fluorophenyl)-2-methylpropanenitrile in anhydrous THF (3 mL) was treated dropwise with borane-dimethyl sulfide complex (2 M, 0.5 mL). The reaction mixture was then heated to reflux for 1 hour. LC-MS indicated completion of the reaction, and the mixture was cooled to 0 °C. The reaction was quenched with MeOH (1 mL), and the solvent was removed under reduced pressure. The residue was purified by preparative thin-layer chromatography to afford the target product (10 mg, two-step yield: 11%).

LC-MS (ESI) m/z: 165.9 $[M+H]^+$

$^1$H NMR (400 MHz, Chloroform-d) $\delta$ 6.95 (m, 1H), 6.78 (m, 1H), 6.68 (m, 1H), 3.73 (s, 2H), 2.77 (s, 2H), 1.28 (s, 6H).

Step 4: Synthesis of T3

Intermediate Int-A (5 mg, 0.008 mmol), 5-(1-amino-2-methylpropan-2-yl)-2-fluoroaniline (14.6 mg, 0.08 mmol), and NaOAc (6.4 mg, 0.08 mmol) were dissolved in anhydrous ethanol (1 mL) and stirred at 25 °C overnight. After completion of the reaction, the solvent was removed under reduced pressure to obtain a crude solid. The crude product was purified by preparative high-performance liquid chromatography to afford the target compound (1.69 mg, yield: 26.7%) as a white solid.

LC-MS (ESI) m/z: 786.3 $[M+H]^+$

$^1$H NMR (400 MHz, DMSO-$d_6$) $\delta$ 8.80 (s, 1H), 6.62 (d, $J$ = 9.7 Hz, 1H), 6.47 (s, 1H), 6.36 (d, $J$ = 13.1 Hz, 1H), 6.26 (s, 1H), 6.16 (s, 1H), 5.05 (s, 1H), 5.03 (s, 2H), 4.47 (m, 2H), 4.20 (d, $J$ = 4.5 Hz, 1H), 4.08 (s, 1H), 3.99 (d, $J$ = 11.2 Hz, 2H), 3.64 (s, 4H), 3.24 - 3.20 (m, 1H), 3.05 (m, 1H), 2.81 (m, 2H), 2.35 (m, 1H), 2.32 (s, 3H), 2.30 - 2.25 (m, 2H), 2.23 (s, 3H), 2.06 (s, 3H), 2.01 (s, 3H).

Example 25: Synthesis of the compound T4

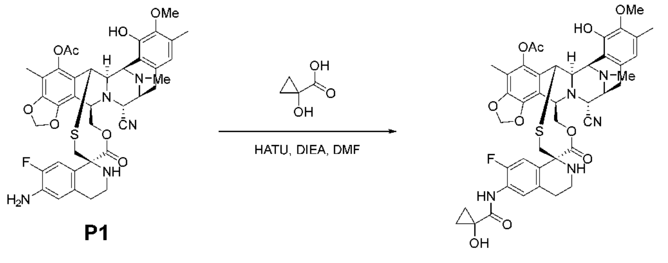


P1

T4

A solution of P1 (5 mg, 0.0066 mmol) and 1-hydroxycyclopropane-1-carboxylic acid (6.74 mg, 0.066 mmol) in DMF (2 mL) was treated with HATU (25.1 mg, 0.066 mmol). After stirring the reaction mixture for 5-10 minutes, DIPEA (8.58 mg, 0.066 mmol) was added. The mixture was stirred at room temperature for 1 hour. The reaction mixture was purified by preparative high-performance liquid chromatography to afford the compound T4 (1.72 mg, yield: 30.9%) as a white solid. LC-MS (ESI) m/z: 842 $[M+H]^+$

Example 26: Synthesis of the compound T5

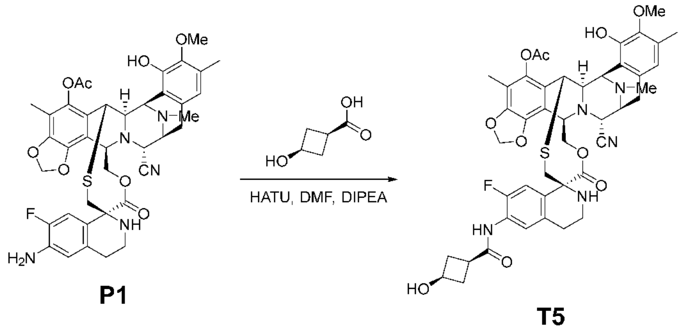


P1

T5

A solution of P1 (4 mg, 0.0053 mmol) and (1S,3S)-3-hydroxycyclobutane-1-carboxylic acid (6 mg, 0.053 mmol) in DMF (2 mL) was treated with HATU (19.8 mg, 0.053 mmol). After stirring the reaction mixture for 5-10 minutes, DIPEA (6.8 mg, 0.053 mmol) was added. The mixture was stirred at room temperature for 1 hour, and the reaction mixture was purified by preparative high-performance liquid chromatography to afford the target compound T5 (1.69 mg, yield: 36.9%) as a white solid.
(ESI) m/z: 856.4 $[M+H]^+$

Example 27: Synthesis of the compound T6

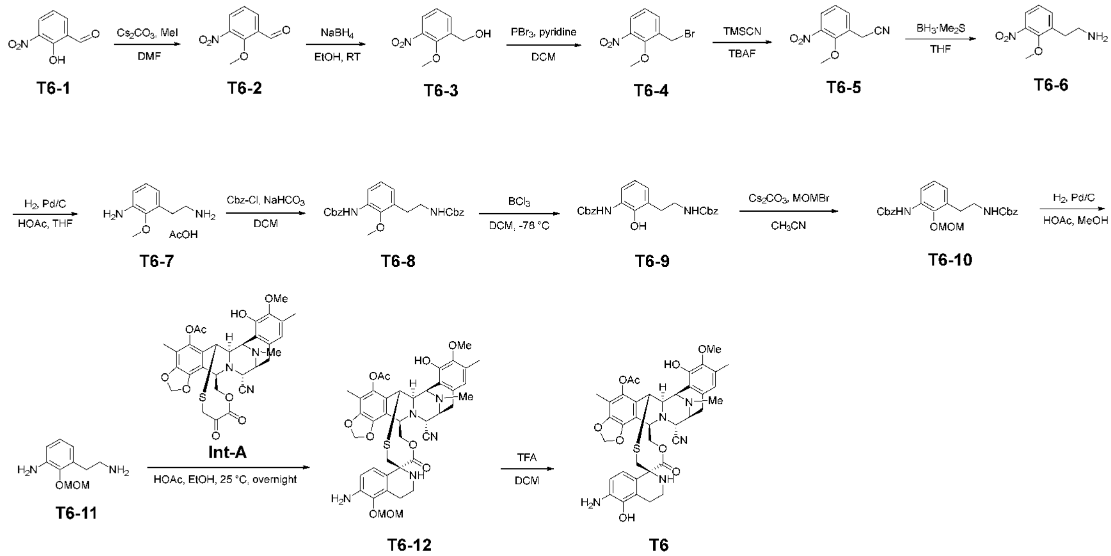


T6-1 T6-2 T6-3 T6-4 T6-5 T6-6 T6-7 T6-8 T6-9 T6-10 Int-A T6-11 T6-12 T6

Step 1: Synthesis of 2-methoxy-3-nitrobenzaldehyde

$Cs_2CO_3$ (29.24 g, 90 mmol, 3 eq) and $CH_3I$ (5.1 g, 36 mmol, 1.2 eq) were added to a solution of 2-hydroxy-3-nitrobenzaldehyde (5 g, 30 mmol, 1.0 eq) in anhydrous DMF (40 mL), and the mixture was stirred at 80°C for 1.5 hours. After completion of the reaction, the solid was filtered off. The filtrate was diluted with water and extracted with MTBE. The organic phase was dried over anhydrous sodium sulfate, filtered, and concentrated to afford the target product (4.02 g, yield: 73.6%) as a yellow solid.
LC-MS (ESI) m/z: 182.2 $[M+H]^+$

Step 2: Synthesis of (2-methoxy-3-nitrophenyl)methanol

2-Methoxy-3-nitrobenzaldehyde (3.5 g, 19.3 mmol, 1.0 eq) was dissolved in ethanol (30 mL), and $NaBH_4$ (804 mg, 21.3 mmol, 1.1 eq) was added. The mixture was stirred at room temperature for 2.5 hours. After completion of the reaction, the pH was adjusted to 3 with 1 N HCl. The mixture was extracted with dichloromethane, and the organic layer was separated, dried over anhydrous sodium sulfate, filtered, and concentrated to afford the target product (2.89 g, crude) as a yellow solid.
LC-MS (ESI) m/z: 184.2 $[M+H]^+$

Step 3: Synthesis of 1-(bromomethyl)-2-methoxy-3-nitrobenzene

(2-Methoxy-3-nitrophenyl)methanol (2.89 g, 15.8 mmol, 1.0 eq) was dissolved in anhydrous dichloromethane (40 mL), and pyridine (891 mg, 11.4 mmol, 0.72 eq) was added. $PBr_3$ (6.4 g, 23.7 mmol, 1.5 eq) was added dropwise to the reaction mixture. The mixture was stirred at room temperature for 3 hours. After completion of the reaction, water was added, and the mixture was extracted with dichloromethane. The combined organic layers were dried over anhydrous sodium sulfate, concentrated, and purified by flash column chromatography on silica gel to afford the target product (1.8 g, yield: 46.3%) as a yellow solid.
LC-MS (ESI) m/z: 245.8 $[M+H]^+$

Step 4: Synthesis of 2-(2-methoxy-3-nitrophenyl)acetonitrile

TBAF (8.1 mmol, 1.1 eq) and trimethylsilyl cyanide (872 mg, 8.8 mmol, 1.2 eq) were dissolved in THF (20 mL) and stirred at room temperature for 1 hour. A solution of 1-(bromomethyl)-2-methoxy-3-nitrobenzene (1.8 g, 7.32 mmol, 1.0 eq) in acetonitrile (20 mL) was then added, and the mixture was stirred at room temperature for an additional 1 hour. The reaction mixture was concentrated, and the crude product was purified by flash column chromatography on silica gel to afford the target compound (1.2 g, yield: 85.7%) as a yellow solid.
LC-MS (ESI) m/z: 193 $[M+H]^+$

Step 5: Synthesis of 2-(2-methoxy-3-nitrophenyl)ethylamine

2-(2-Methoxy-3-nitrophenyl)acetonitrile (1.1 g, 5.7 mmol, 1.0 eq) was dissolved in anhydrous THF (40 mL), and 2.5 M borane-dimethyl sulfide complex (14.3 mL, 2.5 eq) was added. The reaction mixture was refluxed for 1 hour. The reaction was quenched with MeOH, and the solvent was removed under reduced pressure. The residue was purified by flash column chromatography on silica gel to afford the target product (650 mg, yield: 55%).

LC-MS (ESI) m/z: 196 $[M+H]^+$

$^1$H NMR (400 MHz, Chloroform-d) δ 7.69 (dd, *J* = 8.1, 1.6 Hz, 1H), 7.45 (dd, *J* = 7.6, 1.7 Hz, 1H), 7.16 (t, *J* = 7.9 Hz, 1H), 3.90 (s, 3H), 3.73 - 3.65 (m, 2H), 2.87 (m, 2H), 1.74 - 1.67 (m, 2H).

Step 6: Synthesis of 3-(2-aminoethyl)-2-methoxyaniline acetate

Under $N_2$ protection, 5% wet Pd/C and HOAc (398 mg, 7 mmol, 2.0 eq) were added to a THF solution of 2-(2-methoxy-3-nitrophenyl)ethylamine (650 mg, 3.57 mmol, 1 eq). The suspension was degassed under vacuum and purged with $H_2$ several times. The mixture was stirred overnight at room temperature under $H_2$ protection. The suspension was filtered through diatomite, and the filter cake was washed with methanol. The combined filtrate was concentrated to afford the crude product (780 mg), which was used directly in the next step.

LC-MS (ESI) m/z: 167 $[M+H]^+$

$^1$H NMR (400 MHz, DMSO-$d_6$) δ 6.76 (m, 1H), 6.58 (m, 1H), 6.39 (m, 1H), 4.86 (brs, 2H), 3.64 (s, 3H), 2.84 (m, 2H), 2.71 (m, 2H).

Step 7: Synthesis of benzyl (3-(2-((benzyloxy)carbonyl)amino)ethyl)-2-methoxyphenyl)carbamate

At 0-10 °C, CbzCl (1.76 g, 10.4 mmol, 3.0 eq) was added to a mixture of 3-(2-aminoethyl)-2-methoxyaniline acetate (780 mg, 3.45 mmol, 1.0 eq) in dichloromethane (20 mL) and saturated $NaHCO_3$ aqueous solution (50 mL). The resulting mixture was stirred at room temperature overnight. LC-MS indicated completion of the reaction. The aqueous phase was separated and extracted twice with dichloromethane (20 mL). The combined organic layers were dried over anhydrous sodium sulfate, filtered, concentrated, and purified by flash column chromatography on silica gel to afford the target product (550 mg, yield: 76.5%) as a yellow solid.

LC-MS (ESI) m/z: 435 $[M+H]^+$

$^1$H NMR (400 MHz, Chloroform-d) δ 8.00 (s, 1H), 7.44 - 7.27 (m, 10H), 7.20 (s, 1H), 7.06 (t, J = 7.9 Hz, 1H), 6.85 (m, 1H), 5.22 (s, 2H), 5.08 (s, 2H), 3.71 (s, 3H), 3.45 (m, 2H), 2.84 (m, 2H).

Step 8: Synthesis of benzyl (3-(2-((benzyloxy)carbonyl)amino)ethyl)-2-hydroxyphenyl)carbamate

At -78 °C, $BCl_3$ (1 M in dichloromethane, 12.7 mL, 12.7 mmol, 10 eq) was added to a solution of benzyl (3-(2-((benzyloxy)carbonyl)amino)ethyl)-2-methoxyphenyl)carbamate (550 mg, 1.27 mmol, 1.0 eq) in dichloromethane (40 mL). The mixture was stirred at the same temperature for 2 hours. LC-MS indicated completion of the reaction. The cooled reaction mixture was quenched with saturated $NaHCO_3$ aqueous solution and extracted with dichloromethane (30 mL). The organic phase was dried over anhydrous sodium sulfate, filtered, concentrated, and the residue was purified by flash column chromatography on silica gel to afford the target product (400 mg, yield: 74.9%) as a yellow solid.

LC-MS (ESI) m/z: 421 $[M+H]^+$

$^1$H NMR (400 MHz, Chloroform-*d*) δ 8.47 (brs, 1H), 7.51 (brs, 1H), 7.44 - 7.28 (m, 10H), 7.19 (m, 1H), 6.80 (m, 2H), 5.22 (s, 2H), 5.14 (s, 2H), 3.35 (m, 2H), 2.89 (m, 2H).

Step 9: Synthesis of benzyl (3-(2-((benzyloxy)carbonyl)amino)ethyl)-2-(methoxymethoxy)phenyl)carbamate

At 0 °C, $Cs_2CO_3$ (235 mg, 0.72 mmol, 2.0 eq) and MOMBr (67.5 mg, 0.54 mmol, 1.5 eq) were added to a solution of benzyl (3-(2-((benzyloxy)carbonyl)amino)ethyl)-2-hydroxyphenyl)carbamate (150 mg, 0.36 mmol, 1.0 eq) in anhydrous acetonitrile (20 mL). The mixture was stirred at room temperature for 4 hours. LC-MS indicated completion of the

reaction. Water was added, and the mixture was extracted with ethyl acetate. The combined organic layers were dried over anhydrous sodium sulfate, filtered, concentrated, and purified by flash column chromatography on silica gel to afford the target product (80 mg, yield: 47.8%) as a yellow liquid.
LC-MS (ESI) m/z: 465 $[M+H]^+$

Step 10: Synthesis of 3-(2-aminoethyl)-2-(methoxymethoxy)aniline

Under $N_2$ protection, 5% wet Pd/C (20 mg) and HOAc (20.4 mg, 0.34 mmol, 2.0 eq) were added to a methanol (5 mL) solution of benzyl (3-(2-((benzyloxy)carbonyl)amino)ethyl)-2-(methoxymethoxy)phenyl)carbamate (80 mg, 0.17 mmol, 1.0 eq). The suspension was degassed under vacuum and purged with $H_2$ several times, then stirred at room temperature under $H_2$ protection for 0.5 hours. The suspension was filtered through diatomite, and the filter cake was washed with methanol. The combined filtrate was concentrated and used directly in the next step.
LC-MS (ESI) m/z: 197 $[M+H]^+$

Step 11: Synthesis of T6-12

The intermediate Int-A (9 mg, 0.015 mmol, 1.0 eq), 3-(2-aminoethyl)-2-(methoxymethoxy)aniline (14.1 mg, 0.072 mmol, 5 eq), and HOAc (9.54 mg, 0.16 mmol, 10 eq) were dissolved in anhydrous ethanol (2 mL) and stirred at room temperature for 2.5 hours. Upon completion of the reaction, the solvent was removed under reduced pressure to afford a solid. The crude product was used directly in the next step without further purification.
LC-MS (ESI) m/z: 800 $[M+H]^+$

Step 12: Synthesis of T6

T6-12 (10 mg) was dissolved in dichloromethane (2 mL), and TFA (1 mL) was added. The mixture was stirred at room temperature overnight. After completion of the reaction, the mixture was neutralized with saturated $NaHCO_3$ aqueous solution and extracted with dichloromethane. The organic layer was separated and concentrated. The crude product was purified by preparative HPLC to afford T6 (3.86 mg, yield: 40.8%) as a brown solid.
LC-MS (ESI) m/z: 756 $[M+H]^+$

Example 28: Synthesis of Compound T7

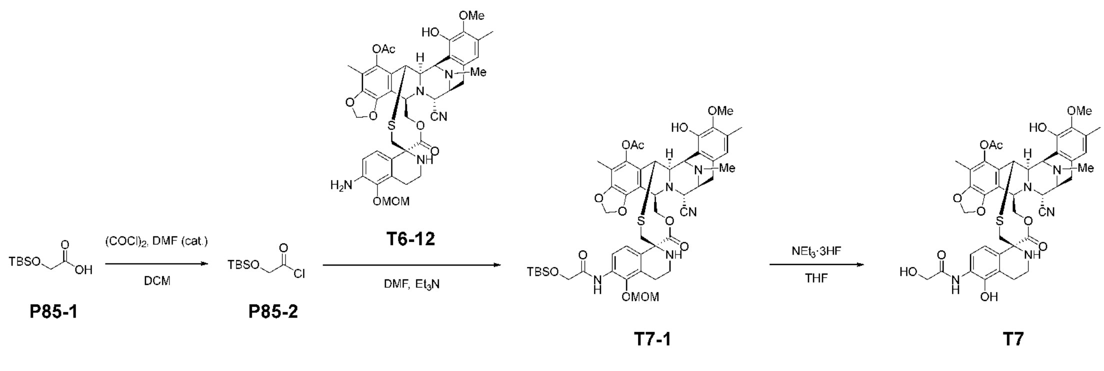

P85-1 P85-2 T6-12 T7-1 T7

Step 1: Synthesis of T7-1

2-((tert-butyldimethylsilyl)oxy)acetic acid (38 mg, 0.2 mmol, 10 eq) was dissolved in anhydrous dichloromethane (2 mL). At 0 °C, oxalyl chloride (2 M in dichloromethane, 0.05 mL, 0.1 mmol, 5 eq) and 2 drops of catalytic DMF were added. The mixture was stirred at 0 °C for 1 hour, then added to a separate flask containing T6-12 (16 mg, 0.02 mmol, 1.0 eq) and triethylamine (20 mg, 0.2 mmol, 10 eq) in anhydrous DMF. The reaction was stirred at room temperature for 2 hours. LC-MS indicated completion of the reaction. The mixture was quenched with water and extracted with ethyl acetate. The combined organic layers were dried, filtered, and purified by flash column chromatography on silica gel to afford the target product (12 mg, yield: 60%) as a yellow liquid.
LC-MS (ESI) m/z: 972 $[M+H]^+$

Step 2: Synthesis of T7

T7-1 (9 mg, 0.0093 mmol, 1.0 eq) was dissolved in anhydrous THF (1 mL), and triethylamine trihydrofluoride (0.5 mL) was added. The mixture was stirred for 1 hour. LC-MS indicated completion of the reaction. The reaction mixture was neutralized with saturated $NaHCO_3$ aqueous solution and extracted with ethyl acetate. The organic layer was dried over anhydrous sodium sulfate, filtered, concentrated, and purified by preparative HPLC to afford the target product T7 (2.29 mg, yield: 30%) as a white solid.
LC-MS (ESI) m/z: 814 $[M+H]^+$

Example 29: Synthesis of Compound T8

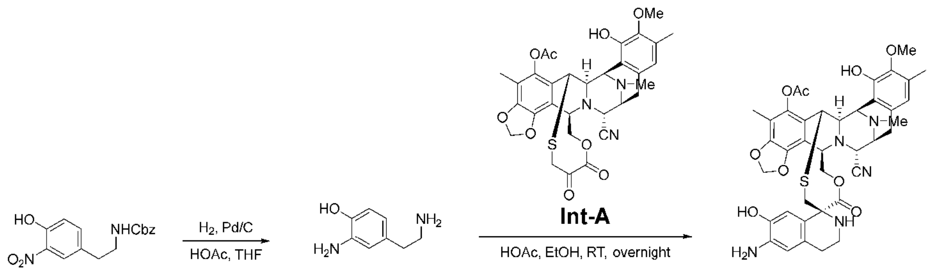


T8-1 T8-2 T8

Step 1: Synthesis of 2-amino-4-(2-aminoethyl)phenol

Under $N_2$ protection, T8-1 (50 mg, 0.16 mmol) and 10% wet Pd/C (30 mg) were added to a methanol (3 mL) solution. The suspension was degassed under vacuum and purged with $H_2$ several times, then stirred at room temperature under $H_2$ protection for 4-5 hours. TLC indicated completion of the reaction. The suspension was filtered through diatomite, and the filter cake was washed with methanol. The combined filtrate was concentrated and used directly in the next step.
LC-MS (ESI) m/z: $[M+H]^+$ 153.2

Step 2: Synthesis of T8

The intermediate Int-A (20 mg, 0.032 mmol), T8-2 (24 mg, 0.16 mmol), and HOAc (20 mg, 0.32 mmol) were dissolved in anhydrous ethanol (1 mL) and stirred at room temperature overnight. Upon completion of the reaction, the mixture was purified by preparative HPLC to afford the target product (10 mg, yield: 41.7%) as a white solid.

LC-MS (ESI) m/z: $[M+H]^+$ 756.4

$^1$H NMR (400 MHz, DMSO-$d_6$) δ 8.78 (s, 1H), 8.70 (s, 1H), 6.44 (s, 1H), 6.28 (s, 1H), 6.21 (d, *J* = 8 Hz, 2H), 6.16 (s, 1H), 5.06 (d, *J* = 12 Hz, 3H), 4.44 (s, 2H), 4.19 (s, 1H), 4.04 (s, 1H), 3.98-3.95 (m, 1H), 3.63 (s, 3H), 3.19-3.08 (m, 5H), 2.79 (s, 2H), 2.58 (s, 2H), 2.56 (s, 1H), 2.43 (s, 1H), 2.29 (s, 3H), 2.21 (s, 3H), 2.03 (s, 3H), 1.99 (s, 3H), 1.95 (s, 1H), 1.69 (d, *J* = 16 Hz, 1H).

Example 30: Synthesis of Compound T9

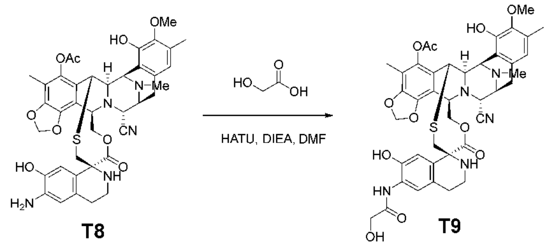


T8 T9

T8 (6 mg, 0.008 mmol), glycolic acid (1.8 mg, 0.024 mmol), DIEA (9.2 mg, 0.072 mmol), and HATU (4.5 mg, 0.012 mmol) were dissolved in anhydrous DMF (0.4 mL) and stirred at room temperature overnight. LC-MS indicated formation of the target product. The reaction mixture was purified by preparative HPLC to afford the target product (3.0 mg, yield: 46.9%) as a white solid.
LC-MS (ESI) m/z: $[M+H]^+$ 814.22

Example 31: Synthesis of Compound T10

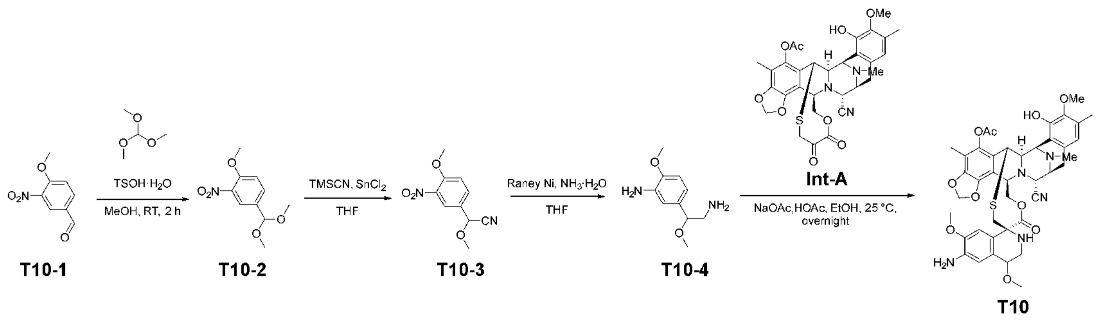


Step 1: Synthesis of 4-(dimethoxymethyl)-1-methoxy-2-nitrobenzene

4-Methoxy-3-nitrobenzaldehyde (2 g, 11 mmol) and trimethyl orthoformate (3.76 g, 25.4 mmol) were dissolved in anhydrous methanol, and p-toluenesulfonyl monohydrate (21 mg, 0.11 mmol) was added. The mixture was stirred at room temperature for 2 hours. TLC indicated completion of the reaction. The reaction was quenched with saturated $NaHCO_3$ aqueous solution and extracted with ethyl acetate. The organic layer was dried over anhydrous sodium sulfate, filtered, and concentrated. The crude product was purified by silica gel flash column chromatography to afford the target compound (2.2 g, yield: 87.7%) as a colorless oil.

LC-MS (ESI) m/z: 197 $[M-CH_3O]^+$

$^1$H NMR (400 MHz, DMSO-$d_6$) δ 7.85 (d, $J$ = 2.2 Hz, 1H), 7.69 - 7.63 (m, 1H), 7.40 (d, $J$ = 8.7 Hz, 1H), 5.44 (s, 1H), 3.95 (s, 3H), 3.27 (s, 6H).

Step 2: Synthesis of 2-methoxy-2-(4-methoxy-3-nitrophenyl)acetonitrile

To a THF (10 mL) solution of 4-(dimethoxymethyl)-1-methoxy-2-nitrobenzene (1 g, 4.4 mmol) and TMSCN (437 mg, 4.4 mmol) was added $SnCl_2$ (66.8 mg, 0.352 mmol). The reaction was monitored by LC-MS until completion. The reaction was quenched with saturated aqueous $NaHCO_3$, extracted with ethyl acetate, and the organic phase was dried over anhydrous $Na_2SO_4$, filtered, and concentrated. The crude product was purified by rapid silica gel column chromatography to afford the target product (690 mg, yield 70.5%).
LC-MS (ESI) m/z: 223 $[M+H]^+$

Step 3: Synthesis of 5-(2-amino-1-methoxyethyl)-2-methoxyaniline

To a THF solution of 2-methoxy-2-(4-methoxy-3-nitrophenyl)acetonitrile (50 mg, 0.22 mmol) was added Raney Ni and 0.1-0.5 equiv of $NH_3 \cdot H_2O$. The mixture was stirred at room temperature for 2 hours. Upon completion, the reaction was filtered and concentrated. The crude product was purified by preparative thin-layer chromatography to afford the target product (34 mg, yield: 77.3%).
LC-MS (ESI) m/z: 197 $[M+H]^+$

Step 4: Synthesis of T10

To an anhydrous ethanol (1 mL) solution were added intermediate Int-A (4 mg, 0.0065 mmol), 5-(2-amino-1-methoxyethyl)-2-methoxyaniline (12.56 mg, 0.065 mmol), HOAc (3.9 mg, 0.065 mmol), and NaOAc (5.25 mg, 0.064 mmol), and the mixture was stirred at 25°C overnight. Upon completion, the solvent was removed under reduced pressure to afford a solid crude product. The crude product was purified by preparative high-performance liquid chromatography to

yield the target product T10 (1.53 mg, yield: 29.7%) as a white solid.
LC-MS (ESI) m/z: 800 [M+H]+

Example 32: Synthesis of compound T11

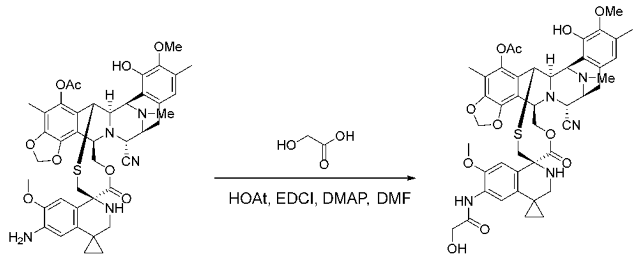


P36 T11

Step 1: Synthesis of T11

P36 (20 mg, 0.025 mmol), glycolic acid (2-hydroxyacetic acid) (3.8 mg, 0.05 mmol), EDCI (23 mg, 0.125 mmol), DMAP (1.5 mg, 0.0125 mmol), and HOAt (16 mg, 0.125 mmol) were dissolved in DMF (0.2 mL) and stirred at room temperature for 2 hours. LC-MS indicated formation of the target product. The crude product was purified by preparative high-performance liquid chromatography to afford the target product (8.3 mg, yield: 38%).

LC-MS (ESI) m/z: 854.3 [M+H]+

$^1$H NMR (400 MHz, DMSO-$d_6$) δ 9.01 (s, 1H), 8.74 (s, 1H), 7.55 (s, 1H), 6.43 (s, 2H), 6.19-6.17 (m, 2H), 6.11 (s, 1H), 5.09-5.05 (m, 1H), 4.52 (s, 2H), 4.23 (s, 1H), 4.14-4.07 (m, 2H), 3.93 (s, 2H), 3.65 (s, 3H), 3.59 (s, 3H), 3.32-3.30 (m, 3H), 2.81-2.77 (m, 2H), 2.32 (s, 3H), 2.23-2.21 (m, 4H), 2.19-1.14 (m, 5H), 2.00 (s, 3H), 1.92-1.90 (m, 1H), 0.85-0.81 (m, 2H), 0.70-0.53 (m, 2H).

Example 33: Synthesis of compound T12

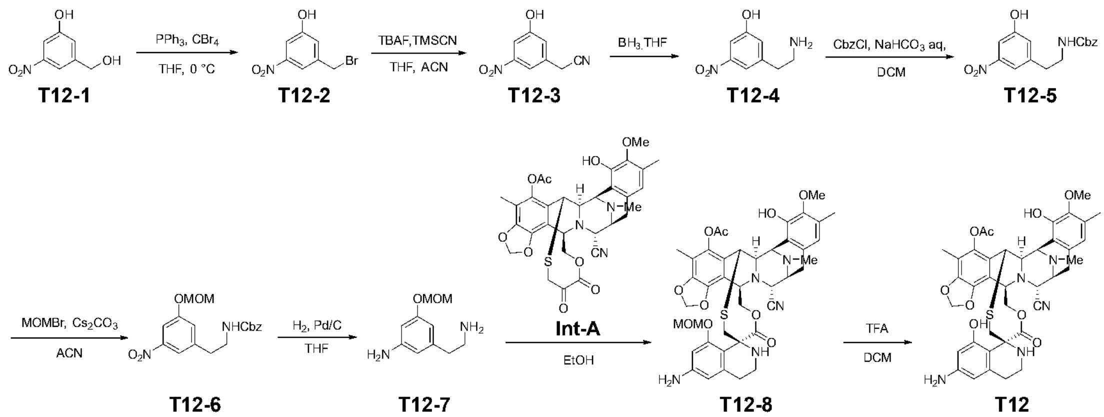


Step 1: Synthesis of 3-(bromomethyl)-5-nitrophenol

At 0°C, to a THF (50 mL) solution of 3-(hydroxymethyl)-5-nitrophenol (3.1 g, 18.33 mmol) was added $PPh_3$ (7.2 g, 27.5 mmol) and $CBr_4$ (9.2 g, 27.5 mmol), and the mixture was stirred at room temperature for 1 hour. LC-MS indicated completion of the reaction. The reaction was quenched with water and extracted twice with ethyl acetate. The organic phase was dried over anhydrous $Na_2SO_4$, filtered, and concentrated. The crude product was purified by rapid silica gel column chromatography to afford the target product (3.7 g, yield: 87.6%) as a yellow solid.

LC-MS (ESI) m/z: 231.7 $[M+H]^+$

Step 2: Synthesis of 2-(3-hydroxy-5-nitrophenyl)acetonitrile

3-(Bromomethyl)-5-nitrophenol (2.7 g, 11.64 mmol) was dissolved in anhydrous acetonitrile (20 mL), and a solution of TBAF (18.63 mL, 18.63 mmol) and TMSCN (2.1 g, 20 mmol) in anhydrous THF was added at room temperature. The reaction was stirred for 4 hours. LC-MS indicated completion of the reaction. The solvent was removed under reduced pressure, and the residue was diluted with ethyl acetate and washed several times with water. The organic phase was dried over $Na_2SO_4$, filtered, and concentrated. The residue was purified by rapid silica gel column chromatography to afford the target product (1.74 g, yield: 84.2%) as a yellow solid.
LC-MS (ESI) m/z: 176.8 $[M-H]^-$

Step 3: Synthesis of 3-(2-aminoethyl)-5-nitrophenol

To an anhydrous THF solution of 2-(3-hydroxy-5-nitrophenyl)acetonitrile (500 mg, 2.8 mmol) was added $BH_3 \cdot THF$ (1 M, 7 mL, 7 mmol), and the mixture was refluxed for 1 hour. LC-MS indicated completion of the reaction. The reaction was quenched with methanol at 0°C and refluxed for an additional 30 minutes. The reaction mixture was concentrated and used directly in the next step.
LC-MS (ESI) m/z: 183.3 $[M+H]^+$

Step 4: Synthesis of benzyl (3-hydroxy-5-nitrophenethyl)carbamate

To an anhydrous dichloromethane solution of the crude 3-(2-aminoethyl)-5-nitrophenol (511 mg, 2.8 mmol) was added Cbz-Cl (860 mg, 5.04 mmol) and an aqueous $NaHCO_3$ solution (283 mg, 3.36 mmol, 1.2 equiv). The mixture was stirred at room temperature for 1.5 hours. LC-MS indicated completion of the reaction. The reaction mixture was washed with water to separate the organic phase, which was then dried over anhydrous $Na_2SO_4$, filtered, and concentrated to afford the target compound (840 mg, crude) as a yellow liquid. The crude product was used directly in the next step without further purification.
LC-MS (ESI) m/z: 317.1 $[M+H]^+$

Step 5: Synthesis of benzyl (3-(methoxymethoxy)-5-nitrophenethyl)carbamate

To an anhydrous acetonitrile (20 mL) solution of benzyl (3-hydroxy-5-nitrophenethyl)carbamate (840 mg, 2.63 mmol) were added $Cs_2CO_3$ (1.7 g, 5.27 mmol) and MOMBr (495 mg, 3.95 mmol), and the mixture was stirred at room temperature for 1 hour. LC-MS indicated completion of the reaction. The reaction was quenched with water and extracted with dichloromethane. The organic phase was separated, dried over anhydrous $Na_2SO_4$, filtered, and concentrated. The residue was purified by rapid silica gel column chromatography to afford the target product (160 mg, yield: 15.8%) as a yellow liquid.

LC-MS (ESI) m/z: 361.0 $[M+H]^+$

$^1$H NMR (400 MHz, Chloroform-d) $\delta$ 7.76 (t, $J$ = 2.2 Hz, 1H), 7.71 (s, 1H), 7.39-7.29 (m, 5H), 7.18 (s, 1H), 5.21 (s, 2H), 5.10 (s, 2H), 3.48 (s, 5H), 2.90 (t, $J$ = 7.0 Hz, 2H).

Step 6: Synthesis of 3-(2-aminoethyl)-5-(methoxymethoxy)aniline

Under $N_2$ protection, to a THF (10 mL) solution of benzyl (3-(methoxymethoxy)-5-nitrophenethyl)carbamate (160 mg, 0.44 mmol) was added 5% wet Pd/C (20 mg) and HOAc (53 mg, 0.88 mmol). The suspension was degassed under vacuum and repeatedly replaced with $H_2$. Under $H_2$ atmosphere, the mixture was stirred at room temperature overnight. TLC indicated completion of the reaction. The suspension was filtered through diatomaceous earth, and the filter cake was washed with methanol. The combined filtrate was concentrated and used directly in the next step.
LC-MS (ESI) m/z: 196.9 $[M+H]^+$

Steps 7 and 8: Synthesis of T12

Intermediate Int-A (10 mg, 0.016 mmol) and 3-(2-aminoethyl)-5-(methoxymethoxy)aniline (10.6 mg, 0.041 mmol) were dissolved in EtOH (10 mL) and stirred at 50°C for 3 hours. LC-MS indicated completion of the reaction. The solvent was removed under reduced pressure, and TFA/DCM = 1:1 (v/v) (1 mL) was added. Upon completion, the reaction

mixture was concentrated and purified by preparative high-performance liquid chromatography to afford the target product (0.46 mg, yield: 3.78%) as a white solid.
LC-MS (ESI) m/z: 756.4 $[M+H]^+$

Example 34: Synthesis of compound T13

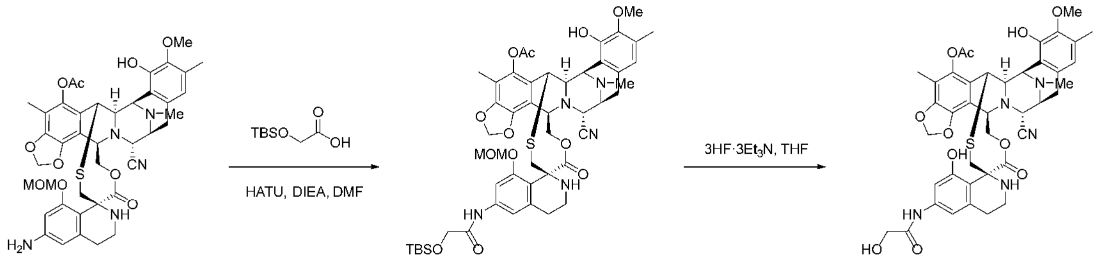


**T12-8** **T13-1** **T13**

Step 1: Synthesis of T13-1

To a DMF (2 mL) solution of the crude T12-8 (20 mg, 0.025 mmol) was added 2-((tert-butyldimethylsilyl)oxy) acetic acid (47.6 mg, 0.25 mmol), HATU (95 mg, 0.25 mmol), and DIEA (32.3 mg, 0.25 mmol). LC-MS indicated completion of the reaction. The crude product was purified by preparative high-performance liquid chromatography to afford the target product (4.2 mg, yield: 13.4%) as a white solid.
LC-MS (ESI) m/z: 972.6 $[M+H]^+$

Step 2: Synthesis of T13

To a THF (0.7 mL) solution of T13-1 (3 mg, 0.003 mmol) was added $3HF \cdot 3Et_3N$ (0.7 mL), and the mixture was stirred at room temperature for 1 hour. LC-MS indicated completion of the reaction. The reaction mixture was then treated with saturated aqueous $NaHCO_3$ and extracted with dichloromethane. The organic phase was separated, dried, filtered, and concentrated, and the residue was dissolved in DCM/TFA = 3:1. Upon completion, the pH of the mixture was adjusted to 7-8 with saturated aqueous $NaHCO_3$. The organic phase was separated, dried over anhydrous $Na_2SO_4$, filtered, and concentrated. The residue was purified by preparative high-performance liquid chromatography to afford the target product (0.97 mg, yield: 38.6%) as a white solid.

LC-MS (ESI) m/z: 814.4 $[M+H]^+$

$^1$H NMR (400 MHz, Chloroform-d) δ 8.00 (s, 1H), 6.86 (s, 1H), 6.65 (s, 1H), 6.57 (s, 1H), 6.06 (d, *J* = 24.5 Hz, 2H), 5.85 (s, 1H), 4.86 (s, 1H), 4.50 (s, 2H), 4.34-4.24 (m, 2H), 4.14 (s, 1H), 4.06 (s, 2H), 3.80 (s, 3H), 3.52 (s, 1H), 3.42 (d, *J* = 8.1 Hz, 1H), 2.97-2.88 (m, 2H), 2.83-2.68 (m, 2H), 2.56 (d, *J* = 16.6 Hz, 2H), 2.33 (s, 3H), 2.30 (s, 3H), 2.25 (s, 1H), 2.22 (s, 4H), 2.06 (s, 3H), 2.04 (s, 1H).

Example 35: Synthesis of compound T14

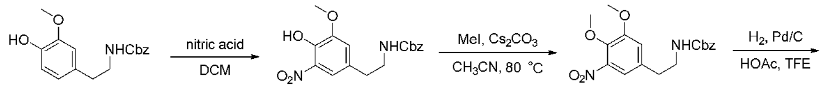


T14-1 T14-2 T14-3

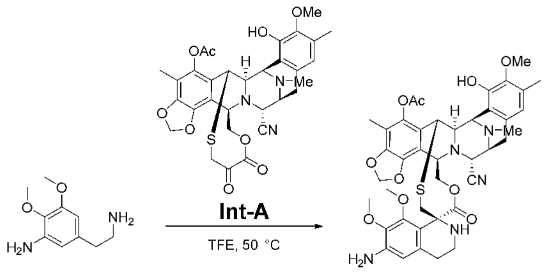


T14-4 T14

Step 1: Synthesis of benzyl (4-hydroxy-3-methoxy-5-nitrophenethyl)carbamate

At 0°C, to a dichloromethane (1.5 mL) solution of benzyl (4-hydroxy-3-methoxyphenethyl)carbamate (300 mg, 0.99 mmol) was slowly added 65% nitric acid (0.6 mL), and the mixture was stirred at the same temperature for 20 minutes. LC-MS indicated completion of the reaction. The reaction mixture was poured into ice water to quench. The mixture was extracted with dichloromethane, and the organic layers were combined, dried, filtered, and concentrated. The residue was purified by rapid silica gel column chromatography to afford the target compound (80 mg, yield: 23%) as a yellow solid.

LC-MS (ESI) m/z: $[M+H]^+$ 347.0

$^1H$ NMR (400 MHz, DMSO-$d_6$) δ 10.18 (s, 1H), 7.39 - 7.24 (m, 7H), 7.15 (d, *J* = 2.0 Hz, 1H), 5.00 (s, 2H), 3.84 (s, 3H), 3.26 (q, *J* = 6.7 Hz, 2H), 2.70 (t, *J* = 7.0 Hz, 2H).

Step 2: Synthesis of benzyl (3,4-dimethoxy-5-nitrophenethyl)carbamate

Benzyl (4-hydroxy-3-methoxy-5-nitrophenethyl)carbamate (80 mg, 0.2 mmol), $Cs_2CO_3$ (197.45 mg, 0.606 mmol), and MeI (34.4 mg, 0.242 mmol) were dissolved in acetonitrile (5 mL) in a sealed tube and stirred at 80°C for 1 hour. LC-MS indicated completion of the reaction. The reaction mixture was filtered, and the filtrate was concentrated. The residue was purified by column chromatography to afford the target compound (55 mg, yield: 60%) as a yellow solid.
LC-MS (ESI) m/z: $[M+H]^+$ 361.1

Step 3: Synthesis of 5-(2-aminoethyl)-2,3-dimethoxyaniline

Under $N_2$ protection, to a TFE (10 mL) solution of benzyl (3,4-dimethoxy-5-nitrophenethyl)carbamate (55 mg, 0.152 mmol) was added HOAc (18.31 mg, 0.305 mmol) and 10% wet Pd/C (20 mg, 20% w/w). The suspension was degassed under vacuum and repeatedly replaced with $H_2$. Under $H_2$ atmosphere, the mixture was stirred at room temperature overnight. TLC indicated completion of the reaction. The suspension was filtered through diatomaceous earth, and the filter cake was washed with methanol. The combined filtrate was concentrated and used directly in the next step.
LC-MS (ESI) m/z: $[M+H]^+$ 197.4

Step 4: Synthesis of T14

Intermediate Int-A (10 mg, 0.016 mmol) and 5-(2-aminoethyl)-2,3-dimethoxyaniline (18.9 mg, 0.096 mmol) were dissolved in 2,2,2-trifluoroethanol (2 mL) and stirred at 50°C overnight. LC-MS indicated completion of the reaction. The crude product was purified by preparative high-performance liquid chromatography to afford the target product (6.72 mg, yield: 52.9%) as a white solid.

LC-MS (ESI) m/z: $[M+H]^+$ 800.5

$^1H$ NMR (400 MHz, Chloroform-d) δ 6.48 (s, 1H), 6.02 (s, 1H), 5.98 (d, *J* = 1.4 Hz, 1H), 5.92 (d, *J* = 1.4 Hz, 1H), 5.68 (s, 1H), 4.62 - 4.44 (m, 2H), 4.24 - 4.18 (m, 1H), 4.18 - 4.15 (m, 1H), 4.12 - 4.05 (m, 1H), 4.04 (d, *J* = 5.2 Hz, 1H), 3.72 (s,

3H), 3.55 (d, $J$ = 1.1 Hz, 3H), 3.49 - 3.43 (m, 1H), 3.29 (d, $J$ = 0.9 Hz, 3H), 3.06 (d, $J$ = 15.0 Hz, 1H), 2.91 - 2.75 (m, 2H), 2.59 - 2.49 (m, 1H), 2.49 - 2.41 (m, 2H), 2.24 (s, 3H), 2.22 (s, 3H), 2.19 - 2.14 (m, 1H), 2.13 (s, 3H), 1.98 (s, 3H).

Example 36: Synthesis of compound T15

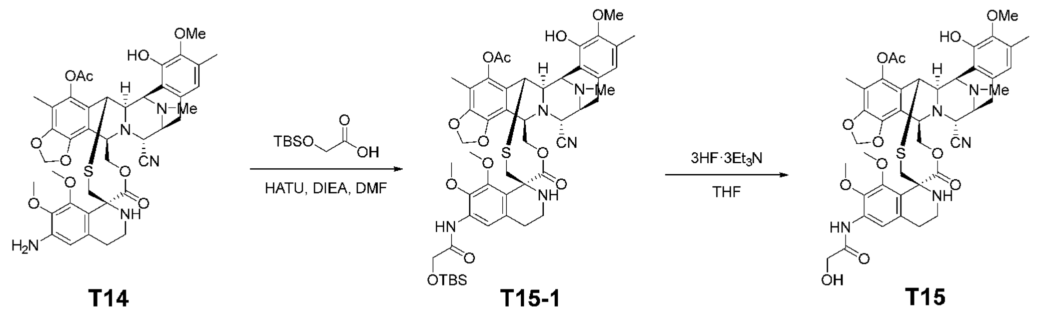


T14 T15-1 T15

Steps 1 and 2: Synthesis of T15

To a DMF (1 mL) solution of T14 (10 mg, 0.0125 mmol) was added 2-((tert-butyldimethylsilyl)oxy)acetic acid (23.9 mg, 0.125 mmol), HATU (47 mg, 0.125 mmol), and DIEA (16.1 mg, 0.125 mmol). LC-MS indicated completion of the reaction. The mixture was diluted with ethyl acetate and washed with water. The organic phase was separated, dried, concentrated, and dissolved in anhydrous THF (1 mL). $3HF\cdot3Et_3N$ (0.9 mL) was then added, and the mixture was stirred at room temperature for 1 hour. LC-MS indicated completion of the reaction. The mixture was treated with saturated aqueous $NaHCO_3$ and dichloromethane. The organic phase was separated, dried over anhydrous $Na_2SO_4$, filtered, and concentrated. The residue was purified by preparative high-performance liquid chromatography to afford the target product (1.6 mg, yield: 14.9%) as a white solid.
LC-MS (ESI) m/z: $[M+H]^+$ 858.5

Example 37: Synthesis of compound T16

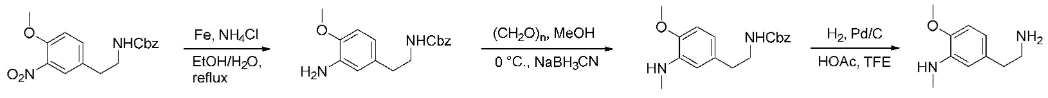


P74-4 T16-2 T16-3 T16-4

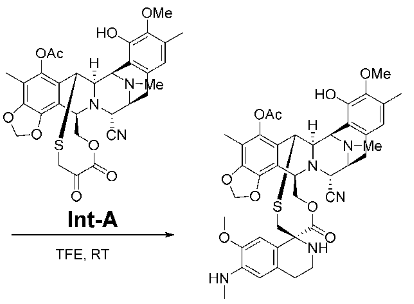
Int-A


T16

Step 1: Synthesis of benzyl (3-amino-4-methoxyphenethyl)carbamate

Benzyl (4-methoxy-3-nitrophenethyl)carbamate (500 mg, 1.51 mmol) was dissolved in an $EtOH/H_2O$ = 4:1 (10 mL) solution, and iron powder (339 mg, 6.05 mmol) and $NH_4Cl$ (320 mg, 6.05 mmol) were added. The mixture was refluxed for 3 hours. LC-MS indicated completion of the reaction. The mixture was filtered, washed with water, and extracted with ethyl acetate. The combined organic phase was dried over anhydrous $Na_2SO_4$, filtered, and concentrated. The residue was used directly in the next step.

LC-MS (ESI) m/z: $[M+H]^+$ 301.2

Step 2: Synthesis of benzyl (4-methoxy-3-(methylamino)phenethyl)carbamate

The crude benzyl (3-amino-4-hydroxyphenethyl)carbamate (200 mg, 0.665 mmol) was dissolved in methanol (5 mL), and paraformaldehyde (79.8 mg, 2.66 mmol) was added. The mixture was stirred at room temperature for 1 hour. At 0°C, $NaBH_3CN$ (209 mg, 3.32 mmol) was added, and the mixture was stirred for an additional 2 hours. LC-MS indicated completion of the reaction. The reaction mixture was diluted with ethyl acetate and water. The organic layer was separated and concentrated. The residue was purified by rapid silica gel column chromatography to afford the target product (50 mg, yield: 22.7%).

LC-MS (ESI) m/z: $[M+H]^+$ 315.1
$^1H$ NMR (400 MHz, Chloroform-d) δ 7.38 (s, 5H), 6.71 (d, *J* = 8.0 Hz, 1H), 6.50 (d, *J* = 8.0 Hz, 1H), 6.43 (s, 1H), 5.13 (s, 2H), 3.86 (s, 3H), 3.49 (d, *J* = 6.9 Hz, 2H), 2.87 (s, 3H), 2.77 (t, *J* = 7.0 Hz, 2H).

Steps 3 and 4: Synthesis of 5-(2-aminoethyl)-2,3-dimethoxyaniline

Under $N_2$ protection, to a TFE (10 mL) solution of benzyl (4-methoxy-3-(methylamino)phenethyl)carbamate (45 mg, 0.143 mmol) was added HOAc (17.16 mg, 0.286 mmol) and 10% wet Pd/C (20 mg, 20% w/w). The suspension was degassed under vacuum and repeatedly replaced with $H_2$. Under $H_2$ atmosphere, the mixture was stirred at room temperature overnight. TLC indicated completion of the reaction. The suspension was filtered, and to the filtrate was added intermediate Int-A (20 mg, 0.0322 mmol). The mixture was stirred at room temperature for 16 hours. LC-MS indicated completion of the reaction. The reaction mixture was purified by preparative high-performance liquid chromatography to afford the target product (11 mg, yield: 43.6%) as a white solid.

LC-MS (ESI) m/z: $[M+H]^+$ 784.3

$^1H$ NMR (400 MHz, Chloroform-d) δ 6.63 (s, 1H), 6.36 (s, 1H), 6.14 (s, 1H), 6.08 (s, 1H), 6.01 (s, 1H), 5.76 (s, 1H), 5.05 (d, *J* = 11.5 Hz, 1H), 4.59 (s, 1H), 4.36 (s, 1H), 4.31 (d, *J* = 4.8 Hz, 1H), 4.22 (s, 1H), 4.13 (d, *J* = 6.4 Hz, 1H), 3.83 (s, 3H), 3.61 (s, 3H), 3.55 (d, *J* = 4.9 Hz, 1H), 3.45 (s, 1H), 3.18 (s, 1H), 2.98 (d, *J* = 9.0 Hz, 2H), 2.78 (s, 3H), 2.67 (s, 1H), 2.51 (d, *J* = 16.4 Hz, 1H), 2.36 (s, 5H), 2.30 (s, 3H), 2.23 (s, 4H), 2.08 (s, 3H).

Example 38: Synthesis of compound T17

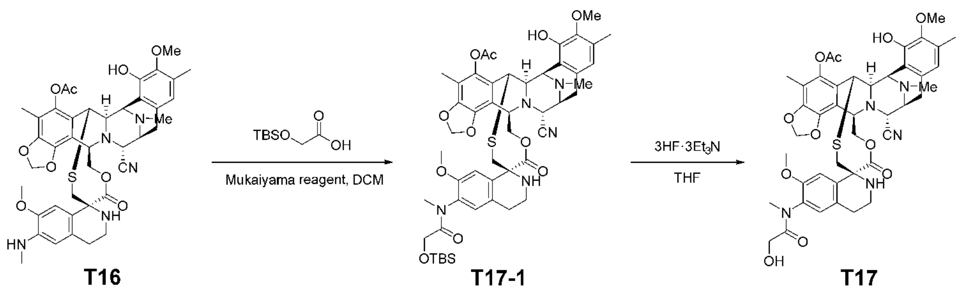


T16 T17-1 T17

Step 1: Synthesis of T17-1

T16 (11 mg, 0.014 mmol), Mukaiyama reagent (7.12 mg, 0.0278 mmol), and 2-((tert-butyldimethylsilyl)oxy)acetic acid (3.99 mg, 0.0209 mmol) were dissolved in anhydrous dichloromethane (3 mL), and $Et_3N$ (7.0 mg, 0.0693 mmol) was added. The mixture was stirred at room temperature for 5 hours. LC-MS indicated completion of the reaction. The reaction mixture was concentrated, and the residue was purified by rapid silica gel column chromatography to afford the target product (6 mg, yield: 44.7%) as a white solid.
LC-MS (ESI) m/z: $[M+H]^+$ 956.7

Step 2: Synthesis of T17

To an anhydrous THF solution of T17-1 (6 mg, 0.00628 mmol) was added 3HF·3$Et_3N$ (1 mL), and the mixture was stirred at room temperature for 30 minutes. LC-MS indicated completion of the reaction. The reaction mixture was quenched with saturated aqueous $NaHCO_3$ and extracted with ethyl acetate. The organic layers were combined, dried over anhydrous $Na_2SO_4$, filtered, and concentrated. The residue was purified by rapid silica gel column chromatography to afford the target product (1.55 mg, yield: 29.3%) as a white solid.
LC-MS (ESI) m/z: $[M+H]^+$ 842.4

Example 39: Synthesis of compound T18

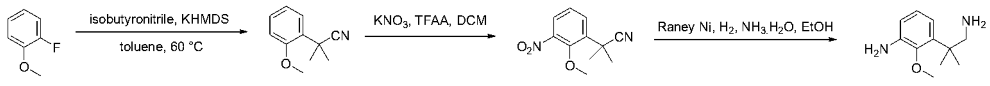


T18-1 T18-2 T18-3 T18-4

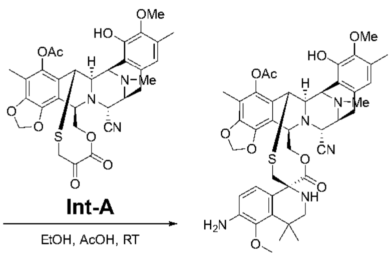


T18

Step 1: Synthesis of 2-(2-methoxyphenyl)-2-methylpropanenitrile

2-Fluorophenyl ether (5 g, 39.66 mmol), isobutyronitrile (10.9 g, 158.67 mmol), and KHMDS (1 M solution in THF, 59.49 mL) were dissolved in dry toluene (50 mL) and stirred at 60°C for 16 hours. Upon completion, the reaction was quenched with saturated aqueous $NH_4Cl$ and extracted with ethyl acetate. The organic layer was washed with saturated brine, dried over anhydrous $Na_2SO_4$, filtered, and concentrated. The residue was purified by rapid silica gel column chromatography to afford the target product (6.5 g, yield: 93.5%) as a colorless liquid.
LC-MS (ESI) m/z: 176.3 $[M+H]^+$

Step 2: Synthesis of 2-(2-methoxy-3-nitrophenyl)-2-methylpropanenitrile

2-(2-Methoxyphenyl)-2-methylpropanenitrile (2 g, 11.4 mmol) and $KNO_3$ (1.5 g, 14.85 mmol) were dissolved in dichloromethane (10 mL). Trifluoroacetic anhydride (10 mL) was added dropwise, and the mixture was stirred at room temperature for 1 hour. Upon completion, the reaction mixture was diluted with 20 mL of water and 20 mL of dichloromethane. The organic layer was separated, dried over anhydrous $Na_2SO_4$, filtered, and concentrated. The residue was purified by rapid silica gel column chromatography to afford the target product (555 mg, yield: 22%) as a yellow solid.

LC-MS (ESI) m/z: 221.5 $[M+H]^+$

$^1H$ NMR (400 MHz, DMSO-$d_6$) δ 7.95 (dd, $J$ = 8.2 Hz, 1.6 Hz, 1H), 7.73 (dd, $J$ = 8.0, 1.6 Hz, 1H), 7.35 (t, $J$ = 8.0 Hz, 1H), 1.74 (s, 6H).

Step 3: Synthesis of 3-(1-amino-2-methylprop-2-yl)-2-methoxyaniline

2-(2-Methoxy-3-nitrophenyl)-2-methylpropanenitrile (10 mg) was dissolved in EtOH (1 mL), and one drop of $NH_3 \cdot H_2O$ was added. A small amount of Raney Ni was added, and the mixture was stirred under $H_2$ atmosphere for 16 hours. Upon completion, the reaction mixture was filtered, and the filtrate was concentrated and used directly in the next step.
LC-MS (ESI) m/z: 195.1 $[M+H]^+$

Step 4: Synthesis of T18

Int-A (10 mg, 0.008 mmol), 3-(1-amino-2-methylprop-2-yl)-2-methoxyaniline (8.8 mg, 0.045 mmol), and AcOH (2.7 mg, 0.045 mmol) were dissolved in anhydrous EtOH (1 mL) and stirred at 25°C overnight. Upon completion, the solvent was removed under reduced pressure. The crude product was purified by preparative high-performance liquid chromatography to afford the target product (2.39 mg, yield: 18.6%) as a white solid.

LC-MS (ESI) m/z: 798.7 $[M+H]^+$

$^1$H NMR (400 MHz, Chloroform-d) δ 6.66 (d, *J* = 8.5 Hz, 1H), 6.61 (s, 1H), 6.44 (d, *J* = 8.5 Hz, 1H), 6.15 (d, *J* = 1.4 Hz, 1H), 6.00 (d, *J* = 1.4 Hz, 1H), 5.79 (s, 1H), 5.00 (d, *J* = 11.5 Hz, 1H), 4.49 (s, 1H), 4.25 (d, *J* = 3.9 Hz, 2H), 4.16 (d, *J* = 2.7 Hz, 1H), 4.05 (dd, *J* = 11.5, 2.0 Hz, 1H), 3.78 (s, 3H), 3.69 (s, 3H), 3.46 - 3.37 (m, 3H), 3.15 (d, *J* = 11.8 Hz, 1H), 3.05 - 2.88 (m, 2H), 2.53 (d, *J* = 14.8 Hz, 1H), 2.34 (s, 4H), 2.28 (s, 3H), 2.23 (d, *J* = 7.9 Hz, 1H), 2.21 (s, 3H), 2.05 (s, 3H), 1.25 (s, 6H).

Synthesis and Characterization of Intermediates

Example 40: Synthesis of compound L1

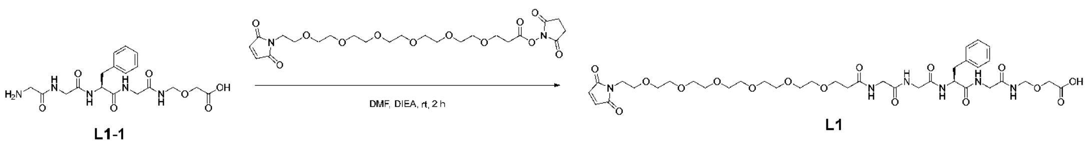


L1-1

L1

At 0°C, (S)-16-amino-10-benzyl-6,9,12,15-tetraoxo-3-oxo-5,8,11,14-tetraazatetradecanoic acid (500 mg, crude) and N,N-diisopropylethylamine (609 mg, 4.72 mmol) were added to a DMF (4.00 mL) solution of 2,5-dioxopyrrolidin-1-yl 1-(2,5-dioxo-2,5-dihydro-1H-pyrrol-1-yl)-3,6,9,12,15,18-hexaoxabenzofuran-21-carboxylate (626 mg, 1.18 mmol). After addition, the reaction mixture was stirred at room temperature for 2 hours. LC-MS indicated completion of the reaction. The mixture was concentrated to afford the crude product, which was purified by preparative high-performance liquid chromatography (0-60% acetonitrile in water) to yield a yellow oily (S)-10-benzyl-38-(2,5-dioxo-2,5-dihydro-1H-pyrrol-1-yl)-6,9,12,15,18-pentaoxo-3,21,24,27,30,33,36-heptaoxa-5,8,11,14,17-pentaazaoctadecanoic acid (90 mg, yield: 9%).

LC-MS (ESI) m/z: 861.4 $[M+Na]^+$.

$^1$H NMR (400 MHz, DMSO-ds): δ 8.51-8.57 (m, 1H), 8.26-8.29 (m, 1H), 8.12-8.15 (m, 1H), 8.08-8.10 (m, 1H), 7.96-7.99 (m, 1H), 7.15-7.27 (m, 6H), 7.01 (s, 2H), 4.61 (d, *J* = 6.8 Hz, 2H), 4.47-4.52 (m, 1H), 3.98 (s, 2H), 3.49-3.73 (m, 32H), 3.03-3.08 (m, 1H), 2.77-2.83 (m, 1H), 2.37-2.41 (m, 2H).

Example 41: Synthesis of compound L2

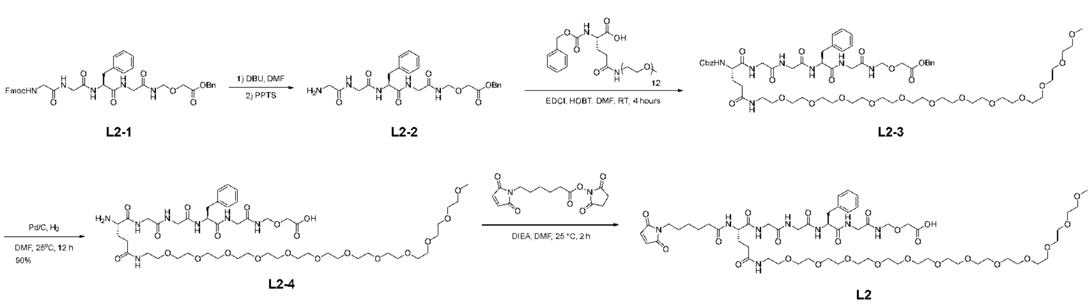


L2-1

L2-2

L2-3

L2-4

L2

Steps 1 and 2: Synthesis of L2-3

Under $N_2$ protection at room temperature, L2-1 (4.2 g, 5.7 mmol, 1.0 eq) was dissolved in anhydrous DMF (15 mL), and DBU (608 mg, 3.99 mmol, 0.7 eq) was added. The mixture was stirred at room temperature for 0.5 hour. TLC (DCM/MeOH = 20/1) was used to monitor the reaction. If starting material remained, an additional 0.2 eq of DBU was added. Upon completion, forming L2-2, 0.7-0.9 eq of 4-methylbenzenesulfonylpyridine was added.

Subsequently, 1.0 eq HOBt and 1.0 eq of (S)-42-(((benzyloxy)carbonyl)amino)-39-oxo-2,5,8,11,14,17,20,23,26,29,32,35-dodecaoxa-38-azatetratriacontane-43-oic acid, and 0.9 eq EDCI were added. The reaction was stirred at room temperature for 1-2 hours. LC-MS indicated completion of the reaction. The reaction mixture was poured into 500 mL water/500 mL DCM, and the layers were separated. The organic phase was concentrated and dissolved in DMF. The crude product was purified by reversed-phase column chromatography ($CH_3CN/H_2O$ = 10%-100%) to afford L2-3 (4 g, yield: 53% over two steps).
LC-MS (ESI) m/z: 681.6 $[M/2+Na]^+$

Step 3: Synthesis of L2-4

Compound L2-3 (4 g, 3 mmol, 1 eq) was dissolved in DMF, and a small amount of Pd/C was added. The reaction mixture was degassed and replaced with $H_2$ using a balloon, then stirred at 70°C for 3 hours. LC-MS indicated completion of the reaction. The mixture was filtered to remove Pd/C, and the filtrate was used directly in the next step.
LC-MS (ESI) m/z: 1094.5 $[M+H]^+$

Step 4: Synthesis of L2

To a DMF solution of compound L2-4 was added 2,5-dioxopyrrolidin-1-yl 6-(2,5-dioxo-2,5-dihydro-1H-pyrrol-1-yl)hexanoate (0.74 g, 2.4 mmol, 0.8 eq) and DIEA (1.17 g, 9 mmol, 3 eq). The mixture was stirred at room temperature for 1 hour. The crude product was purified by reversed-phase column chromatography ($CH_3CN/H_2O$ = 10%-50%) to afford L2 (1.56 g, yield: 40% over two steps).
LC-MS (ESI) m/z: 666.2 $[M/2+Na]^+$

Example 42: Synthesis of compound L3

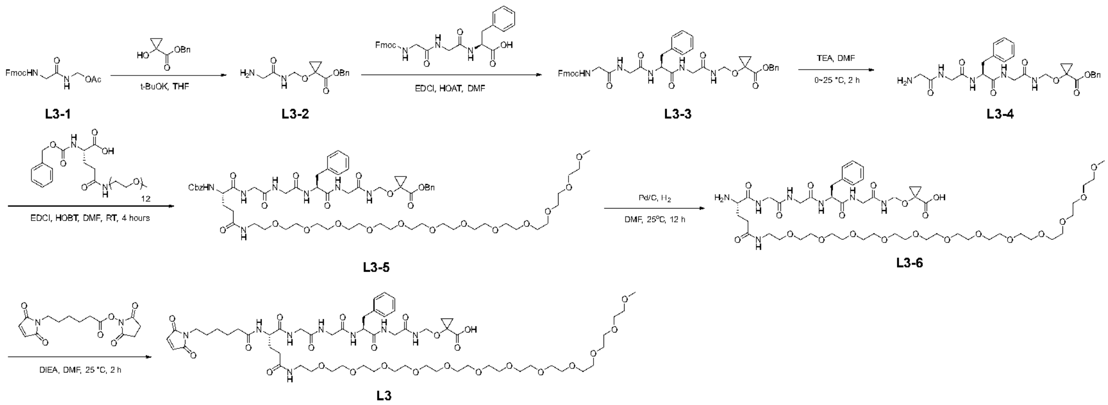


Step 1: Synthesis of 1-((2-(aminoacetyl)amino)methoxy)cyclopropane-1-carboxylic acid benzyl ester

At 0-5°C, t-BuOK (300 mg, 2.7 mmol) was added to a THF (15 mL) solution of L3-1 (500 mg, 1.4 mmol) and 1-hydroxycyclopropane-1-carboxylic acid benzyl ester (500 mg, 2.6 mmol). The reaction mixture was then allowed to warm to room temperature and stirred for 10 minutes. Ice water was added to quench the reaction, and the mixture was extracted three times with ethyl acetate. The combined organic layers were dried over anhydrous $Na_2SO_4$, filtered, and concentrated. The resulting residue was used directly in the next step.
LC-MS (ESI) m/z: 279 $[M+H]^+$

Step 2: Synthesis of (S)-1-((11-benzyl-1-(9H-fluoren-9-yl)-3,6,9,12,15-pentaoxa-2-oxo-4,7,10,13,16-pentaazaheneicos-17-yl)oxy)cyclopropane-1-carboxylic acid benzyl ester

L3-2 (920 mg, crude), (((9H-fluoren-9-yl)methoxy)carbonyl)glycylglycyl-L-phenylalanine (701 mg, 1.4 mmol), EDCI (382 mg, 2.0 mmol), and HOAT (270 mg, 2.0 mmol) were dissolved in DMF (15 mL) and stirred at room temperature overnight. LC-MS indicated formation of the target product, which was used directly in the next step without further purification.
LC-MS (ESI) m/z: 762 $[M+H]^+$

Step 3: Synthesis of benzyl (S)-1-((13-amino-7-benzyl-3,6,9,12-tetraoxa-2,5,8,11-tetraazatriacontyloxy)cyclopropane-1-carboxylate

Triethylamine (0.3 mL) was added to the reaction solution of L3-3, and the mixture was stirred overnight. LC-MS indicated formation of the target product. The reaction mixture was purified by preparative high-performance liquid chromatography to afford the target product (123 mg, three-step yield: 12%) as a white solid.
LC-MS (ESI) m/z: 540 $[M+H]^+$

Step 4: Synthesis of L3-5

L3-4 (100 mg, 1.85 mmol), (S)-42-(((benzyloxy)carbonyl)amino)-39-oxo-2,5,8,11,14,17,20,23,26,29,32,35-dodecaoxa-38-azatetratriacontane-43-oic acid (180 mg, 2.25 mmol), EDCI (95 mg, 5.55 mmol), and HOBt (65 mg, 5.55 mmol) were dissolved in DMF (0.6 mL) and stirred at room temperature overnight. LC-MS indicated formation of the target product. The reaction mixture was purified by preparative high-performance liquid chromatography to afford the target product (60 mg, yield: 40.2%) as a white solid.
LC-MS (ESI) m/z: 1344.5 $[M+H]^+$

Step 5: Synthesis of L3-6

Under $N_2$ protection, L3-5 (60 mg, 0.045 mmol) and 10% wet Pd/C (10 mg) were added to DMF (2 mL). The suspension was degassed under vacuum and repeatedly replaced with $H_2$. Under $H_2$ atmosphere, the mixture was stirred at room temperature for 4-5 hours. TLC indicated completion of the reaction, and the reaction mixture was used directly in the next step.

Step 6: Synthesis of L3

6-(Maleimide)hexanoic acid N-hydroxysuccinimide ester (20 mg, 0.065 mmol) and DIEA (0.02 mL, 0.11 mmol) were added to a DMF solution of L3-6, and the mixture was stirred at room temperature overnight. The reaction mixture was purified by preparative high-performance liquid chromatography to afford the target compound (18 mg, two-step yield: 30%).
LC-MS (ESI) m/z: 1313.3 $[M+H]^+$

Example 43: Synthesis of compound L4

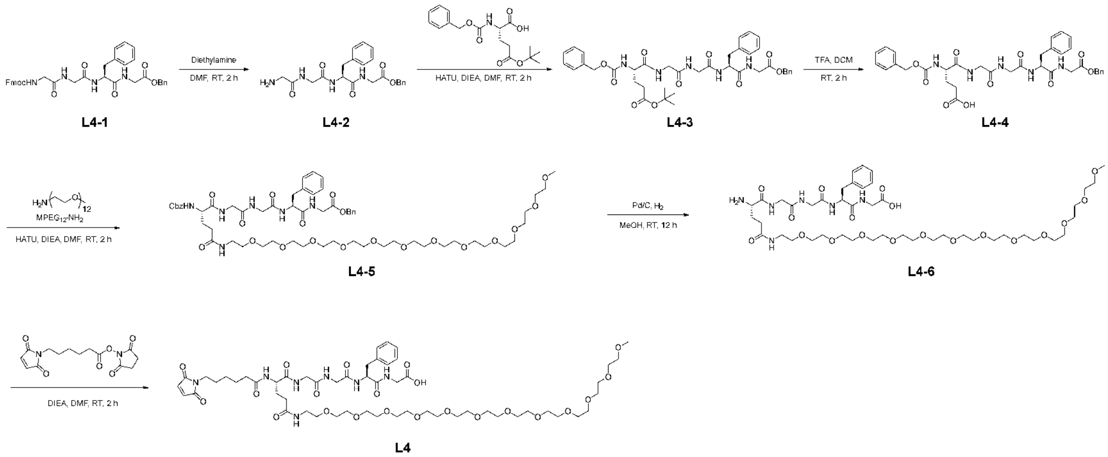


L4-1 L4-2 L4-3 L4-4 L4-5 L4-6 L4

<u>Step 1: Synthesis of (S)-benzyl 2-(2-(2-(aminoacetyl)amino)acetylamino)-3-phenylpropanamido)acetate</u>

At room temperature, diethylamine (508 mg, 6.95 mmol) was added to a DMF (20.0 mL) solution of (S)-benzyl 11-benzyl-1-(9H-fluoren-9-yl)-3,6,9,12-tetraoxo-2-oxa-4,7,10,13-tetraazapentadecan-15-oate (3.00 g, 4.63 mmol). The mixture was stirred at room temperature for 2 hours. LC-MS indicated completion of the reaction. The mixture was filtered, and the filtrate was concentrated under vacuum to yield a yellow oily (S)-benzyl 2-(2-(2-(aminoacetyl)amino) acetylamino)-3-phenylpropanamido)acetate (2.50 g, crude), which was used directly in the next step without further purification.
LC-MS (ESI) m/z: 426.9 $[M+H]^+$

<u>Step 2: Synthesis of (5S,14S)-1-benzyl 17-tert-butyl 5-benzyl-14-(((benzyloxy)carbonyl)amino)-4,7,10,13-tetraoxo-3,6,9,12-tetraazapentadecane-1,17-dioate</u>

At 0°C, (S)-2-(2-(2-(aminoacetyl)amino)acetylamino)-3-phenylpropanamido)acetate (2.50 g, crude) and HATU (4.46 g, 11.7 mmol) were dissolved in DMF (30.0 mL). To this solution were added (S)-2-(((benzyloxy)carbonyl) amino)-5-(tert-butoxy)-5-oxopentanoic acid (2.96 g, 8.78 mmol) and DIEA (3.03 g, 23.5 mmol) at 0°C. The mixture was stirred at room temperature for 2 hours, then concentrated. The crude product was purified by silica gel flash column chromatography (MeOH/DCM = 0-10%) to afford (5S,14S)-1-benzyl 17-tert-butyl 5-benzyl-14-(((benzyloxy)carbonyl) amino)-4,7,10,13-tetraoxo-3,6,9,12-tetraazapentadecane-1,17-dioate (1.20 g, yield: 27%) as a yellow solid.
LC-MS (ESI) m/z: 745.7 $[M+H]^+$

<u>Step 3: Synthesis of (7S,16S)-7-benzyl-16-(((benzyloxy)carbonyl)amino)-3,6,9,12,15-pentaoxo-1-phenyl-2-oxa-5,8,11,14-tetraazanonadecane-19-oic acid</u>

At room temperature, TFA (5.00 mL) was added to a $CH_2Cl_2$ (5.00 mL) solution of (5S,14S)-1-benzyl 17-tert-butyl 5-benzyl-14-(((benzyloxy)carbonyl)amino)-4,7,10,13-tetraoxo-3,6,9,12-tetraazapentadecane-1,17-dioate (1.20 g, 1.61 mmol). The reaction mixture was stirred at room temperature for 2 hours. LC-MS indicated completion of the reaction. The mixture was concentrated to afford (7S,16S)-7-benzyl-16-(((benzyloxy)carbonyl)amino)-3,6,9,12,15-pentaoxo-1-phenyl-2-oxa-5,8,11,14-tetraazanonadecane-19-oic acid (1.20 g, crude) as a yellow oily material, which was used directly in the next step without further purification.
LC-MS (ESI) m/z: 690.4 $[M+H]^+$

<u>Step 4: Synthesis of (42S,51S)-benzyl 51-benzyl-42-(((benzyloxy)carbonyl)amino)-39,43,46,49,52-pentaoxo-2,5,8,11,14,17,20,23,26,29,32,35-dodecaoxa-38,44,47,50,53-pentaaza-pentacosapentaenoate</u>

A solution of amino dodecanol monomethyl ether (778 mg, 1.39 mmol) and DIEA (898 mg, 6.96 mmol) was added at 0°C to a DMF (20 mL) solution of (7S,16S)-7-benzyl-16-(((benzyloxy)carbonyl)amino)-3,6,9,12,15-pentaoxo-1-phenyl-2-oxa-5,8,11,14-tetraazanonadecane-19-oic acid (1.20 g, crude) and HATU (1.32 g, 3.47 mmol). The mixture was stirred at room temperature for 2 hours, then concentrated to afford a crude product. The crude was purified by reversed-phase column chromatography ($CH_3CN/H_2O$ = 0-65%) to give (42S,51S)-benzyl 51-benzyl-42-(((benzyloxy)carbonyl) amino)-39,43,46,49,52-pentaoxo-2,5,8,11,14,17,20,23,26,29,32,35-dodecaoxa-38,44,47,50,53-pentaaza-pentacosapentaenoate (645 mg, two-step yield: 30%) as a white solid.

LC-MS (ESI) m/z: 616.3 $[M+H]^+$

Step 5: Synthesis of (42S,51S)-42-amino-51-benzyl-39,43,46,49,52-pentaoxo-2,5,8,11,14,17,20,23,26,29,32,35-dodecaoxa-38,44,47,50,53-pentaaza-pentacosane-55-oic acid

Under a nitrogen atmosphere, 10% wet Pd/C was added to a MeOH (10.0 mL) solution of benzyl ((S)-42-(((benzyloxy)carbonyl)amino)-39-oxo-2,5,8,11,14,17,20,23,26,29,32,35-dodecaoxa-38-azatricyclobutane-43-carbonyl)glycylglycyl-L-phenylalanine glycine (645 mg, 0.52 mmol). The suspension was evacuated and backfilled with $H_2$ three times, then stirred at room temperature for 12 hours. LC-MS indicated completion of the reaction. The mixture was filtered to remove Pd/C, and the filtrate was concentrated to afford (42S,51S)-42-amino-51-benzyl-39,43,46,49,52-pentaoxo-2,5,8,11,14,17,20,23,26,29,32,35-dodecaoxa-38,44,47,50,53-pentaaza-pentacosane-55-oic acid (320 mg, crude) as a yellow oily material, used directly in the next step without further purification.
LC-MS (ESI) m/z: 1006.9 $[M+H]^+$

Step 6: Synthesis of (42S,51S)-51-benzyl-42-(6-((2,5-dioxo-2,5-dihydro-1H-pyrrol-1-yl)hexanoyl)amino)-39,43,46,49,52-pentaoxo-2,5,8,11,14,17,20,23,26,29,32,35-dodecaoxa-38,44,47,50,53-pentaaza-pentacosapentaenoic acid

At 0°C, DIEA (164 mg, 1.27 mmol) was added to a DMF (4.00 mL) solution of (42S,51S)-42-amino-51-benzyl-39,43,46,49,52-pentaoxo-2,5,8,11,14,17,20,23,26,29,32,35-dodecaoxa-38,44,47,50,53-pentaaza-pentacosapentaenoic acid (320 mg, crude) and 6-(maleimido)hexanoic acid N-hydroxysuccinimide ester (147 mg, 0.48 mmol). The reaction mixture was stirred at room temperature for 2 hours. The mixture was concentrated to afford a crude product, which was purified by preparative HPLC to give (42S,51S)-51-benzyl-42-(6-((2,5-dioxo-2,5-dihydro-1H-pyrrol-1-yl)hexanoyl)amino)-39,43,46,49,52-pentaoxo-2,5,8,11,14,17,20,23,26,29,32,35-dodecaoxa-38,44,47,50,53-pentaaza-pentacosapentaenoic acid (100 mg, two-step yield: 16%) as a yellow oily material.

LC-MS (ESI) m/z: 1201.6 $[M+H]^+$

$^1H$ NMR (400 MHz, DMSO-ds): δ 8.36-8.38 (m, 1H), 8.10-8.13 (m, 2H), 8.01-8.03 (m, 2H), 7.85-7.87 (m, 1H), 7.19-7.33 (m, 5H), 7.01 (s, 2H), 4.52-4.58 (m, 1H), 4.19-4.23 (m, 1H), 3.59-3.79 (m, 44H), 3.36-3.46 (m, 9H), 3.25 (s, 3H), 3.01-3.08 (m, 1H), 2.75-2.81 (m, 1H), 2.10-2.12 (m, 5H), 1.86-1.89 (m, 2H), 1.70-1.75 (m, 1H), 1.47-1.51 (m, 5H), 1.19-1.22 (m, 2H).

Example 44: Synthesis of compound L5

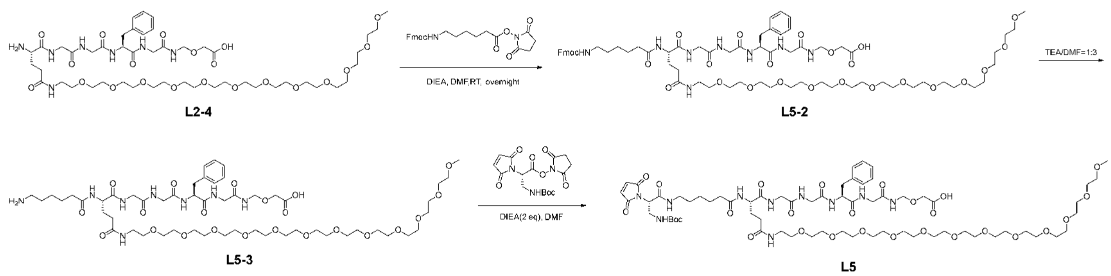

L2-4
L5-2
L5-3
L5

Step 1: Synthesis of L5-2

L2-4 (2.0 g, 1.83 mmol), 2,5-dioxopyrrolidin-1-yl 6-((((9H-fluoren-9-yl)methoxy)carbonyl)amino)hexanoate (846 mg, 1.9 mmol), and DIEA (0.63 mL, 3.7 mmol) were dissolved in DMF (10 mL) and stirred at room temperature overnight. LC-MS indicated formation of the target product, which was used directly in the next step without purification.
LC-MS (ESI) m/z: 1429.7 $[M+H]^+$

Step 2: Synthesis of L5-3

Triethylamine (3 mL) was added to the above solution, and the reaction mixture was stirred at room temperature overnight. LC-MS indicated formation of the target product. The target product was purified by preparative high-performance liquid chromatography to give L5-3 (2.0 g, yield: 87%) as an oil.
LC-MS (ESI) m/z: 1428.4 [M+H]+

Step 3: Synthesis of L5

L5-3 (1.5 g, 1.3 mmol), 2,5-dioxopyrrolidin-1-yl (S)-3-(((tert-butoxycarbonyl)amino)-2-(2,5-dihydro-2,5-dioxo-1H-pyrrol-1-yl)propanoate) (104 mg, 1.3 mmol), and DIEA (0.45 mL, 2.6 mmol) were dissolved in DMF (2 mL) and stirred at room temperature overnight. LC-MS indicated completion of the reaction. The reaction mixture was purified by preparative high-performance liquid chromatography to obtain the target compound L5 (1.07 mg, yield: 58.6%) as a white solid.
LC-MS (ESI) m/z: 1473.3 [M+H]+

Example 45: Synthesis of compound L6

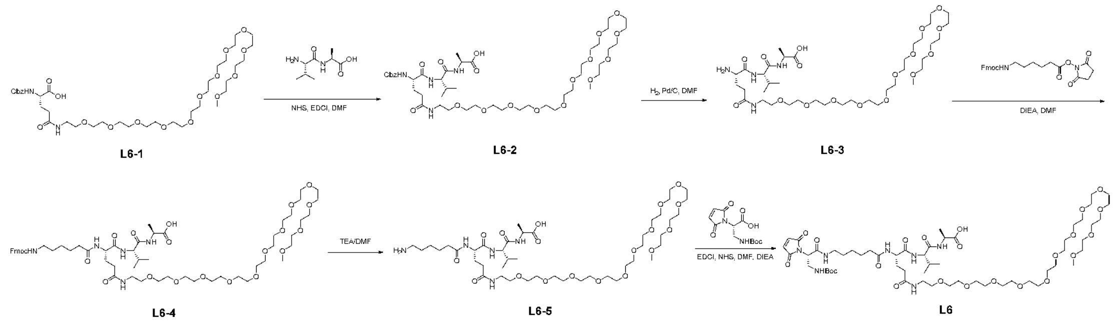


L6-1 L6-2 L6-3 L6-4 L6-5 L6

Step 1: Synthesis of L6-2

At room temperature, L6-1 (600 mg, 0.73 mmol), NHS (84 mg, 0.73 mmol), and EDCI (140 mg, 0.73 mmol) were dissolved in DMF (2 mL) and stirred for 3 hours. LC-MS indicated completion of the reaction. Valyl-alanine (135 mg, 0.72 mmol) and DIEA (0.249 mL, 1.45 mmol) were then added, and the mixture was stirred overnight. LC-MS confirmed the reaction was complete. The reaction mixture was purified by preparative high-performance liquid chromatography to afford the target product L6-2 (394 mg, yield: 54%) as a white solid.
LC-MS (ESI) m/z: 993 [M+H]+

Step 2: Synthesis of L6-3

Under $N_2$ protection, L6-3 (300 mg, 0.29 mmol) and 5% wet Pd/C (30 mg) were added to DMF (2 mL). The suspension was degassed under vacuum and repeatedly exchanged with $H_2$. The mixture was stirred at 45°C under $H_2$ overnight. LC-MS confirmed the reaction was complete. The reaction mixture was filtered and used directly in the next step.
LC-MS (ESI) m/z: 859.4 [M+H]+

Step 3: Synthesis of L6-4

L6-3 (258 mg, 0.3 mmol), 2,5-dioxopyrrolidin-1-yl 6-(((9H-fluoren-9-yl)methoxy)carbonyl)amino)hexanoate (135 mg, 0.3 mmol), and DIEA (0.105 mL, 0.6 mmol) were dissolved in DMF (2 mL) and stirred at room temperature for 3 hours. LC-MS indicated the reaction was complete. The reaction mixture was used directly in the next step.
LC-MS (ESI) m/z: 598.0 [M+H]+

Step 4: Synthesis of L6-5

Triethylamine (0.5 mL) was added to the reaction mixture of L6-4, and the mixture was stirred overnight. LC-MS indicated the reaction was complete. The reaction mixture was purified by preparative HPLC to afford the target product (112 mg, three-step yield: 33%) as an oil.
LC-MS (ESI) m/z: 972 [M+H]+

Step 5: Synthesis of L6

(S)-3-((tert-Butoxycarbonyl)amino)-2-(2,5-dioxo-2,5-dihydro-1H-pyrrol-1-yl)propanoic acid (16 mg, 0.056 mmol), EDCI (10.6 mg, 0.056 mmol), and NHS (6.5 mg, 0.056 mmol) were dissolved in DMF (0.5 mL) and stirred at room temperature for 2 hours. Then, L6-5 (50 mg, 0.051 mmol) and DIEA (0.02 mL, 0.12 mmol) were added to the reaction mixture, and the mixture was stirred at room temperature for 3 hours. LC-MS indicated the reaction was complete. The reaction mixture was purified by preparative HPLC to afford the target product (45 mg, yield: 71%) as an oil.
LC-MS (ESI) m/z: 1238.6 [M+H]$^+$

Example 46: Synthesis of compound L7

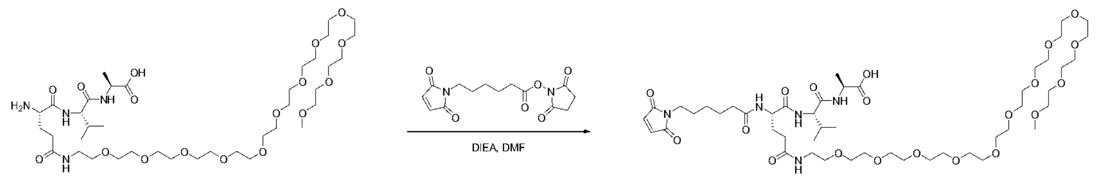


L6-3 L7

Step: Synthesis of the target compound

At room temperature, L6-3 (60 mg, 0.09 mmol), 6-(maleimido)hexanoic acid N-hydroxysuccinimide ester (23 mg, 0.07 mmol), and DIEA (0.034 mL, 0.2 mmol) were added to a DMF (2 mL) solution and stirred overnight. LC-MS indicated the reaction was complete. The reaction mixture was purified by preparative HPLC to afford the target compound (24 mg, yield: 33%) as a white solid.
LC-MS (ESI) m/z: [M/2+H]$^+$ 902.8

Synthesis and Characterization of Linker-Drug Conjugates

Example 47: Synthesis of the Compound LP1

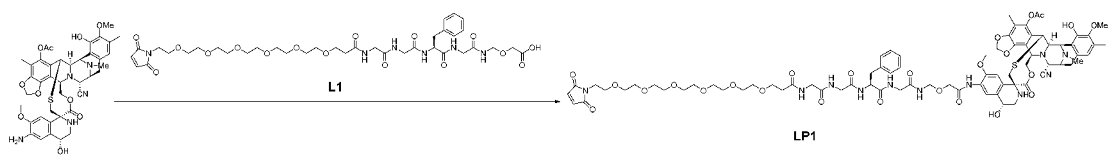


P13 LP1

P13 (30 mg, 0.038 mmol, 1 eq.), L1 (96.06 mg, 0.114 mmol, 3 eq.), HOAt (25.86 mg, 0.19 mmol, 5 eq.), and DMAP (2.32 mg, 0.019 mmol, 0.5 eq.) were dissolved in 1.5 mL of anhydrous DMF, and EDCI (19.66 mg, 0.19 mmol, 5 eq.) was added. The mixture was stirred at room temperature for 2 hours, and LC-MS indicated that the reaction was complete. The target product was obtained as a white solid (34.58 mg, yield: 56%) after purification by preparative high-performance liquid chromatography.

LC-MS (ESI) m/z: 803.4 [M/2+H]$^+$

$^1$H NMR (400 MHz, DMSO-$d_6$) δ 8.76 (s, 1H), 8.71 (s, 1H), 8.63 (t, $J$ = 6.7 Hz, 1H), 8.28 (t, $J$ = 5.7 Hz, 1H), 8.14 (t, $J$ = 5.7 Hz, 1H), 8.09 (d, $J$ = 7.9 Hz, 1H), 8.02 - 7.94 (m, 2H), 7.28 - 7.14 (m, 6H), 7.00 (s, 2H), 6.42 (d, $J$ = 10.3 Hz, 2H), 6.13 (s, 2H), 5.03 (d, $J$ = 11.6 Hz, 1H), 4.77 (d, $J$ = 7.8 Hz, 1H), 4.67 (d, $J$ = 6.8 Hz, 2H), 4.46 (s, 3H), 4.19 (d, $J$ = 4.7 Hz, 1H), 4.15 (d, $J$ = 7.4 Hz, 1H), 4.11 (s, 1H), 4.06 (d, $J$ = 11.3 Hz, 1H), 4.02 (s, 2H), 3.78 - 3.67 (m, 5H), 3.64 (s, 3H), 3.60 (d, $J$ = 6.7 Hz, 3H), 3.57 (s, 4H), 3.55 (d, $J$ = 4.9 Hz, 2H), 3.51 (d, $J$ = 5.0 Hz, 3H), 3.48 (d, $J$ = 1.7 Hz, 10H), 3.46 (s, 10H), 3.45 (d, $J$ = 2.4 Hz, 5H), 3.28 (s, 2H), 3.04 (d, $J$ = 9.6 Hz, 2H), 2.78 (dd, $J$ = 25.0, 14.5 Hz, 2H), 2.67 (s, 1H), 2.38 (t, $J$ = 6.5 Hz, 2H), 2.29 (s, 3H), 2.22 (s, 3H), 2.04 (s, 3H), 1.97 (s, 3H).

Example 48: Synthesis of the Compound LP62

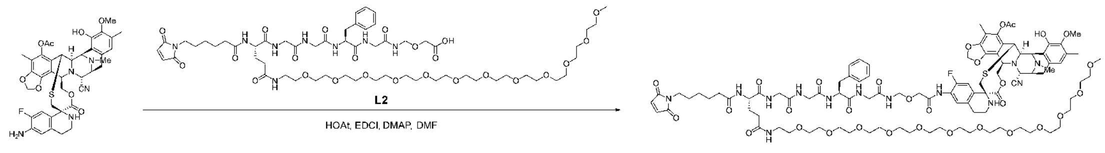


P1 LP62

Step 1: Synthesis of LP62

P1 (8 mg, 0.011 mmol), L2 (141.6 mg, 0.11 mmol), DMAP (0.68 mg, 0.0055 mmol), and HOAt (15 mg, 0.11 mmol) were added to 1 mL of anhydrous DMF, followed by the portionwise addition of EDCI (11.4 mg, 0.11 mmol). After the addition, the reaction mixture was stirred at room temperature overnight. The target product was obtained as a brown oily substance (1.4 mg, yield: 29%) after purification by preparative high-performance liquid chromatography.
LC-MS (ESI) m/z: 1014.3 $[M/2+H]^+$

Example 49: Synthesis of the Compound LP63

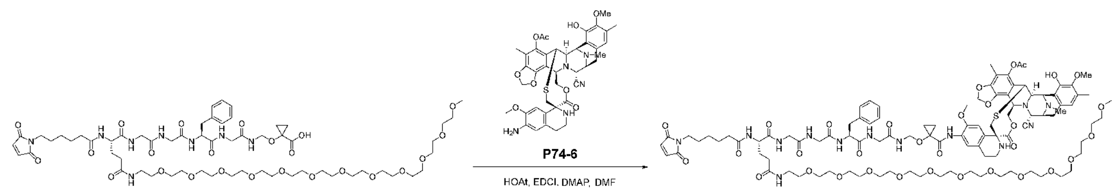


L3 LP63

L3 (18 mg, 0.014 mmol), P74-6 (8.2 mg, 0.014 mmol), DMAP (0.2 mg, 0.007 mmol), and HOAt (9 mg, 0.07 mmol) were dissolved in 200 μL of anhydrous DMF, followed by the addition of EDCI (13 mg, 0.07 mmol). The reaction mixture was stirred at room temperature overnight. LC-MS indicated the formation of the target product. The target product was obtained as a white solid (10 mg, yield: 45.5%) after purification by preparative high-performance liquid chromatography.

LC-MS (ESI) m/z: $[M/2+H]^+$ 1033.51

$^1$H NMR (400 MHz, DMSO-$d_6$) δ 9.14 (s, 2H), 8.75-8.70 (m, 1H), 8.47 (s, 1H), 8.32 (s, 1H), 8.13 (s, 2H), 8.09 (s, 1H), 8.09 (s, 1H), 7.98 (s, 1H), 7.85 (s, 1H), 7.59 (s, 1H), 7.26-7.17 (m, 6H), 6.98 (s, 2H), 6.44 (s, 2H), 6.11 (s, 2H), 5.05 (d, *J* = 12 Hz, 1H), 4.51 (s, 2H), 4.49 (s, 3H), 4.21 (s, 2H), 4.11-4.04 (m, 2H), 3.71-3.64 (m, 12H), 3.57-3.50 (m, 54H), 3.43-3.36 (m, 8H), 3.23 (s, 3H), 3.20-3.15 (m, 3H), 3.04-2.99 (m, 2H), 2.37 (s, 3H), 2.33 (s, 3H), 2.21-2.05 (m, 9H), 1.96 (s, 3H), 1.95-1.86 (m, 2H), 1.49 (s, 4H), 1.21-1.14 (m, 6H).

Example 50: Synthesis of the Compound LP64

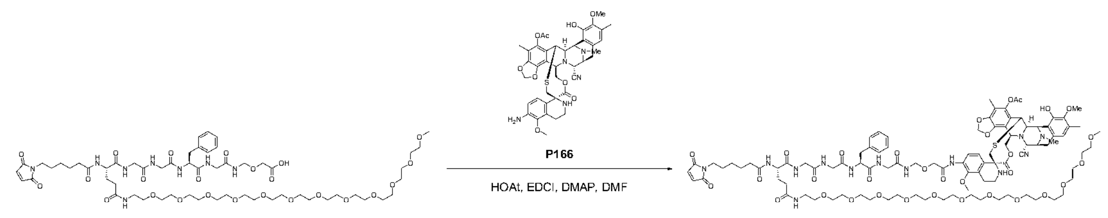


L2 LP64

P166 (25 mg, 0.032 mmol), L2 (125.41 mg, 0.097 mmol), DMAP (7 mg, 0.006 mmol), and HOAt (43.55 mg, 0.32 mmol) were dissolved in 1 mL of DMF, followed by the portionwise addition of EDCI (61.34 mg, 0.32 mmol). After the

addition, the reaction mixture was stirred at room temperature overnight. LC-MS indicated the formation of the target product. The reaction mixture was purified by preparative high-performance liquid chromatography to afford the target product LP64 (18.5 mg, yield: 27.9%) as a white solid.

LC-MS (ESI) m/z: $[M/2+H]^+$ 1020

$^1$H NMR (400 MHz, DMSO-$d_6$) δ 8.87 (s, 1H), 8.75 (s, 1H), 8.68 (t, *J* = 6.7 Hz, 1H), 8.32 (m, 2H), 8.12 (m, 2H), 8.07-7.96 (m, 2H), 7.85 (t, *J* = 5.7 Hz, 1H), 7.77 (d, *J* = 8.8 Hz, 1H), 7.29-7.12 (m, 5H), 6.99 (s, 2H), 6.73 (d, *J* = 8.9 Hz, 1H), 6.66 (brs, 1H), 6.47 (s, 1H), 6.26 (s, 1H), 6.18 (s, 1H), 5.33 (t, *J* = 4.9 Hz, 1H), 5.07 (d, *J* = 11.5 Hz, 1H), 4.68 (d, *J* = 6.6 Hz, 2H), 4.54-4.41 (m, 3H), 4.24-4.13 (m, 2H), 4.13-3.97 (m, 4H), 3.71 (m, 6H), 3.64 (s, 3H), 3.60 (s, 3H), 3.51 (d, *J* = 1.4 Hz, 48H), 3.45-3.38 (m, 8H), 3.24 (s, 3H), 3.17 (m, 3H), 3.14-2.99 (m, 3H), 2.80 (m, 3H), 2.42-2.32 (m, 2H), 2.28 (s, 3H), 2.24 (s, 3H), 2.10 (t, *J* = 7.8 Hz, 5H), 2.05 (s, 3H), 2.02 (d, *J* = 7.4 Hz, 2H), 1.99 (s, 3H), 1.88 (m, 1H), 1.72 (m, 2H), 1.53-1.38 (m, 6H).

Example 51: Synthesis of the Compound LP65

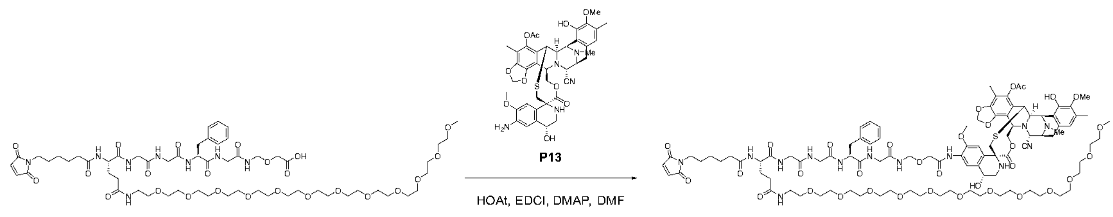


L2 LP65

P13 (13 mg, 0.016 mmol, 1.0 eq), L2 (63.89 mg, 0.049 mmol, 3.0 eq), DMAP (0.97 mg, 0.008 mmol, 0.5 eq), and HOAt (21.77 mg, 0.16 mmol, 10 eq) were dissolved in 1 mL of DMF, followed by the addition of EDCI (16.56 mg, 0.16 mmol, 10 eq). After the addition, the reaction mixture was stirred at room temperature overnight. The reaction mixture was purified by preparative high-performance liquid chromatography to afford the target product (1.3 mg, yield: 3.7%) as a white solid.

LC-MS (ESI) m/z: 1028 $[M/2+H]^+$

$^1$H NMR (400 MHz, DMSO-$d_6$) δ 8.78 (s, 1H), 8.74 (s, 1H), 8.16 (m, 3H), 8.02 (m, 2H), 7.88 (s, 1H), 7.26 (m, 5H), 7.20 (s, 1H), 7.01 (s, 2H), 6.47 (s, 1H), 6.40 (s, 1H), 6.16 (s, 2H), 5.20 (m, 1H), 5.07 (m, 1H), 4.69 (m, 2H), 4.52 (m, 3H), 4.21 (m, 3H), 4.13 (s, 1H), 3.73 (s, 3H), 3.65 (s, 3H), 3.59 (s, 2H), 3.52 (m, 44H), 3.44 (m, 2H), 3.26 (m, 2H), 3.19 (m, 4H), 2.91 (m, 3H), 2.81 (m, 5H), 2.75 (m, 2H), 2.69 (m, 1H), 2.35 (m, 2H), 2.31 (m, 3H), 2.24 (s, 3H), 2.12 (t, 3H), 2.07 (s, 2H), 1.99 (s, 3H), 1.48(m, 6H).

Example 52: Synthesis of the Compound LP66

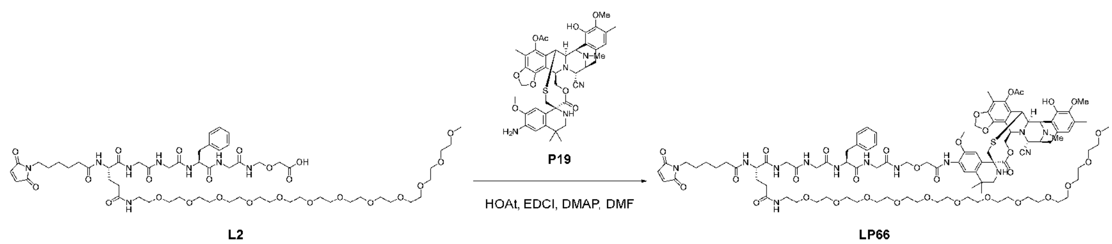


L2 LP66

L2 (18 mg, 0.014 mmol), P19 (11 mg, 0.014 mmol), DMAP (0.8 mg, 0.007 mmol), and HOAt (9 mg, 0.07 mmol) were dissolved in 400 μL of anhydrous DMF, followed by the addition of EDCI (13 mg, 0.07 mmol). The reaction mixture was stirred at room temperature overnight. LC-MS indicated the formation of the target product. The target product was obtained as a white solid (12 mg, yield: 41.5%) after purification by preparative high-performance liquid chromatography.

LC-MS (ESI) m/z: 1034.14 $[M/2+H]^+$

$^1H$ NMR (400 MHz, DMSO-$d_6$) δ 8.83 (s, 2H), 8.69 (s, 1H), 8.37 (s, 1H), 8.16 (s, 2H), 8.05 (s, 2H), 8.02 (s, 1H), 7.88 (s, 1H), 7.25-7.22 (m, 4H), 7.19-7.17 (m, 1H), 7.00 (s, 2H), 6.50 (s, 1H), 6.25 (s, 1H), 6.19-6.17 (m, 1H), 5.06 (s, 1H), 4.68 (s, 1H), 4.58 (s, 1H), 4.50 (s, 1H), 4.26 (s, 1H), 4.20 (s, 2H), 4.10-4.08 (m, 2H), 4.04 (s, 2H), 3.64 (s, 5H), 3.56 (s, 4H), 3.50 (s, 45H), 3.43-3.33 (m, 10H), 2.24 (s, 3H), 3.19-2.77 (m, 7H), 2.32 (s, 3H), 2.23 (s, 3H), 2.11-2.06 (m, 7H), 1.98 (s, 3H), 1.48-1.45 (m, 2H), 1.45 (s, 4H), 1.24-1.08 (m, 10H).

Example 53: Synthesis of the Compound LP67

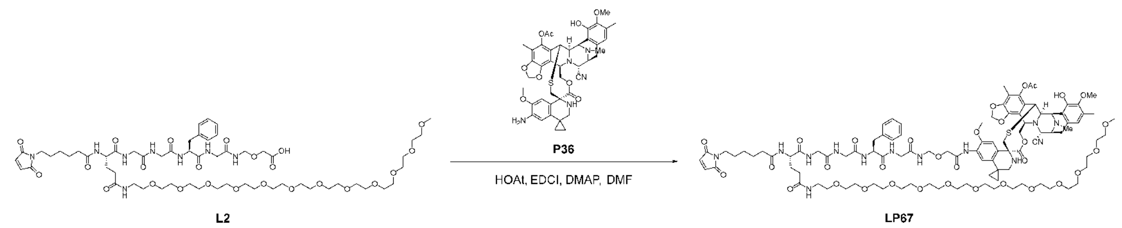


A mixture of P36 (18 mg, 0.022 mmol), L2 (29 mg, 0.014 mmol), DMAP (0.8 mg, 0.007 mmol), and HOAt (9 mg, 0.07 mmol) was added to 0.6 mL of anhydrous DMF, followed by the portionwise addition of EDCI (13 mg, 0.07 mmol). The reaction mixture was stirred at room temperature overnight. LC-MS indicated the formation of the target product. The reaction mixture was purified by preparative high-performance liquid chromatography to afford the target product (14 mg, yield: 30%) as a white solid.

LC-MS (ESI) m/z: $[M/2+H]^+$ 1033.3

$^1H$ NMR (400 MHz, DMSO-$d_6$) δ 8.86 (s, 2H), 8.67-8.70 (m, 1H), 8.34-8.40 (m, 1H), 8.12-8.15 (m, 1H), 8.00-8.05 (m, 2H), 7.85-7.87 (m, 1H), 7.27-7.19 (m, 5H), 7.01 (s, 2H), 6.51 (s, 1H), 6.29 (s, 1H), 6.23 (s, 1H), 6.19 (s, 1H), 5.20-5.22 (m, 1H), 4.68 (d, *J* = 8 Hz, 1H), 4.59 (s, 1H), 4.50-4.55 (m, 1H), 4.19-4.27 (m, 4H), 4.04 (s, 2H), 3.77-3.52 (m, 61H), 3.45-3.36 (m, 9H), 3.26 (s, 3H), 3.22-3.19 (m, 2H), 3.06-3.03 (m, 1H), 2.84-2.69 (m, 4H), 2.35 (s, 3H), 2.24 (s, 3H), 2.14-2.10 (m, 4H), 2.04-2.00 (m, 6H), 1.95-1.86 (m, 2H), 1.52-1.46 (m, 4H), 1.26-1.19 (m, 4H), 1.03 (s, 2H).

Example 54: Synthesis of the Compound LP68

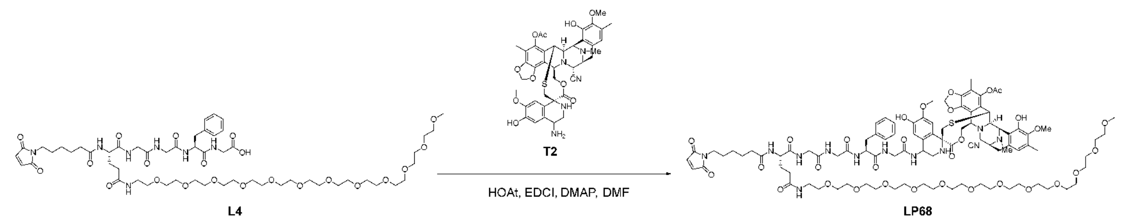


L4 (3 mg, 0.0025 mmol), T2 (2 mg, 0.0025 mmol), DMAP (0.14 mg, 0.0012 mmol), and HOAt (4.5 mg, 0.035 mmol) were dissolved in 0.40 mL of anhydrous DMF, followed by the addition of EDCI (6.5 mg, 0.035 mmol). The reaction mixture was stirred at room temperature overnight. The target product was obtained as a white solid (0.27 mg, yield: 5.4%) after purification by preparative high-performance liquid chromatography.
LC-MS (ESI) m/z: 984.67 $[M/2+H]^+$

Example 55: Synthesis of the Compound LP69

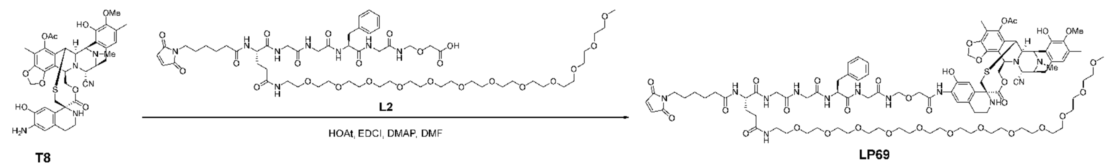


T8 LP69

Step 1: Synthesis of LP69

At room temperature, a mixture of T8 (3 mg, 0.004 mmol), L2 (5 mg, 0.004 mmol), EDCI (7 mg, 0.04 mmol), HOAt (5 mg, 0.04 mmol), and DMAP (0.3 mg, 0.002 mmol) was dissolved in 0.4 mL of anhydrous DMF and stirred for 2 hours. LC-MS indicated the formation of the target product. The target product was obtained as a white solid (1.1 mg, yield: 13.7%) after purification by preparative high-performance liquid chromatography.
LC-MS (ESI) m/z: 1012.8 $[M/2+H]^+$

Example 56: Synthesis of the Compound LP70

Step 1: Synthesis of LP70

EDCI (2.4 mg, 0.0125 mmol) was added to a solution of P241 (2 mg, 0.0025 mmol), L2 (5 mg, 0.0038 mmol), HOAt (1.7 mg, 0.0125 mmol), and DMAP (0.15 mg, 0.0012 mmol) in 0.4 mL of anhydrous DMF. The mixture was stirred at room temperature overnight. LC-MS indicated the reaction was complete. The target product was obtained as a white solid (0.3 mg, yield: 5.7%) after purification by preparative high-performance liquid chromatography.
LC-MS (ESI) m/z: 1032.8 $[M/2+H]^+$

Example 57: Synthesis of the Compound LP71

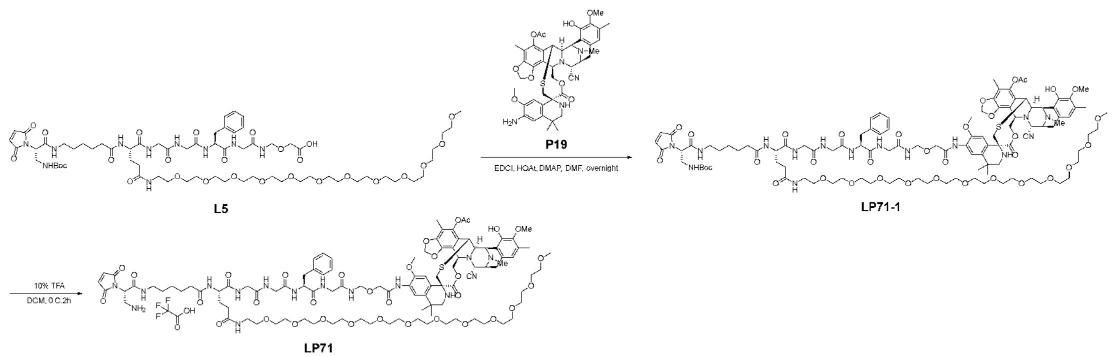


L5 LP71-1 LP71

Step 1: Synthesis of LP71-1

At room temperature, a solution of L5 (28 mg, 0.019 mmol), P19 (14 mg, 0.017 mmol), EDCI (16 mg, 0.085 mmol), DMAP (1 mg, 0.009 mmol), and HOAt (11 mg, 0.085 mmol) in 0.4 mL of DMF was stirred for 2 hours. LC-MS indicated the formation of the target product. The target compound was obtained as a white solid (16 mg, yield: 41%) after purification by preparative high-performance liquid chromatography.
LC-MS (ESI) m/z: 1127.4 $[M/2+H]^+$

Step 2: Synthesis of LP71

LP71-1 (14 mg, 0.006 mmol) was dissolved in 10% TFA/DCM (4 mL) and stirred at 0°C for 3 hours. TLC indicated the reaction was complete, and DMF (2 mL) was added. The mixture was concentrated to remove TFA and dichloromethane, and the residue was purified by preparative high-performance liquid chromatography to afford the target product (6.3 mg, yield: 46%) as a white solid.
LC-MS (ESI) m/z: 1077.4 $[M/2+H]^+$

Example 58: Synthesis of the Compound LP72

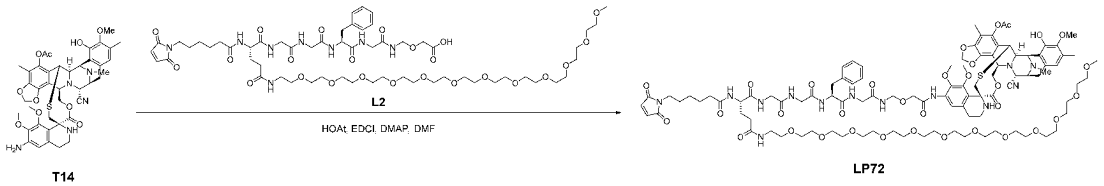


T14 L2 LP72

EDCI (23 mg, 0.06 mmol) was added to a solution of T14 (10 mg, 0.012 mmol), L2 (38.6 mg, 0.03 mmol), HOAt (16.3 mg, 0.06 mmol), and DMAP (0.73 mg, 0.006 mmol) in 2 mL of anhydrous DMF. The reaction mixture was stirred at 40°C for 1.5 hours. The target compound was obtained as a white solid (1.50 mg, yield: 11.5%) after purification by preparative high-performance liquid chromatography.
LC-MS (ESI) m/z: $[M/2+H]^+$ 1035.4

Example 59: Synthesis of the Compound LP73

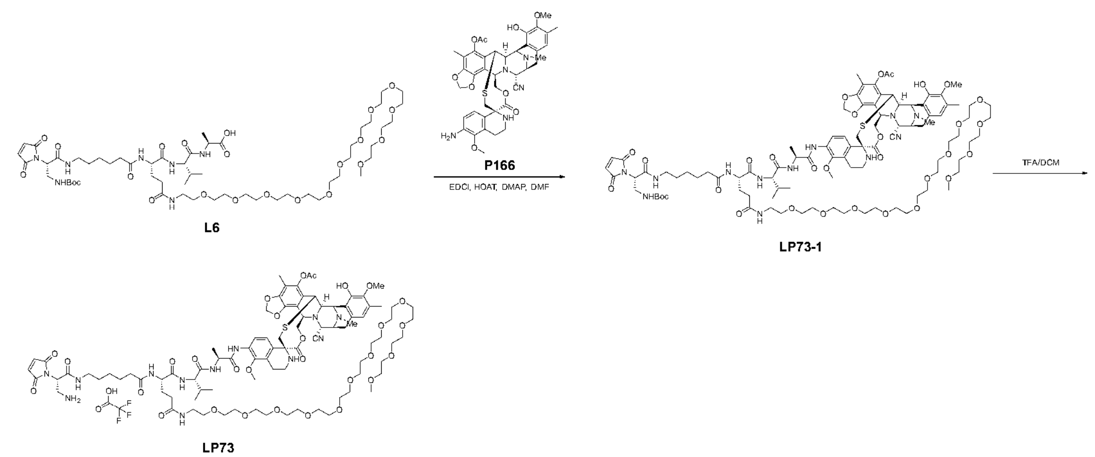


L6 P166 LP73-1 LP73

Step 1: Synthesis of LP73-1

L6 (16 mg, 0.013 mmol), P166 (10 mg, 0.015 mmol), EDCI (12 mg, 0.065 mmol), DMAP (0.8 mg, 0.007 mmol), and HOAt (9 mg, 0.056 mmol) were added to 0.8 mL of DMF, and the mixture was stirred at room temperature for 2 hours. LC-MS indicated the formation of the target product. The target product was obtained as a white solid (3 mg, yield: 11.6%) after purification by preparative high-performance liquid chromatography.
LC-MS (ESI) m/z: 995.5 $[1/2M+H]^+$

Step 2: Synthesis of LP73

A solution of LP73-1 (3 mg, 0.006 mmol) in 10% TFA/DCM (1 mL) was stirred at 0°C for 3 hours. TLC indicated the reaction was complete, and DMF (2 mL) was added. The mixture was concentrated to remove TFA and dichloromethane, and the residue was purified by preparative high-performance liquid chromatography to afford the target product (2.6 mg, yield: 83%) as a white solid.
LC-MS (ESI) m/z: 946.2 $[M/2+H]^+$

Example 60: Synthesis of the Compound LP74

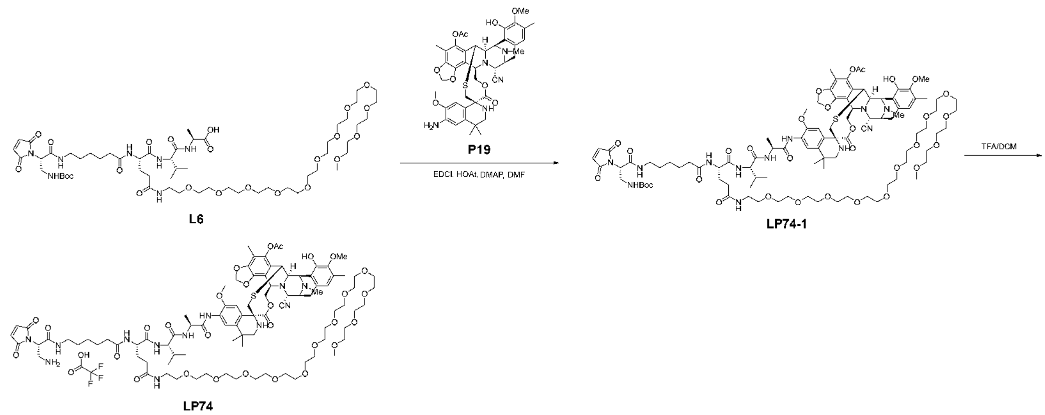


Step 1: Synthesis of LP74-1

L6 (16 mg, 0.013 mmol), P19 (10 mg, 0.013 mmol), EDCI (12 mg, 0.065 mmol), DMAP (0.8 mg, 0.007 mmol), and HOAt (9 mg, 0.056 mmol) were dissolved in 0.8 mL of DMF and stirred at room temperature for 2 hours. LC-MS indicated the reaction was complete. The target product was obtained as a white solid (6 mg, yield: 23%) after purification by preparative high-performance liquid chromatography.
LC-MS (ESI) m/z: 993.6 $[M/2+H]^+$

Step 2: Synthesis of LP74

A solution of LP74-1 (6 mg, 0.0019 mmol) in 10% TFA/DCM (1 mL) was stirred at 0°C for 3 hours. TLC indicated the reaction was complete, and DMF (2 mL) was added. The mixture was concentrated to remove TFA and dichloromethane, and the residue was purified by preparative high-performance liquid chromatography to afford the target product (1.4 mg, yield: 22%) as a white solid.
LC-MS (ESI) m/z: 960.2 $[M/2+H]^+$

Example 61: Synthesis of the Compound LP75

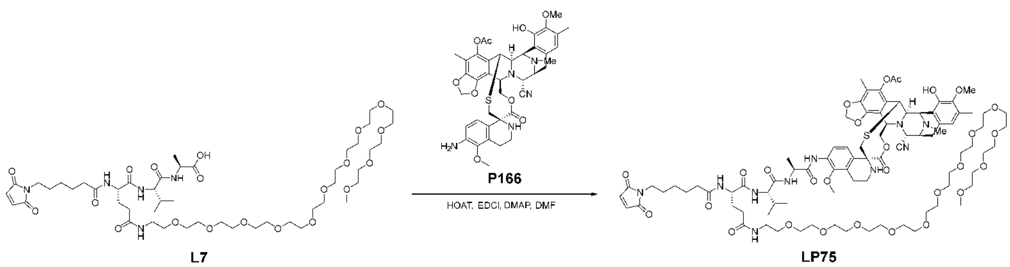


At room temperature, a solution of L7 (10 mg, 0.01 mmol), P166 (8 mg, 0.01 mmol), EDCI (16 mg, 0.085 mmol), DMAP (1 mg, 0.009 mmol), and HOAt (11 mg, 0.08 mmol) in 0.4 mL of DMF was stirred for 2 hours. LC-MS indicated the reaction was complete. The reaction mixture was purified by preparative high-performance liquid chromatography to afford the target product (1.7 mg, yield: 8.5%) as a white solid.
LC-MS (ESI) m/z: $[M/2+H]^+$ 1052.5

Example 62: Synthesis of the Compound LP76

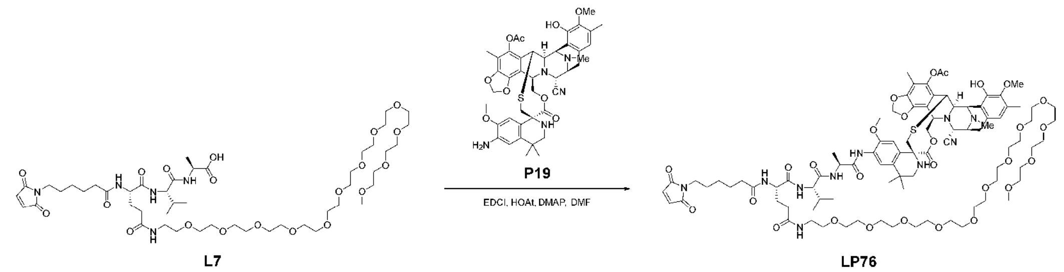


At room temperature, a solution of L7 (10 mg, 0.01 mmol), P19 (8 mg, 0.01 mmol), EDCI (16 mg, 0.085 mmol), DMAP (1 mg, 0.009 mmol), and HOAt (11 mg, 0.08 mmol) in 0.4 mL of DMF was stirred for 2 hours. LC-MS indicated the reaction was complete. The reaction mixture was purified by preparative high-performance liquid chromatography to afford the target product (3.4 mg, yield: 20%) as a white solid.
LC-MS (ESI) m/z: $[M/2+H]^+$ 916.9

Example 63: Synthesis of the Compound LP77

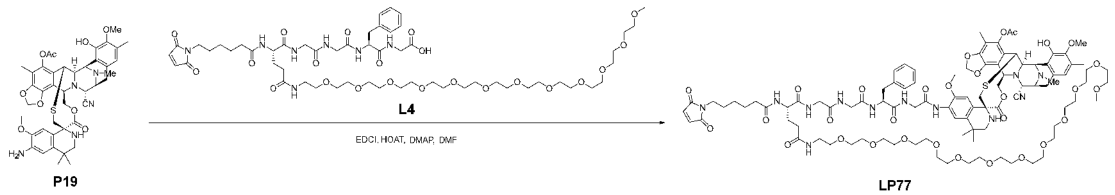


L4 (2 mg, 0.0017 mmol), P19 (1.5 mg, 0.0017 mmol), EDCI (1.6 mg, 0.008 mmol), DMAP (0.1 mg, 0.009 mmol), and HOAt (1.1 mg, 0.008 mmol) were added to 0.4 mL of anhydrous DMF, and the reaction mixture was stirred at room temperature for 2 hours. LC-MS indicated the reaction was complete. The target product was obtained as a white solid (0.57 mg, yield: 17%) after purification by preparative high-performance liquid chromatography.
LC-MS (ESI) m/z: 1981.6 $[M+H]^+$

Example 64: Synthesis of the Compound LP78

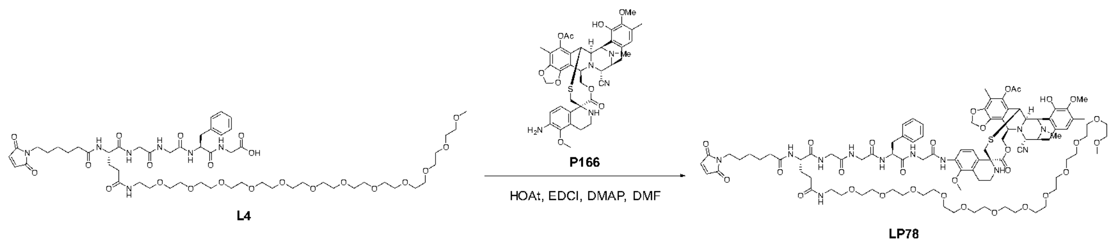


Step: Synthesis of LP78

L4 (33 mg, 0.03 mmol), P166 (15 mg, 0.03 mmol), EDCI (30 mg, 0.15 mmol), DMAP (1.8 mg, 0.015 mmol), and HOAt (21 mg, 0.15 mmol) were added to 0.4 mL of DMF and stirred at room temperature for 2 hours. LC-MS indicated the formation of the target product. The reaction mixture was purified by preparative high-performance liquid chromatography to afford the target product (14.7 mg, yield: 38%) as a white solid.

LC-MS (ESI) m/z: 1953.6 $[M+H]^+$

$^1$H NMR (400 MHz, DMSO-$d_6$) δ 9.5 (s, 1H), 8.95(s, 1H), 8.41-8.38 (m, 1H),8.06-8.02(m, 2H), 7.95-7.90(m, 2H), 7.79-7.76(m, 1H), 7.71(s, 1H), 7.16-7.14(m, 4H), 7.11-7.08(s, 1H), 6.92(s, 2H), 6.7(s, 1H), 6.45(s, 1H), 6.19(s, 1H), 5.13(s, 1H), 4.49-4.44(m, 3H), 4.19-4.08(m, 4H), 3.86 (d, $J$ = 1.0 Hz, 2H), 3.68-3.53(m, 13H), 3.46-3.42(m, 46H),

3.31-3.27(m, 3H), 3.17(s, 3H), 3.12-3.08(m, 3H), 3.30-2.95(m, 2H), 2.80-2.59(m, 6H), 2.22-2.21(m, 6H), 2.04-1.98(m, 7H), 1.91 (s, 3H),1.84-1.76(m, 1H), 1.68-1.61 (m, 1H), 1.43-1.35(m, 4H), 1.17-1.08(m, 3H).

Preparation and Characterization of Antibody-Drug Conjugates

The following are exemplary methods for the preparation and analytical characterization of antibody-drug conjugates. It should be noted that the following examples are merely some embodiments and are not limited to the embodiments described herein. For example, the antibody is not limited to the antibodies shown here and may be any other antibody.

General Conjugation Procedure for Preparing Antibody-Drug Conjugates Based on Novel Ecteinascidin Derivatives

The monoclonal antibody (1.0 eq.) was placed in a 0.5 mL centrifuge tube, and 25 mM $Na_2B_4O_7$, 25 mM NaCl, 1 mM DTPA buffer was added to dilute the antibody concentration to 5-10 mg/mL. After mixing by vortexing, a 2 mg/mL TCEP aqueous solution (5-8 eq.) was added to reduce the antibody. After vortexing, the reaction mixture was placed on a thermoregulated shaker and reduced at 20-25°C for 2-3 hours. An equal volume of 500 mM Tris, pH 8.8, or another suitable buffer was then added, followed by a toxin-linker solution dissolved in DMSO at 10 mg/mL (5-8 eq.). DMSO was supplemented to 10-20% of the total final reaction volume. After vortexing, the mixture was incubated on a thermoregulated shaker at 25°C for 16-24 hours to effect conjugation.

Residual toxin-linker not conjugated to the antibody in the reaction mixture was removed using dextran-coated activated carbon (300 mg/mL, supplier: Sigma). A 10% (v/v) activated carbon solution was added to the reaction mixture, mixed by vortexing, and incubated at 4°C with shaking for 2 hours. The supernatant was collected to measure the residual toxin-linker content. If the free toxin-linker content exceeded 1%, the above procedure was repeated multiple times until the measured levels met the standard (typically, three treatments are sufficient). After treatment, the mixture was centrifuged, and the supernatant was collected through a syringe and hydrophilic membrane filter to remove the activated carbon. The buffer of the desired antibody-drug conjugate was exchanged to a suitable storage buffer through four rounds of ultrafiltration and stored at -80°C.

General Characterization Methods for Antibody-Drug Conjugates

(a) HIC-HPLC Analysis of ADC DAR Value

High-Performance Liquid Chromatography System: e2695 HPLC system or Agilent 1260 HPLC system.

Chromatographic Column: MabPac™ HIC-Butyl 5 μm, 4.6 × 100 mm (supplier: Thermo).

Mobile phase: Mobile phase A (MPA): 1.5 M (NH4)2SO4 + 50 mM potassium phosphate (pH 7.0); mobile phase B (MPB): 50 mM sodium phosphate (pH 7.0)/isopropanol (75:25 V/V); elution was carried out according to the following gradient program (5%-95%), where 0-2 min, the volume of mobile phase A was 100%-95% and the volume of mobile phase B was 0%-5%; 2-22 min, the volume of mobile phase A was 95%-5% and the volume of mobile phase B was 5%-95%; 22-24 min, the volume of mobile phase A was 5%-0% and the volume of mobile phase B was 95%-100%; 24-26 min, the volume of mobile phase A was 0%-100% and the volume of mobile phase B was 100%-0%; 26-30 min, the volume of mobile phase A was 100%-100% and the volume of mobile phase B was 0%-0%.

Detection conditions: The flow rate of the mobile phase was set at 1 mL/min, the detection wavelength was 280 nm, and the column temperature was 30°C.

Experimental procedure: Take 50 μg of the conjugated sample (volume determined according to the sample concentration), inject it into the high-performance liquid chromatography system, elute using the above-described gradient program, and record the chromatogram.

$$\text{DAR calculation formula: DAR} = \Sigma(\text{relative peak area} \times \text{number of loaded drugs})/100$$

(b) C18-HPLC analysis of Free Linker-Drug (mol/mol%)

High-performance liquid chromatography system: e2695 high-performance liquid chromatography system.

Column: XBridge® C18 3.5 μm 4.6 × 150 mm (manufacturer: Waters).

Mobile phase: Mobile phase A (MPA): 0.1% TFA-$H_2O$; mobile phase B (MPB): 0.1% TFA-ACN; elution was carried out according to the following gradient program (5%-95%), where 0-30 min, the volume of mobile phase A was

90%-20% and the volume of mobile phase B was 10%-80%; 30-31 min, the volume of mobile phase A was 20%-90% and the volume of mobile phase B was 80%-10%; 31-35 min, the volume of mobile phase A was 90%-90% and the volume of mobile phase B was 10%-10%.

Detection conditions: The flow rate of the mobile phase was set at 0.5 mL/min, the detection wavelength was 254 nm, and the column temperature was 30°C.

Reagent I preparation example: Measure 30 mL of anhydrous methanol and 50 mL of acetonitrile, weigh 10 g of sodium chloride, mix in a container, stir at room temperature for more than 1 hour, then let stand for 1 hour. Collect the supernatant, filter through a 0.22 μm organic membrane, and store at room temperature; shelf life: 3 months.

Reagent II preparation example: Measure 100 mL of reagent I, 15 mL of DMSO, and 85 mL of ADC sample storage buffer, mix uniformly in a container, and store at room temperature; shelf life: 2 months.

Experimental procedure:

Sample solution preparation:

1) Free toxin reference: Take the toxin corresponding to the test article as the reference, and prepare it with the above reagent II to a final concentration of 1.0 mg/mL.

2) Take 10 μL of the 1.0 mg/mL toxin reference prepared above, add it to 90 μL of reagent II to prepare a concentration of 100 μg/mL; then, according to the following table, serially dilute the sample with reagent II to prepare samples at the required concentrations.

| Sample No. | Concentration of sample to be taken | Volume of sample to be taken | Volume of reagent II to be taken | Required concentration for sample preparation |
|---|---|---|---|---|
| 1 | 100 μg/ml | 40 μl | 360 μl | 10 μg/ml |
| 2 | 10 μg/ml | 180 μl | 180 μl | 5.0 μg/ml |
| 3 | 5.0 μg/ml | 120 μl | 180 μl | 2.0 μg/ml |
| 4 | 2.0 μg/ml | 180 μl | 180 μl | 1.0 μg/ml |
| 5 | 1.0 μg/ml | 180 μl | 180 μl | 0.5 μg/ml |
| 6 | 0.5 μg/ml | 120 μl | 180 μl | 0.2 μg/ml |
| 7 | 0.2 μg/ml | 180 μl | 180 μl | 0.1 μg/ml |
| Note: Inject the samples prepared at the required concentrations into the HPLC in ascending order of concentration. | | | | |

Sample testing: Take 85 μg of the conjugated sample, mix with 3 μL of DMSO for 5 min, then add 60 μL of reagent I to the sample system, mix for 5-10 min, centrifuge at 2000 rpm for 2 min, collect 20 μL of the supernatant, inject into the high-performance liquid chromatography system, elute using the above-described gradient program, and record the chromatogram.

Based on the calculated standard linear regression equation, the content of free toxin in the ADC was determined:

$$\text{Free Drug (mol/mol\%)} = \text{residual small molecule molar concentration / antibody molar concentration} \times 100.$$

(c) Determination of free linker-drug content by LC-MS/MS method

Internal standard compounds: 50 ng/mL tolbutamide, labetalol, etofesalamide, and butylspirocyclohexanone.

Extraction solvent: ethyl acetate.

Standard solution serial dilution: Dilute the standard solutions with 50% methanol-water to 20,000, 16,000, 10,000, 4,000, 1,000, 200, 40, and 20 ng/mL.

Sample pretreatment before LC-MS/MS analysis: Take 20 μL of the sample, add 20 μL of blank buffer and 240 μL of extraction solvent. Vortex mix and allow to stand until phase separation occurs. Take 160 μL of the supernatant into a 96-well plate and evaporate to dryness. Add 200 μL of 50% acetonitrile-water, mix uniformly, and then analyze by LC-MS/MS.

Standard solution pretreatment before LC-MS/MS analysis: Take 2 μL of the working solution, add it to 38 μL of blank buffer, then add 240 μL of extraction solvent. Vortex to mix and allow to stand until phase separation occurs. Take 160 μL of the supernatant into a 96-well plate and evaporate to dryness. Add 200 μL of 50% acetonitrile-water and mix uniformly.

Mass spectrometry conditions:

| Liquid chromatography-mass spectrometry system | LC-MS/MS (API6500+ ABSciex ExionAC: SH-LCMS-005) | | | |
|---|---|---|---|---|
| Liquid Chromatography Method | | | | |
| Mobile phase A | 0.1%FA & 5mM NH4FA in Water | | | |
| Mobile phase B | 0.5%FA in ACN | | | |
| Chromatographic column | ZORBAX Eclipse XDB-C18 2.1×50mm 3.5μm | | | |
| Liquid chromatography | AB Sciex | | | |
| Automatic sampler | AB Sciex | | | |
| Loading volume (μL) | 20 | | | |
| Elution methods: | Time (minutes) | Flow rate (mL/min) | Mobile phase A (%) | Mobile phase B (%) |
| | 0 | 0.600 | 70 | 30 |
| | 0.30 | 0.600 | 70 | 30 |
| | 2.00 | 0.600 | 5 | 95 |
| | 2.50 | 0.600 | 5 | 95 |
| | 2.51 | 0.600 | 70 | 30 |
| | 3.00 | 0.600 | 70 | 30 |
| Mass spectrometry Method | | | | |
| Ion source | ESI | | | |
| Scanning | MRM | | | |
| Polarization | Positive | | | |
| Mass spectrometry parameters | CUR (psi) | | | 40 |
| | CAD | | | 8 |
| | IS (V) | | | 5500 |
| | TEM (°C) | | | 500 |
| | GS1 (psi) | | | 50 |
| | GS2 (psi) | | | 50 |
| | EP (V) | | | 10 |
| | CXP (V) | | | 6 |

Data processing: A standard curve was generated based on the concentrations of the standard solutions and the corresponding mass spectrometry responses. Using the linear fitting curve (equation) and the mass spectrometry response of the test samples, the residual amount of free linker-drug was calculated.

(d) SEC-HPLC analysis of ADC aggregates

High-performance liquid chromatography system: e2695 high-performance liquid chromatography system or 1260 Agilent liquid chromatography system.

Column: Waters Xbridge BEH200 SEC (7.8 × 300 mm, 3.5 μm)
Mobile phase: 50 mM PB + 200 mM Arg (pH 6.80) + 10% IPA, elution was carried out according to the following program: 0-30 min, the volume of mobile phase A was 100%-100%.

Detection conditions: The flow rate of the mobile phase was set at 0.5 mL/min, the detection wavelength was 280 nm, and the column temperature was 26°C.

Experimental procedure: Take 20 μg of the conjugated sample (volume determined according to the sample concentration), inject into the high-performance liquid chromatography system, elute using the above-described program, and record the chromatogram.

Calculation formulas:

Monomer purity (%) = A_monomer / A_total × 100%; Aggregate purity (%) = A_aggregate / A_total × 100%.

Example 65: Preparation of antibody-drug conjugate ADC-1

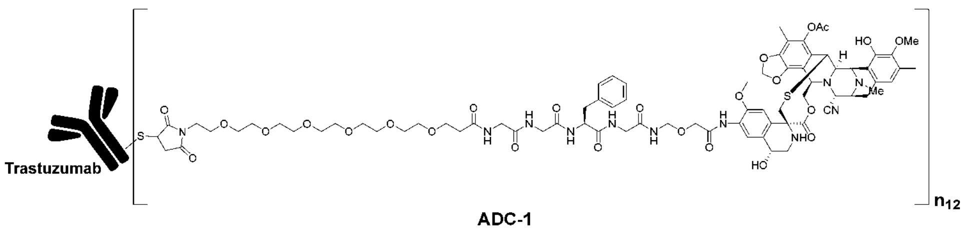

ADC-1

,

$n_{12}$=2.3

According to the general conjugation process for preparing antibody-drug conjugates, TCEP (10 mM, 1.8 eq.) aqueous solution was added to a solution of trastuzumab (1.0 mg, 38.9 mg/mL, 1.0 eq.) in 50 mM PBS pH 7.4, and the mixture was reduced at 22°C for 2 hours; then, LP1 (10 mg/mL, 4.0 eq.) in DMSO was added, and the conjugation reaction was carried out at 22°C for 1 hour. After three rounds of purification using dextran-coated activated carbon, the buffer of the desired antibody-drug conjugate was concentrated by eight rounds of ultrafiltration to obtain ADC-1 ($c_{ADC}$ (mg/mL): 10.56, V (mL): 0.08, yield 84.5%).

Using the general characterization methods for antibody-drug conjugates, the following characterization results were obtained:

HIC-DAR: 2.30, SEC purity: 98.85%, Free Linker-Drug (mol/mol%): not detected.

Example 66: Preparation of antibody-drug conjugate ADC-85

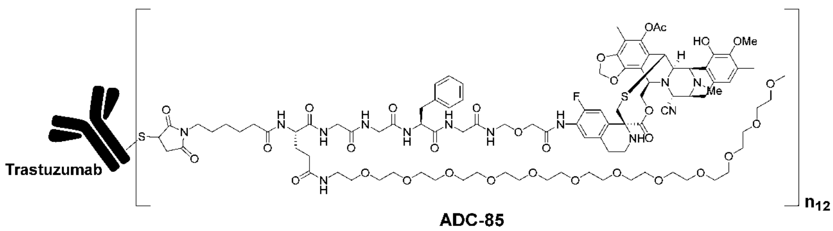

ADC-85

,

$n_{12}$=1.2

According to the general conjugation process for preparing antibody-drug conjugates, TCEP (10 mM, 2 eq.) aqueous solution was added to a solution of trastuzumab (1.0 mg, 21.07 mg/mL, 1.0 eq.) in 50 mM PBS pH 7.0, and the

mixture was reduced at 22°C for 2 hours; then, LP62 (10 mg/mL, 5.0 eq.) in DMSO was added, and the conjugation reaction was carried out at 22°C for 1 hour. After three rounds of purification using dextran-coated activated carbon, the buffer of the desired antibody-drug conjugate was concentrated by five rounds of ultrafiltration to obtain ADC-85 ($c_{ADC}$ (mg/mL): 6.68, V (mL): 0.1, yield 66.8%).

Using the general characterization methods for antibody-drug conjugates, the following characterization results were obtained:
HIC-DAR: 1.20, SEC purity: 100%, Free Linker-Drug (mol/mol%): not detected.

Example 67: Preparation of antibody-drug conjugate ADC-86

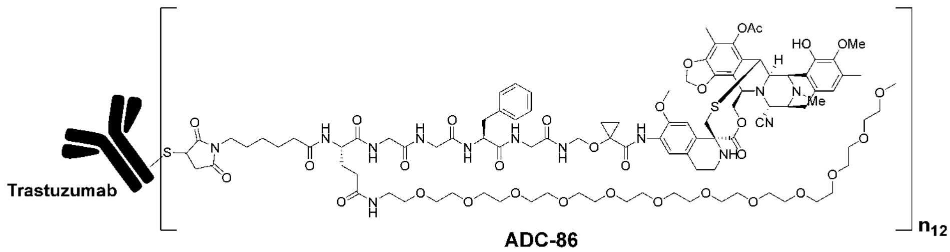

ADC-86

,

$n_{12}$=6.5

According to the general conjugation process for preparing antibody-drug conjugates, TCEP (1 mM, 15 eq.) aqueous solution was added to a solution of trastuzumab (1.5 mg, 10 mg/mL, 1.0 eq.) in 20 mM PBS pH 7.4, and the mixture was reduced at 37°C for 1.5 hours; the solution was then placed on ice for 5 minutes, followed by the addition of LP63 (1 mM, 15.0 eq.) in DMSO, and the conjugation reaction was carried out at 22°C for 1 hour. After three rounds of purification using dextran-coated activated carbon, the buffer of the desired antibody-drug conjugate was concentrated by five rounds of ultrafiltration to obtain ADC-86 ($c_{ADC}$ (mg/mL): 8, V (mL): 0.08, yield 40%).

Using the general characterization methods for antibody-drug conjugates, the following characterization results were obtained:
HIC-DAR: 6.5, SEC purity: 65%, Free Linker-Drug (mol/mol%): not detected.

Example 68: Preparation of antibody-drug conjugate ADC-87

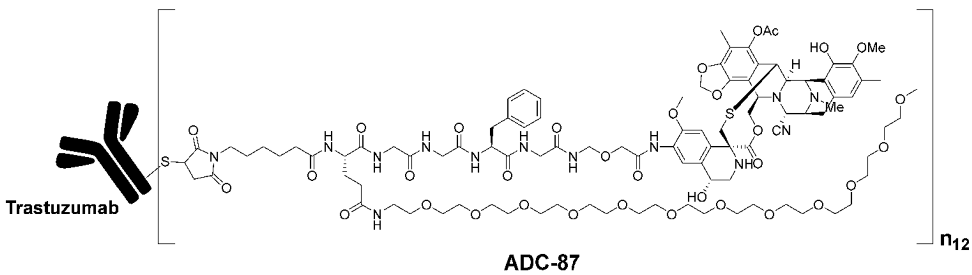

ADC-87

,

$n_{12}$=8.0

According to the general conjugation process for preparing antibody-drug conjugates, TCEP (10 mM, 8 eq.) aqueous solution was added to a solution of trastuzumab (1.0 mg, 21.07 mg/mL, 1.0 eq.) in 50 mM PBS pH 7.0, and the mixture was reduced at 22°C for 2 hours; then, LP65 (10 mg/mL, 12.0 eq.) in DMSO was added, and the conjugation reaction was carried out at 22°C for 3 hours. After three rounds of purification using dextran-coated activated carbon, the buffer of the desired antibody-drug conjugate was concentrated by five rounds of ultrafiltration to obtain ADC-87 ($c_{ADC}$ (mg/mL): 5.53, V (mL): 0.15, yield 63.6%).

Using the general characterization methods for antibody-drug conjugates, the following characterization results were obtained:
HIC-DAR: 8.0, SEC purity: 89%, Free Linker-Drug (mol/mol%): not detected.

Example 69: Preparation of antibody-drug conjugate ADC-88

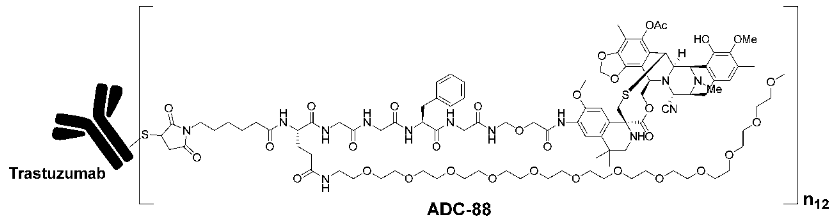

ADC-88

,

$n_{12}$=4.0

According to the general conjugation process for preparing antibody-drug conjugates, TCEP (10 mM, 2 eq.) aqueous solution was added to a solution of trastuzumab (1.0 mg, 21.07 mg/mL, 1.0 eq.) in 50 mM PBS pH 7.0, and the mixture was reduced at 22°C for 2 hours; then, LP66 (10 mg/mL, 8.0 eq.) in DMSO was added, and the conjugation reaction was carried out at 22°C for 1 hour. After three rounds of purification using dextran-coated activated carbon, the buffer of the desired antibody-drug conjugate was concentrated by five rounds of ultrafiltration to obtain ADC-88 ($c_{ADC}$ (mg/mL): 6.09, V (mL): 0.11, yield 66.9%).

Using the general characterization methods for antibody-drug conjugates, the following characterization results were obtained:

HIC-DAR: 4.0, SEC purity: 99.08%, Free Linker-Drug (mol/mol%): not detected.

Example 70: Preparation of antibody-drug conjugate ADC-89

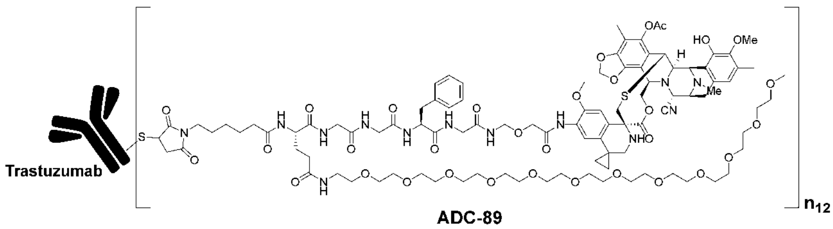

ADC-89

,

$n_{12}$=3.8

According to the general conjugation process for preparing antibody-drug conjugates, TCEP (10 mM, 2 eq.) aqueous solution was added to a solution of trastuzumab (1.0 mg, 21.07 mg/mL, 1.0 eq.) in 50 mM PBS pH 7.0, and the mixture was reduced at 22°C for 2 hours; then, LP67 (10 mg/mL, 8.0 eq.) in DMSO was added, and the conjugation reaction was carried out at 22°C for 1 hour. After three rounds of purification using dextran-coated activated carbon, the buffer of the desired antibody-drug conjugate was concentrated by five rounds of ultrafiltration to obtain ADC-89 ($c_{ADC}$ (mg/mL): 4.73, V (mL): 0.12, yield 56.8%).

Using the general characterization methods for antibody-drug conjugates, the following characterization results were obtained:

HIC-DAR: 3.8, SEC purity: 92.87%, Free Linker-Drug (mol/mol%): not detected.

Example 71: Preparation of antibody-drug conjugate ADC-90

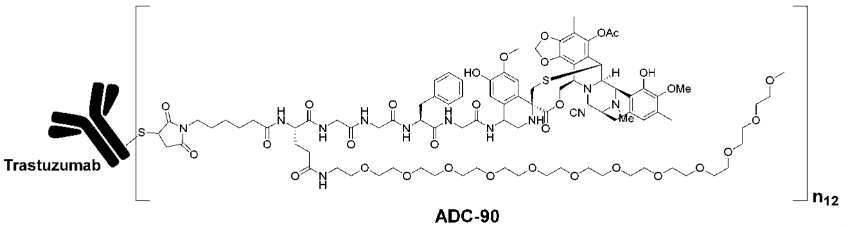

ADC-90

,

$n_{12}$=2.1

According to the general conjugation process for preparing antibody-drug conjugates, TCEP (10 mM, 2 eq.) aqueous solution was added to a solution of trastuzumab (1.0 mg, 21.07 mg/mL, 1.0 eq.) in 50 mM PBS pH 7.0, and the mixture was reduced at 22°C for 2 hours; then, LP68 (10 mg/mL, 9.0 eq.) in DMSO was added, and the conjugation reaction was carried out at 22°C for 1 hour. After three rounds of purification using dextran-coated activated carbon, the buffer of the desired antibody-drug conjugate was concentrated by five rounds of ultrafiltration to obtain ADC-90 ($c_{ADC}$ (mg/mL): 2.81, V (mL): 0.12, yield 33.7%).

Using the general characterization methods for antibody-drug conjugates, the following characterization results were obtained:

HIC-DAR: 2.08, SEC purity: 93.64%, Free Linker-Drug (mol/mol%): not detected.

Example 72: Preparation of antibody-drug conjugate ADC-91

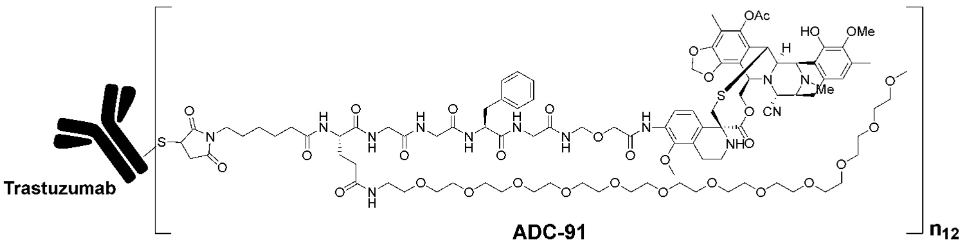

ADC-91

,

$n_{12}$=7.9

According to the general conjugation procedure for preparing antibody-drug conjugates, TCEP (10 mM, 2 eq.) aqueous solution was added to a solution of trastuzumab (1.0 mg, 21.07 mg/mL, 1.0 eq.) in 50 mM PBS buffer at pH 7.0, and the reduction was carried out at 22°C for 2 hours; LP64 (10 mg/mL, 5.0 eq.) in DMSO was then added, and the conjugation reaction was carried out at 22°C for 1 hour. After purification by three treatments with dextran-coated activated carbon, the buffer of the desired antibody-drug conjugate was subjected to 8 rounds of ultrafiltration to afford ADC-91 ($C_{ADC}$ (mg/mL): 8.18, V (mL): 0.13, yield: 70.9%).

By applying the general characterization methods for antibody-drug conjugates, the following characterization results were obtained:

HIC-DAR: 7.93, SEC purity: 95.63%, Free Linker Drug (mol/mol%): not detected.

Example 73: Preparation of antibody-drug conjugate ADC-92

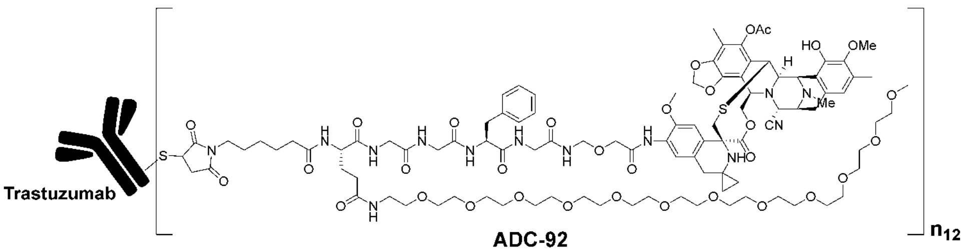

ADC-92

,

$n_{12}$=4.6

According to the general conjugation procedure for preparing antibody-drug conjugates, TCEP (10 mM, 2 eq.) aqueous solution was added to a solution of trastuzumab (1.0 mg, 21.07 mg/mL, 1.0 eq.) in 50 mM PBS buffer at pH 7.0, and the reduction was carried out at 22°C for 2 hours; LP70 (10 mg/mL, 5.0 eq.) in DMSO was then added, and the conjugation reaction was carried out at 22°C for 1 hour. After purification by three treatments with dextran-coated activated carbon, the buffer of the desired antibody-drug conjugate was subjected to 8 rounds of ultrafiltration to afford ADC-92 ($C_{ADC}$ (mg/mL): 5.82, V (mL): 0.1, yield: 58.2%).

By applying the general characterization methods for antibody-drug conjugates, the following characterization results were obtained:

HIC-DAR: 4.6, SEC purity: 98.92%, Free Linker Drug (mol/mol%): not detected.

Example 74: Preparation of antibody-drug conjugate ADC-93

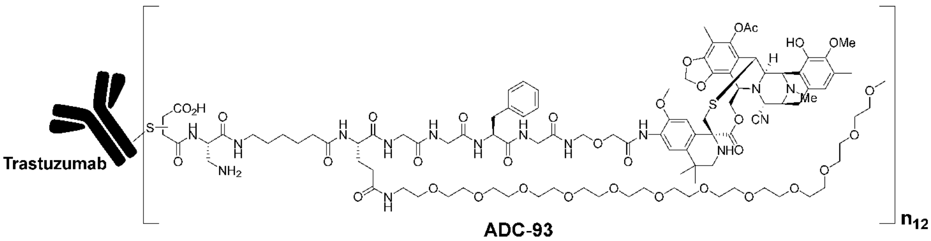

ADC-93 ,

$n_{12}$=1.6

According to the general conjugation procedure for preparing antibody-drug conjugates, TCEP (10 mM, 2 eq.) aqueous solution was added to a solution of trastuzumab (2.0 mg, 21.07 mg/mL, 1.0 eq.) in 50 mM PBS buffer at pH 7.0, and the reduction was carried out at 22°C for 2 hours; LP71 (10 mg/mL, 5.0 eq.) in DMSO was then added, and the conjugation reaction was carried out at 22°C for 1 hour. Subsequently, 500 mM Tris-HCl (pH 8.8) solution at 80% of the total ADC volume was added to adjust the pH to 8.0, and the mixture was incubated at 22°C for 4 hours. After purification by three treatments with dextran-coated activated carbon, the buffer of the desired antibody-drug conjugate was subjected to 8 rounds of ultrafiltration to afford ADC-93 ($C_{ADC}$ (mg/mL): 8.76, V (mL): 0.1, yield: 43.8%).

By applying the general characterization methods for antibody-drug conjugates, the following characterization results were obtained:

HIC-DAR: 1.6, SEC purity: 94.6%, Free Linker Drug (mol/mol%): not detected.

Example 75: Preparation of antibody-drug conjugate ADC-94

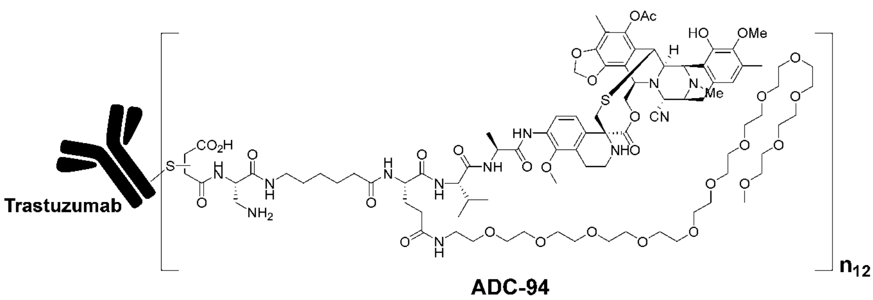

ADC-94 ,

$n_{12}$=1.6

According to the general conjugation procedure for preparing antibody-drug conjugates, TCEP (10 mM, 2 eq.) aqueous solution was added to a solution of trastuzumab (2.0 mg, 21.07 mg/mL, 1.0 eq.) in 50 mM PBS buffer at pH 7.0, and the reduction was carried out at 22°C for 2 hours; LP73 (10 mg/mL, 5.0 eq.) in DMSO was then added, and the conjugation reaction was carried out at 22°C for 1 hour. 500 mM Tris-HCl (pH 8.8) solution at 80% of the total ADC volume was added to adjust the pH to 8.0, and the mixture was incubated at 22°C for 4 hours. After purification by three treatments with dextran-coated activated carbon, the buffer of the desired antibody-drug conjugate was subjected to 8 rounds of ultrafiltration to afford ADC-94 ($C_{ADC}$ (mg/mL): 8.99, V (mL): 0.1, yield: 45%).

By applying the general characterization methods for antibody-drug conjugates, the following characterization results were obtained:
HIC-DAR: 1.6, SEC purity: 97.67%, Free Linker Drug (mol/mol%): not detected.

Example 76: Preparation of antibody-drug conjugate ADC-95

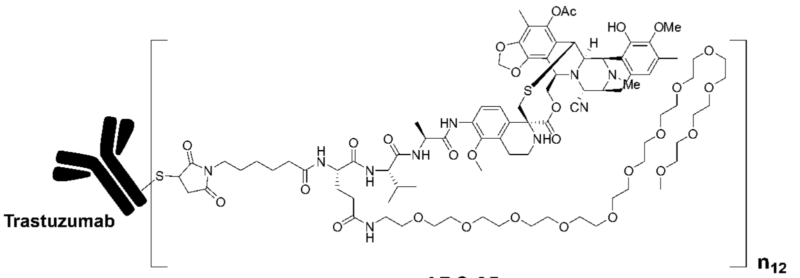

ADC-95

,

$n_{12}$=2.9

According to the general conjugation procedure for preparing antibody-drug conjugates, TCEP (10 mM, 2 eq.) aqueous solution was added to a solution of trastuzumab (1.5 mg, 21.07 mg/mL, 1.0 eq.) in 50 mM PBS buffer at pH 7.0, and the reduction was carried out at 22°C for 2 hours; LP75 (10 mg/mL, 5.0 eq.) in DMSO was then added, and the conjugation reaction was carried out at 22°C for 1 hour. After purification by three treatments with dextran-coated activated carbon, the buffer of the desired antibody-drug conjugate was subjected to 8 rounds of ultrafiltration to afford ADC-95 ($C_{ADC}$ (mg/mL): 6.18, V (mL): 0.11, yield: 45.3%).

By applying the general characterization methods for antibody-drug conjugates, the following characterization results were obtained:
HIC-DAR: 2.9, SEC purity: 99.61%, Free Linker Drug (mol/mol%): not detected.

Example 77: Preparation of antibody-drug conjugate ADC-96

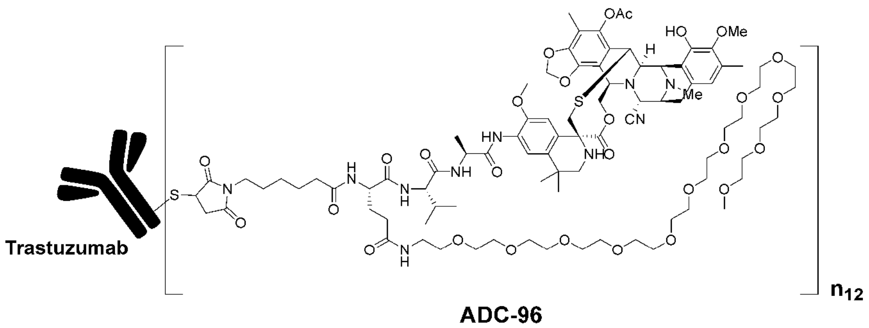

ADC-96

,

$n_{12}$=4.3

According to the general conjugation procedure for preparing antibody-drug conjugates, TCEP (10 mM, 2 eq.) aqueous solution was added to a solution of trastuzumab (1.5 mg, 21.07 mg/mL, 1.0 eq.) in 50 mM PBS buffer at pH 7.0, and the reduction was carried out at 22°C for 2 hours; LP76 (10 mg/mL, 5.0 eq.) in DMSO was then added, and the conjugation reaction was carried out at 22°C for 1 hour. After purification by three treatments with dextran-coated activated carbon, the buffer of the desired antibody-drug conjugate was subjected to 8 rounds of ultrafiltration to afford ADC-96 ($C_{ADC}$ (mg/mL): 6.82, V (mL): 0.11, yield: 50%).

By applying the general characterization methods for antibody-drug conjugates, the following characterization results were obtained:
HIC-DAR: 4.3, SEC purity: 98.38%, Free Linker Drug (mol/mol%): not detected.

Example 78: Preparation of antibody-drug conjugate ADC-97

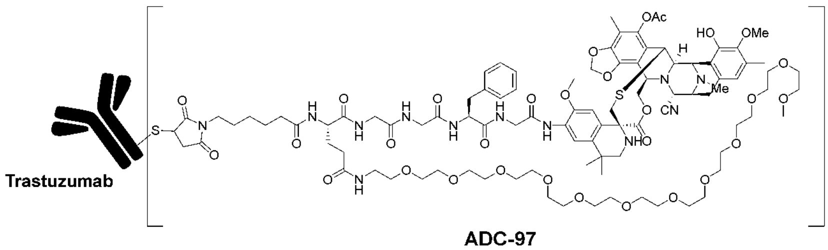

ADC-97

,

$n_{12}$=3.4

According to the general conjugation procedure for preparing antibody-drug conjugates, TCEP (10 mM, 2 eq.) aqueous solution was added to a solution of trastuzumab (0.8 mg, 21.07 mg/mL, 1.0 eq.) in 50 mM PBS buffer at pH 7.0, and the reduction was carried out at 22°C for 2 hours; LP77 (10 mg/mL, 5.0 eq.) in DMSO was then added, and the conjugation reaction was carried out at 22°C for 1 hour. After purification by three treatments with dextran-coated activated carbon, the buffer of the desired antibody-drug conjugate was subjected to 8 rounds of ultrafiltration to afford ADC-97 ($C_{ADC}$ (mg/mL): 3.46, V (mL): 0.1, yield: 43.3%).

By applying the general characterization methods for antibody-drug conjugates, the following characterization results were obtained:

HIC-DAR: 3.4, SEC purity: 98.9%, Free Linker Drug (mol/mol%): not detected.

Example 79: Preparation of antibody-drug conjugate ADC-98

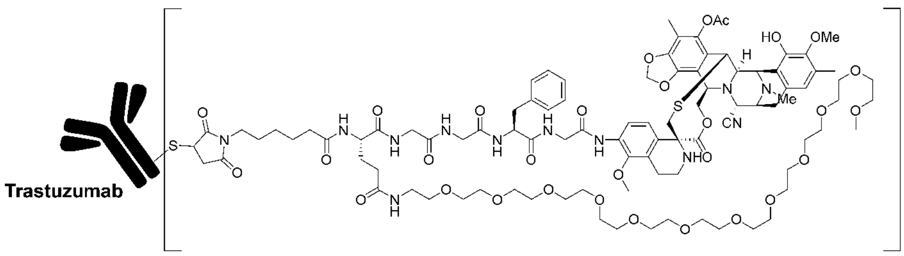

ADC-98

,

$n_{12}$=1.7

According to the general conjugation procedure for preparing antibody-drug conjugates, TCEP (10 mM, 2 eq.) aqueous solution was added to a solution of trastuzumab (1.0 mg, 21.07 mg/mL, 1.0 eq.) in 50 mM PBS buffer at pH 7.0, and the reduction was carried out at 22°C for 2 hours; LP78 (10 mg/mL, 7.0 eq.) in DMSO was then added, and the conjugation reaction was carried out at 22°C for 1 hour. After purification by three treatments with dextran-coated activated carbon, the buffer of the desired antibody-drug conjugate was subjected to 3 rounds of ultrafiltration to afford ADC-98 ($C_{ADC}$ (mg/mL): 6.21, V (mL): 0.1, yield: 62.1%).

By applying the general characterization methods for antibody-drug conjugates, the following characterization results were obtained:

HIC-DAR: 1.7, SEC purity: 97.85%, Free Linker Drug (mol/mol%): not detected.

Biological Evaluation

1) Tumor Cell Proliferation Inhibition Assay of Novel Ecteinascidin Derivatives

MDA-MB-231, OV90, and NCI-N87 cells at defined cell densities (1,500 cells per well, 80 μL volume) were seeded into 96-well plates. The cells were cultured overnight in a constant-temperature incubator (37°C, 5% $CO_2$). Ecteinascidin derivative solutions at a maximum concentration of 2 mM were first prepared in DMSO, and serial 1:4 gradient dilutions were performed. 1 μL of each DMSO-prepared dilution was added to 99 μL of PBS containing 10% FBS

per well and mixed thoroughly. The diluted solutions (2 μL of diluted solution and 18 μL of PBS containing 10% FBS per well) were then co-incubated with the cells (37°C, 5% $CO_2$) for five days, after which CellTiter-Glo reagent (40 μL per well) was added, and fluorescence values were measured using an i3x microplate reader.

The in-vitro cytotoxicity of the ecteinascidin derivatives of the present invention (++++: < 1 nM; +++: 1-10 nM; ++: 10-200 nM; +: > 200 nM)

| Compound No. | Structure | MDA-MB-231 | $IC_{50}$ (nM) OV90 | NCI-N87 |
|---|---|---|---|---|
| Trabectedin | 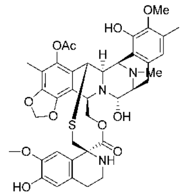 | +++ | ++ | ++++ |
| Lurbinectedin | 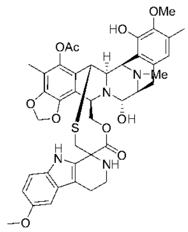 | +++ | +++ | ++++ |
| P1 | 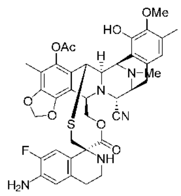 | ++++ | ++++ | ++++ |
| P5 | 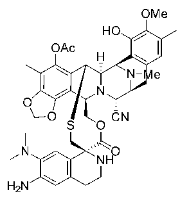 | +++ | +++ | +++ |
| P9 | 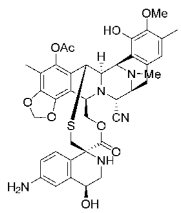 | ++++ | ++++ | ++++ |
| P10 | 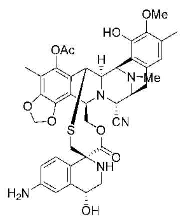 | ++++ | ++++ | ++++ |
| P12 | 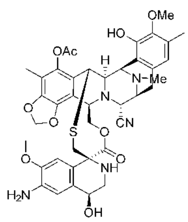 | ++++ | ++++ | ++++ |

(continued)

| Compound No. | Structure | MDA-MB-231 | $IC_{50}$ (nM) OV90 | NCI-N87 |
|---|---|---|---|---|
| P13 | 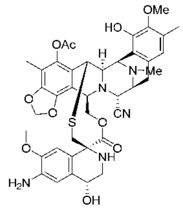 | ++++ | ++++ | ++++ |
| P14 | 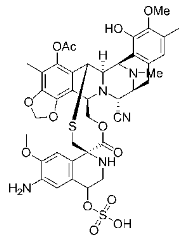 | + | + | + |
| P19 | 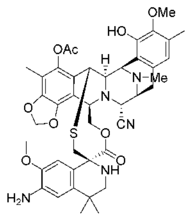 | ++++ | ++++ | ++++ |
| P30 | 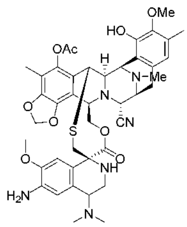 | ++++ | ++++ | ++++ |
| P36 | 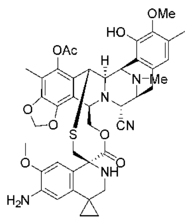 | ++++ | ++++ | ++++ |
| P52 | 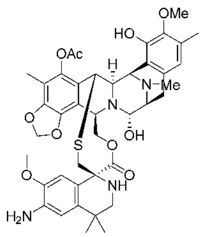 | ++++ | ++++ | ++++ |
| P58 | 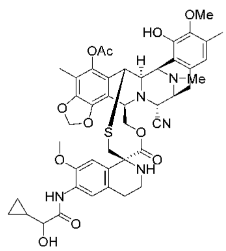 | ++++ | ++++ | ++++ |

(continued)

| Compound No. | Structure | MDA-MB-231 | IC$_{50}$ (nM) OV90 | NCI-N87 |
|---|---|---|---|---|
| P65 | 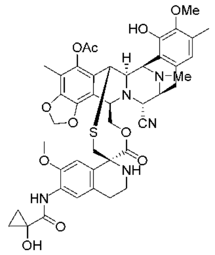 | ++++ | ++++ | ++++ |
| P74 | 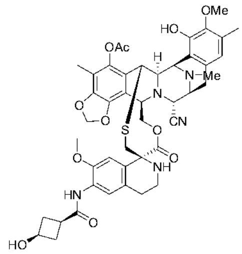 | ++++ | ++++ | ++++ |
| P75 | 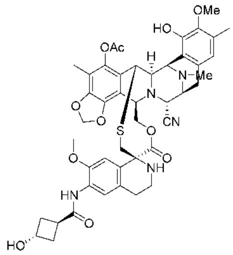 | ++++ | ++++ | ++++ |
| P85 | 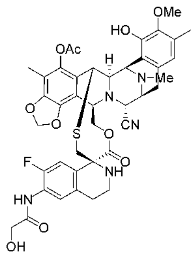 | ++++ | ++++ | ++++ |
| P90 | 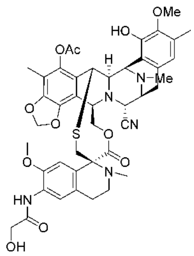 | ++++ | ++++ | ++++ |
| P96 | 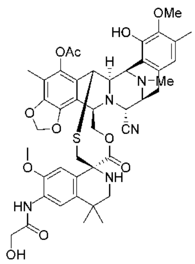 | ++++ | ++++ | ++++ |

(continued)

| Compound No. | Structure | MDA-MB-231 | $IC_{50}$ (nM) OV90 | NCI-N87 |
|---|---|---|---|---|
| P166 | 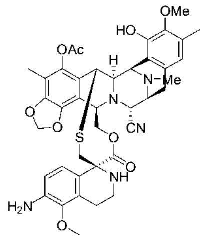 | ++++ | ++++ | ++++ |
| P170 | 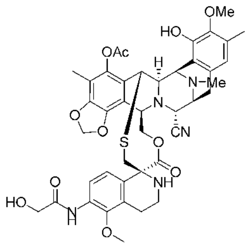 | ++++ | ++++ | ++++ |
| P172 | 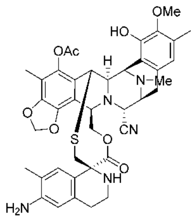 | ++++ | ++++ | ++++ |
| P241 | 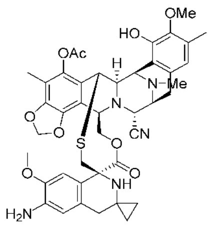 | ++++ | +++ | ++++ |
| P261 | 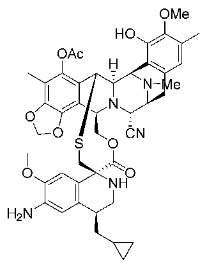 | +++ | ++ | ++ |
| P262 | 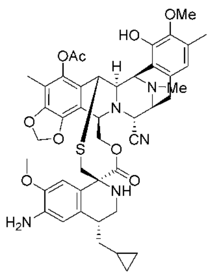 | +++ | +++ | +++ |

(continued)

| Compound No. | Structure | MDA-MB-231 | $IC_{50}$ (nM) OV90 | NCI-N87 |
|---|---|---|---|---|
| T1 | 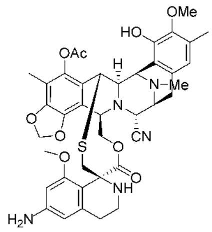 | ++++ | ++++ | ++++ |
| T2 | 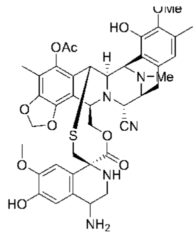 | +++ | ++ | ++ |
| T3 | 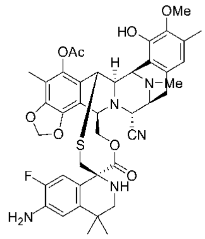 | ++++ | ++++ | ++++ |
| T4 | 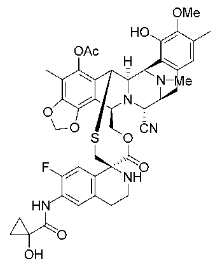 | ++++ | ++++ | ++++ |
| T5 | 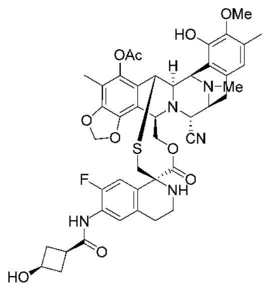 | ++++ | ++++ | +++ |
| T6 | 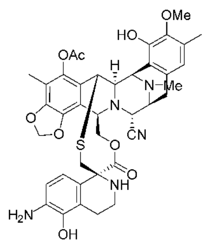 | +++ | +++ | +++ |

(continued)

| Compound No. | Structure | MDA-MB-231 | $IC_{50}$ (nM) OV90 | NCI-N87 |
|---|---|---|---|---|
| T7 | 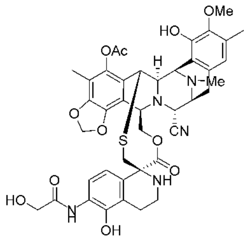 | +++ | +++ | +++ |
| T8 | 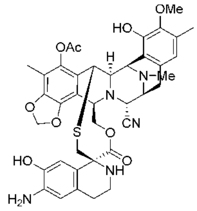 | +++ | +++ | +++ |
| T9 | 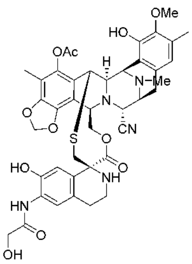 | +++ | +++ | +++ |
| T10 | 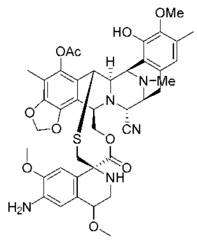 | ++++ | ++++ | ++++ |
| T11 | 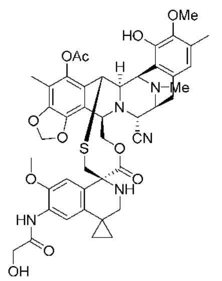 | ++++ | ++++ | ++++ |
| T12 | 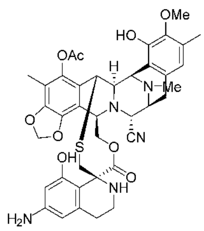 | +++ | +++ | +++ |

(continued)

| Compound No. | Structure | MDA-MB-231 | $IC_{50}$ (nM) OV90 | NCI-N87 |
|---|---|---|---|---|
| T13 | 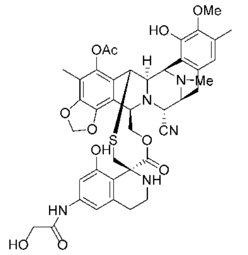 | ++ | ++ | ++ |
| T14 | 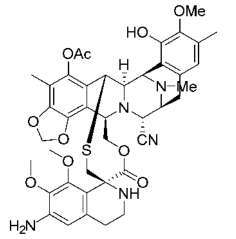 | +++ | ++ | ++ |
| T15 | 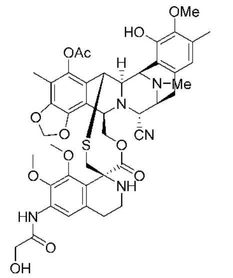 | ++ | ++ | ++ |
| T16 | 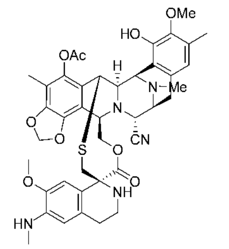 | +++ | +++ | ++++ |
| T17 | 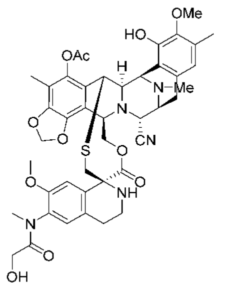 | ++++ | ++++ | ++++ |
| T18 | 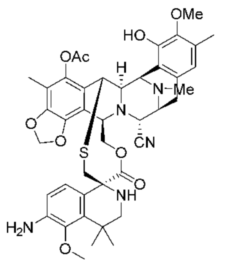 | ++++ | +++ | ++++ |

All ecteinascidin derivatives exhibited certain antitumor activity, among which the in-vitro cytotoxic activities of P1, P9, P10, P12, P13, P19, P30, P36, P52, P58, P65, P74, P75, P85, P90, P96, P166, P170, P172, T1, T3, T4, T10, T11,

and T17 were superior to or comparable to those of trabectedin and lurbinectedin.

2) Tumor Cell Proliferation Inhibition Assay of Antibody-Drug Conjugates Based on Novel Ecteinascidin Derivatives

1,500 cells were seeded into 96-well plates (excluding the PBS-filled edge wells), and cultured in 80 μL of appropriate culture medium in a constant-temperature cell culture incubator at 37°C with 5% $CO_2$. After overnight culture in the constant-temperature incubator (37°C, 5% $CO_2$), 20 μL of the diluted antibody-drug conjugate solution based on novel ecteinascidin derivatives was added to each well, such that the final concentration range of the conjugate was 3.8 pM - 250 nM. After co-incubation of the cells with the conjugate for 5 days, the 96-well plate was equilibrated at room temperature for 30 minutes. 40 μL of CellTiter-Glo® (Promega, G7572) reagent was added to each well, and after a 10-minute light-protected reaction, fluorescence values were measured using a SpectraMax i3x microplate reader. The luminescence values were plotted against conjugate concentration (nM) and the curve was fitted using GraphPad Prism software.

The in-vitro cytotoxicity of the antibody-drug conjugates (++++: < 1 nM; +++: 1-10 nM; ++: 10-200 nM; +: > 200 nM)

| ADC No. | Antibody | Linker-drug compound No. | IC50 (nM) |
|---|---|---|---|
| | | | NCI-N87 |
| ADC-1 | Trastuzumab | LP1 | +++ |
| ADC-85 | Trastuzumab | LP62 | ++ |
| ADC-86 | Trastuzumab | LP63 | +++ |
| ADC-87 | Trastuzumab | LP65 | ++++ |
| ADC-88 | Trastuzumab | LP66 | +++ |
| ADC-89 | Trastuzumab | LP67 | +++ |
| ADC-90 | Trastuzumab | LP68 | ++++ |
| ADC-91 | Trastuzumab | LP64 | +++ |
| ADC-92 | Trastuzumab | LP70 | +++ |
| ADC-93 | Trastuzumab | LP71 | +++ |
| ADC-94 | Trastuzumab | LP73 | ++++ |
| ADC-95 | Trastuzumab | LP75 | +++ |
| ADC-96 | Trastuzumab | LP76 | ++ |
| ADC-97 | Trastuzumab | LP77 | +++ |
| ADC-98 | Trastuzumab | LP78 | ++ |

All antibody-drug conjugates (ADCs) exhibited certain antitumor activity; for example, in NCI-N87 cells, the in-vitro cytotoxic activities of ADC-1, ADC-86, ADC-88, ADC-89, ADC-91, ADC-92, ADC-93, ADC-95, and ADC-97 were in the range of 1-10 nM; while those of ADC-87, ADC-90, and ADC-94 were less than 1 nM.

The main advantages of the present invention are as follows:

1. Among the novel ecteinascidin derivatives provided by the present invention, some exhibit potent in-vitro cytotoxicity (1-10 nM), and some exhibit even more potent in-vitro cytotoxicity (< 1 nM).

2. The antibody-drug conjugates based on novel ecteinascidin derivatives provided by the present invention exhibit potent in-vitro cytotoxicity (1-10 nM), and some of the antibody-drug conjugates exhibit even more potent in-vitro cytotoxicity (< 1 nM).

## Claims

**1.** An ecteinascidin derivative, **characterized in that** the structure thereof is a compound as shown in general formula (I):

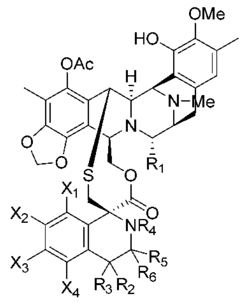

General formula (I):

wherein $R_1$ is selected from hydroxyl or cyano;

$R_2$ and $R_3$ are the same or different, and are each independently selected from hydrogen, deuterium, hydroxyl, mercapto, halogen, deuterated alkyl, haloalkyl, haloalkoxy, hydroxyl-substituted alkyl, amino-substituted alkyl, alkylamino-substituted alkyl, alkyl, alkoxy, alkylthio, $C_{2-6}$ alkenyl, $C_{2-6}$ alkynyl, cycloalkyl, alkyl-substituted amino, cycloalkyl-substituted alkyl, heterocyclyl, aryl, heteroaryl, sulfonyl,

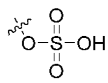, $-\S-NR_aR_b$, or $-\S-(CH_2)_x-X_a$;

$R_2$ and $R_3$ together with the carbon atoms to which they are attached form a saturated carbocycle, a saturated heterocycle, cycloalkyl, heterocycloalkyl, or do not form a ring; the x is selected from an integer of 1-10;

$R_a$ and $R_b$ are each independently selected from hydrogen, deuterium, hydroxyl, hydroxyl-substituted alkyl, alkyl, alkoxy, cycloalkyl, heterocyclyl, aryl, heteroaryl, alkyl-substituted sulfonyl, aryl-substituted sulfonyl, or heteroaryl-substituted sulfonyl; $R_a$ and $R_b$ together with the nitrogen atoms to which they are attached form a saturated heterocycle, heterocycloalkyl, or do not form a ring;

$R_4$ is selected from hydrogen, alkyl, deuterated alkyl, haloalkyl, amino-substituted alkyl, alkylamino-substituted alkyl, or

$$-\S-(CH_2)_s-X_c;$$

s is selected from an integer of 1-10;

$R_5$ and $R_6$ are both hydrogen or alkyl, and $R_5$ and $R_6$ together with the carbon atoms to which they are attached form a saturated carbocycle, a saturated heterocycle, cycloalkyl, heterocycloalkyl, or do not form a ring; or $R_5$ and $R_6$ are different, and are each independently selected from hydrogen, alkyl, cycloalkyl, or cycloalkyl-substituted alkyl;

$X_a$, $X_c$, $X_1$, $X_2$, $X_3$, and $X_4$ are each independently selected from hydrogen, halogen, $C_{1-4}$ alkyl, or

$$-\S-A-(Y)_{0-1}-(R_c)_{0-1}-(CH_2)_n-Z,$$

wherein A is selected from oxygen, sulfur, -NH, $-NCD_3$, $-NCF_3$, $-NCH_3$, $-NCH_2CH_3$, or $-NCH_2CH_2CH_3$; Y is selected from -C(=O)-, $-S(=O)_2-$, or $-CHCF_3$; $R_c$ is selected from a 3- to 10-membered saturated or partially unsaturated heterocyclyl, a $C_{3-10}$ saturated or partially unsaturated carbocycle, or

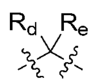;

n is selected from an integer of 0-10, and Z is selected from hydrogen, hydroxyl, mercapto, $-NH_2$, $-NHCH_3$, $-NHCH_2CH_3$, - $NHCH_2CH_2CH_3$, $-CD_3$, or $-CF_3$;

$R_d$ and $R_e$ are the same or different, and are each independently selected from hydrogen, deuterium, trifluor-

omethyl, alkyl, $C_{2-6}$ alkenyl, $C_{2-6}$ alkynyl, cycloalkyl, cycloalkyl-substituted alkyl, heterocyclyl, aryl, or heteroaryl; $R_d$ and $R_e$ together with the carbon atoms to which they are attached form a saturated carbocycle, a saturated heterocycle, cycloalkyl, heterocycloalkyl, or do not form a ring;
when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

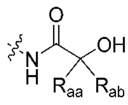, 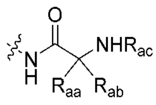,

$-NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

**2.** The ecteinascidin derivative according to claim 1, wherein in the general formula (I):

$R_1$ is selected from hydroxyl or cyano;
$R_2$ and $R_3$ are the same or different, and are each independently selected from hydrogen, deuterium, hydroxyl, mercapto, halogen, deuterated alkyl, haloalkyl, haloalkoxy, hydroxyalkyl, amino-substituted alkyl, alkylamino-substituted alkyl, alkyl, alkoxy, alkylthio, $C_{2-6}$ alkenyl, $C_{2-6}$ alkynyl, cycloalkyl, alkyl-substituted amino, cycloalkylalkyl, heterocyclyl, aryl, heteroaryl, sulfonyl group,

$$\xi\text{-}NR_aR_b$$

or

$$\xi-(CH_2)_x\text{-}X_a\,;$$

$R_2$ and $R_3$ together with the carbon atoms to which they are attached form cycloalkyl, heterocycloalkyl, or do not form a ring;
x is selected from an integer of 1-10;
$R_a$ and $R_b$ are each independently selected from hydrogen, deuterium, hydroxyl, hydroxyalkyl, alkyl, alkoxy, cycloalkyl, heterocyclyl, aryl, heteroaryl, alkylsulfonyl, arylsulfonyl, or heteroarylsulfonyl; $R_a$ and $R_b$ together with the nitrogen atoms to which they are attached form heterocycloalkyl or do not form a ring;
$R_4$ is selected from hydrogen, alkyl, deuterated alkyl, haloalkyl, amino-substituted alkyl, alkylamino-substituted alkyl, or

$$\xi-(CH_2)_s\text{-}X_c\,;$$

s is selected from an integer of 1-10;
$R_5$ and $R_6$ are both hydrogen or alkyl, and $R_5$ and $R_6$ together with the carbon atoms to which they are attached form cycloalkyl, heterocycloalkyl, or do not form a ring; or $R_5$ and $R_6$ are different, and are each independently selected from hydrogen, alkyl, cycloalkyl, or cycloalkylalkyl;
$X_1$ and $X_4$ are hydrogen;
$X_a$, $X_c$, $X_2$, and $X_3$ are each independently selected from hydrogen, halogen, $C_{1-4}$ alkyl, or

$$-\xi-A\text{-}(Y)_{0-1}\text{-}(R_c)_{0-1}\text{-}(CH_2)_n\text{-}Z\,,$$

wherein A is selected from oxygen, sulfur, -NH, $-NCD_3$, $-NCF_3$, $-NCH_3$, $-NCH_2CH_3$, or $-NCH_2CH_2CH_3$; Y is selected from C(=O), $S(=O)_2$, or $-CHCF_3$; $R_c$ is selected from a 3- to 10-membered saturated or partially unsaturated heterocyclyl, a $C_{3-10}$ saturated or partially unsaturated carbocycle, or

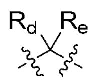;

n is selected from an integer of 0-10, and Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;
$R_d$ and $R_e$ are the same or different, and are each independently selected from hydrogen, deuterium, trifluoromethyl, alkyl, $C_{2-6}$ alkenyl, $C_{2-6}$ alkynyl, cycloalkyl, cycloalkylalkyl, heterocyclyl, aryl, or heteroaryl; Rd and Re together with the carbon atoms to which they are attached form cycloalkyl, heterocycloalkyl, or do not form a ring;
when one of $R_2$ or $R_3$ is hydrogen, the other is not $C_{1-4}$ alkyl; when both $R_2$ **and** $R_3$ **are** hydrogen, $X_2$ or $X_3$ is not

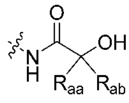, 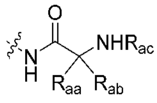,

$-NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

**3.** The ecteinascidin derivative according to claim 1, wherein in the general formula (I):

$R_1$ is selected from hydroxyl or cyano;
$R_2$ and $R_3$ are the same or different, and are each independently selected from hydrogen, deuterium, hydroxyl, mercapto, halogen, deuterated alkyl, haloalkyl, haloalkoxy, hydroxyl-substituted alkyl, amino-substituted alkyl, alkylamino-substituted alkyl, alkyl, alkoxy, alkylthio, $C_{2-6}$ alkenyl, $C_{2-6}$ alkynyl, cycloalkyl, alkyl-substituted amino, cycloalkyl-substituted alkyl, heterocyclyl, aryl, heteroaryl,

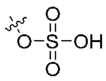, $-\S-NR_aR_b$, or $-\S-(CH_2)_x-X_a$;

$R_2$ **and** $R_3$ **together** with the carbon atoms to which they are attached form a saturated carbocycle, a saturated heterocycle, or do not form a ring; x is selected from an integer of 1-10;
$R_a$ and $R_b$ are each independently selected from hydrogen, deuterium, hydroxyl, hydroxyl-substituted alkyl, alkyl, alkoxy, cycloalkyl, heterocyclyl, aryl, heteroaryl, alkyl-substituted sulfonyl, aryl-substituted sulfonyl, or heteroaryl-substituted sulfonyl; $R_a$ and $R_b$ together with the nitrogen atoms to which they are attached form a saturated heterocycle or do not form a ring;
$R_4$ is selected from hydrogen, alkyl, deuterated alkyl, haloalkyl, amino-substituted alkyl, alkylamino-substituted alkyl, or

$$-\S-(CH_2)_s-X_c;$$

s is selected from an integer of 1-10;
$R_5$ and $R_6$ are both hydrogen or alkyl, and $R_5$ and $R_6$ together with the carbon atoms to which they are attached form a saturated carbocycle, a saturated heterocycle, or do not form a ring; or $R_5$ and $R_6$ are different, and are each independently selected from hydrogen, alkyl, cycloalkyl, or cycloalkyl-substituted alkyl;
$X_a$, $X_c$, $X_1$, $X_2$, $X_3$, and $X_4$ are each independently selected from hydrogen, halogen, $C_{1-4}$ alkyl, or

$$-\S-A-(Y)_{0-1}-(R_c)_{0-1}-(CH_2)_n-Z,$$

wherein A is selected from oxygen, sulfur, -NH, $-NCD_3$, $-NCF_3$, $-NCH_3$, $-NCH_2CH_3$, or $-NCH_2CH_2CH_3$; Y is selected from -C(=O)-, $-S(=O)_2-$, or $-CHCF_3$; $R_c$ is selected from a 3- to 10-membered saturated or partially unsaturated heterocyclyl, a $C_{3-10}$ saturated or partially unsaturated carbocycle, or

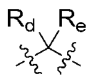;

n is selected from an integer of 0-10, and Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;
$R_d$ and $R_e$ are the same or different, and are each independently selected from hydrogen, deuterium, trifluor-

omethyl, alkyl, $C_{2-6}$ alkenyl, $C_{2-6}$ alkynyl, cycloalkyl, cycloalkyl-substituted alkyl, heterocyclyl, aryl, or heteroaryl; $R_d$ and $R_e$ together with the carbon atoms to which they are attached form a saturated carbocycle, a saturated heterocycle, or do not form a ring;

when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

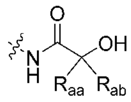, 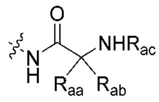,

$-NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

**4.** The ecteinascidin derivative according to claim 1 or 3, wherein in the general formula (I):

$R_1$ is selected from hydroxyl or cyano;

$R_2$ and $R_3$ are the same or different, and are each independently selected from hydrogen, deuterium, hydroxyl, mercapto, fluorine, chlorine, deuterated $C_{1-6}$ alkyl, halo $C_{1-6}$ alkyl, halo $C_{1-6}$ alkoxy, $C_{1-6}$ hydroxy-substituted alkyl, amino-substituted $C_{1-6}$ alkyl, $C_{1-6}$ alkylamino-substituted $C_{1-6}$ alkyl, $C_{1-6}$ alkyl, $C_{1-6}$ alkoxy, $C_{1-6}$ alkylthio, $C_{2-6}$ alkenyl, $C_{2-6}$ alkynyl, $C_{3-8}$ cycloalkyl, $C_{1-6}$ alkyl-substituted amino, cycloalkyl-substituted alkyl, heterocyclyl, aryl, heteroaryl,

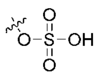, 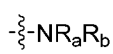,

or

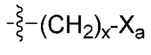;

$R_2$ **and** $R_3$ **together** with the carbon atoms to which they are attached form a 3- to 10-membered saturated carbocycle, a 3- to 10-membered saturated heterocycle, or do not form a ring; x is selected from an integer of 1-6; $R_a$ and $R_b$ are each independently selected from hydrogen, hydroxyl, hydroxyl-substituted alkyl, alkyl, cycloalkyl, aryl, alkyl-substituted sulfonyl, or aryl-substituted sulfonyl; $R_a$ and $R_b$ together with the nitrogen atoms to which they are attached form a saturated heterocycle or do not form a ring;

$R_4$ is selected from hydrogen, $C_{1-6}$ alkyl, deuterated $C_{1-6}$ alkyl, halo $C_{1-6}$ alkyl, amino-substituted $C_{1-6}$ alkyl, or $C_{1-6}$ alkylamino-substituted $C_{1-6}$ alkyl, or

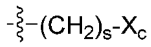;

s is selected from an integer of 1-6;

$R_5$ and $R_6$ are both hydrogen or $C_{1-6}$ alkyl; $R_5$ and $R_6$ together with the carbon atoms to which they are attached form a 3- to 10-membered saturated carbocycle, a 3- to 10-membered saturated heterocycle, or do not form a ring; or $R_5$ and $R_6$ are different, and are each independently selected from hydrogen, $C_{1-6}$ alkyl, $C_{3-10}$ cycloalkyl, or cycloalkyl-substituted alkyl;

$X_a$, $X_c$, $X_1$, $X_2$, $X_3$, and $X_4$ are each independently selected from hydrogen, fluorine, chlorine, methyl, ethyl, or

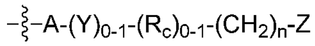,

wherein A is selected from oxygen, sulfur, -NH, $-NCD_3$, $-NCF_3$, $-NCH_3$, $-NCH_2CH_3$, or $-NCH_2CH_2CH_3$; Y is selected from -C(=O)-; $R_c$ is selected from a 3- to 6-membered saturated heterocyclyl or a $C_{3-6}$ saturated carbocycle, or

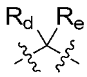;

n is selected from an integer of 0-6, and Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, trifluoromethyl, alkyl, $C_{2-6}$ alkenyl, $C_{2-6}$ alkynyl, cycloalkyl, cycloalkyl-substituted alkyl, or aryl; $R_d$ and $R_e$ together with the carbon atoms to which they are attached form a saturated carbocycle, a saturated heterocycle, or do not form a ring; when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

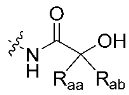, 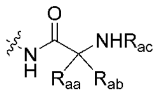,

$-NHCH_3$, - $NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

**5.** The ecteinascidin derivative according to claim 1 or 3, wherein in the general formula (I):

$R_1$ is selected from hydroxyl or cyano;

$R_2$ and $R_3$ are the same or different, and are each independently selected from hydrogen, hydroxyl, mercapto, fluorine, chlorine, trideuteromethyl, trifluoromethyl, trifluoromethoxy, $C_{1-6}$ hydroxy-substituted alkyl, amino-substituted $C_{1-6}$ alkyl, $C_{1-6}$ alkylamino-substituted $C_{1-6}$ alkyl, $C_{1-6}$ alkyl, $C_{1-6}$ alkoxy, $C_{1-6}$ alkylthio, $C_{2-6}$ alkenyl, $C_{2-6}$ alkynyl, $C_{3-8}$ cycloalkyl, $C_{1-6}$ alkyl-substituted amino, cycloalkyl-substituted alkyl, heterocyclyl, aryl, heteroaryl,

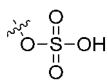, -ξ-$NR_aR_b$,

or

-ξ-$(CH_2)_x$-$X_a$;

$R_2$ **and** $R_3$ **together** with the carbon atoms to which they are attached form a 3- to 6-membered saturated carbocycle, a 3- to 6-membered saturated heterocycle, or do not form a ring; x is selected from an integer of 1-6;

$R_a$ and $R_b$ are each independently selected from hydrogen, hydroxyl, hydroxyl-substituted alkyl, alkyl, cycloalkyl, aryl, or alkyl-substituted sulfonyl; $R_a$ and $R_b$ together with the nitrogen atoms to which they are attached form a saturated heterocycle or do not form a ring;

$R_4$ is selected from hydrogen, $C_{1-6}$ alkyl, trideuteromethyl, trifluoromethyl, amino-substituted $C_{1-6}$ alkyl, or $C_{1-6}$ alkylamino-substituted $C_{1-6}$ alkyl, or

-ξ-$(CH_2)_s$-$X_c$;

s is selected from an integer of 1-6;

$R_5$ and $R_6$ are both hydrogen or $C_{1-6}$ alkyl; $R_5$ and $R_6$ together with the carbon atoms to which they are attached form a 3- to 6-membered saturated carbocycle, a 3- to 6-membered saturated heterocycle, or do not form a ring; or $R_5$ and $R_6$ are different, and are each independently selected from hydrogen, $C_{1-6}$ 6 alkyl, $C_{3-6}$ cycloalkyl, or cycloalkyl-substituted alkyl;

$X_a$, $X_c$, $X_1$, $X_2$, $X_3$, and $X_4$ are each independently selected from hydrogen, fluorine, chlorine, methyl, ethyl, or

–ξ–A-$(Y)_{0-1}$-$(R_c)_{0-1}$-$(CH_2)_n$-Z,

wherein A is selected from oxygen, sulfur, -NH, $-NCD_3$, $-NCH_3$, $-NCH_2CH_3$, or $-NCH_2CH_2CH_3$; Y is selected from -C(=O)-; $R_c$ is selected from a 3- to 6-membered saturated heterocyclyl, a $C_{3-6}$ saturated carbocycle, or

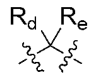

;

n is selected from an integer of 0-6, and Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;

$R_d$ and $R_e$ are the same or different, and are each independently selected from hydrogen, trifluoromethyl, alkyl, $C_{2-6}$ alkenyl, $C_{2-6}$ alkynyl, cycloalkyl, cycloalkyl-substituted alkyl, or aryl; $R_d$ and $R_e$ together with the carbon atoms to which they are attached form a saturated carbocycle, a saturated heterocycle, or do not form a ring; when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

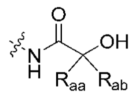

,

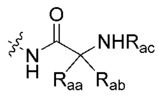

,

$-NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

**6.** The ecteinascidin derivative according to claim 1 or 3, wherein in the general formula (I):

$R_1$ is selected from hydroxyl or cyano;

$R_2$ and $R_3$ are the same or different, and are each independently selected from hydrogen, hydroxyl, fluorine, trifluoromethyl, trifluoromethoxy, $C_{1-6}$ hydroxy-substituted alkyl, amino-substituted $C_{1-6}$ alkyl, $C_{1-6}$ alkylamino-substituted $C_{1-6}$ alkyl, $C_{1-6}$ alkyl, $C_{1-6}$ alkoxy, $C_{3-8}$ cycloalkyl, $C_{1-6}$ alkyl-substituted amino,

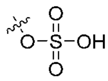

,

-ξ-$NR_aR_b$, or -ξ-$(CH_2)_x$-$X_a$;

$R_2$ and $R_3$ together with the carbon atoms to which they are attached form a 3- to 6-membered saturated carbocycle, a 3- to 6-membered saturated heterocycle, or do not form a ring; x is selected from an integer of 1-2;

$R_a$ and $R_b$ are each independently selected from hydrogen, hydroxyl-substituted alkyl, alkyl, or alkyl-substituted sulfonyl; $R_a$ and $R_b$ together with the nitrogen atoms to which they are attached form a saturated heterocycle or do not form a ring;

$R_4$ is selected from hydrogen, $C_{1-4}$ alkyl, amino-substituted $C_{1-4}$ alkyl, $C_{1-4}$ alkylamino-substituted $C_{1-4}$ alkyl, or

-ξ-$(CH_2)_s$-$X_c$;

s is selected from an integer of 1-2;

$R_5$ and $R_6$ are both hydrogen or $C_{1-6}$ alkyl; $R_5$ and $R_6$ together with the carbon atoms to which they are attached form a 3- to 6-membered saturated carbocycle, a 3- to 6-membered saturated heterocycle, or do not form a ring; or $R_5$ and $R_6$ are different, and are each independently selected from hydrogen, $C_{1-6}$ alkyl, $C_{3-6}$ cycloalkyl, or cycloalkyl-substituted alkyl;

$X_a$, $X_c$, $X_1$, $X_2$, $X_3$, and $X_4$ are each independently selected from hydrogen, fluorine, methyl, or

-ξ-A-$(Y)_{0-1}$-$(R_c)_{0-1}$-$(CH_2)_n$-Z,

wherein A is selected from oxygen, $-NCH_3$, or -NH; Y is selected from -C(=O)-; $R_c$ is selected from a $C_{3-6}$ saturated carbocycle or

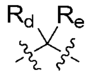;

n is selected from an integer of 0-1, and Z is selected from hydrogen or hydroxyl;

$R_d$ and $R_e$ are the same or different, and are each independently selected from hydrogen, trifluoromethyl, alkyl, cycloalkyl, or cycloalkyl-substituted alkyl; $R_d$ and $R_e$ together with the carbon atoms to which they are attached form a saturated carbocycle, a saturated heterocycle, or do not form a ring;

when one of $R_2$ or $R_3$ is hydrogen, the other is not $C_{1-4}$ alkyl; or when $R_2$, $R_3$, $X_1$, and $X_4$ are all hydrogen, $X_2$ or $X_3$ is not

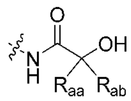, 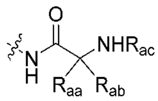,

$-NHCH_3$, - $NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

**7.** The ecteinascidin derivative according to claim 1 or 3, wherein in the general formula (I):

$R_1$ is selected from hydroxyl or cyano;

$R_2$ and $R_3$ are the same or different, and are each independently selected from hydrogen, deuterium, hydroxyl, mercapto, halogen, deuterated alkyl, haloalkyl, haloalkoxy, hydroxyl-substituted alkyl, amino-substituted alkyl, alkylamino-substituted alkyl, alkyl, alkoxy, alkylthio, $C_{2-6}$ alkenyl, $C_{2-6}$ alkynyl, cycloalkyl, alkyl-substituted amino, cycloalkyl-substituted alkyl, heterocyclyl, aryl, heteroaryl,

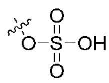, 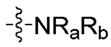, or 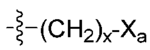;

$R_2$ and $R_3$ together with the carbon atoms to which they are attached form a saturated carbocycle, a saturated heterocycle, or do not form a ring;

x is selected from an integer of 1-10;

$R_a$ and $R_b$ are each independently selected from hydrogen, deuterium, hydroxyl, hydroxyl-substituted alkyl, alkyl, alkoxy, cycloalkyl, heterocyclyl, aryl, heteroaryl, alkyl-substituted sulfonyl, aryl-substituted sulfonyl, or heteroaryl-substituted sulfonyl; $R_a$ and $R_b$ together with the nitrogen atoms to which they are attached form a saturated heterocycle or do not form a ring;

$R_4$ is selected from hydrogen, alkyl, deuterated alkyl, haloalkyl, amino-substituted alkyl, or alkylamino-substituted alkyl, or

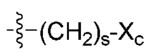;

s is selected from an integer of 1-10;

$R_5$ and $R_6$ are both hydrogen or alkyl, and $R_5$ and $R_6$ together with the carbon atoms to which they are attached form a saturated carbocycle, a saturated heterocycle, or do not form a ring; or $R_5$ and $R_6$ are different, and are each independently selected from hydrogen, alkyl, cycloalkyl, or cycloalkyl-substituted alkyl;

$X_1$ and $X_4$ are hydrogen;

$X_a$, $X_c$, $X_2$, and $X_3$ are each independently selected from hydrogen, halogen, $C_{1-4}$ alkyl, or

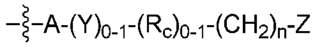,

wherein A is selected from oxygen, sulfur, -NH, $-NCD_3$, $-NCF_3$, $-NCH_3$, $-NCH_2CH_3$, or $-NCH_2CH_2CH_3$; Y is selected from -C(=O)-, $-S(=O)_2-$, or $-CHCF_3$; $R_c$ is selected from a 3- to 10-membered saturated or partially unsaturated heterocyclyl, a $C_{3-10}$ saturated or partially unsaturated carbocycle, or

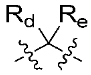;

n is selected from an integer of 0-10, and Z is selected from hydrogen, hydroxyl, mercapto, $-NH_2$, $-NHCH_3$, $-NHCH_2CH_3$, $-NHCH_2CH_2CH_3$, $-CD_3$, or $-CF_3$;
$R_d$ and $R_e$ are the same or different, and are each independently selected from hydrogen, deuterium, trifluoromethyl, alkyl, $C_{2-6}$ alkenyl, $C_{2-6}$ alkynyl, cycloalkyl, cycloalkyl-substituted alkyl, heterocyclyl, aryl, or heteroaryl;
$R_d$ and $R_e$ together with the carbon atoms to which they are attached form a saturated carbocycle, a saturated heterocycle, or do not form a ring;
when one of $R_2$ or $R_3$ is hydrogen, the other is not $C_{1-4}$ alkyl; when $R_2$ or $R_3$ are both hydrogen, $X_2$ or $X_3$ is not

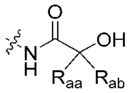, 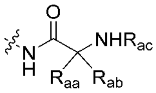,

$-NHCH_3$, $-NHCH_2CH_3$, or $-NHCH_2CH_2CH_3$, wherein $R_{aa}$, $R_{ab}$, and $R_{ac}$ are each independently selected from hydrogen or $C_{1-4}$ alkyl.

**8.** The ecteinascidin derivative according to claim 1 or 3, wherein in the general formula (I):

$R_1$ is selected from hydroxyl or cyano;
$R_2$ and $R_3$ are the same or different, and are each independently selected from hydrogen, hydroxyl, fluorine, trifluoromethyl, trifluoromethoxy, methyl, methoxy, cyclopropyl-substituted methyl,

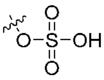, $-\xi-NR_aR_b$, or $-\xi-(CH_2)_x-X_a$;

$R_a$ and $R_b$ are each independently selected from hydrogen or methyl; $R_2$ and $R_3$ together with the carbon atoms to which they are attached form a 3-membered saturated carbocycle, a 4-membered oxygen-containing saturated heterocycle, or a 4-membered sulfur-containing saturated heterocycle; x is selected from an integer of 1-2;
$R_4$ is selected from hydrogen or methyl;
$R_5$ and $R_6$ are each independently selected from hydrogen or methyl; when both $R_5$ and $R_6$ are methyl, they together with the carbon atoms to which they are attached form a 3-membered saturated carbocycle;
$X_1$ and $X_4$ are each independently selected from hydrogen or methoxy;
$X_a$, $X_2$, and $X_3$ are each independently selected from hydrogen, fluorine, methyl, or

$$-\xi-A-(Y)_{0-1}-(R_c)_{0-1}-(CH_2)_n-Z,$$

wherein A is selected from oxygen, $-NCH_3$, or -NH; Y is selected from -C(=O)-; $R_c$ is selected from a $C_{3-6}$ saturated carbocycle or

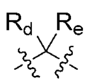;

n is selected from an integer of 0-1, and Z is selected from hydrogen or hydroxyl;
$R_d$ and $R_e$ are the same or different, and are each independently selected from hydrogen, trifluoromethyl, cyclopropyl, or cyclopropyl-substituted methyl; $R_d$ and $R_e$ together with the carbon atoms to which they are attached form a 3- to 5-membered saturated carbocycle;
when one of $R_2$ or $R_3$ is hydrogen, the other is not methyl; or when $R_2$, $R_3$, $X_1$, and $X_4$ are all hydrogen, $X_2$ or $X_3$ is not

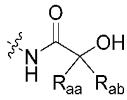, 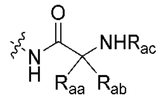,

-$NHCH_3$, - $NHCH_2CH_3$, or -$NHCH_2CH_2CH_3$, wherein $R_{aa}$, $R_{ab}$, and $R_{ac}$ are each independently selected from hydrogen or $C_{1-4}$ alkyl.

**9.** The ecteinascidin derivative according to claim 1, wherein the compound in the general formula (I) is selected from at least one of the following structural formula compounds,

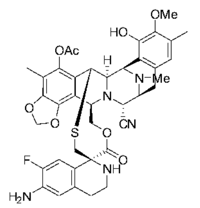
**P1**

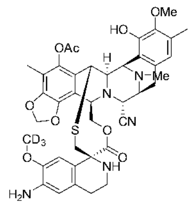
**P2**

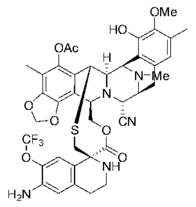
**P3**

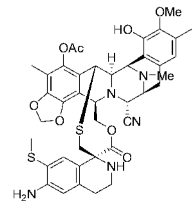
**P4**

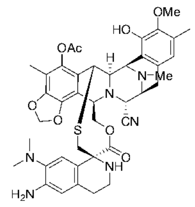
**P5**

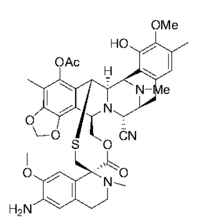
**P6**

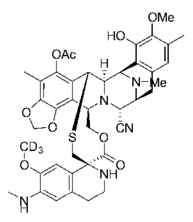
**P7**

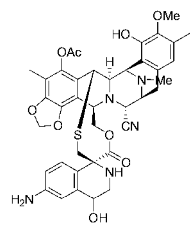
**P8**

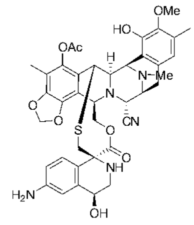
**P9**

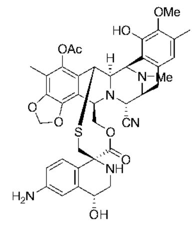
**P10**

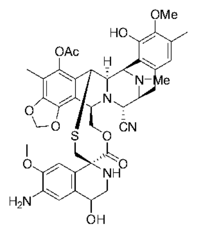
**P11**

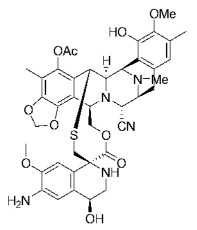
**P12**

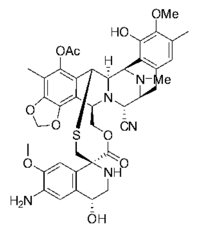
**P13**

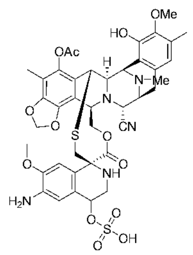
**P14**

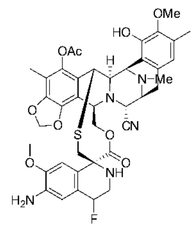
**P15**

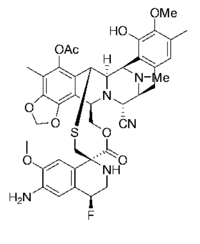
**P16**

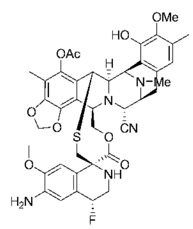
**P17**

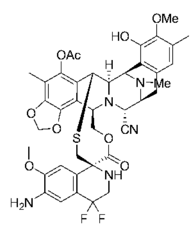
**P18**

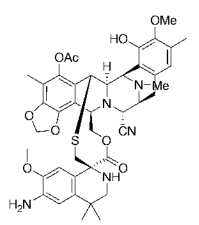
**P19**

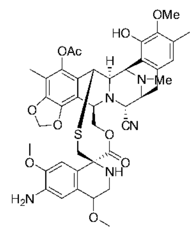
**P20**

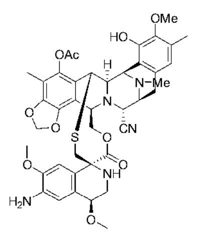
**P21**

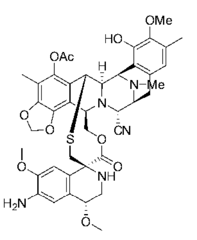
**P22**

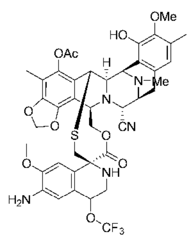
**P23**

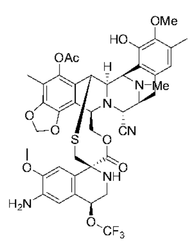
**P24**

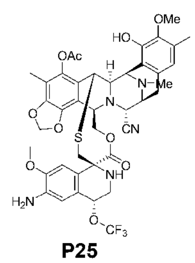
**P25**

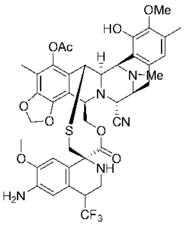
P26

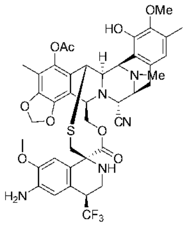
P27

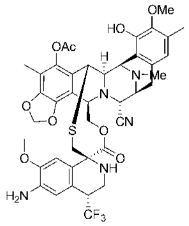
P28

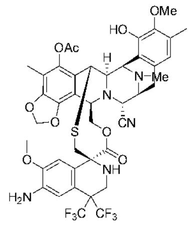
P29

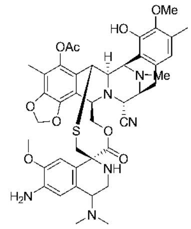
P30

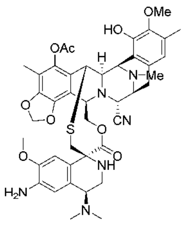
P31

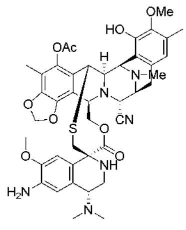
P32

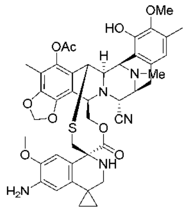
P36

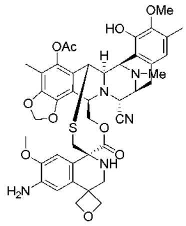
P37

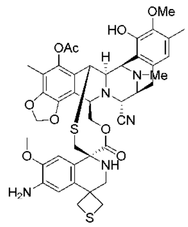
P38

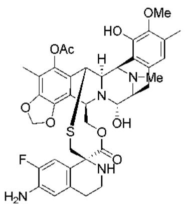
P43

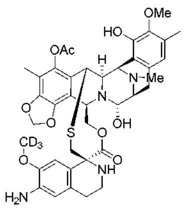
P44

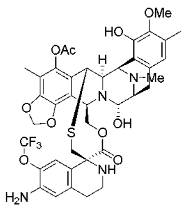
P45

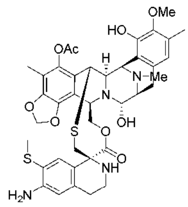
P46

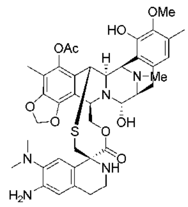
P47

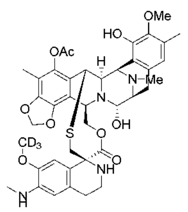
P48

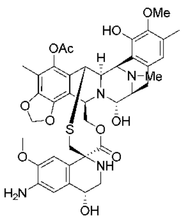
P49

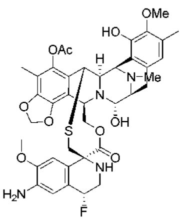
P50

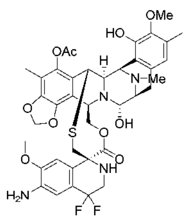
P51

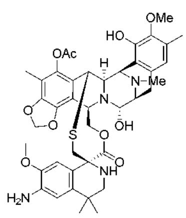
P52

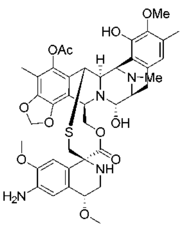
P53

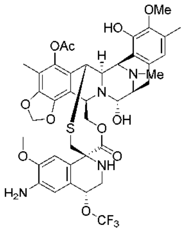
P54

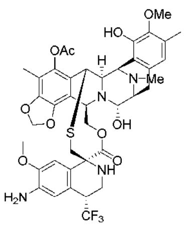
P55

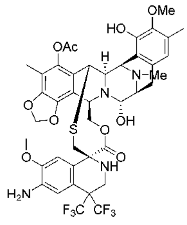
P56

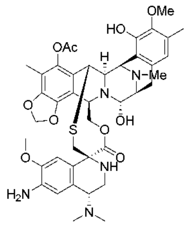
P57

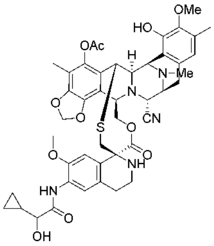
P58

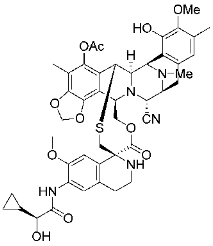
P59

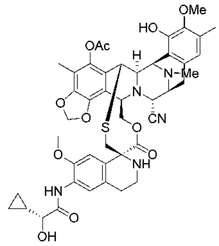
P60

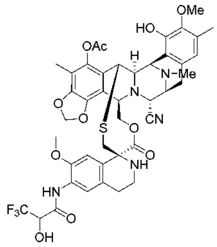
P61

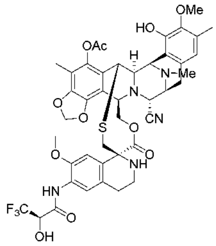
P62

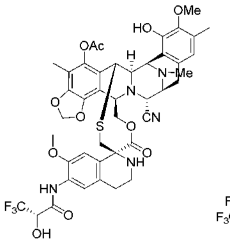
P63
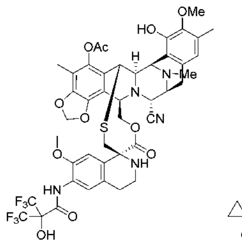
P64
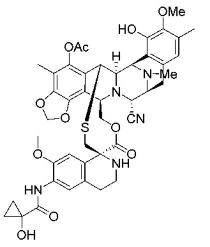
P65
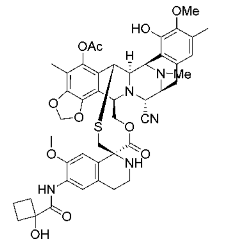
P66
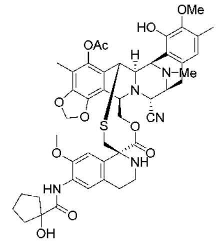
P67

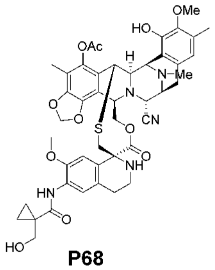
P68
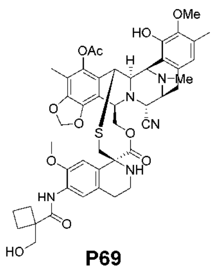
P69
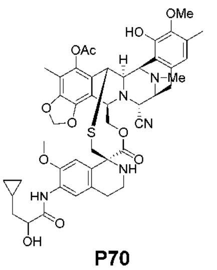
P70
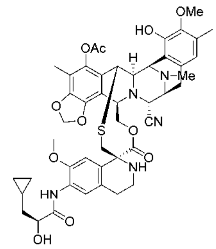
P71
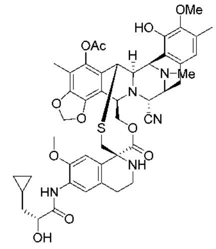
P72

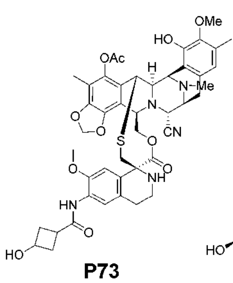
P73
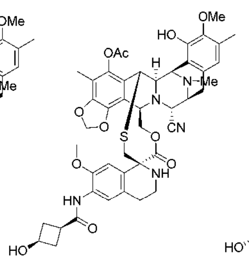
P74
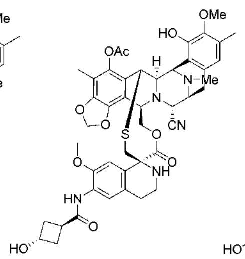
P75
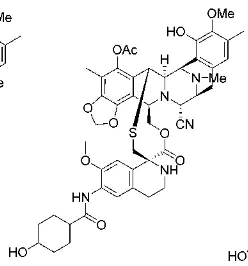
P76
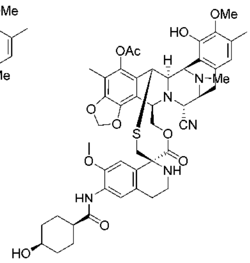
P77

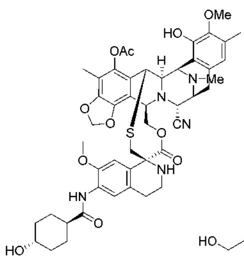
P78
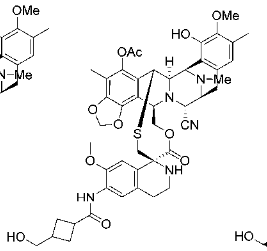
P79
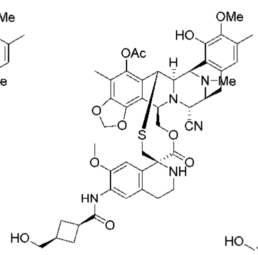
P80
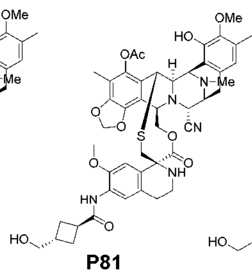
P81
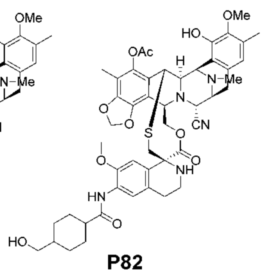
P82

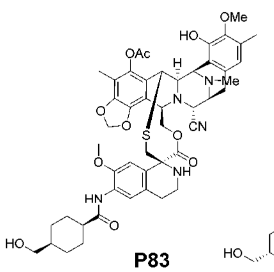
P83
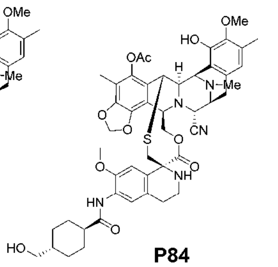
P84
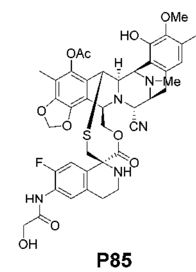
P85
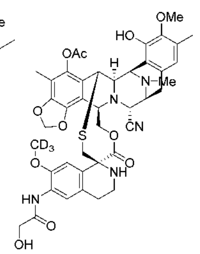
P86
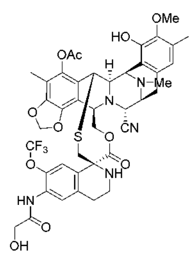
P87

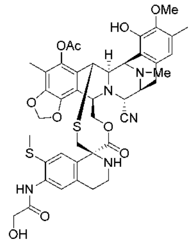
P88

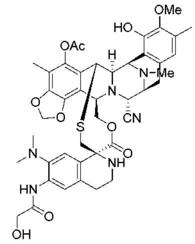
P89

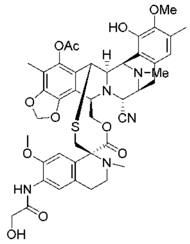
P90

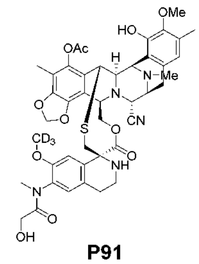
P91

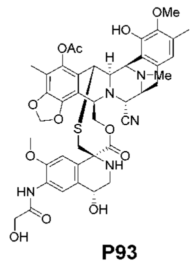
P93

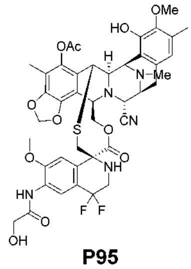
P95

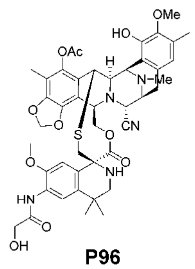
P96

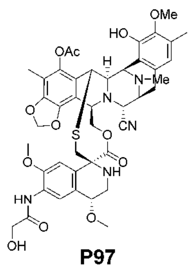
P97

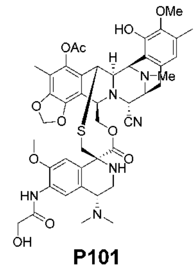
P101

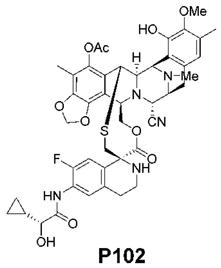
P102

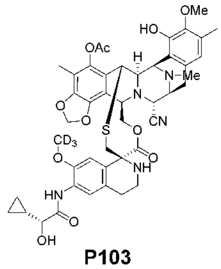
P103

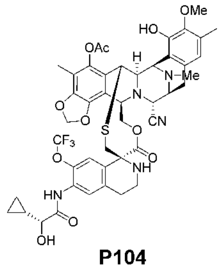
P104

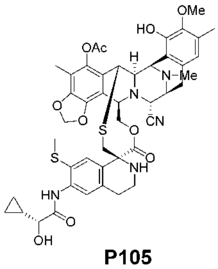
P105

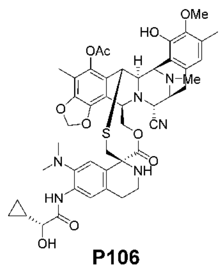
P106

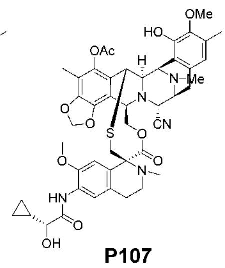
P107

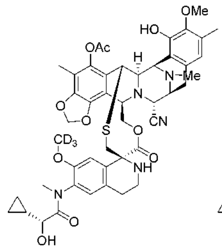
P108

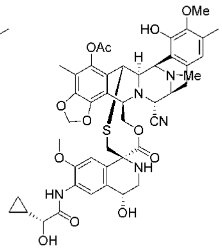
P110

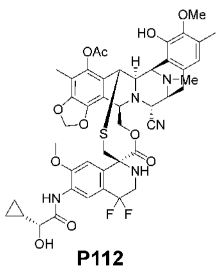
P112

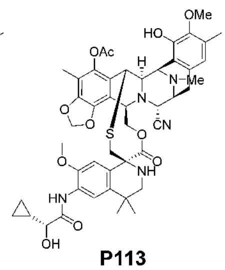
P113

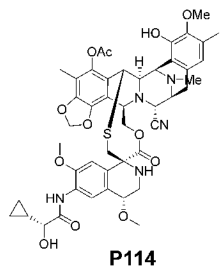
P114

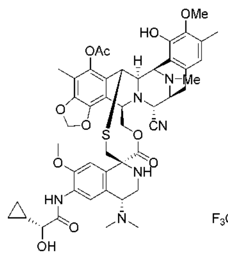
P118

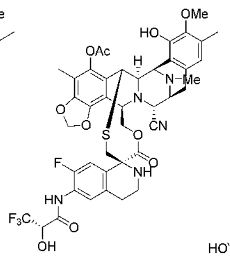
P119

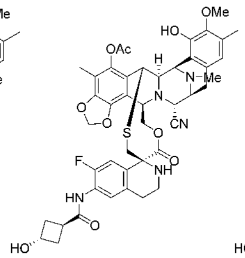
P136

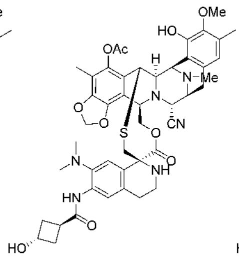
P140

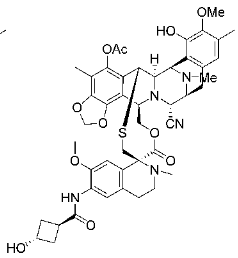
P141

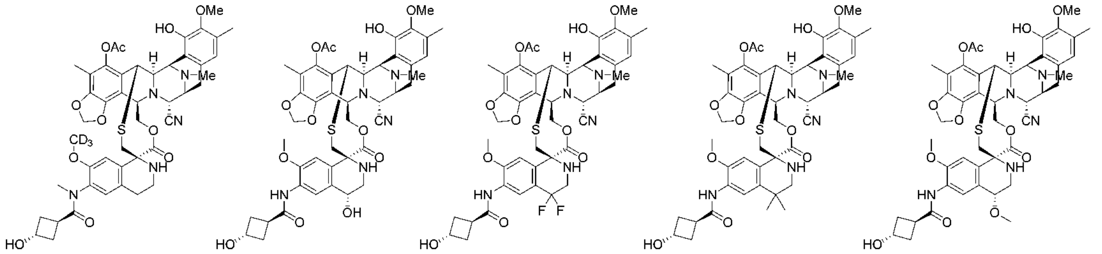

P142 P144 P146 P147 P148

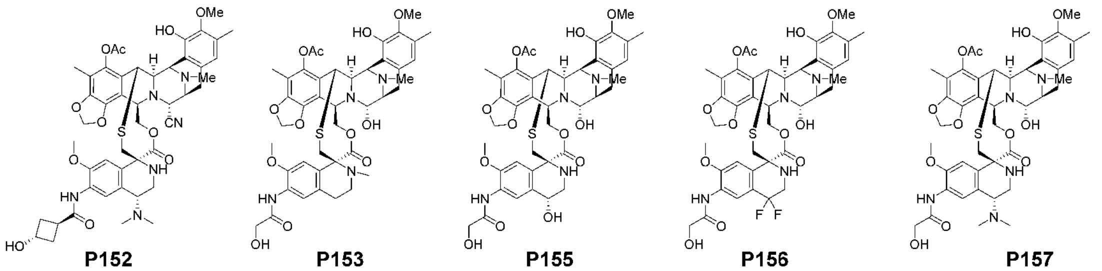

P152 P153 P155 P156 P157

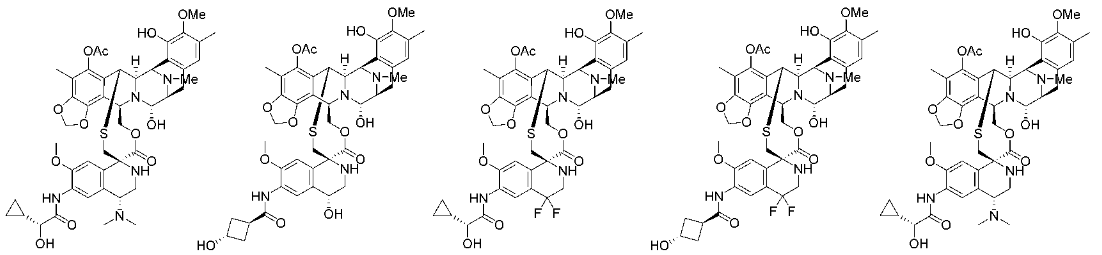

P158 P160 P163 P164 P165

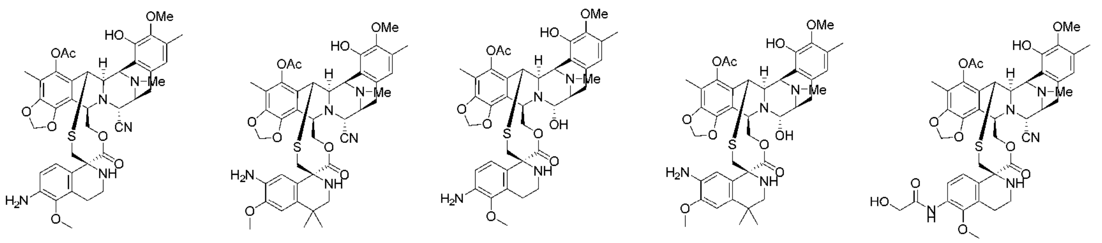

P166 P167 P168 P169 P170

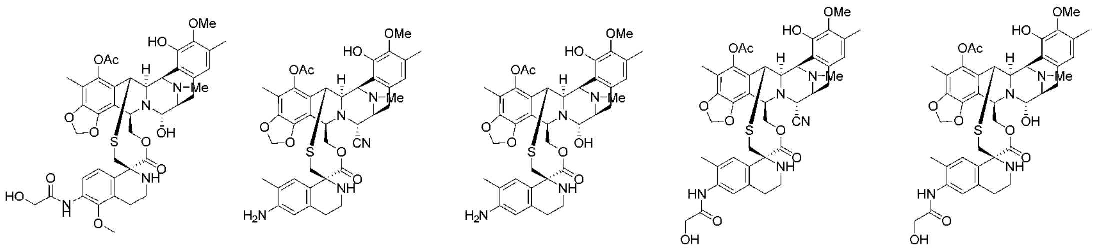

P171 P172 P173 P174 P175

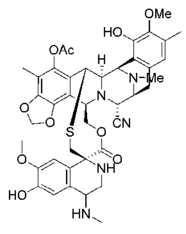
P186

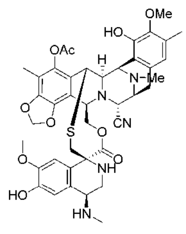
P187

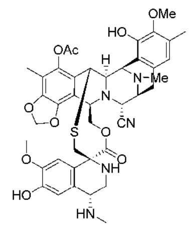
P188

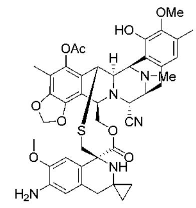
P241

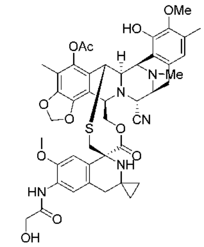
P253

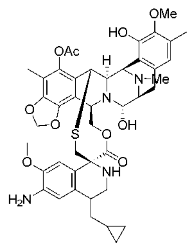
P260

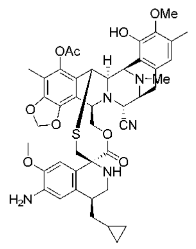
P261

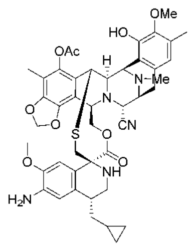
P262

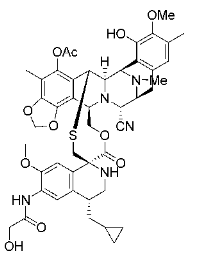
P264

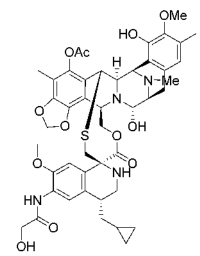
P265

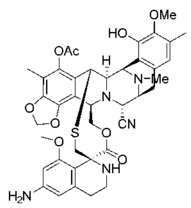
T1

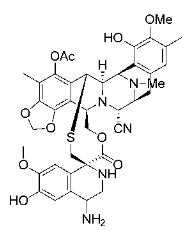
T2

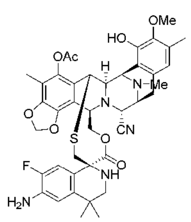
T3

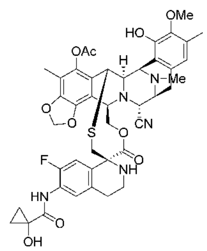
T4

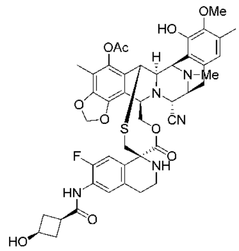
T5

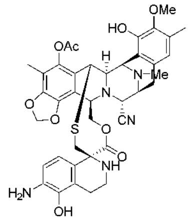
T6

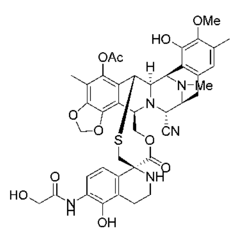
T7

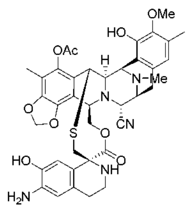
T8

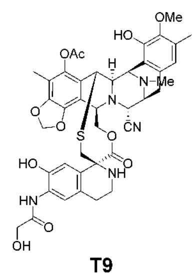
T9

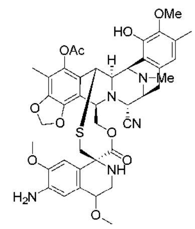
T10

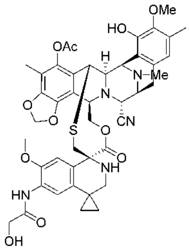
T11

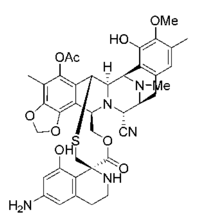
T12

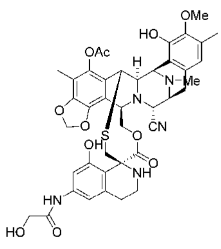
T13

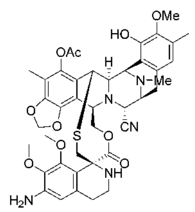
T14

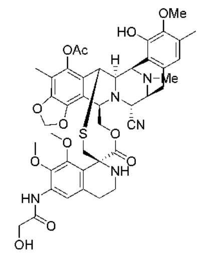
T15

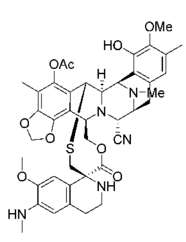 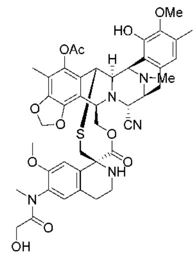 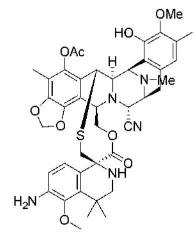 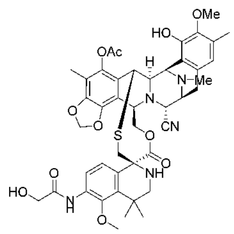

T16 T17 T18 T19

**10.** The ecteinascidin derivative according to claim 9, wherein in the compounds P1-P32, P36-P38, P58-P91, P93, P95-P97, P101-P108, P110, P112-P114, P118, P119, P136, P140-P142, P144, P146-P148, P152, P166, P167, P170, P172, P174, P186-P188, P241, P253, P261-P262, P264, and T1-T19, the - CN at the $R_1$ position is replaced by -OH.

**11.** The ecteinascidin derivative according to claim 9, wherein the derivative comprises a single chiral component or a mixture of non-enantiomeric isomers.

**12.** The ecteinascidin derivative according to any one of claims 1 to 3, wherein the ecteinascidin derivative further comprises a pharmaceutically acceptable salt, a tautomer, or a stereoisomer of the compound of the general formula (I).

**13.** An application of the ecteinascidin derivative according to any one of claims 1 to 12 in the preparation of an anticancer drug.

**14.** An antibody-drug conjugate, **characterized in that** the antibody-drug conjugate comprises the ecteinascidin derivative according to any one of claims 1 to 12 and an antibody.

**15.** An application of the antibody-drug conjugate according to claim 14 in the preparation of an anticancer drug.

**16.** A compound of general formula (III), or a pharmaceutically acceptable salt, a tautomer, or a stereoisomer thereof, **characterized in that**:

$$M—L_1—L_2—D$$

General formula (III)

wherein M is selected from

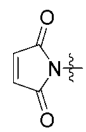, 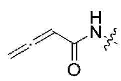, 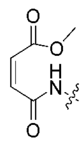, 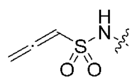,

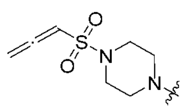, 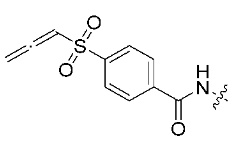, 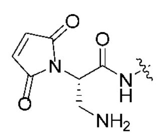, 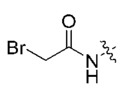 or 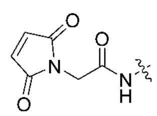;

$L_1$ is selected from

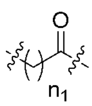, 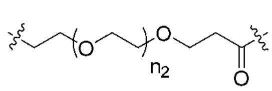,

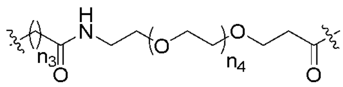, 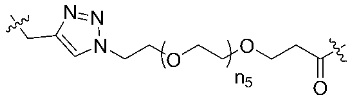,

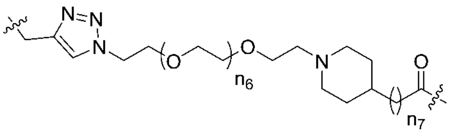,

or a combination thereof; $n_1$ is an integer of 1-20, $n_2$ is an integer of 0-20, $n_3$ is an integer of 1-20, $n_4$ is an integer of 0-20, $n_5$ is an integer of 0-20, $n_6$ is an integer of 0-20, and $n_7$ is an integer of 0-20;

$L_2$ is

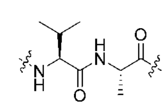, 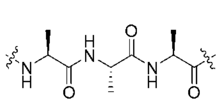, 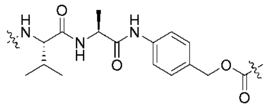,

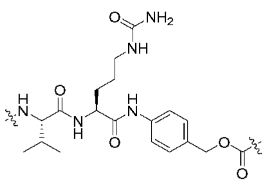, 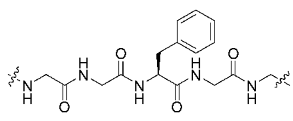, 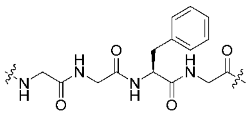,

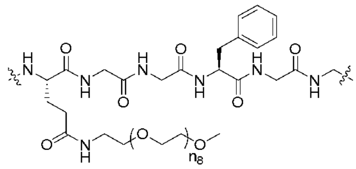, 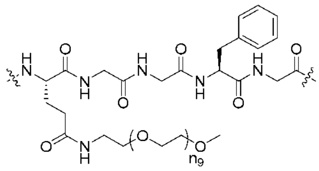, 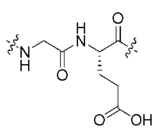,

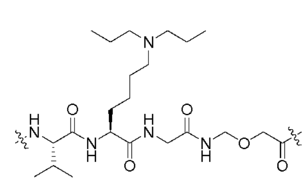, 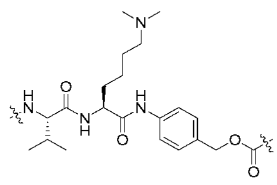, 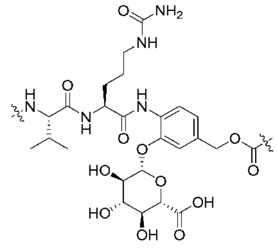,

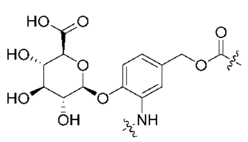, 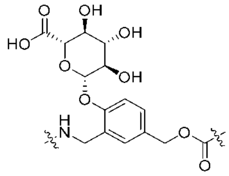, 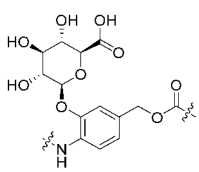, 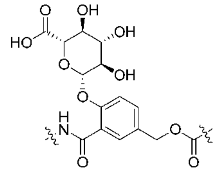,

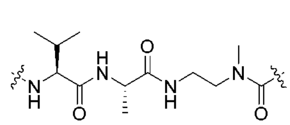, 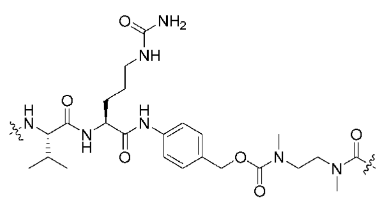,

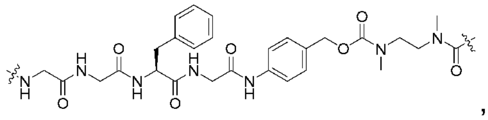, 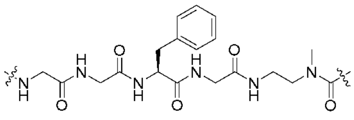,

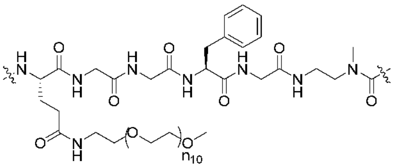 or 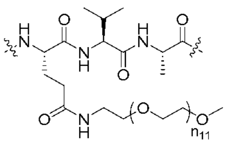;

$n_8$ is an integer of 0-20, $n_9$ is an integer of 0-20, $n_{10}$ is an integer of 0-20, and $n_{11}$ is an integer of 0-20;
D is selected from a product moiety formed by the ecteinascidin derivative according to any one of claims 1 to 12 by loss of one or more atoms or groups;
wherein D is covalently bonded to $L_2$ through a nitrogen or oxygen atom in D.

**17.** The compound according to claim 16, wherein in general formula (III),

wherein M is selected from

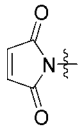, 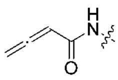, 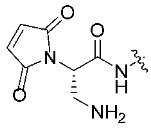, 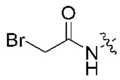 or

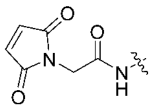;

$L_1$ is selected from

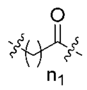, 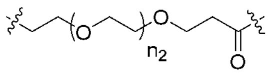, 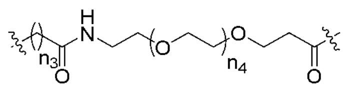,

or a combination thereof; $n_1$ is an integer of 1-20, nz is an integer of 0-20, $n_3$ is an integer of 1-20, or $n_4$ is an integer of 0-20;

$L_2$ is 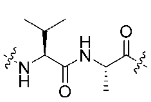, 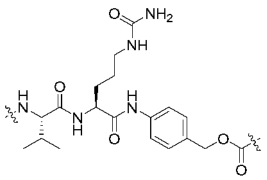, 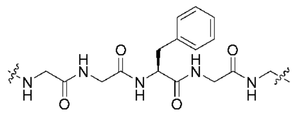,

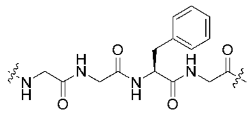, 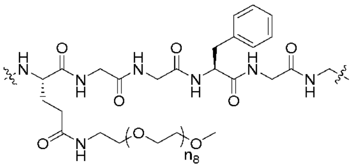, 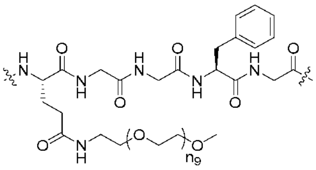,

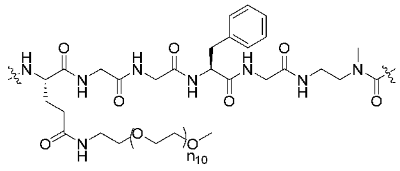 or 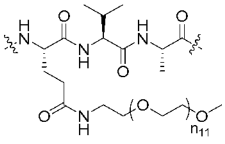 ;

$n_8$ is an integer of 0-20, $n_{10}$ is an integer of 0-20, or $n_{11}$ is an integer of 0-20;
D is selected from a product moiety formed by the ecteinascidin derivative according to any one of claims 1 to 12 by loss of one or more atoms or groups;
D is covalently bonded to L2 through a nitrogen or oxygen atom in any one of $X_1$, $X_2$, $X_3$, $X_4$, $R_2$, $R_3$, or $R_4$ in D.

**18.** The compound according to claim 16, wherein in general formula (III),

wherein M is selected from

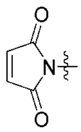 , 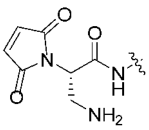 , 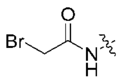 or 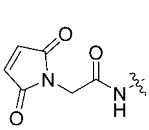 ;

$L_1$ is selected from

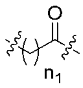 , 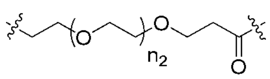 , 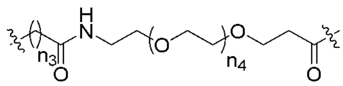 ,

or a combination thereof; $n_1$ is an integer of 1-18, nz is an integer of 0-18, $n_3$ is an integer of 1-18, $n_4$ is an integer of 0-18;
$L_2$ is

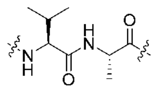 , 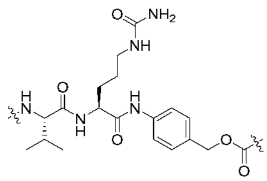 , 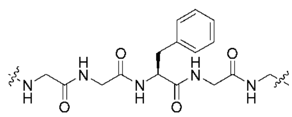 ,

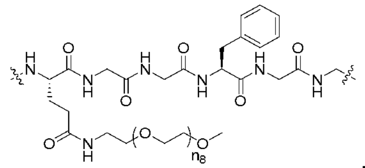 , 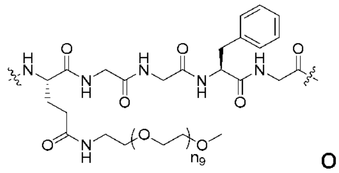 or 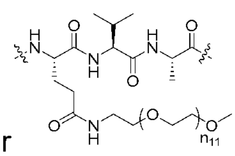 ;

$n_8$ is an integer of 0-18, or $n_{11}$ is an integer of 0-18;
D is selected from a product moiety formed by the ecteinascidin derivative according to any one of claims 1 to 12 by loss of one or more atoms or groups;
D is covalently bonded to $L_2$ through a nitrogen or oxygen atom in any one of $X_1$, $X_2$, $X_3$, $X_4$, $R_2$, $R_3$, or $R_4$ in D.

**19.** The compound according to claim 16, wherein in general formula (III),

wherein M is selected from

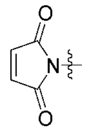, 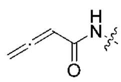 or 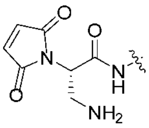;

$L_1$ is selected from

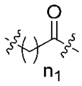, 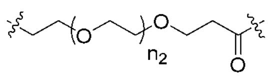, 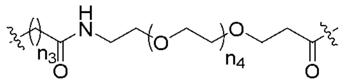,

or a combination thereof; $n_1$ is an integer of 1-20, nz is an integer of 1-20, $n_3$ is an integer of 1-20, or $n_4$ is an integer of 1-20;

$L_2$ is 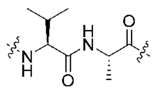, 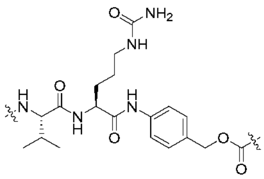, 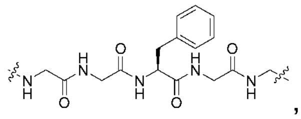,

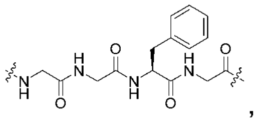, 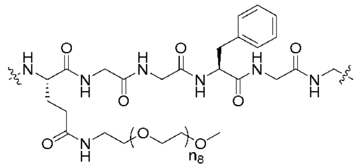 or 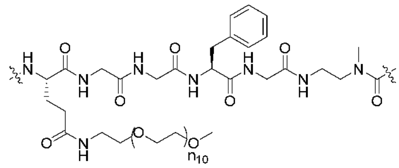;

$n_8$ is an integer of 1-20, $n_{10}$ is an integer of 1-20;
D is selected from a product moiety formed by the ecteinascidin derivative according to any one of claims 1 to 12 by loss of one or more atoms or groups;
D is covalently bonded to $L_2$ through a nitrogen or oxygen atom in any one of $X_1$, $X_2$, $X_3$, $X_4$, $R_2$, $R_3$, or $R_4$ in D.

**20.** The compound according to claim 16, wherein the compound of general formula (III) is selected from at least one of the compounds in the following table:

| No. | Structure |
|---|---|
| LP1 | 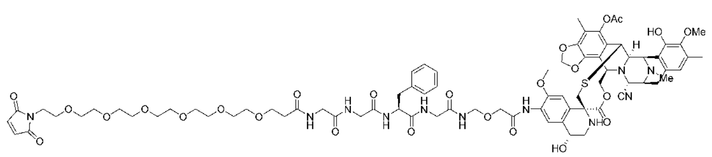 |
| LP2 | 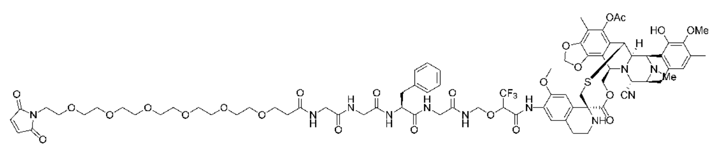 |
| LP3 | 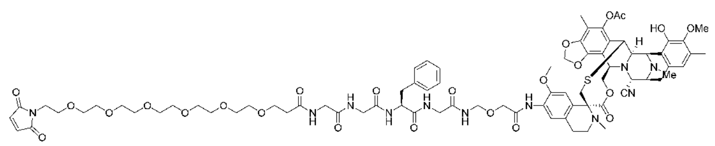 |

(continued)

| No. | Structure |
|---|---|
| LP4 | 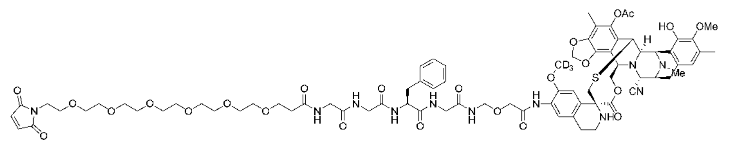 |
| LP5 | 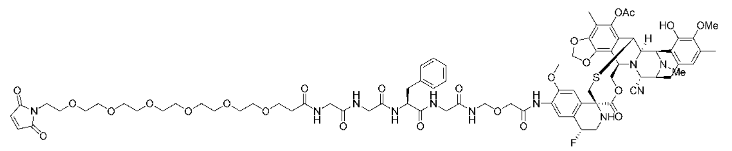 |
| LP6 | 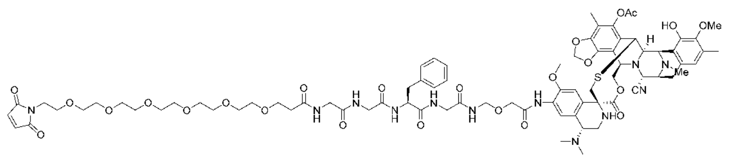 |
| LP7 | 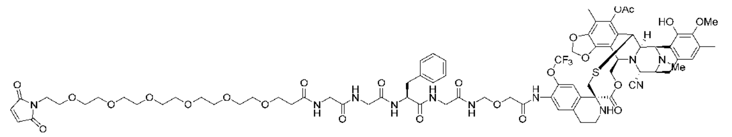 |
| LP8 | 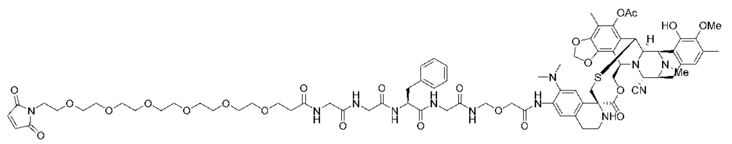 |
| LP9 | 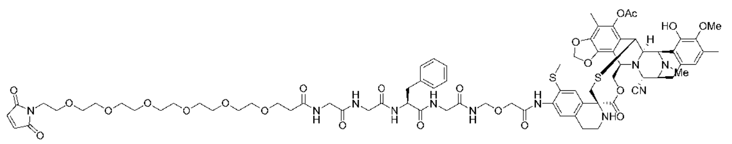 |
| LP10 | 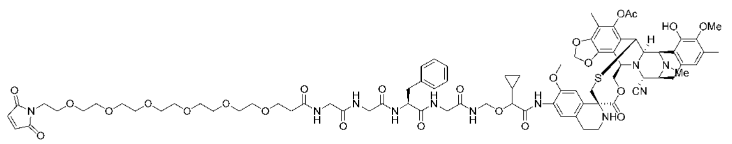 |
| LP11 | 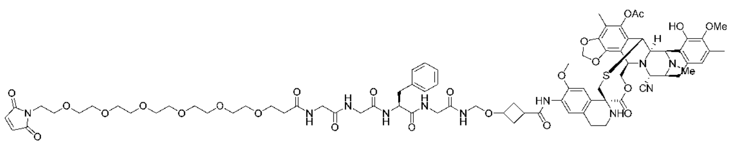 |
| LP12 | 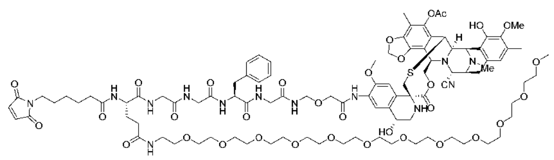 |
| LP13 | 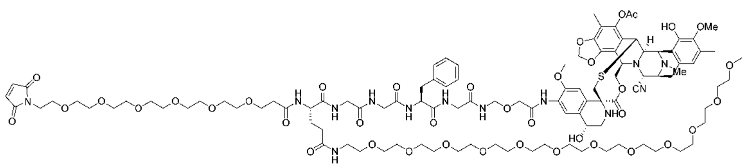 |

(continued)

| No. | Structure |
|---|---|
| LP14 | 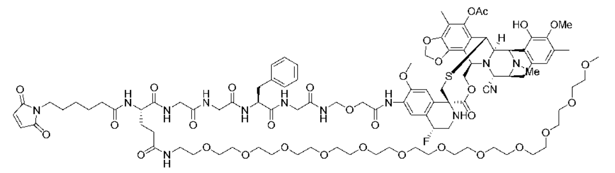 |
| LP15 | 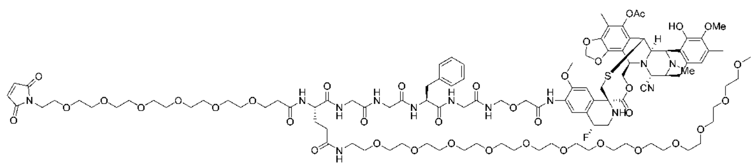 |
| LP16 | 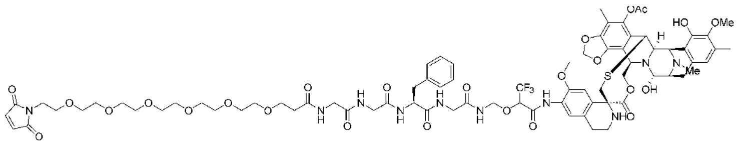 |
| LP17 | 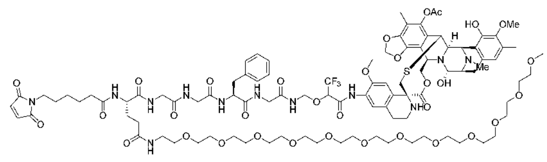 |
| LP18 | 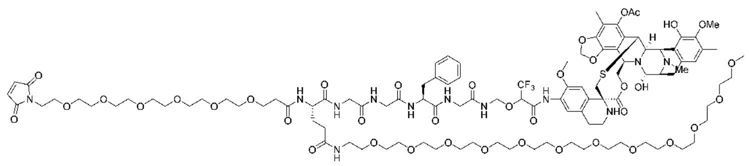 |
| LP19 | 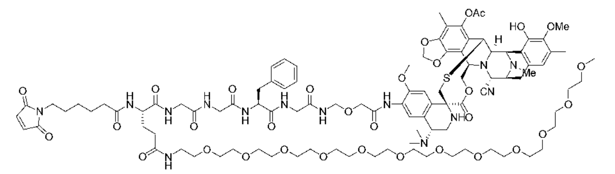 |
| LP27 | 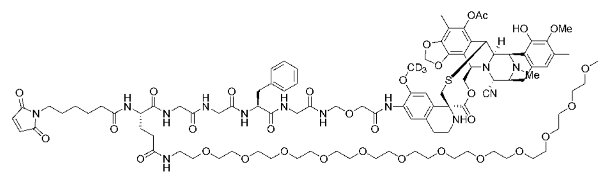 |
| LP28 | 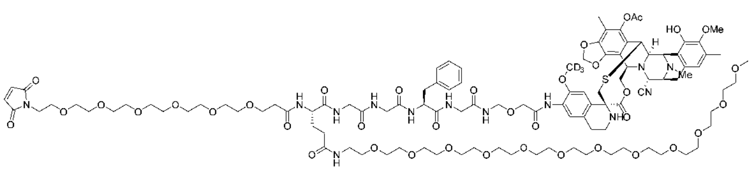 |
| LP29 | 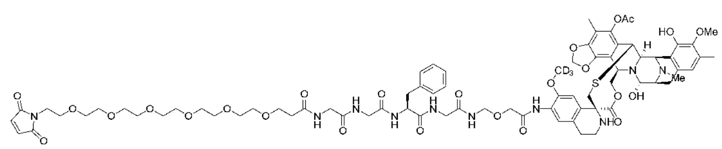 |

(continued)

| No. | Structure |
|---|---|
| LP30 | 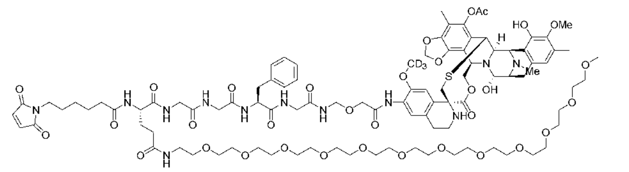 |
| LP31 | 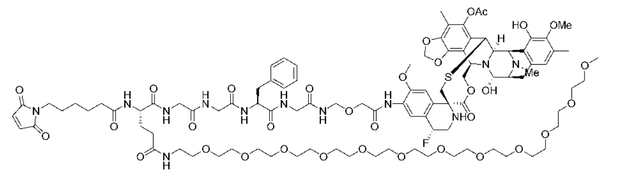 |
| LP34 | 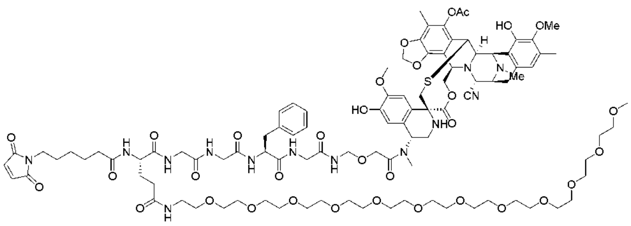 |
| LP58 | 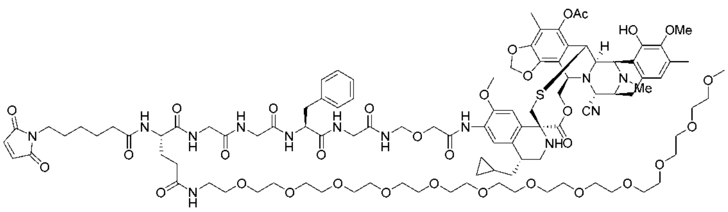 |
| LP59 | 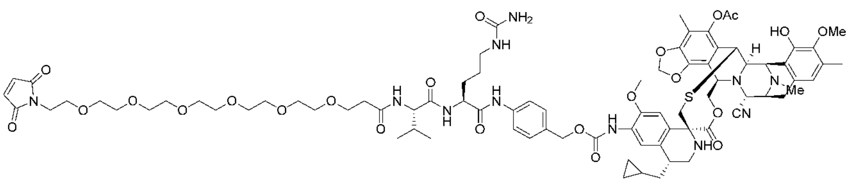 |
| LP62 | 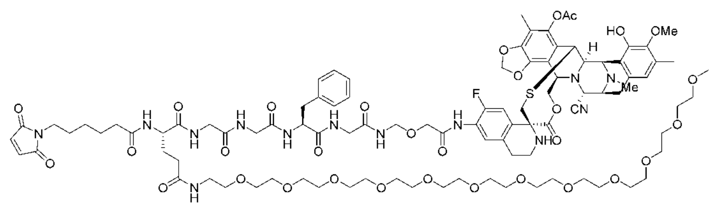 |
| LP63 | 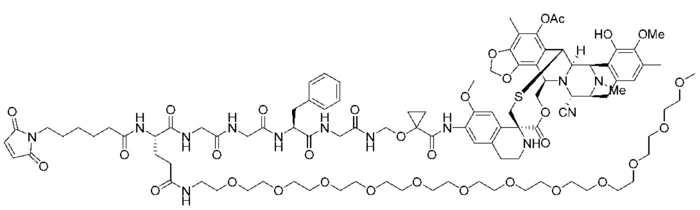 |
| LP64 | 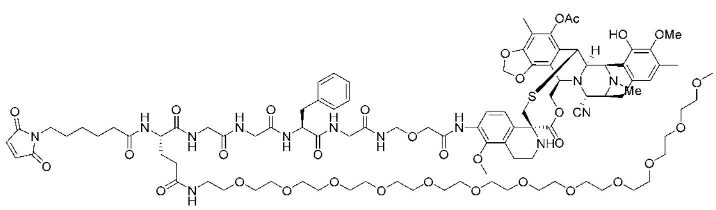 |

(continued)

| No. | Structure |
|---|---|
| LP65 | 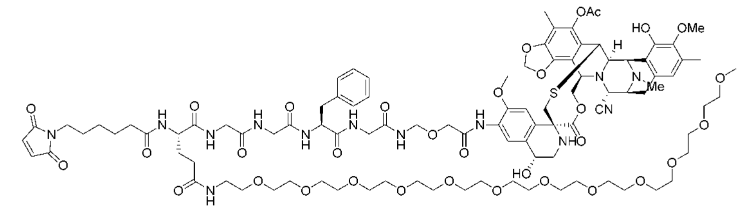 |
| LP66 | 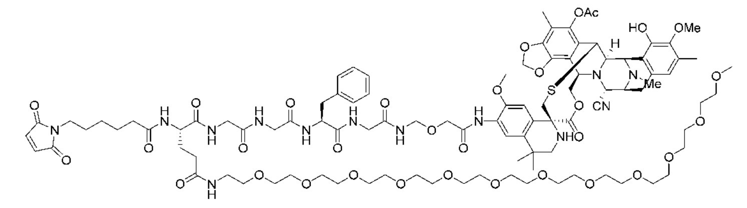 |
| LP67 | 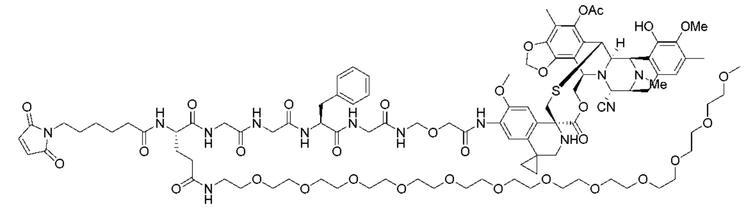 |
| LP68 | 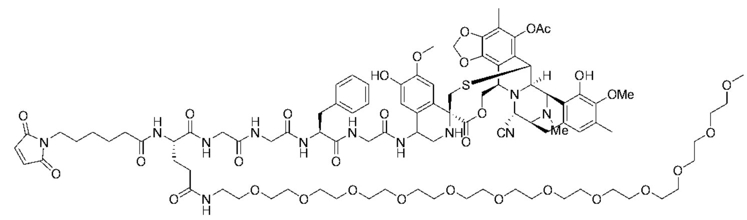 |
| LP69 | 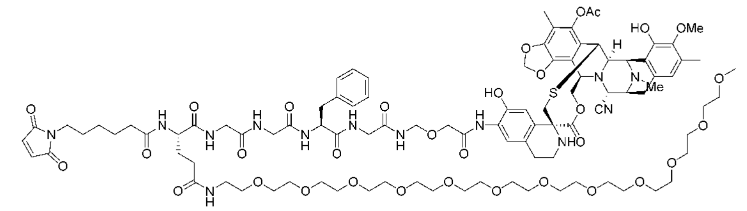 |
| LP70 | 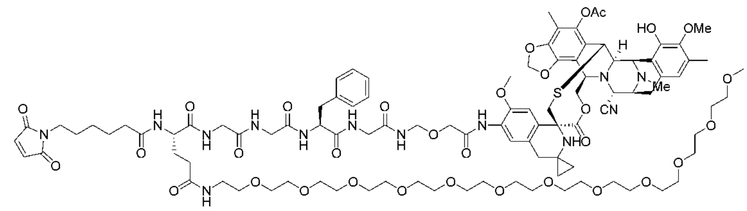 |
| LP71 | 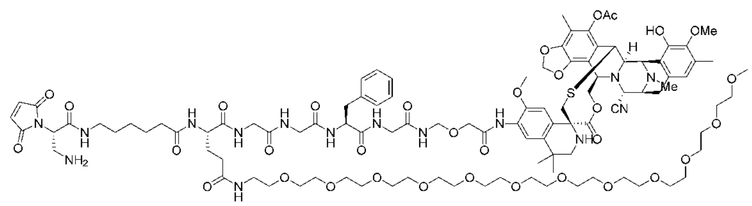 |

(continued)

| No. | Structure |
|---|---|
| LP72 | 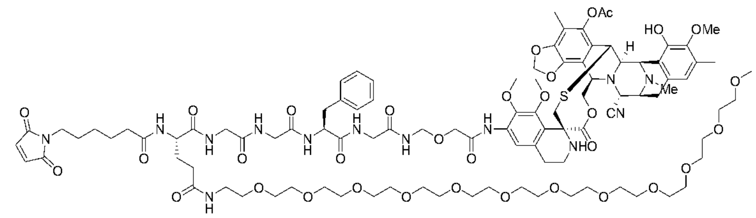 |
| LP73 | 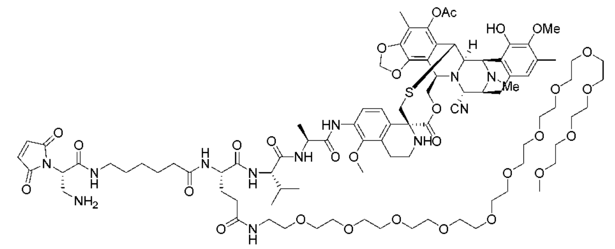 |
| LP74 | 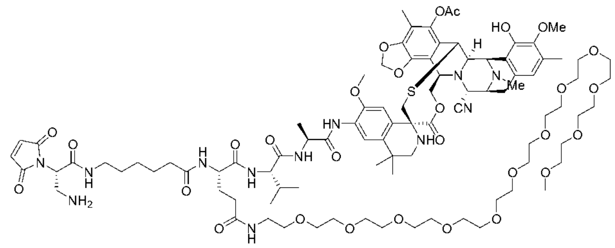 |
| LP75 | 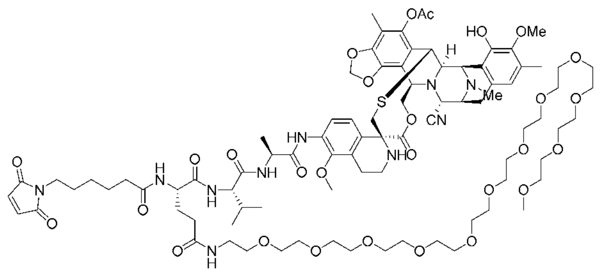 |
| LP76 | 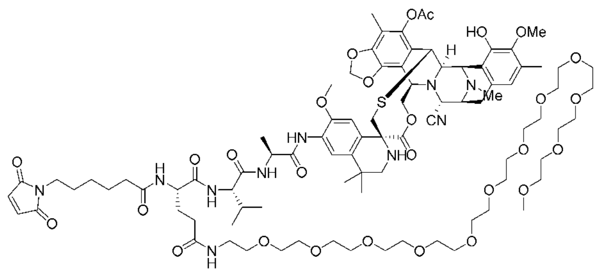 |
| LP77 | 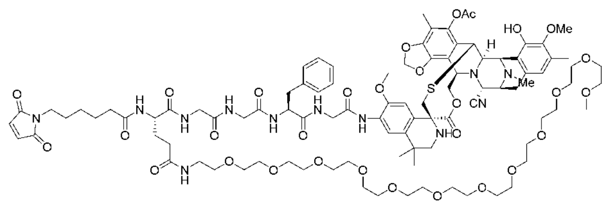 |

(continued)

| No. | Structure |
|---|---|
| LP78 | 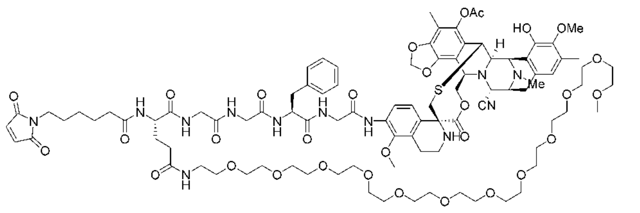 |
| LP79 | 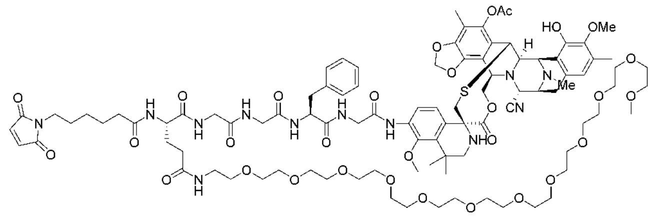 |
| LP80 | 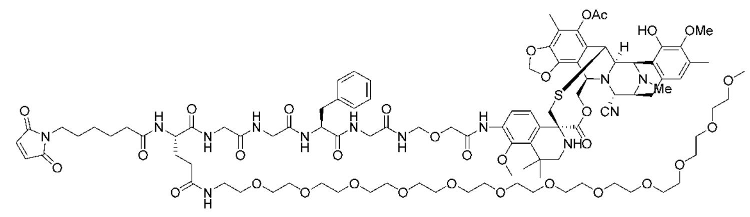 |

**21.** An antibody-drug conjugate, **characterized in that** the antibody-drug conjugate comprises a structure as shown in general formula (IV):

$$Ab\left(-M-L_1-L_2-D\right)_{n_{12}}$$

General formula (IV)

wherein Ab is an antibody, an antibody fragment, or a protein, wherein the antibody is selected from a murine antibody, a rabbit antibody, a phage display-derived antibody, a yeast display-derived antibody, a chimeric antibody, a humanized antibody, a fully human antibody, an antibody fragment, a bispecific antibody, and a multispecific antibody; M-$L_1$-$L_2$-D is selected from any compound according to any one of claims 16 to 20, or a pharmaceutically acceptable salt thereof, or a tautomer or stereoisomer thereof; and $n_{12}$ is any numerical value selected from 0 to 20.

**22.** The antibody-drug conjugate according to claim 21, **characterized in that** $n_{12}$ is any numerical value selected from 1 to 20.

**23.** The antibody-drug conjugate according to claim 21, **characterized in that** in general formula (IV), the Ab is selected from Abciximab, Alemtuzumab, Anetumab, Atezolizumab, Avelumab, Basiliximab, Bevacizumab, Blinatomumab, Brentuximab, Catumaxomab, Cetuximab, Cirmtuzumab, Coltuximab, Daclizumab, Daratumumab, Denintuzumab, Denosumab, Depatuxizumab, Dinutuximab, Durvalumab, Elotuzumab, Enfortumab, Glembatumumab, Gemtuzumab, Ibritumomab, Indatuximab, Indusatumab, Inotuzumab, Ipilimumab, Labetuzumab, Ladiratuzumab, Laprituximab, Lifastuzumab, Lorvotuzumab, Milatuzumab, Mirvetuximab, Naratuximab, Necitumumab, Nimotuzumab, Nivolumab, Obinutuzumab, Ofatumumab, Olaratumab, Omalizumab, Palivizumab, Panitumumab, Patritumab, Pembrolizumab, Pertuzumab, Pinatuzumab, Polatuzumab, Ramucirumab, Rovalpituzumab, Sacituzumab, Siltuximab, Sirtratumab, Sofituzumab, Vadastuximab, Vorsetuzumab, Trastuzumab, an anti-CD4 antibody, an anti-CD5 antibody, an anti-CD13 antibody, an anti-CD30 antibody, an antigen-binding fragment thereof, or an immunologically active portion thereof; M-$L_1$-$L_2$-D is selected from a compound according to any one of claims 16 to 20, or a pharmaceutically acceptable salt thereof, or a tautomer or stereoisomer thereof; and $n_{12}$ is any numerical value selected from 0 to 12.

**24.** The antibody-drug conjugate according to claim 21, wherein in general formula (IV), the Ab is selected from Patritumab, Sacituzumab, Mirvetuximab, Cetuximab, Trastuzumab, or Cirmtuzumab; M-$L_1$-$L_2$-D is selected from a compound according to any one of claims 16 to 20, or a pharmaceutically acceptable salt thereof, or a tautomer or stereoisomer thereof; and $n_{12}$ is any numerical value selected from 0 to 8.

**25.** The antibody-drug conjugate according to claim 21, wherein the antibody-drug conjugate in general formula (IV) is selected from at least one compound of the following structural formulas:

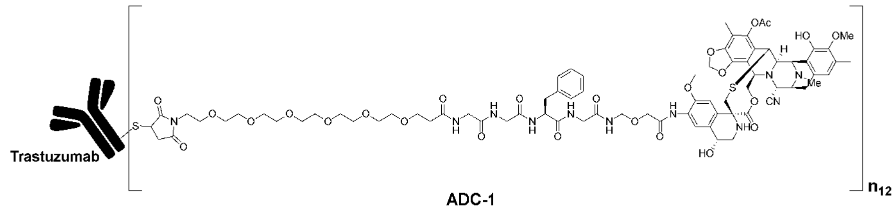

ADC-1

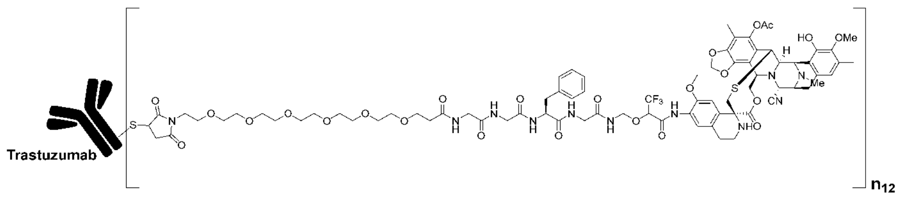

ADC-2

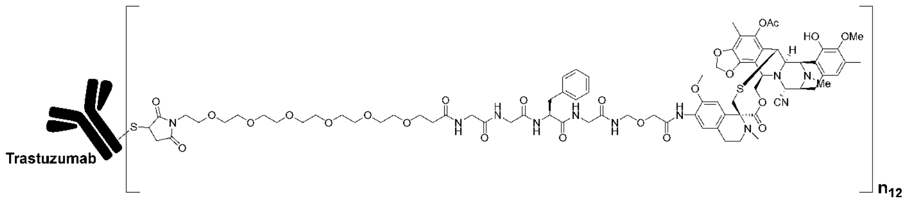

ADC-3

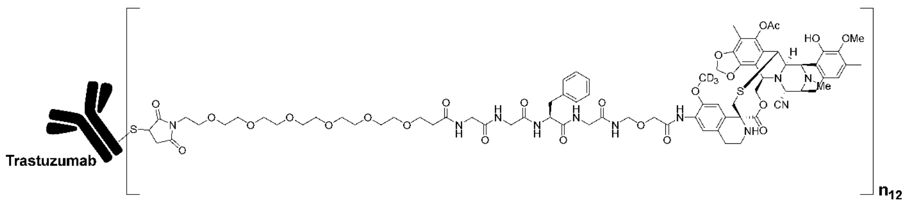

ADC-4

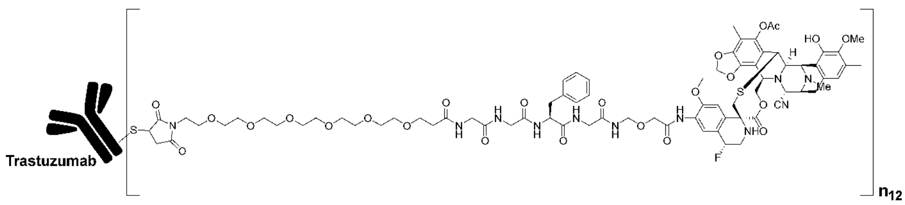

ADC-5

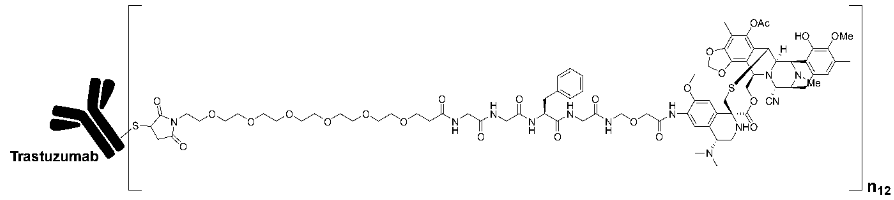

ADC-6

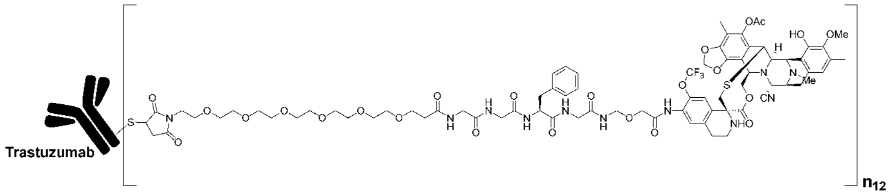

ADC-7

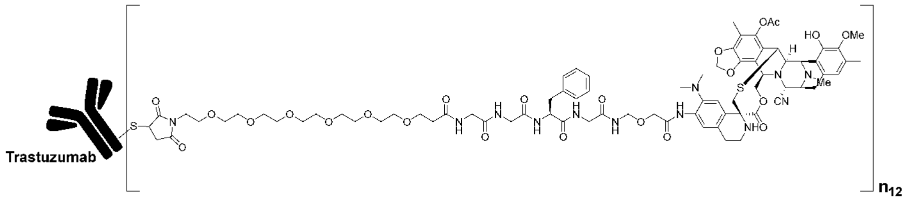

ADC-8

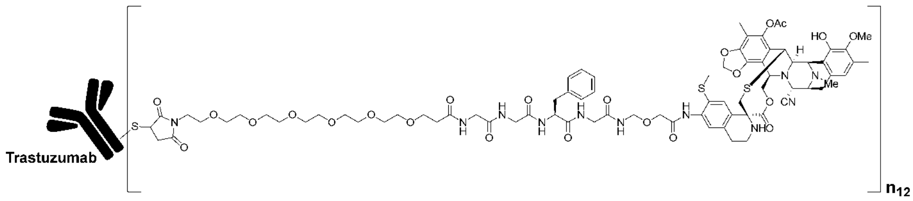

ADC-9

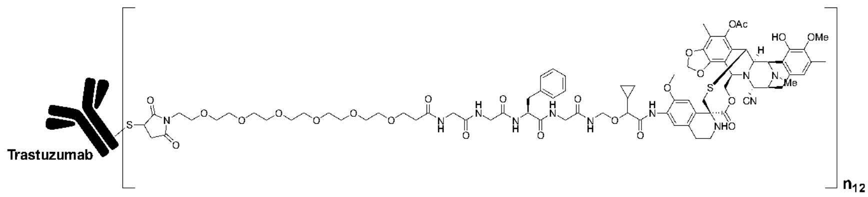

ADC-10

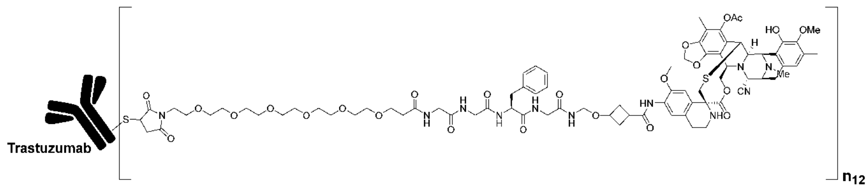

ADC-11

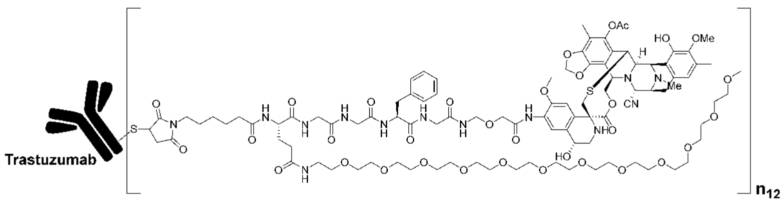

ADC-12

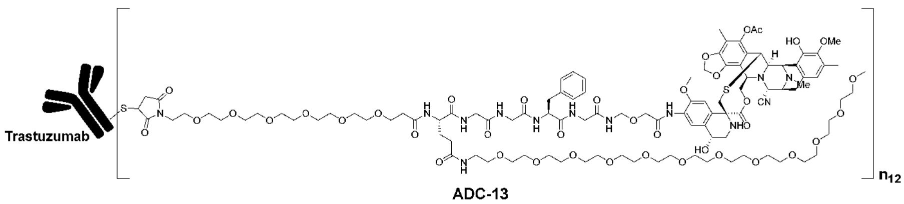

ADC-13

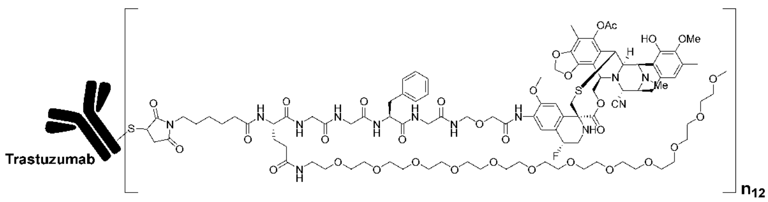

ADC-14

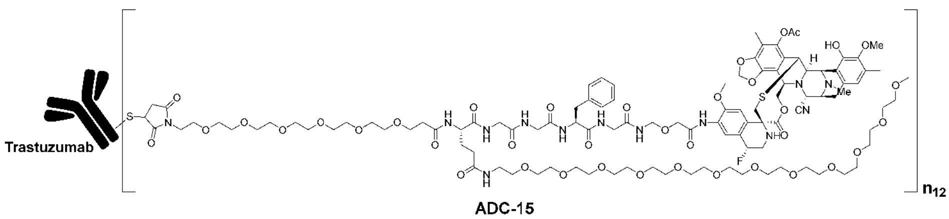

ADC-15

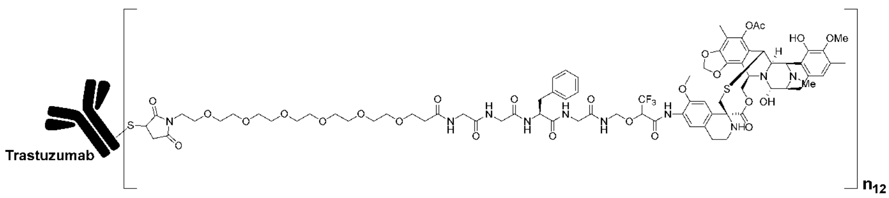

ADC-16

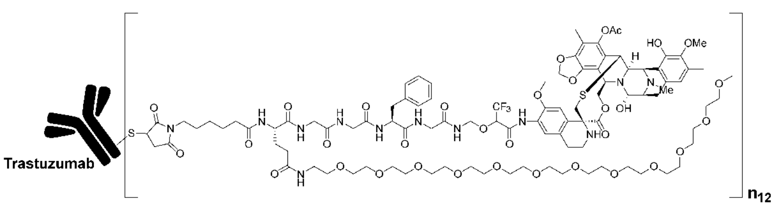

ADC-17

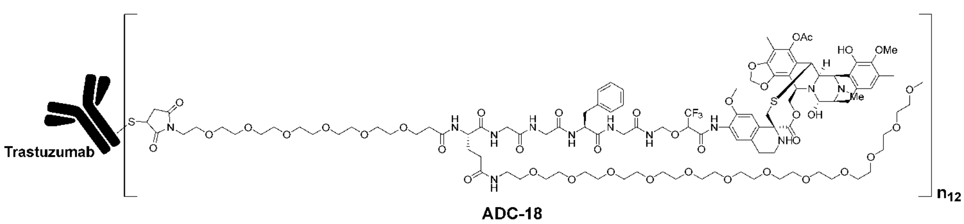

ADC-18

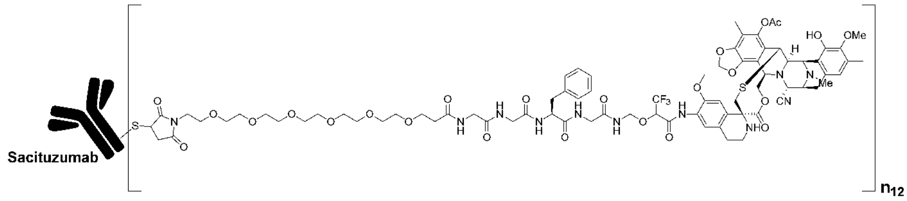

ADC-26

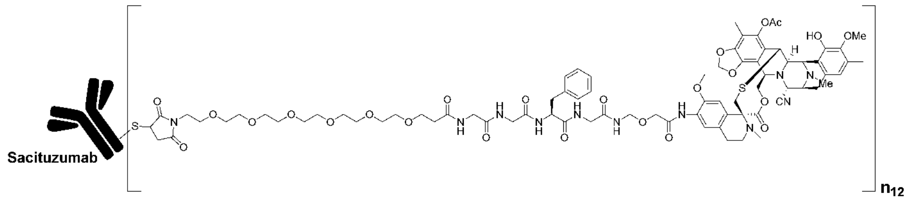

ADC-27

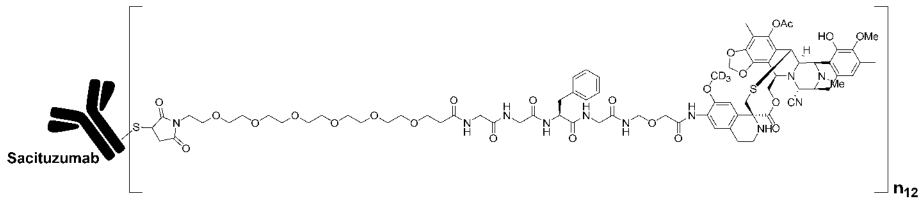

ADC-28

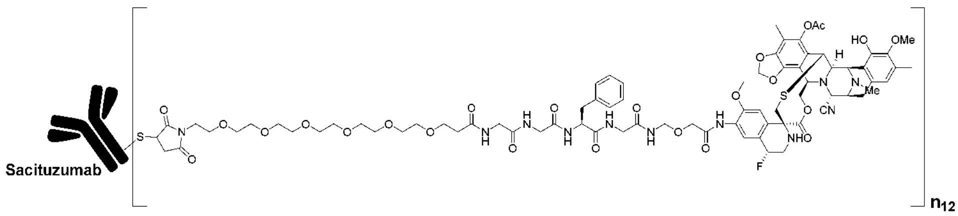

ADC-29

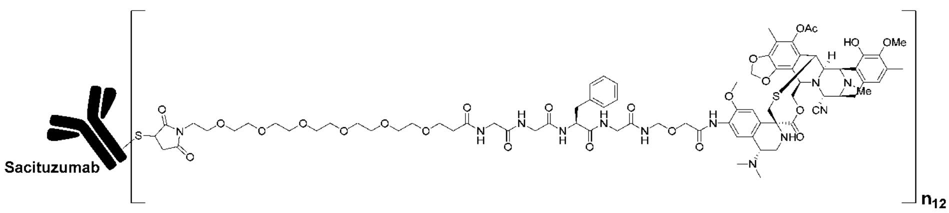

ADC-30

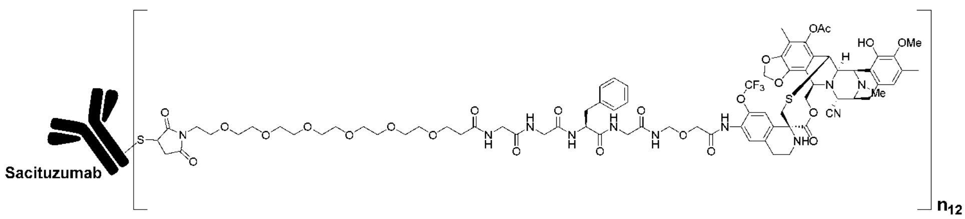

ADC-31

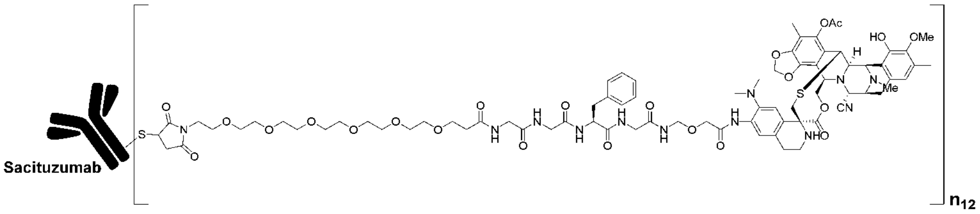

ADC-32

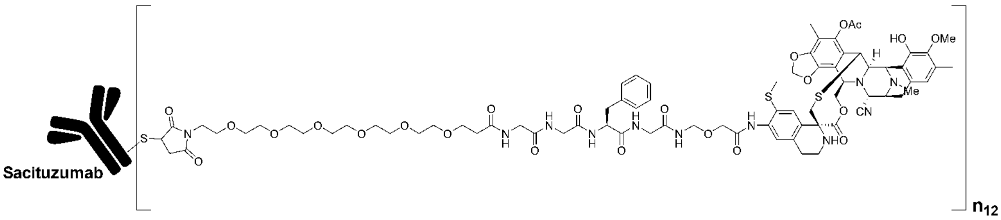

ADC-33

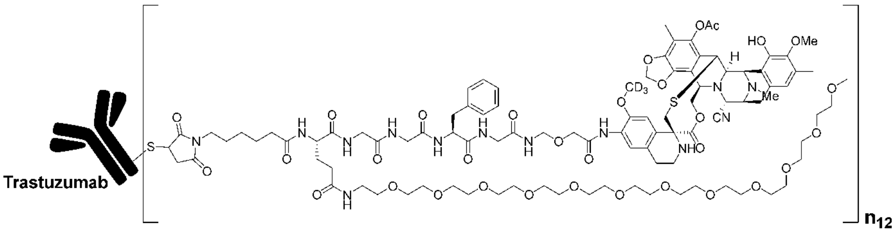

ADC-34

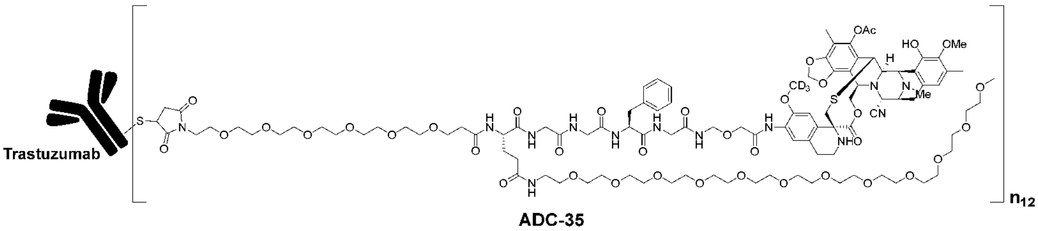

ADC-35

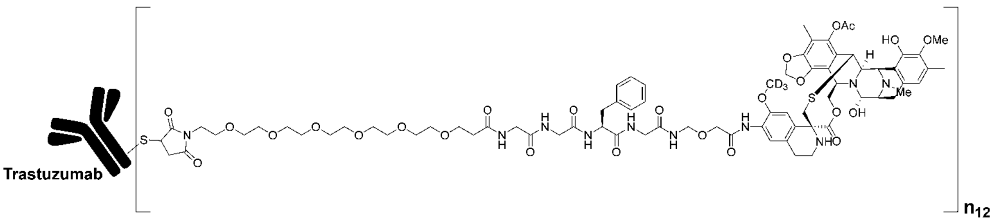

ADC-36

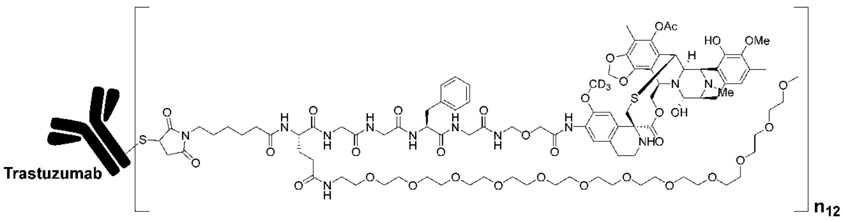

ADC-37

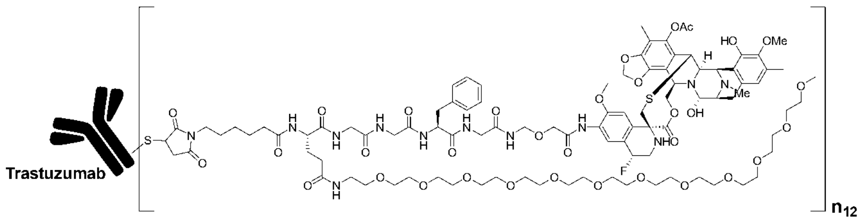

ADC-38

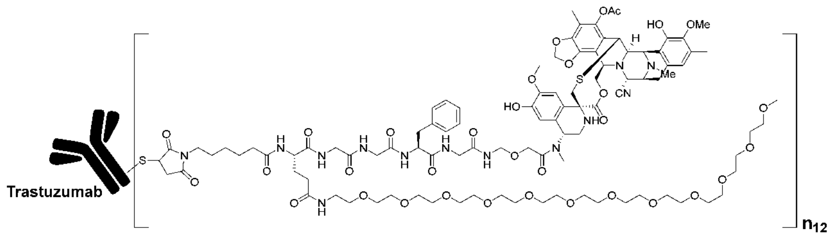

ADC-41

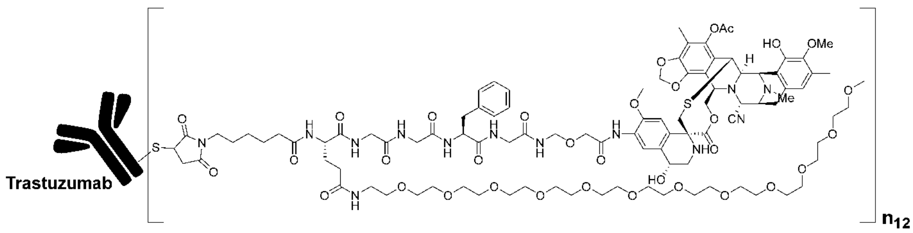

ADC-55

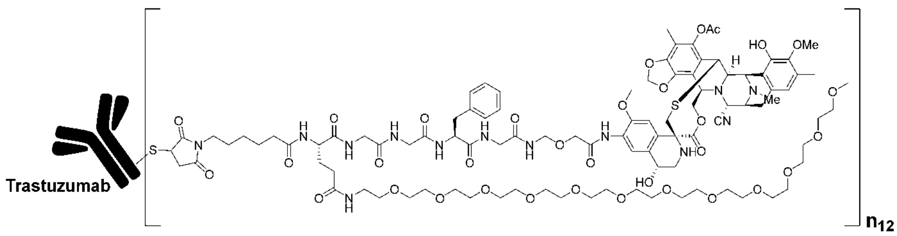

ADC-56

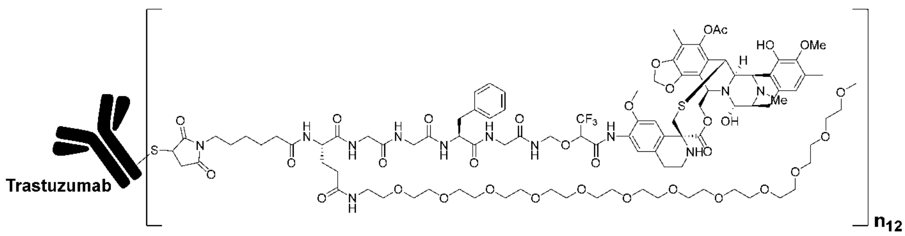

ADC-57

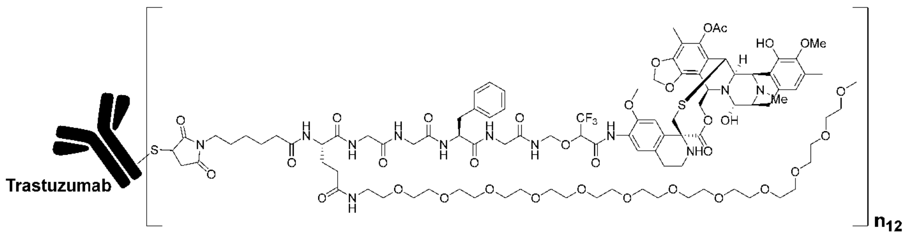

ADC-58

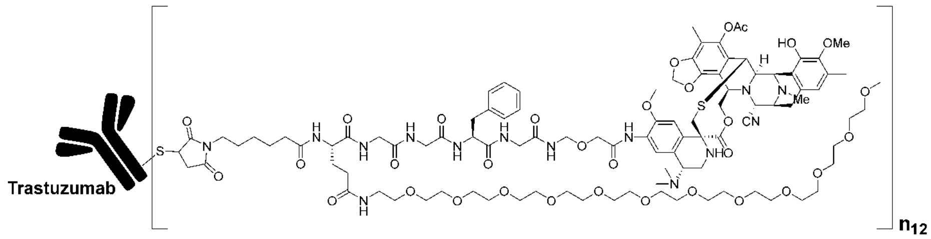

ADC-59

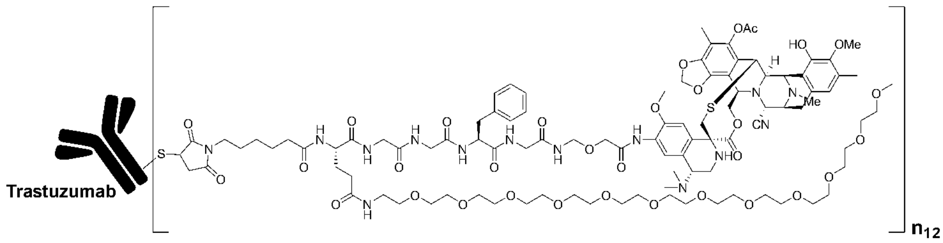

ADC-60

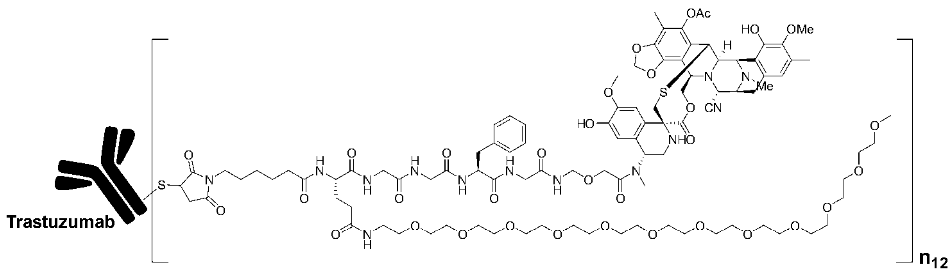

ADC-61

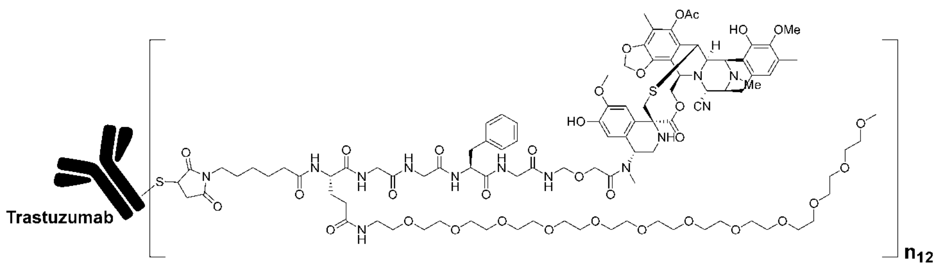

ADC-62

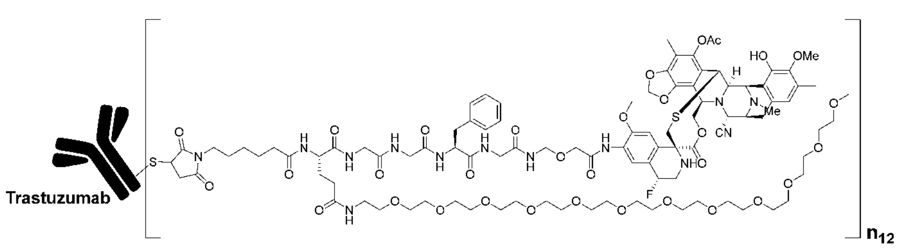

ADC-65

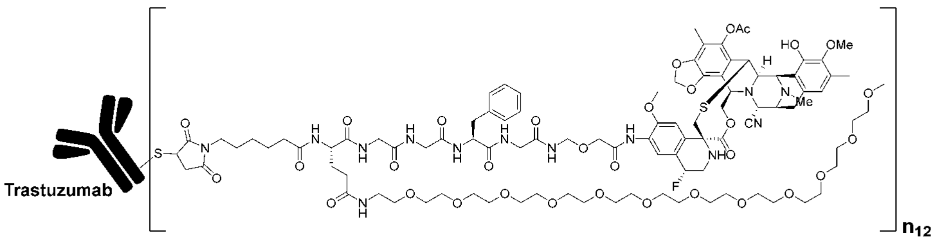

ADC-66

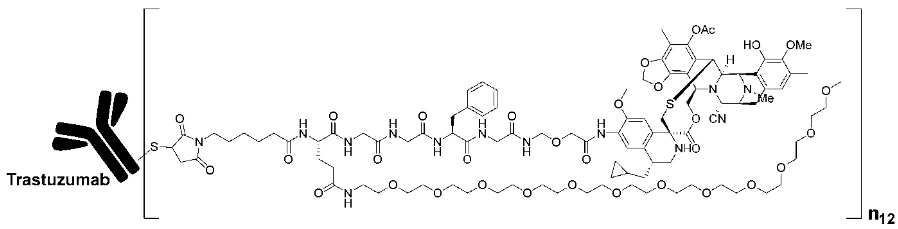

ADC-79

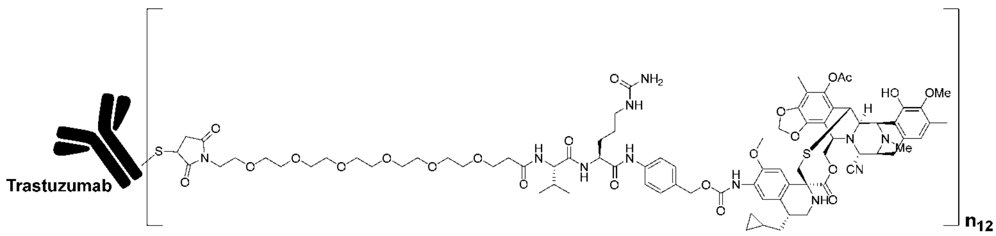

ADC-80

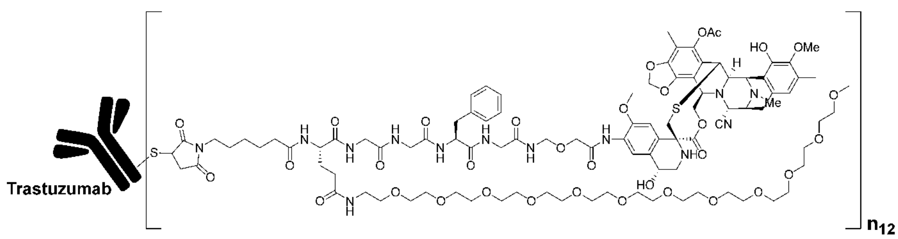

ADC-83

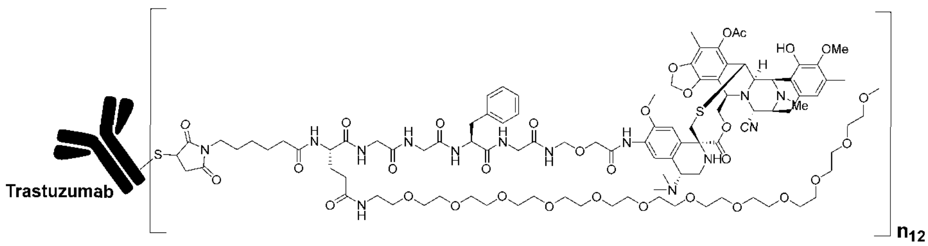

ADC-84

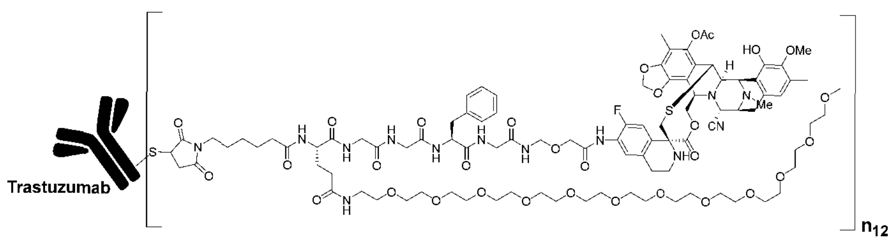

ADC-85

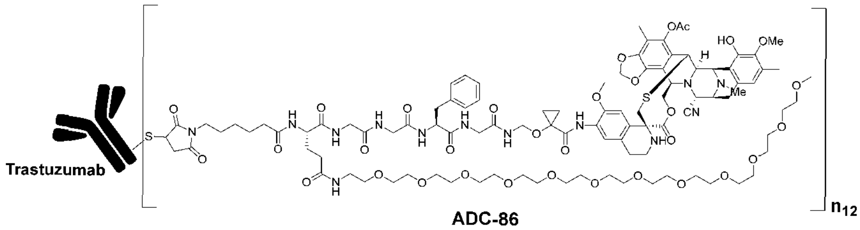

ADC-86

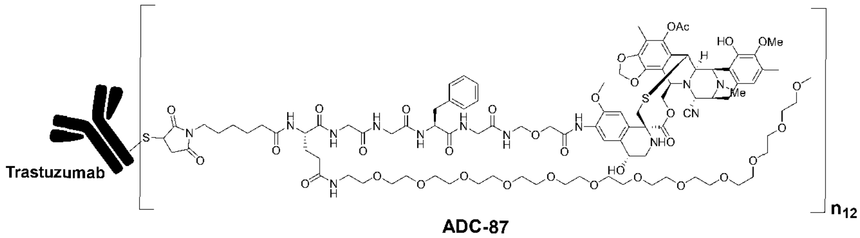

ADC-87

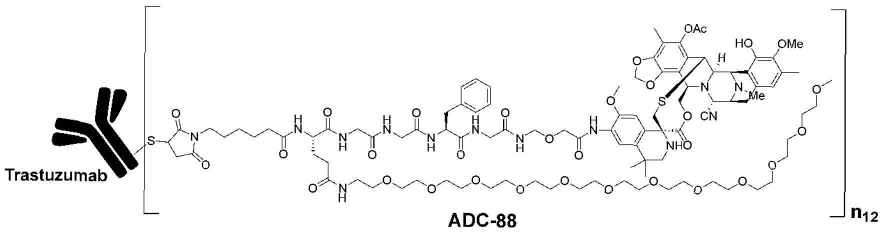

ADC-88

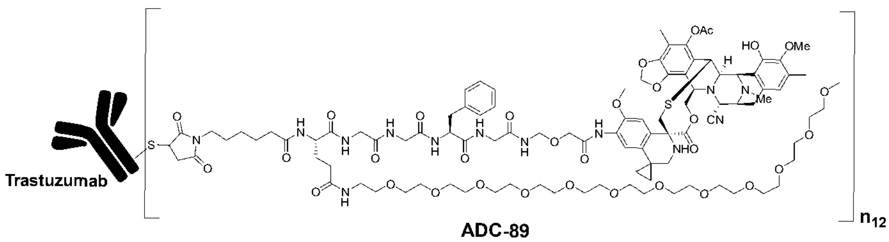

ADC-89

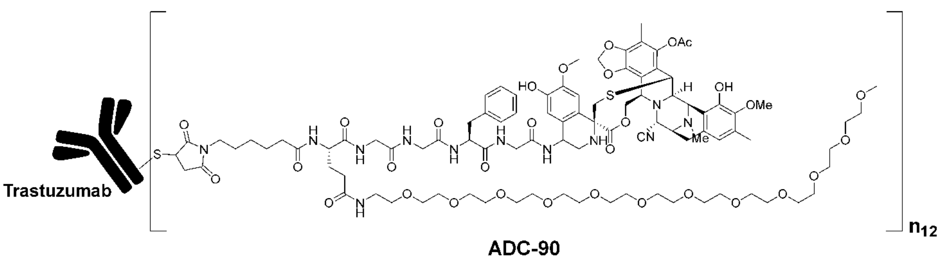

ADC-90

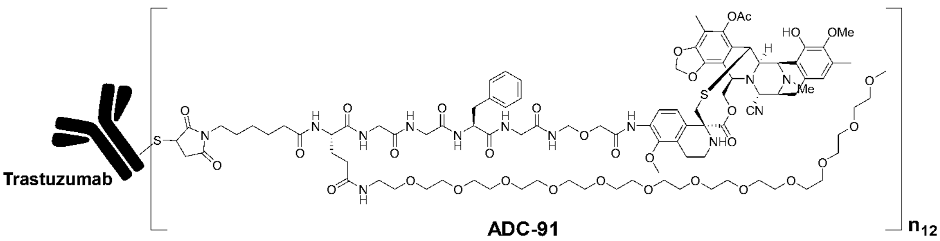

ADC-91

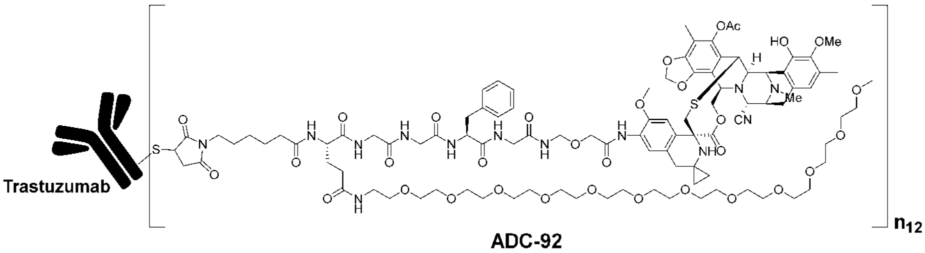

ADC-92

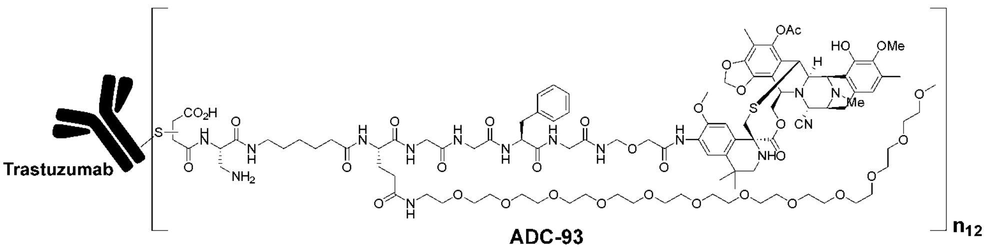

ADC-93

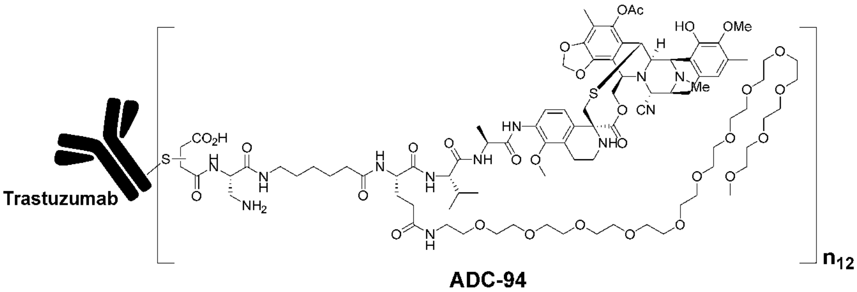

ADC-94

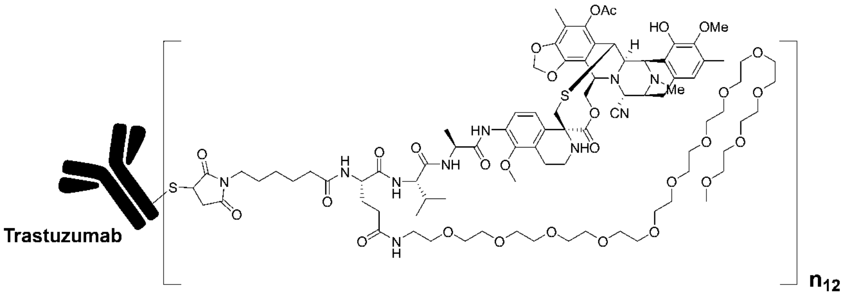

ADC-95

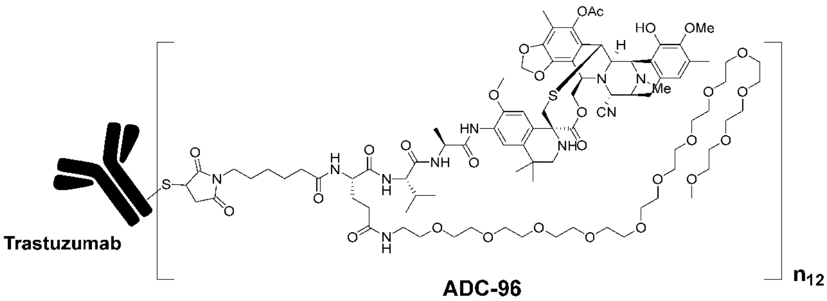

ADC-96

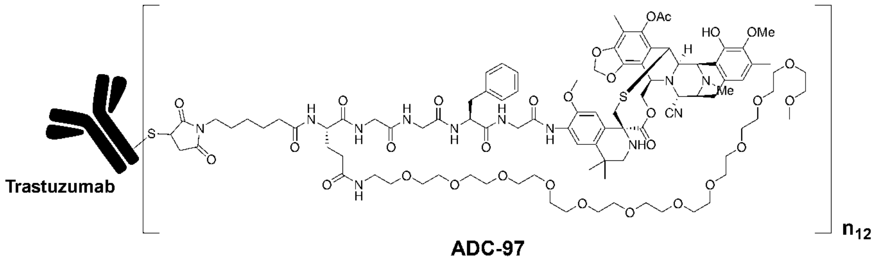

ADC-97

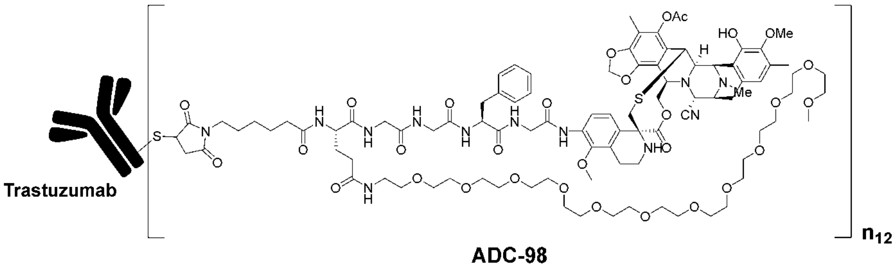

ADC-98

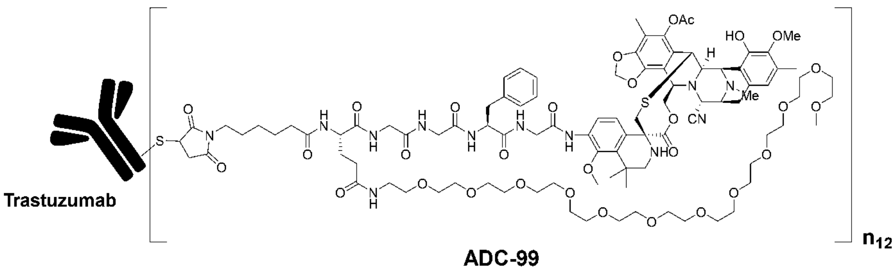

ADC-99

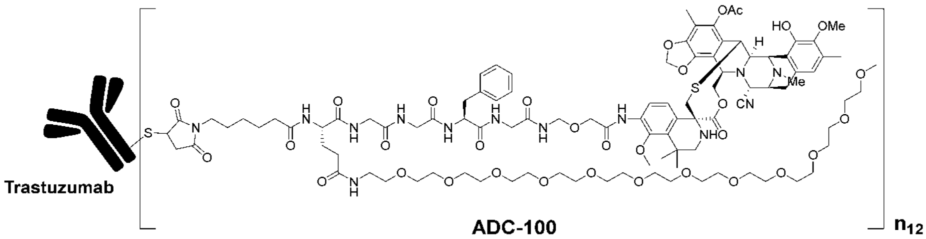

ADC-100

wherein $n_{12}$ is any value selected from 0 to 8.

**26.** The antibody-drug conjugate according to claim 25, wherein $n_{12}$ is any value selected from 1 to 8.

## INTERNATIONAL SEARCH REPORT

International application No.
**PCT/CN2024/118098**

**A. CLASSIFICATION OF SUBJECT MATTER**

C07D 515/22(2006.01)i； A61P 35/00(2006.01)i； A61K 31/4995(2006.01)i

According to International Patent Classification (IPC) or to both national classification and IPC

**B. FIELDS SEARCHED**

Minimum documentation searched (classification system followed by classification symbols)

IPC:C07D; A61K; A61P

Documentation searched other than minimum documentation to the extent that such documents are included in the fields searched

Electronic data base consulted during the international search (name of data base and, where practicable, search terms used)

REGISTRY(STN), CAPLUS(STN), MARPAT(STN), DWPI, CNKI, CNTXT, 百度, BAIDU: 泰诚思生物医药有限公司, 邹斌, 杜平, 布莱恩, 海鞘素, 抗体药物, 偶联, 抗体偶联, 四氢异喹啉, 曲贝替定, 阿昔单抗, ET-743, ET-770, ecteinascidin, trabectedin, ADC, abciximab, trastuzumab, cancer, anticancer, tumour, antitumoral, 结构检索, structural search

**C. DOCUMENTS CONSIDERED TO BE RELEVANT**

| Category* | Citation of document, with indication, where appropriate, of the relevant passages | Relevant to claim No. |
|---|---|---|
| X | CN 116284054 A (MEDILINK THERAPEUTICS (SUZHOU) CO., LTD.) 23 June 2023 (2023-06-23)<br>description, pages 1-20 and 44-47, and claims 1-10 | 1-26 |
| X | WO 03008423 A1 (PHARMA MAR, S.A. et al.) 30 January 2003 (2003-01-30)<br>description, pages 1-106 and 189-190 | 1-8, 12-13 |
| X | CN 114805398 A (JIANGSU HENGRUI PHARMACEUTICALS CO., LTD.) 29 July 2022 (2022-07-29)<br>description, pages 1 and 5 | 1-8, 12-13 |
| X | CN 112574234 A (JIANGSU HENGRUI PHARMACEUTICALS CO., LTD. et al.) 30 March 2021 (2021-03-30)<br>claims 8 and 12-13 | 1-8, 12-13 |
| A | CN 115427081 A (PHARMA MAR, S.A.) 02 December 2022 (2022-12-02)<br>claims 1 and 62-91 | 1-26 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 December 2024** | **23 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

## INTERNATIONAL SEARCH REPORT

International application No.
**PCT/CN2024/118098**

**C. DOCUMENTS CONSIDERED TO BE RELEVANT**

| Category* | Citation of document, with indication, where appropriate, of the relevant passages | Relevant to claim No. |
|---|---|---|
| E | WO 2024186263 A1 (AXCYNSIS THERAPEUTICS PTE. LTD.) 12 September 2024 (2024-09-12)<br>entire description | 1-26 |

Form PCT/ISA/210 (second sheet) (July 2022)

**INTERNATIONAL SEARCH REPORT**
**Information on patent family members**

International application No.
**PCT/CN2024/118098**

| Patent document cited in search report | | | Publication date (day/month/year) | Patent family member(s) | | | Publication date (day/month/year) |
|---|---|---|---|---|---|---|---|
| CN | 116284054 | A | 23 June 2023 | None | | | |
| WO | 03008423 | A1 | 30 January 2003 | SI | 2298780 | T1 | 31 January 2013 |
| | | | | ES | 2394772 | T3 | 05 February 2013 |
| | | | | PT | 1854800 | E | 25 September 2012 |
| | | | | GB | 0117402 | D0 | 05 September 2001 |
| | | | | EP | 2298780 | A1 | 23 March 2011 |
| | | | | EP | 2298780 | B1 | 12 September 2012 |
| | | | | PT | 1406907 | E | 10 December 2007 |
| | | | | CY | 1107809 | T1 | 19 June 2013 |
| | | | | MXPA | 04000506 | A | 23 July 2004 |
| | | | | ATE | 371661 | T1 | 15 September 2007 |
| | | | | AU | 2008207381 | A1 | 11 September 2008 |
| | | | | AU | 2008207381 | B2 | 14 July 2011 |
| | | | | ES | 2389671 | T3 | 30 October 2012 |
| | | | | PT | 2298780 | E | 17 December 2012 |
| | | | | EP | 1854800 | A1 | 14 November 2007 |
| | | | | EP | 1854800 | B1 | 27 June 2012 |
| | | | | AU | 2002317967 | B2 | 05 June 2008 |
| | | | | AU | 2002317967 | C1 | 22 January 2009 |
| | | | | CA | 2453991 | A1 | 30 January 2003 |
| | | | | CA | 2453991 | C | 20 December 2011 |
| | | | | US | 2010197695 | A1 | 05 August 2010 |
| | | | | US | 8076337 | B2 | 13 December 2011 |
| | | | | DK | 1406907 | T3 | 04 February 2008 |
| | | | | US | 2006106021 | A1 | 18 May 2006 |
| | | | | US | 7759345 | B2 | 20 July 2010 |
| | | | | DK | 1854800 | T3 | 01 October 2012 |
| | | | | NO | 20040189 | L | 16 March 2004 |
| | | | | NO | 335262 | B1 | 27 October 2014 |
| | | | | SI | 1854800 | T1 | 30 November 2012 |
| | | | | CY | 1113359 | T1 | 22 June 2016 |
| | | | | EP | 1406907 | A1 | 14 April 2004 |
| | | | | EP | 1406907 | B1 | 29 August 2007 |
| | | | | ES | 2291479 | T3 | 01 March 2008 |
| | | | | JP | 2004536132 | A | 02 December 2004 |
| | | | | JP | 4653950 | B2 | 16 March 2011 |
| | | | | NO | 20140711 | L | 16 March 2004 |
| | | | | NO | 340213 | B1 | 20 March 2017 |
| | | | | DK | 2298780 | T3 | 07 January 2013 |
| | | | | DE | 60222135 | D1 | 11 October 2007 |
| | | | | DE | 60222135 | T2 | 21 May 2008 |
| CN | 114805398 | A | 29 July 2022 | None | | | |
| CN | 112574234 | A | 30 March 2021 | None | | | |
| CN | 115427081 | A | 02 December 2022 | CA | 3175426 | A1 | 28 October 2021 |
| | | | | US | 2024131180 | A1 | 25 April 2024 |
| | | | | TW | 202203980 | A | 01 February 2022 |
| | | | | CL | 2022002917 | A1 | 30 June 2023 |
| | | | | EP | 4138923 | A1 | 01 March 2023 |
| | | | | AR | 121894 | A1 | 20 July 2022 |
| | | | | DOP | 2022000224 | A | 15 March 2023 |

Form PCT/ISA/210 (patent family annex) (July 2022)

**INTERNATIONAL SEARCH REPORT**
**Information on patent family members**

International application No.
**PCT/CN2024/118098**

| Patent document cited in search report | Publication date (day/month/year) | Patent family member(s) | Publication date (day/month/year) |
|---|---|---|---|
| | | MX 2022013298 A | 30 November 2022 |
| | | ECSP 22088584 A | 30 December 2022 |
| | | CR 20220581 A | 09 January 2023 |
| | | CO 2022016548 A2 | 07 March 2023 |
| | | WO 2021214126 A1 | 28 October 2021 |
| | | BR 112022020823 A2 | 29 November 2022 |
| | | PE 20230785 A1 | 11 May 2023 |
| | | KR 20230004714 A | 06 January 2023 |
| | | IL 297028 A | 01 December 2022 |
| | | JP 2023522259 A | 29 May 2023 |
| | | AU 2021260792 A1 | 24 November 2022 |
| WO 2024186263 A1 | 12 September 2024 | WO 2024186264 A1 | 12 September 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **KENNETH L.** Rinehart from the Caribbean tunicate Ecteinascidia turbinata. It can now be prepared by artificial synthesis. *Nat. Prod. Rep.*, 2015, vol. 32, 328-347